# EUROPEAN PATENT APPLICATION

(11) **EP 4 741 375 A1**
(43) Date of publication of application: **13.05.2026**
(21) Application number: 24306869.9
(22) Date of filing: 06.11.2024
(51) Int. Cl.: C07D 209/44, C07D 217/02, C07D 217/22, C07D 401/04, C07D 401/14, C07D 407/14, C07D 413/04, C07D 417/04, A61K 31/4035, A61K 31/472, A61K 31/4725, A61P 35/00

(54) **SULFONYLUREA DERIVATIVES AND THEIR USE FOR THE TREATMENT OF CANCER**

(71) Applicant: Qubit Pharmaceuticals, 75014 Paris (FR)
(72) Inventor: El Hage, Krystel, 75014 Paris (FR); Nicolaï, Eric, 75014 Paris (FR)
(74) Representative: A.P.I. Conseil

(57) **Abstract**

The present disclosure describes sulfonylurea derivatives compounds of Formula (I), and pharmaceutically acceptable salts thereof, compositions, methods, and uses thereof. Such compounds are believed to be therapeutically useful as inhibitors of KAT6A with improved selectivity particularly in the treatment and/or prevention of diseases and disorders mediated by KAT6A in a subject.

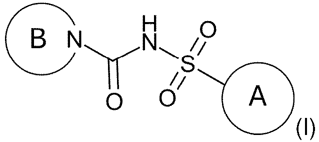

## Description

### FIELD OF THE INVENTION

The present invention relates to the field of pharmaceuticals and concerns compounds capable of modulating KAT6 activity and/or KAT6-signaling, including their synthesis methods and therapeutic applications. In addition, the invention relates to incorporation of these compounds into a pharmaceutical formulation, and its application as a KAT6 inhibitor in the development of therapeutic agents for the treatment and/or prevention of cancer.

### BACKGROUND OF THE INVENTION

KAT6A, also designated as MYST3 or MOZ, is a member of the MYST family of histone acetyltransferases (HATs) that play a role in the regulation of gene expression and chromatin modification. KAT6A functions as an epigenetic regulator by acetylating histone H3 at lysine 9 (H3K9) and lysine 14 (H3K14), post-translational modifications associated with transcriptionally active chromatin. Through these modifications, KAT6A modulates chromatin structure and accessibility, thereby influencing the transcription of genes involved in key cellular processes such as differentiation, proliferation, and survival (Lv, D. *et al.,* 2017).

Beyond its role in normal cellular function, KAT6A has been implicated in oncogenesis. Alterations in KAT6A activity, including mutations, chromosomal translocations, and aberrant expression, are associated with the initiation and progression of various cancers. Notably, chromosomal translocations involving KAT6A, such as t(8;16)(p11 ;p13), result in fusion proteins like KAT6A-CREBBP, which are linked to acute myeloid leukemia (AML) (Coenen, E. A. *et al.,* 2013). Additionally, overexpression of KAT6A has been observed in solid tumors, including breast and colon cancers, where it contributes to increased cellular proliferation and survival (Hu, Z. *et al.,* 2019).

The oncogenic potential of KAT6A is mediated through its capacity to modify the transcriptional landscape in a manner that supports tumor progression. KAT6A, as a histone acetyltransferase, acetylates histone H3 at lysine residues K9, K14, and K23, thereby facilitating the expression of genes involved in cellular proliferation and survival while repressing those associated with differentiation and apoptosis. Additionally, KAT6A seems to interact with other epigenetic regulators, including the NuA4 complex and the p300/CBP coactivator, to modulate the expression of oncogenes and tumor suppressor genes. These interactions collectively contribute to the establishment of a transcriptional environment that promotes oncogenic processes. Thus, targeting KAT6A presents a viable therapeutic strategy for cancers characterized by KAT6A dysregulation. Inhibition of KAT6A's acetyltransferase activity has the potential to disrupt the transcriptional programs that drive cancer progression (Weber, L. M. *et al.,* 2023).

Cancers driven by KAT6A dysregulation, including AML, breast cancer, and colon cancer, frequently present aggressive phenotypes and exhibit resistance to conventional treatments like chemotherapy and radiation. Compounds such as WM-1119 demonstrate moderate efficacy *in vitro* but lack sufficient potency *in vivo* to achieve robust therapeutic outcomes due to issues such as low bioavailability, or poor pharmacokinetic properties (Sharma S. *et al.,* 2023). A primary challenge in developing KAT6A inhibitors is achieving selectivity due to structural homology with other MYST family members, including KAT6B (MORF), KAT5 (Tip60), and KAT7 (HBO1). This similarity, particularly within the acetyl-CoA binding pocket, complicates the design of inhibitors that specifically target KAT6A without affecting other HATs, which are essential for normal cellular functions. Indeed, non-selective inhibition may lead to off-target effects and toxicity, thereby narrowing the therapeutic window. The development of novel KAT6A inhibitors with enhanced selectivity and potency could provide new therapeutic options for these cancers, particularly those resistant to existing treatments. Additionally, such inhibitors could be integrated into combination therapies to improve treatment efficacy and mitigate drug resistance.

Recent advancements include the discovery of CTx-648 (PF-9363), a highly potent, and orally bioavailable inhibitor of KAT6A/B histone acetyltransferases. CTx-648 has demonstrated antitumor activity in breast cancer models with high KAT6A expression and estrogen receptor positivity (ER+). By specifically inhibiting KAT6A/B, CTx-648 reduces the acetylation of histone H3 at lysine 23 (H3K23Ac), thereby suppressing tumor growth. The efficacy of CTx-648 in preclinical trials highlights the potential of targeted KAT6A inhibition as a therapeutic approach for cancers with KAT6A dysregulation. However, in the phase 1 clinical trial of PF-07248144 for ER+HER2- metastatic breast cancer, neutropenia and anemia were among the most frequently reported treatment-related adverse events. These occurred in 59.8% and 48.6% of patients, respectively, with 35.5% and 13.1 % experiencing grade 3 or 4 severity (Mukohara T. *et al.,* 2024). This high incidence stresses the critical role of the KAT families in hematopoiesis and in particular KAT6 and KAT7. KAT6A/B enzymes are involved in the regulation of gene expression for the proliferation and differentiation of hematopoietic stem cells (HSC) and show a high degree of cooperation in the regulation of hematopoiesis. Like KAT6A/B and KAT7 also have a critical role in maintaining HSC function as it confers differentiation down the erythroid or alternative lineages. However, KAT6A/B, but not KAT7, regulates B cell development. The inhibition of KAT6B and KAT7 alongside KAT6A disrupts normal blood cell development, leading to adverse effects such as anemia and neutropenia (Bergamasco M.I. *et al.,* 2024). The occurrence of neutropenia and anemia emphasizes the necessity for developing compounds with greater specificity to minimize adverse effects while maintaining therapeutic efficacy.

Recently, a class of acylsulfonamide compounds has been introduced, disclosing their potential as inhibitors specifically targeting the KAT6A protein (WO2024189598). These compounds are suggested to have therapeutic value in conditions associated with KAT6A activity, including various cancers and proliferative disorders. However, the patent does not provide details regarding their specificity toward KAT6A or differentiate between KAT6A and its close relative, KAT6B. This raises questions about the selectivity of these compounds, such specificity is crucial for the development of cancer-specific inhibitors with high therapeutic index that can precisely target KAT6A-driven malignancies.

In summary, KAT6A is involved in normal gene regulation and cancer pathogenesis through its histone acetyltransferase activity. While existing inhibitors have laid important groundwork, there remains a pressing need for more selective and potent inhibitors that specifically target KAT6A-driven cancers with a selective inhibition of KAT6A in preference to KAT6B.

### SUMMARY OF THE INVENTION

The invention aims to address these drawbacks and in particular aims at developing compounds with a better therapeutic index. The following provides a simplified summary of selected aspects, embodiments, and examples of the present invention to offer a basic understanding of it. However, this summary is not intended to provide a comprehensive overview of all aspects, embodiments, and examples of the invention. Its sole purpose is to present selected aspects, embodiments, and examples in a concise manner, serving as an introduction to the more detailed description of the aspects, embodiments, and examples that follow this summary.

The present disclosure aims to provide a compound of general formula (I): 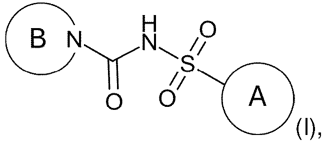 or a pharmaceutically acceptable salt thereof, wherein:
A represents an aryl group, such as a phenyl group or a benzo-fused cycloalkyl group, or a heteroaryl group, such as a benzo-fused heterocyclic group;
wherein A is optionally substituted by one or more members selected from the group consisting of:
   - alkoxy groups, such as C₁-C₄ alkoxy groups;
   - alkyl groups, such as C₁-C₄ alkyl groups, optionally substituted by 1 to 3 fluorine atoms;
   - halogens;
   - unsubstituted or substituted amino groups, such as dialkylamino groups, such as a dimethylamino group (-N(CH₃)₂);
   - heterocyclyl groups, preferably a 4- to 6-member heterocycloalkyl group, such as azetidine or pyrrolidine, optionally substituted by one or more alkyl groups, oxo groups and/or halogens;
   - phenyl groups, optionally substituted by one or more alkyl groups and/or halogens; and
   - cycloalkyl groups optionally substituted by one or more alkyl groups and/or halogens, such as a C₃-C₅ cycloalkyl group optionally substituted by 1 to 2 fluorine atoms;
      and
B represents a partially saturated nitrogen containing bicyclic ring system, such as tetrahydro-isoquinoline or isoindoline, substituted by one or more members selected from the group consisting of:
   - halogens;
   - dialkylamino groups, such as a dimethylamino group (-N(CH₃)₂);
   - cycloalkyl groups optionally substituted by one or more alkyl groups and/or halogens, such as a C₃-C₅ cycloalkyl group optionally substituted by 1 to 2 fluorine atoms;
   - alkoxy groups such as a C₁-C₄ alkoxy group;
   - 3- to 6-member cycloalkyloxy groups, such as cyclopropyloxy group, optionally substituted by one or more alkyl groups and/or halogens;
   - 4- to 6-member heterocycloalkyl groups, optionally substituted by one or more alkyl groups and/or halogens, which is directly bonded to the bicyclic ring system via a covalent bond or linked through a spacer, preferably chosen from a C₁-C₄ alkylene chain, or a single oxygen atom; for example a 4- to 6-member heterocyclyl group, such as azetidine, optionally substituted by one or more alkyl groups and/or halogens;
   - 5- or 6-member heteroaryl groups, optionally substituted by one or more alkyl groups and/or halogens, which is directly bonded to the bicyclic ring system via a covalent bond or linked through a spacer, preferably chosen from a C₁-C₄ alkylene chain, or a single oxygen atom; for example a 5- or 6-member heteroaryl group, such as pyrazole, isoxazole, thiazole, pyridine or oxazole, optionally substituted by one or more alkyl groups and/or halogens; and
   - alkyl groups, such as a C₁-C₄ alkyl group, optionally substituted by 1 to 3 fluorine atoms.
As it is shown in the examples, the compounds of formula (I) are effective in modulating or inhibiting the KAT6 signaling pathway. Moreover, the compounds of the invention show selective inhibition of KAT6A in preference to KAT6B.
According to other optional features of the compound of the invention, it may optionally include one or more of the following features, either individually or in combination:
   - A represents an aryl group, such as a phenyl group or a benzo-fused cycloalkyl group, or a heteroaryl group, such as a benzo-fused heterocyclic group; substituted by one or more alkoxy groups such as a C₁-C₄ alkoxy group, and/or alkyl groups, such as a C₁-C₄ alkyl group, optionally substituted by 1 to 3 fluorine atoms;
      wherein A is optionally further substituted by one or more members selected from the group consisting of:
      - alkoxy groups, such as a C₁-C₄ alkoxy group;
      - alkyl groups, such as a C₁-C₄ alkyl group, optionally substituted by 1 to 3 fluorine atoms;
      - halogens;
      - unsubstituted or substituted amino groups, such as dialkylamino groups, such as a dimethylamino group (-N(CH₃)₂);
      - heterocyclyl groups, preferably a 4- to 6-member heterocycloalkyl group, such as azetidine or pyrrolidine, optionally substituted by one or more alkyl groups, oxo groups and/or halogens;
      - phenyl groups, optionally substituted by one or more alkyl groups and/or halogens; and
      - cycloalkyl groups optionally substituted by one or more alkyl groups and/or halogens, such as a C₃-C₅ cycloalkyl group optionally substituted by 1 to 2 fluorine atoms.
   - B represents a partially saturated nitrogen containing bicyclic ring system, such as tetrahydro-isoquinoline or isoindoline, comprising an aryl part substituted by a 5- or 6-member heteroaryl group, which is directly bonded to the aryl part of the bicyclic ring system via a covalent bond or linked through a spacer, said spacer being preferably chosen from a C₁-C₄ alkylene chain or a single oxygen atom; wherein B is optionally further substituted by one or more members selected from the group consisting of:
      - halogens;
      - dialkylamino groups, such as a dimethylamino group (-N(CH₃)₂).
      - cycloalkyl groups optionally substituted by one or more alkyl groups and/or halogens, such as a C₃-C₅ cycloalkyl group optionally substituted by 1 to 2 fluorine atoms;
      - alkoxy groups, such as a C₁-C₄ alkoxy group;
      - 3- to 6-member cycloalkyloxy groups, such as cyclopropyloxy, optionally substituted by one or more alkyl groups and/or halogens; and
      - alkyl groups, such as a C₁-C₄ alkyl group, optionally substituted by 1 to 3 fluorine atoms.
   - the compound is of formula (II): 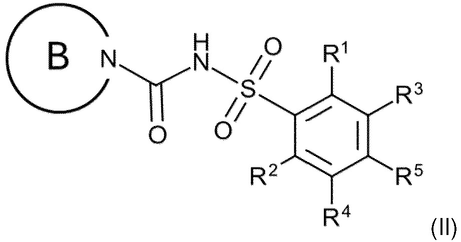 or a pharmaceutically acceptable salt thereof, wherein:
      R¹, R², R³, R⁴, and R⁵ each independently represent H; an alkyl group, such as a C₁-C₄ alkyl group, optionally substituted by 1 to 3 fluorine atoms; a cycloalkyl group, optionally substituted by one or more alkyl groups and/or halogens; a heterocyclyl group, preferably a 4- to 6-member heterocyclyl group, optionally substituted by one or more alkyl groups, oxo groups and/or halogens; a halogen;
      an alkoxy group such as a C₁-C₄ alkoxy group; a cycloakyloxy group, optionally substituted by one or more alkyl groups and/or halogens; a phenyl group,
      optionally substituted by one or more alkyl groups and/or halogens; or a substituted or unsubstituted amino group, such as dialkylamino groups, such as a dimethylamino group (-N(CH₃)₂);
      alternatively, any one pair selected from R' and R³, R² and R⁴, R⁴ and R⁵, or R³ and R⁵, taken together, form a 5- or 6-member heterocycloalkyl group or an heteroaromatic ring or a 5- or 6-member cycloalkyl group, optionally substituted by one or more alkyl groups and/or halogens, while the remaining R¹, R², R³, R⁴,
      and R⁵, each independently, represent H; an alkyl group, optionally substituted by 1 to 3 fluorine atoms; a cycloalkyl group, optionally substituted by one or more alkyl groups and/or halogens; a heterocyclyl group, preferably a 4- to 6-member heterocyclyl group, optionally substituted by one or more alkyl groups, oxo groups and/or halogens; a halogen; an alkoxy group, such as a C₁-C₄ alkoxy group; a cycloakyloxy group, optionally substituted by one or more alkyl groups and/or halogens; a phenyl group, optionally substituted by one or more alkyl groups and/or halogens; or a substituted or unsubstituted amino group, such as dialkylamino groups, such as a dimethylamino group (-N(CH₃)₂).

The compounds of formula (II) allow for an enhanced affinity towards the target
- the compound is of formula (III): 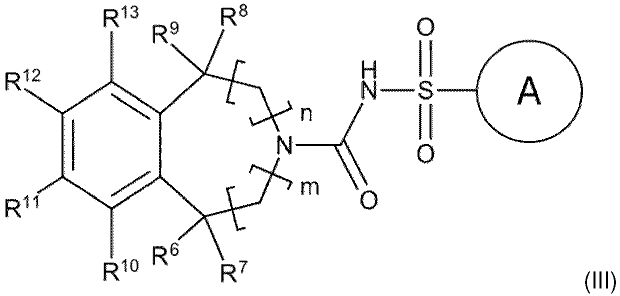 or a pharmaceutically acceptable salt thereof, wherein:
   R⁶, R⁷, R⁸, and R⁹ each independently represent H; or an alkyl group;
   n and m each independently represent 0 or 1;
   R¹⁰ and R¹³ each independently represent H; a halogen; an alkoxy group; a 4- to 6-member heterocycloalkyl group or a 5- or 6-member heteroaryl group, optionally substituted by one or more alkyl groups and/or halogens; a cycloalkyl group, optionally substituted by one or more alkyl groups and/or halogens; or an alkyl group, optionally substituted by 1 to 3 fluorine atoms; and
   R¹¹ and R¹² each independently represent H; a halogen; a dialkylamino group, such as a dimethylamino group (-N(CH₃)₂); a cycloalkyl group, optionally substituted by one or more alkyl groups and/or halogens; a 4- to 6-member heterocycloalkyl group, optionally substituted by one or more alkyl groups and/or halogens, such as azetidine, optionally substituted by one or more alkyl groups and/or halogens; or a 4- to 6-member heteroaryl group, optionally substituted by one or more alkyl groups and/or halogens, which is directly bonded to the aryl via a covalent bond or linked through a spacer, such as an alkyl chain; such as pyrazole or oxazole, optionally substituted by one or more alkyl groups and/or halogens.

The compounds of formula (III) exhibit higher selectivity against KAT6A vs KAT6B as well as KAT7.
- the compound is of formula (Illf): 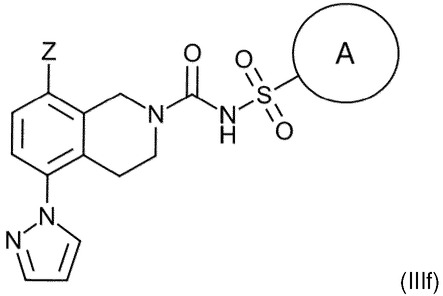
   or a pharmaceutically acceptable salt thereof,
   wherein Z represents a halogen; an alkyl group optionally substituted by 1 to 3 fluorine atoms; an alkoxy group; or a cycloalkyl group optionally substituted by one or more alkyl groups and/or halogens.
- the compound is of formula (IV): 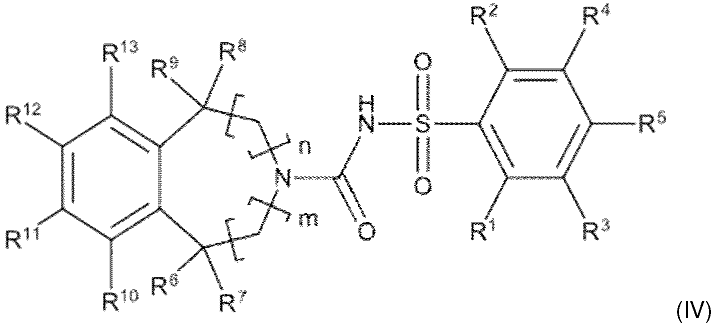 or a pharmaceutically acceptable salt thereof, wherein:
   R¹, R², R³, R⁴, and R⁵ each independently represent H; an alkyl group, such as a C₁-C₄ alkyl group, optionally substituted by 1 to 3 fluorine atoms; a cycloalkyl group, optionally substituted by one or more alkyl groups and/or halogens; a heterocyclyl group, preferably a 4- to 6-member heterocyclyl group, optionally substituted by one or more alkyl groups, oxo groups and/or halogens; a halogen; an alkoxy group, such as a C₁-C₄ alkoxy group; a cycloakyloxy group, optionally substituted by one or more alkyl groups and/or halogens; a phenyl group, optionally substituted by one or more alkyl groups and/or halogens; or a substituted or unsubstituted amino group, such as dialkylamino groups, such as a dimethylamino group (-N(CH₃)₂);
   alternatively, any one pair selected from R¹ and R³, R² and R⁴, R⁴ and R⁵, or R³ and R⁵, taken together, form a 5- or 6-member heterocycloalkyl group or an heteroaromatic ring or a 5- or 6-member cycloalkyl group, optionally substituted by one or more alkyl groups and/or halogens, while the remaining R¹, R², R³, R⁴, and R⁵, each independently, represent H; an alkyl group, optionally substituted by 1 to 3 fluorine atoms; a cycloalkyl group, optionally substituted by one or more alkyl groups and/or halogens; a heterocyclyl group, preferably a 4- to 6-member heterocyclyl group, optionally substituted by one or more alkyl groups, oxo groups and/or halogens; a halogen; an alkoxy group, such as a C₁-C₄ alkoxy group; a cycloakyloxy group, optionally substituted by one or more alkyl groups and/or halogens; a phenyl group, optionally substituted by one or more alkyl groups and/or halogens; or a substituted or unsubstituted amino group, such as dialkylamino groups, such as a dimethylamino group (-N(CH₃)₂);
   R⁶, R⁷, R⁸, and R⁹ each independently represent H; or an alkyl group; n and m each independently represent 0 or 1;
   R¹⁰ and R¹³ each independently represent H; a halogen; an alkoxy group; a 4- to 6-member heterocycloalkyl group or a 5- or 6-member heteroaryl group, optionally substituted by one or more alkyl groups and/or halogens; a cycloalkyl group, optionally substituted by one or more alkyl groups and/or halogens; an alkyl group, optionally substituted by 1 to 3 fluorine atoms; and
   R¹¹ and R¹² each independently represent H; a halogen; a dialkylamino group, such as a dimethylamino group (-N(CH₃)₂); a cycloalkyl group, optionally substituted by one or more alkyl groups and/or halogens; a 4- to 6-member heterocycloalkyl group, optionally substituted by one or more alkyl groups and/or halogens, such as azetidine, optionally substituted by one or more alkyl groups and/or halogens; or a 4- to 6-member heteroaryl group, optionally substituted by one or more alkyl groups and/or halogens, which is directly bonded to the aryl via a covalent bond or linked through a spacer such as an alkyl chain; such as pyrazole or oxazole, optionally substituted by one or more alkyl groups and/or halogens.

The compounds according to formula (IV) exhibit higher selectivity against KAT6A vs KAT6B, as well as KAT7, KAT5 and KAT8.
- B is a tetrahydro-isoquinoline substituted on its aryl part of the bicyclic ring system by a 5- or 6-member heteroaryl group, which is directly bonded to the aryl part via a covalent bond or linked through a spacer such as an alkyl chain; wherein B is optionally further substituted by one or more members selected from the group consisting of:
   - halogens;
   - dialkylamino groups, such as a dimethylamino group (-N(CH₃)₂);
   - cycloalkyl groups optionally substituted by one or more alkyl groups and/or halogens, such as a C₃-C₅ cycloalkyl group optionally substituted by 1 to 2 fluorine atoms;
   - alkoxy groups, such as a C₁-C₄ alkoxy group;
   - 3- to 6-member cycloalkyloxy groups, such as cyclopropyloxy, optionally substituted by one or more alkyl groups and/or halogens; and
   - alkyl groups, such as a C₁-C₄ alkyl group, optionally substituted by 1 to 3 fluorine atoms.
- The compound or a pharmaceutically acceptable salt thereof which is selected from: 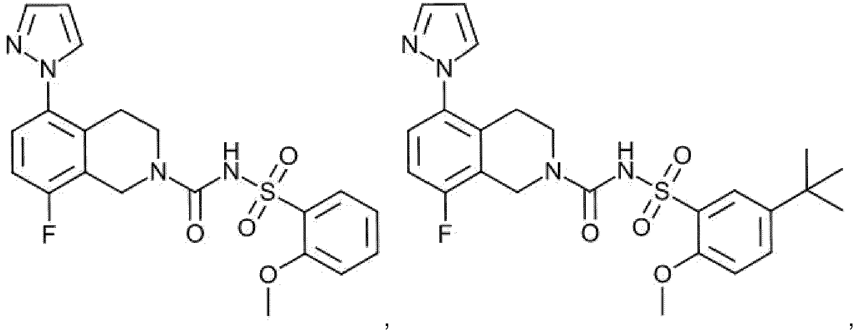 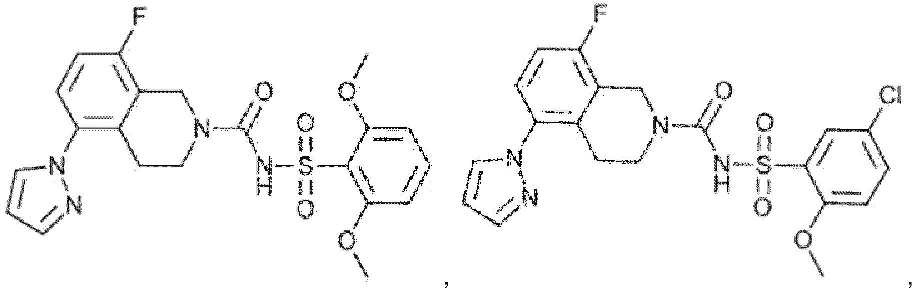 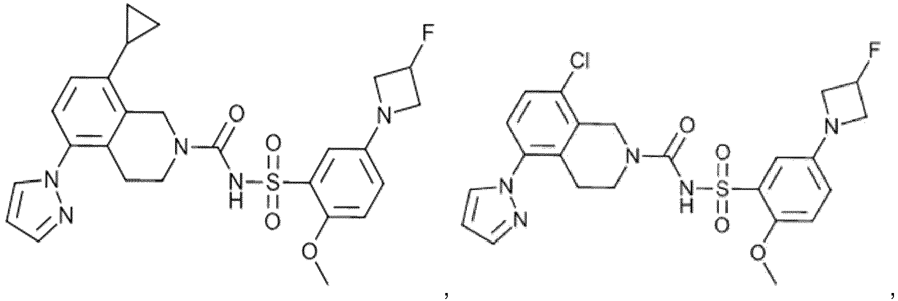 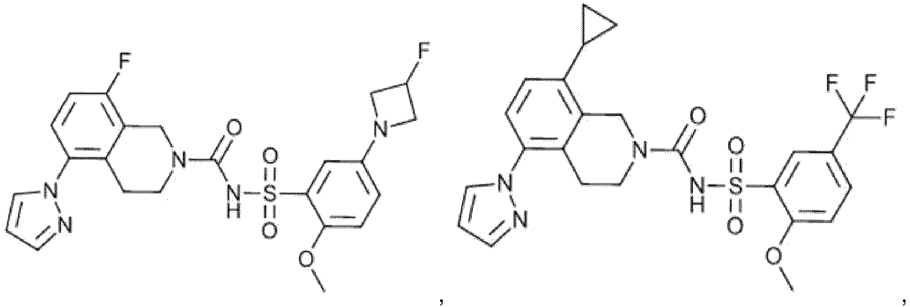 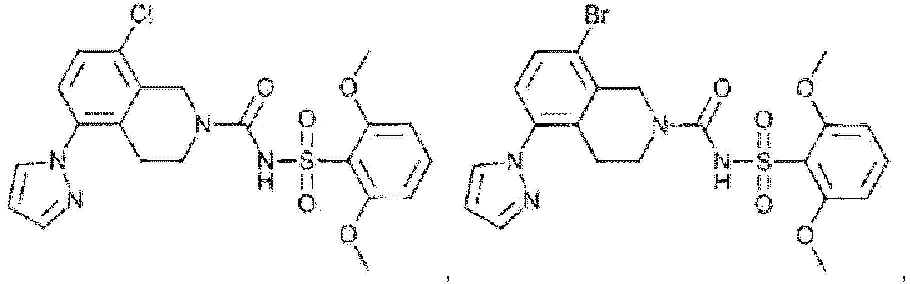 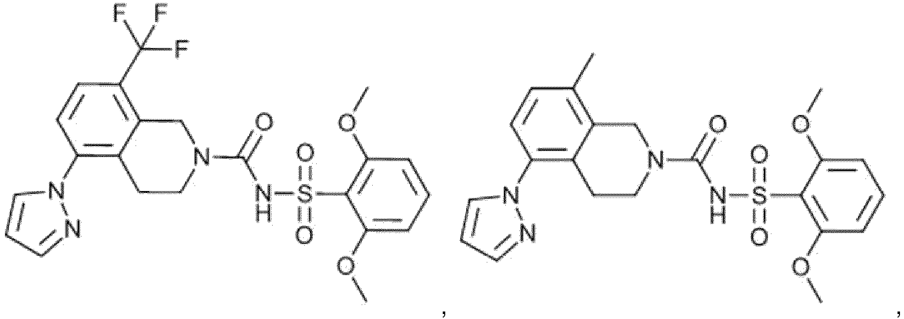 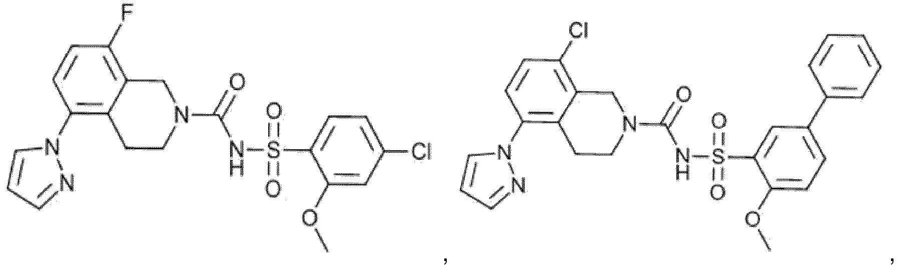 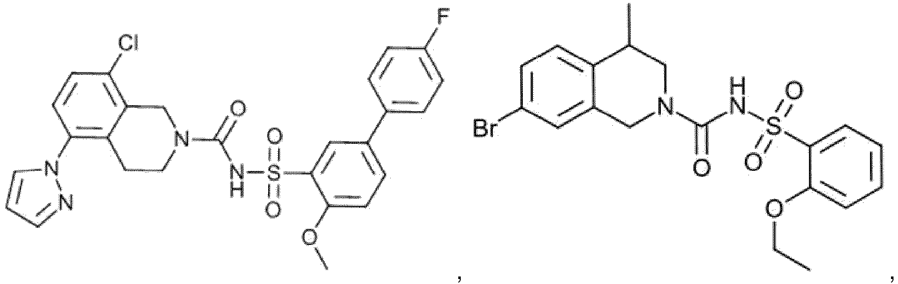 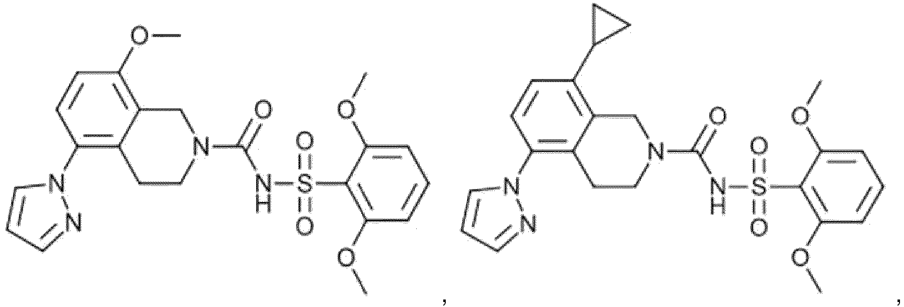 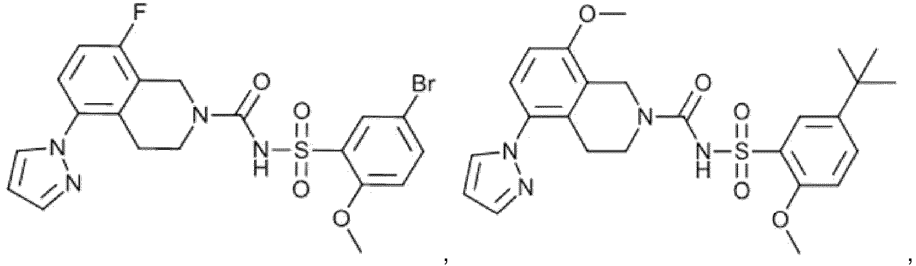 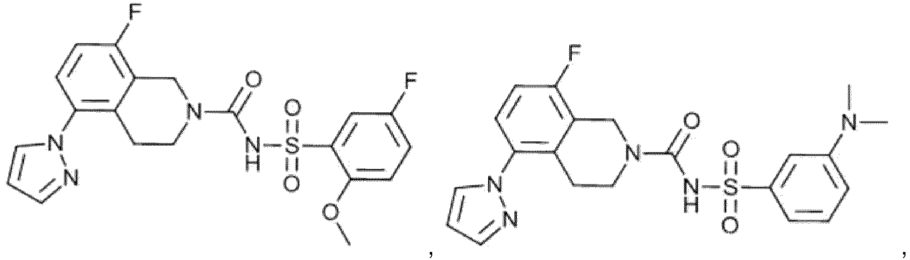 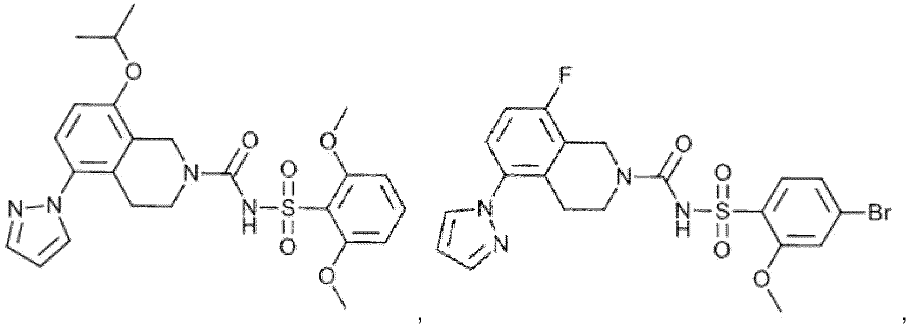 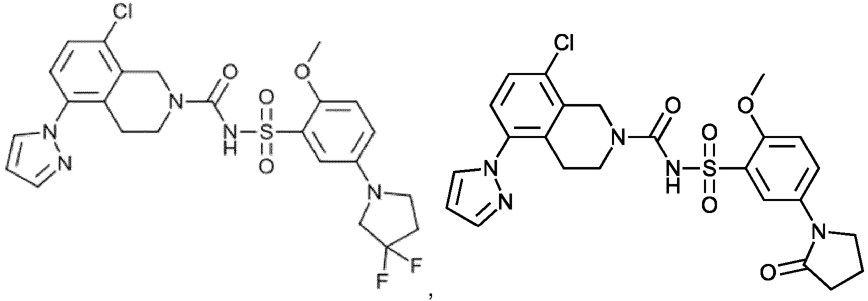 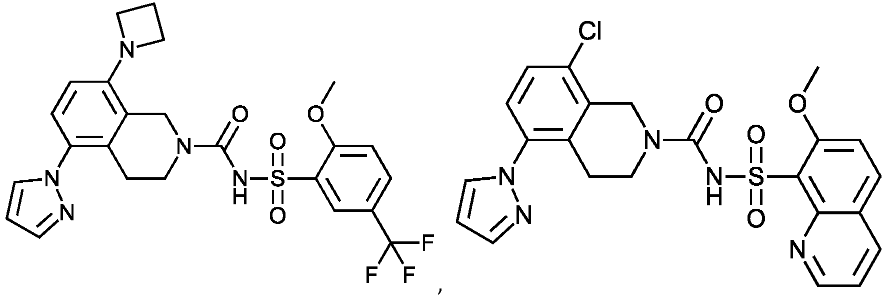 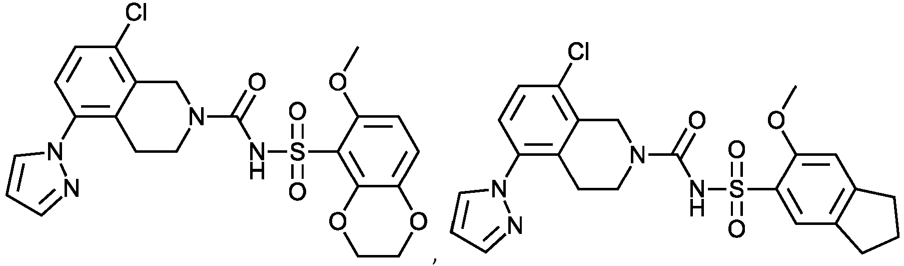 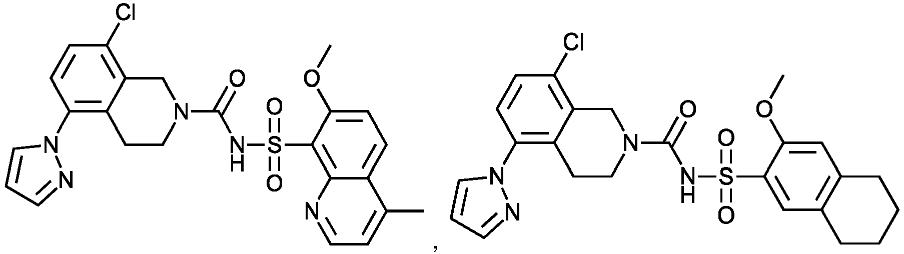 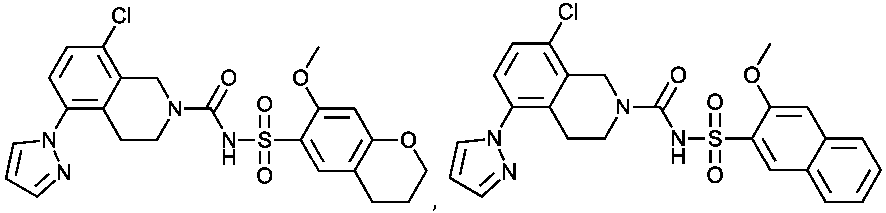 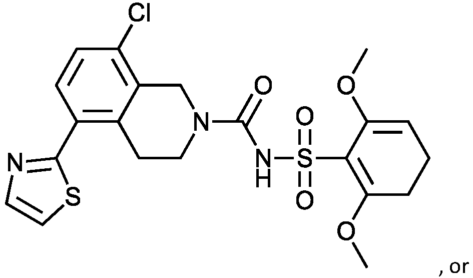 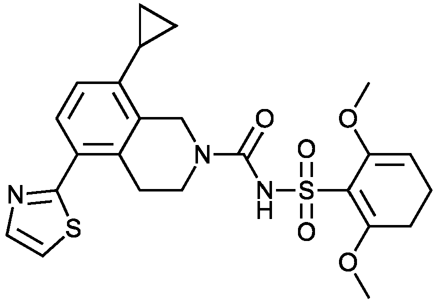
- The compound or a pharmaceutically acceptable salt thereof which is selected from: 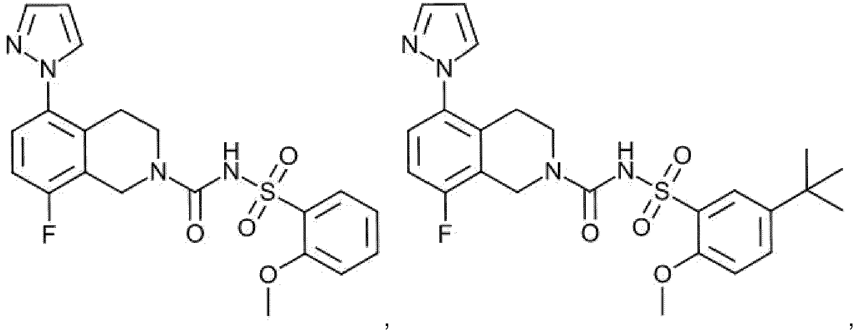 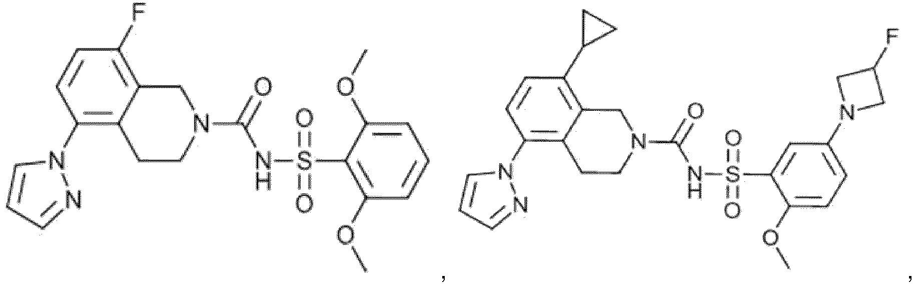 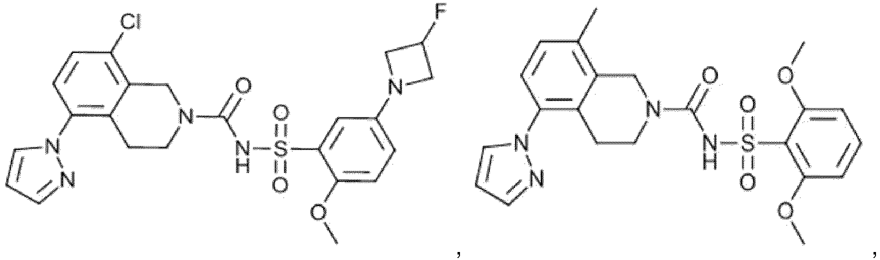 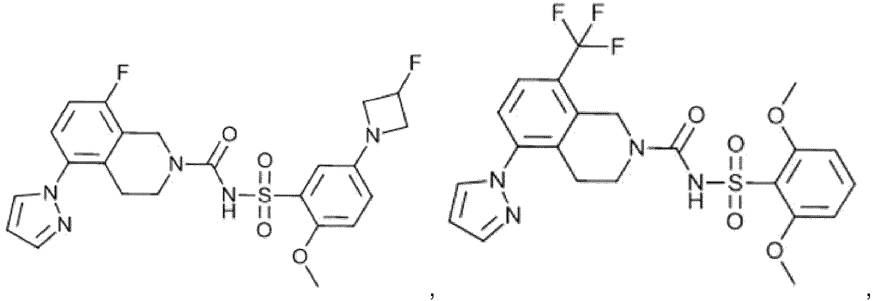 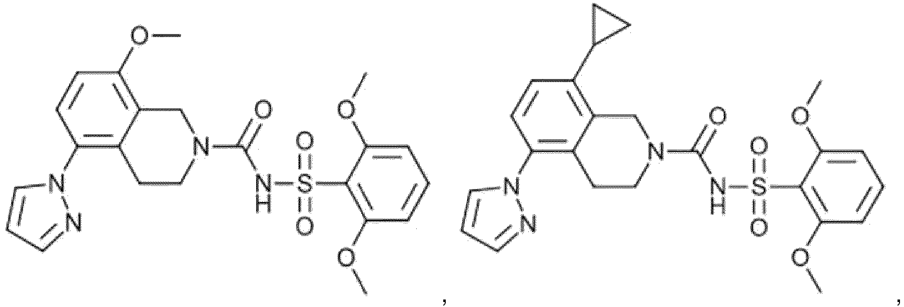 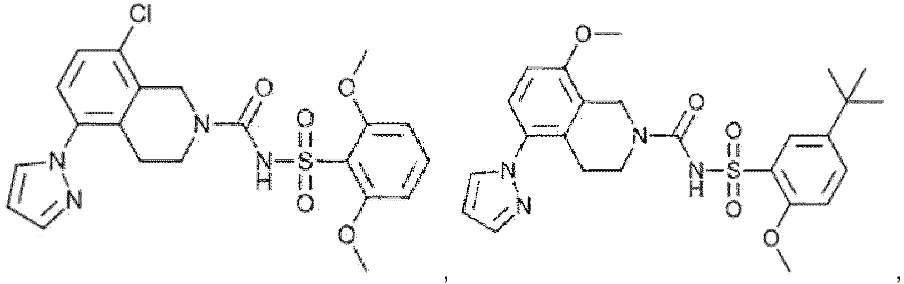 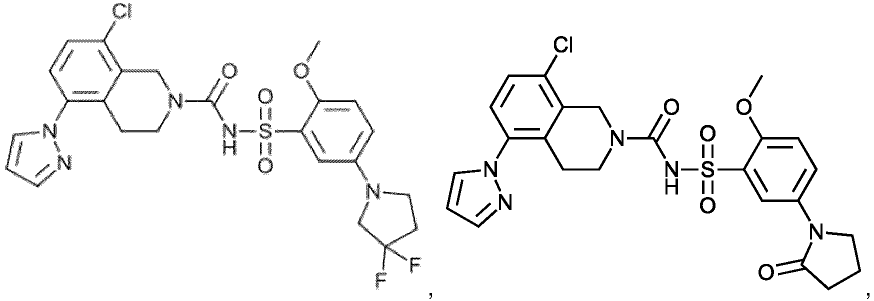 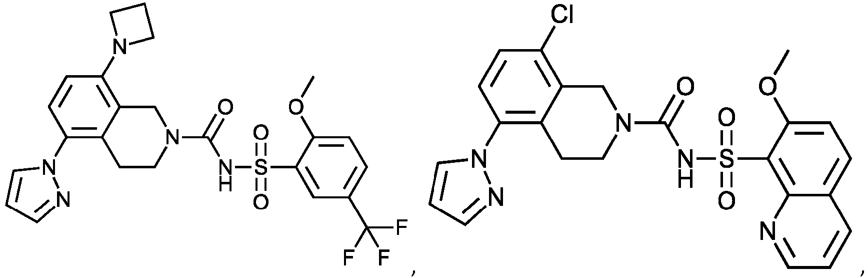 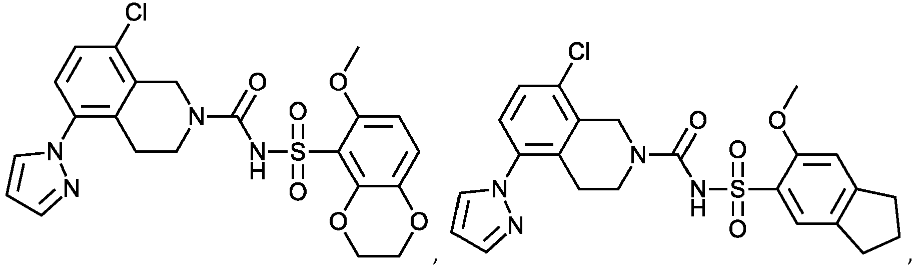 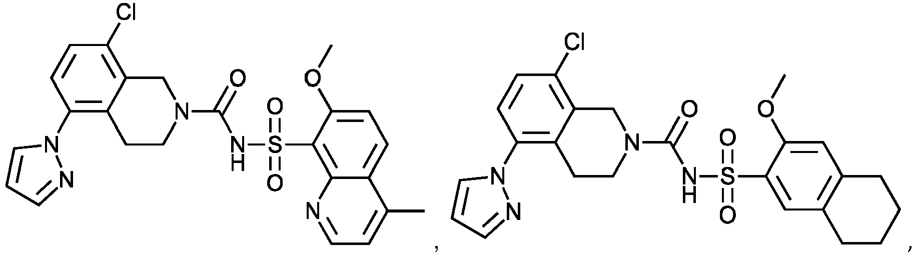 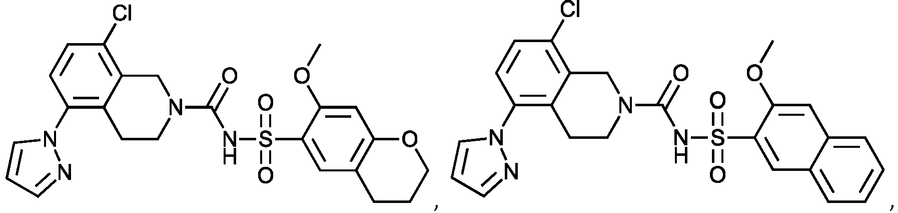 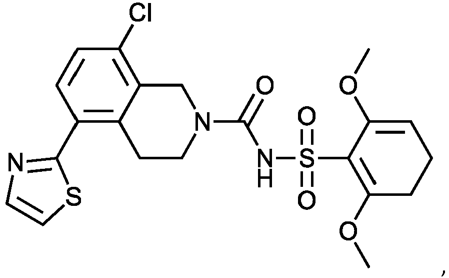 or

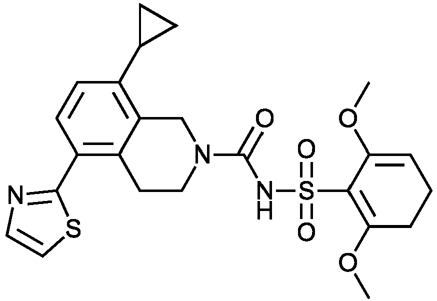

According to a **second aspect,** the invention relates to a pharmaceutical composition comprising a compound according to the invention, or a pharmaceutically acceptable salt thereof, and one or more pharmaceutically acceptable excipients.

**According to another aspect,** the invention relates to a compound according to the invention or a pharmaceutically acceptable salt thereof, or the pharmaceutical composition according to the invention, for use in the treatment or prophylaxis of a disease, preferably wherein the disease is a hyperproliferative disorder.

The compounds of the invention are particularly effective for use in the treatment or prophylaxis of a disease at modulating or inhibiting the KAT6 activity or KAT6 signaling pathway, particularly KAT6A.

The invention can in particular relate to the compound, the pharmaceutically acceptable salt or the pharmaceutical composition for use according to the invention, wherein it is for use in combination with a selective estrogen receptor degrader (SERD) inhibitor such as fulvestrant or oral alternatives like camizestrant; immune checkpoint inhibitors targeting PD1, PDL1, or CTLA4 and/or a cyclin-dependent kinase (CDK) inhibitor, including CDK2, CDK4, CDK6 or dual CDK2/4 or, CDK4/6 inhibitors, such as palbociclib, ribociclib, or abemaciclib, for enhanced therapeutic efficacy in estrogen receptor-positive cancer. The compounds of the invention are particularly effective for use in combination with a selective estrogen receptor degrader (SERD) inhibitor; immune checkpoint inhibitors targeting PD1, PDL1, or CTLA4 inhibitor and/or a cyclin-dependent kinase (CDK) inhibitor.

According to **another aspect,** the invention relates to a method for treating or prophylaxis of a disease, preferably wherein the disease is a hyperproliferative disorder, comprising administering to a patient in need thereof a compound of general formula (I), or a pharmaceutically acceptable salt thereof, according to the invention, or a pharmaceutically acceptable composition according to the invention. The method of the invention is particularly effective for use in the treatment or prophylaxis of a disease at modulating or inhibiting the KAT6 activity or KAT6 signaling pathway, particularly KAT6A.

The invention can in particular relate to the method for treating or prophylaxis of a disease according to the invention, wherein it further comprises administering to the patient in need thereof a Selective Estrogen Receptor Degrader (SERD) inhibitor such as fulvestrant or oral alternatives like elacestrant or camizestrant; immune checkpoint inhibitors targeting PD1, PDL1, or CTLA4 and/or a cyclin-dependent kinase (CDK) inhibitor, including CDK2, CDK4, CDK6 or dual CDK2/4 or, CDK4/6 inhibitors, such as palbociclib, ribociclib, or abemaciclib, for enhanced therapeutic efficacy in estrogen receptor-positive cancer, PD1, PDL1, CTLA4 and/or a Cyclin-Dependent Kinase (CDK) inhibitor, such as CDK2, CDK4, CDK6 or dual CDK2/4, CDK4/6 inhibitors, such as palbociclib, ribociclib, or abemaciclib.

The method of the invention is particularly effective for use in combination with a selective estrogen receptor degrader (SERD) such as fulvestrant or oral alternatives like elacestrant or camizestrant; immune checkpoint inhibitors targeting PD1, PDL1, or CTLA4 inhibitor and/or a cyclin-dependent kinase (CDK) inhibitor, including such as CDK2, CDK4, CDK6 or dual CDK2/4 or, CDK4/6 inhibitors, such as palbociclib, ribociclib, or abemaciclib., for enhanced therapeutic efficacy in estrogen receptor-positive cancer, PD1, PDL1, CTLA4 inhibitor and/or a Cyclin-Dependent Kinase (CDK) inhibitor, such as CDK2, CDK4, CDK6 or dual CDK2/4, CDK4/6 inhibitors, such as palbociclib, ribociclib, or abemaciclib, for enhanced therapeutic efficacy in estrogen receptor-positive cancer.

### DETAILED DESCRIPTION AND ADDITIONAL EMBODIMENTS

Below, we provide a summary of the invention and its associated terminology, followed by a discussion of the drawbacks of the prior art. Finally, we will detail how the invention effectively addresses these issues.

Unless otherwise stated, a reference to a physical measurement value in the form of an interval shall be understood to include the limits.

As used herein, the singular form **"a", "an",** and **"the"** include plural references unless indicated otherwise. For example, **"a"** substituent includes one or more substituents.

The expression **"hyperproliferative disorders"** within the meaning of the invention can refer to any diseases characterized by excessive and uncontrolled cell proliferation in humans or animals. This includes a wide range of conditions where abnormal cell growth leads to the formation of tumors or other tissue anomalies. Examples encompass various forms of cancer such as carcinomas, sarcomas, leukemias, lymphomas, and melanomas.

The term **"cancer"** within the meaning of the invention, can refer to a diverse group of diseases or disorders characterized by the uncontrolled growth and spread of abnormal cells. Cancer can manifest in various tissues and organs, presenting as solid tumors or affecting blood-forming tissues, such as in leukemia. This term encompasses a wide range of malignancies, including but not limited to carcinomas, sarcomas, leukemias, lymphomas, and melanomas. Specifically, it includes primary cancers as well as metastatic diseases, wherein cancer cells spread from the original (primary) site to other parts of the body. Examples of cancers covered by this term include lung, breast, colorectal, prostate, pancreatic, liver, stomach, esophageal, ovarian, cervical, bladder, and skin carcinomas; osteosarcoma, chondrosarcoma, liposarcoma, and rhabdomyosarcoma sarcomas; acute and chronic lymphoblastic and myeloid leukemias; Hodgkin and non-Hodgkin lymphomas; central nervous system cancers such as glioblastoma and medulloblastoma; melanomas; germ cell tumors including testicular and ovarian cancers; neuroendocrine tumors; multiple myeloma; thyroid cancers; mesotheliomas; head and neck cancers; gastrointestinal stromal tumors (GIST); Kaposi's sarcoma; and vulvar, vaginal, and penile cancers. Additionally, the term includes rare and mixed types of cancers, as well as metastatic cancers. Furthermore, it encompasses KATs overexpressing cancers, particularly those exhibiting elevated levels of KAT6, which contribute to the malignancy's progression and resistance to conventional therapies.

The term **"tumor"** within the meaning of the invention, can refer to an abnormal mass of tissue that arises from excessive and uncontrolled cell proliferation. Tumors can be classified as either benign or malignant. A benign tumor is non-cancerous and does not invade surrounding tissues or metastasize to other parts of the body. In contrast, a malignant tumor, also known as a cancerous tumor, possesses the potential to invade nearby tissues and spread (metastasize) to distant sites within the body. Hence, the term "tumor" preferably refers to solid masses that may develop in various organs or tissues, as well as neoplastic growths affecting any part of the body, including soft tissues, bones, and blood-forming organs.

The expression **"KATs overexpressing cancer"** within the meaning of the invention, can refer to cancers in which one or more lysine acetyltransferases (KATs) are produced at abnormally high levels compared to normal tissue counterparts. Specifically, this includes cancers that exhibit overexpression of KAT6, a member of the KAT family involved in chromatin remodeling and the regulation of gene expression. Overexpression of KAT6 and other KATs can drive oncogenic processes such as enhanced cell proliferation, resistance to apoptosis, metastasis, and the maintenance of stem cell-like properties in cancer cells. Examples of KATs overexpressing cancers include certain subtypes of leukemia, such as acute myeloid leukemia (AML) with KAT6A or KAT6B rearrangements, as well as solid tumors like specific forms of breast cancer, colorectal cancer, and pancreatic cancer that display elevated KAT6 expression. The expression **"KAT6A overexpressing cancer"** within the meaning of the invention, can refer to cancers in which KAT6A is produced at higher levels compared to normal tissue counterparts. In breast cancer, *KAT6A* was found to be amplified in 12-15% of tumors as part of the 8p11-12 amplicon, and is associated with poor outcomes for primary breast cancer patients, mostly of luminal subtype (Turner-Ivey, B. et al. 2017). Additionally, neuroendocrine tumors and other malignancies where KAT6 overexpression contributes to tumor progression and therapy resistance are encompassed within this definition.

The terms **"patient"** or **"subject"** within the meaning of the invention, can refer to any individual, whether human or non-human, who is undergoing treatment, diagnosis, or observation related to a medical condition or therapeutic intervention. This includes individuals currently diagnosed with a disease, disorder, or condition, as well as those who are at risk of developing such conditions. Additionally, it encompasses individuals participating in clinical trials or research studies, those receiving preventive care, and those undergoing monitoring for potential health issues. Both symptomatic and asymptomatic individuals fall within this definition.

The expressions **"patient in need thereof"** or **"subject in need thereof"** within the meaning of the invention, can refer to any individual, whether human or non-human, who requires therapeutic intervention, diagnosis, or monitoring for a specific medical condition or disease addressed by the invention. This includes individuals who are diagnosed with the targeted disease or condition and require treatment, as well as those who are at elevated risk of developing the disease and thus require preventive measures. Additionally, it encompasses individuals undergoing therapy or intervention aimed at managing, mitigating, or curing the targeted disease or condition, as well as those participating in diagnostic or monitoring procedures related to the disease. Furthermore, it includes individuals who may benefit from improved therapeutic outcomes, enhanced disease management, or reduced side effects through the use of the invention.

The terms **"treatment"** or **"treating"** within the meaning of the invention, can refer to any medical intervention aimed at managing, alleviating, reducing, or eliminating symptoms of a disease, disorder, or condition, as well as preventing its progression or recurrence. This may include the administration of pharmaceutical compounds, therapeutic agents, medical procedures, or other interventions that directly or indirectly modify the course of the condition. The terms may encompass both curative approaches, aimed at eradicating the condition, and palliative approaches, intended to relieve symptoms without necessarily curing the underlying disease. Treatment can also involve preventive measures to inhibit the onset of a disease in individuals at risk.

The terms **"prophylaxis"** within the meaning of the invention, can refer to the use of a compound that, in a statistical sample, reduces the occurrence of the disorder or condition in the treated sample relative to an untreated control sample, or delays the onset or reduces the severity of one or more symptoms of the disorder or condition relative to the untreated control sample, when administered prior to the onset of the disorder or condition.

The expression **"pharmaceutical composition"** within the meaning of the invention, can refer to a formulation that contains one or more active therapeutic agents selected from the compounds described herein, their pharmaceutically acceptable salts, prodrugs, or derivatives thereof, in combination with pharmaceutically acceptable excipients, carriers, adjuvants, or other chemical components. These compositions are prepared in a form suitable for administration to a patient and are designed to deliver the active ingredient effectively while ensuring stability, bioavailability, and eventually controlled release. The pharmaceutical composition encompasses a variety of dosage forms, including but not limited to solid forms such as tablets, capsules, granules, and powders; liquid forms such as solutions, suspensions, emulsions, and syrups; parenteral forms including injectable solutions or suspensions; topical forms like creams, ointments, gels, and lotions; and inhalable forms such as aerosols and dry powders. Additionally, these compositions are formulated for multiple routes of administration, including oral, intravenous (IV), intramuscular (IM), subcutaneous (SC), transdermal, and inhalation.

The expression **"pharmaceutically acceptable"** within the meaning of the invention, can refer to substances, compounds, or compositions that are suitable for use in humans or animals without producing significant adverse effects, toxicity, or undesirable reactions. These acceptable components are compatible with the active pharmaceutical ingredients and other formulation components, ensuring safety and efficacy in therapeutic applications. Characteristics of pharmaceutically acceptable substances include being non-toxic and non-irritating at the concentrations used, chemically and physically compatible with active ingredients and other excipients, and not adversely affecting the stability of the pharmaceutical composition.

The expression **"pharmaceutically acceptable salt"** within the meaning of the invention, can refer to a salt form of an active pharmaceutical compound that is suitable for therapeutic use in humans or animals. These salts are derived from the reaction of the active compound with pharmaceutically acceptable inorganic or organic acids or bases, enhancing properties such as solubility, stability, or bioavailability without introducing significant toxicity. Examples of pharmaceutically acceptable salts include those formed by reacting the active compound with inorganic acids like hydrochloric acid or sulfuric acid, organic acids such as citric acid or acetic acid, inorganic bases like sodium hydroxide or potassium hydroxide, and organic bases including ammonia or triethylamine. These salts may be prepared through direct neutralization during the final stages of purification or via solvent-based methods that facilitate salt formation during crystallization or precipitation. The pharmaceutically acceptable salts described herein are further detailed in Stahl, P. H., & Wermuth, C. G. (Eds.). (2011), which is incorporated by reference.

The term **"excipients",** within the meaning of the invention, can refer to pharmaceutically acceptable inert substances that are incorporated into a pharmaceutical composition alongside the active compound described herein. Excipients serve various essential functions in the formulation, including but not limited to facilitating the manufacturing process, enhancing the stability and shelf-life of the active ingredient, aiding in the delivery and absorption of the active compound, improving the taste or appearance of the pharmaceutical formulation, and ensuring uniformity and proper dosage.

The expression **"pharmaceutically acceptable excipient, carrier, adjuvant, or vehicle"** within the meaning of the invention, can refer to any substance that facilitates the delivery, administration, stability, absorption, or effectiveness of an active pharmaceutical ingredient (API) without causing significant adverse effects or negatively interacting with the API. Carriers provide a medium for the API, ensuring uniform distribution within the dosage form, while excipients serve various functions such as fillers, binders, disintegrants, lubricants, and preservatives. Adjuvants may enhance the therapeutic effect of the API, and vehicles act as solvents or dispersing agents to deliver the API in liquid or semi-solid formulations. Characteristics of pharmaceutically acceptable carriers, adjuvants, or vehicles include biocompatibility, ensuring they are non-toxic and non-irritating to the patient; compatibility, meaning they do not react adversely with the API or other formulation components; and stability, maintaining the integrity and efficacy of the pharmaceutical composition over its intended shelf life.

The term **"halogen,"** as used here, refers to either the elemental forms fluorine, chlorine, bromine, or iodine, as well as their corresponding radicals when acting as substituents in chemical compounds. Specifically, it encompasses fluoro (F), chloro (CI), bromo (Br), or iodo (I) groups that can be incorporated into the compounds of the invention.

The expression **"alkyl group",** within the meaning of the invention, can refer to saturated monovalent hydrocarbon radicals. It is derived from an alkane by removing one hydrogen atom, resulting in a structure with the general formula CₙH₂ₙ₊₁. Alkyl groups can vary in size and structure, ranging from simple methyl (CH₃-) and ethyl (C₂H₅-) groups to larger, more complex chains such as propyl (C₃H₇-), butyl (C₄H₉-), pentyl (C₅H₁₁-), and hexyl (C₆H₁₃-) groups. Alkyl groups may also include branched or ramified structures like isopropyl (CH(CH₃)₂-) and isobutyl (CH₂CH(CH₃)₂-). The terms "C₁₋₄ alkyl group" or "C₁-C₄ alkyl group" refer to both linear and branched alkyl groups containing between one and four carbon atoms. Similarly, "C₁₋₆ alkyl group" or "C₁-C₆ alkyl group" encompasses alkyl groups with one to six carbon atoms. Examples of alkyl groups include, but are not limited to, methyl, ethyl, propyl, butyl, pentyl, hexyl, isopropyl, and isobutyl groups.

The expression **"alkoxy group",** as used herein, refers to an alkyl radical bonded to an oxygen atom, represented as alkyl-O-. The alkoxy radical is attached to a molecule through the oxygen atom. Alkoxy groups can vary in size and structure, encompassing both straight and branched alkyl moieties. The terms "C₁-C₄ alkoxy" and "C₁-C₃ alkoxy" denote alkoxy radicals containing from one to four carbon atoms and from one to three carbon atoms, respectively. Examples of alkoxy groups include, but are not limited to, methoxy (CH₃O-), ethoxy (C₂H₅O-), propoxy (C₃H₇O-), isopropoxy ((CH₃)₂CHO-), and butoxy (C₄H₉O-) groups.

The expression **"amino group"** or the expression **"substituted or unsubstituted amino group"** within the meaning of the invention, can refer to a functional group consisting of a nitrogen atom bonded to one or more substituents. Specifically, it can denote primary amino group (-NH₂), secondary amino group (-NHR) where a nitrogen atom is bonded to one hydrogen atom and one alkyl or aryl group, or a tertiary amino group (-NR₂) where a nitrogen atom is bonded to two alkyl or aryl groups. In particular, an amino group according to the invention can comprise a dialkylamino group, such as a dimethylamino group (-N(CH₃)₂).

The expression **"phenyl group"** within the meaning can refer to a functional group derived from benzene, an aromatic hydrocarbon, by the removal of one hydrogen atom, resulting in the formula (-CH₅). It comprises a six-carbon aromatic ring, specifically a benzene ring, which can be attached to other atoms or functional groups in a molecule through the position where the hydrogen atom was removed. The phenyl group can be substituted at different positions on the ring, leading to derivatives such as ortho-, meta-, and para-substituted phenyl compounds. Examples of compounds containing a phenyl group include, but are not limited to, 1,3-dimethoxybenzene, where the phenyl group is attached to two methoxy group; 1-methoxy-4-(trifluoromethyl)benzene, where it is attached to a methoxy and a trifluoromethyl.

The expression **"cyclic group"** within the meaning of the invention can refer to a functional group comprising one or more ring structures formed by atoms connected in a closed loop. These rings can be saturated or unsaturated, aromatic or non-aromatic, and can consist of carbon atoms alone (carbocyclic) or a combination of carbon and heteroatoms such as nitrogen, oxygen, or sulfur (heterocyclic).

The expression **"ring system"** within the meaning of the invention, can refer to a cyclic structure composed of one or more connected rings. Ring systems can be monocyclic (a single ring) or polycyclic (multiple rings fused together or linked). Ring systems can be carbocyclic, consisting entirely of carbon atoms, or heterocyclic, containing one or more heteroatoms such as nitrogen, oxygen, or sulfur. Ring systems include, but are not limited to, saturated monocyclic rings, saturated polycyclic rings, aromatic monocyclic rings or aromatic polycyclic rings.

The term **"carbocyclic ring system"** refers to a ring composed exclusively of carbon atoms. Carbocyclic ring systems can be monocyclic or polycyclic, and can comprise saturated (alicyclic), as in cycloalkyl groups, and/or aromatic, as in aryl groups.

The expression **"cycloalkyl group"** within the meaning of the invention, can refer to saturated cyclic hydrocarbon radical derived from cycloalkanes by the removal of one hydrogen atom, resulting in a structure with the general formula CₙH₂ₙ₋₁, where n typically ranges from three to six. Examples of cycloalkyl group include, but are not limited to, C3-C6 cycloalkyl.

The expression **"cycloalkyloxy group"** within the meaning of the invention, can refer to an alkoxy group in which the alkyl moiety is a cycloalkyl group. It is represented as cycloalkyl-O-, where the cycloalkyl portion is a saturated cyclic hydrocarbon group. Examples of cycloalkyloxy groups include, but are not limited to, cyclopropyloxy, cyclobutyloxy, cyclopentyloxy, or cyclohexyloxy.

The expression **"aryl group"** within the meaning of the invention, can refer to a functional group derived from an aromatic hydrocarbon. It comprises an aromatic ring, typically a benzene ring (C₆H₅-), which can be attached to other atoms or functional groups in a molecule. The term "C₆-C₁₀ aryl" encompasses aryl groups containing from six to ten carbon atoms. Examples of aryl groups include, but are not limited to, phenyl (C₆H₅-), naphthyl (C₁₀H₇-), anthracyl (C₁₄H₁₀-), and their respective isomers such as 1-naphthyl and 2-anthracyl. Additionally, "aryl" includes fused polycyclic aromatic ring systems, such as bicyclic group, where an aromatic ring is fused to one or more additional rings, aromatic or not, such as heterocyclic rings containing heteroatoms, whether saturated or partially saturated. Examples include, but are not limited to, indanyl (2,3-dihydro-1H-indene) and tetrahydronaphthyl (1,2,3,4-tetrahydronaphthyl), where the point of attachment is on the aromatic ring.

The expression **"heterocyclic ring system"** refers to a ring system containing at least one heteroatom (nitrogen, oxygen, or sulfur) within the ring. Heterocyclic ring systems can be monocyclic or polycyclic, and can be saturated, partially saturated, or aromatic. The ring size can vary, typically containing 3 to 7 ring atoms per ring.

The term **"heteroatom"** within the meaning of the invention, can refer to an atom within a molecule that is not carbon or hydrogen. Specifically, it includes atoms such as nitrogen (N), oxygen (O), sulfur (S), phosphorus (P), silicon (Si), and other elements that can substitute for carbon in organic compounds.

The expression **"heterocyclyl group"** within the meaning of the invention, can refer to a substituent derived from a heterocycle by the removal of one hydrogen atom, resulting in a group that can be attached to other atoms or functional groups within a molecule. A heterocycle refers to a cyclic structure, saturated, partially saturated, or aromatic ring, in which one or more of the atoms in the ring are elements other than carbon, such as nitrogen, oxygen, or sulfur. Heterocycles can be aromatic or non-aromatic and may contain various numbers of heteroatoms within the ring. Examples of heterocyclyl group include, but are not limited to, 4- to 6-member heterocyclyl group, such as azetidine (derived from azetidine, a four-membered ring containing one nitrogen atom), imidazolyl (derived from imidazole, a five-membered ring containing two nitrogen atoms), pyrrolidinyl (derived from pyrrolidine, a five-membered ring containing one nitrogen atom), or thiazolyl (derived from thiazole, a five-membered ring containing both nitrogen and sulfur atoms). These heterocyclyl groups may be optionally substituted by an alkyl group, an oxo group or a halogen, allowing for further diversification of the compound's structure, optionally substituted by an alkyl group and/or a halogen.

The expression **"heteroaryl group"** within the meaning of the invention, can refer to an aryl group in which one or more carbon atoms in the aromatic ring are replaced by heteroatoms such as nitrogen, oxygen, or sulfur. These heteroatoms can be incorporated into the aromatic ring in various positions, leading to structures such as pyridyl (where a nitrogen replaces a carbon in a benzene ring), thiophenyl (an aryl group with sulfur substitution in the aromatic ring), furanyl (an aryl group with oxygen substitution in the aromatic ring) groups, or indolyl (an aryl group derived from indole, containing both nitrogen and fused aromatic rings). Heteroaryl groups maintain the aromatic character of the ring. Additionally, "heteroaryl" includes fused polycyclic aromatic ring systems where an aromatic ring comprising one or more heteroatoms is fused to one or more additional rings, aromatic or not.

The expression **"heterocycloalkyl group"** within the meaning of the invention, can refer to a cycloalkyl group in which one or more carbon atoms are replaced by heteroatoms such as nitrogen, oxygen, or sulfur, thereby forming a heterocyclic ring. Examples of heterocycloalkyl groups include, but are not limited to, a 4- to 6-member heterocycloalkyl group such as azetidinyl (a four-membered ring containing one nitrogen atom, derived from azetidine), morpholinyl (a six-membered ring containing both nitrogen and oxygen atoms, derived from morpholine), piperidinyl (a six-membered ring containing one nitrogen atom, derived from piperidine) or thiazolidinyl (a five-membered ring containing both nitrogen and sulfur atoms, derived from thiazolidine). Heterocycloalkyl groups may be optionally substituted by one or more alkyl groups, oxo groups and/or halogens, allowing for further structural diversification and modulation of the compound's pharmacological properties.

The expression **"bicyclic ring"** can refer to a ring system composed of two fused rings, which can be both carbocyclic, both heterocyclic, or a combination of carbocyclic and heterocyclic rings. The rings can be saturated, partially saturated, or aromatic. Bicyclic ring can be homocyclic (both rings of the same type) or heterocyclic (rings of different types). Bicyclic ring can be further substituted by cyclic group.

The expression **"polycyclic ring"** can refer to a ring system composed of at least two fused rings, which can be both carbocyclic, both heterocyclic, or a combination of carbocyclic and heterocyclic rings. The rings can be saturated, partially saturated, or aromatic. Polycyclic ring can be homocyclic (both rings of the same type) or heterocyclic (rings of different types). Polycyclic ring can be further substituted by cyclic group.

The expression **"benzo-fused alicyclic group"** refers to a polycyclic ring system formed by the fusion of a benzene ring with a heterocycloalkyl ring or a cycloalkyl ring. The heterocycloalkyl or cycloalkyl portion are a saturated or partially saturated ring. Such expression encompasses the benzo-fused heterocycloalkyl group and benzo-fused cycloalkyl group. Examples include dihydrobenzofuran (a partially saturated furan ring fused to a benzene ring).

The expression **"benzo-fused heterocyclic group"** can refer to a polycyclic ring comprising a benzene ring which is fused to a heterocyclic ring containing at least one heteroatom. The heterocyclic ring can be five- to seven-membered, it is partially saturated. Examples include quinoline (benzene fused to a pyridine ring), indoline (benzene fused to a pyrrolidine ring), dihydrobenzofuran (benzene fused to a tetrahydrofuran ring), and dihydrobenzothiophene (benzene fused to a tetrahydrothiophene ring). A "benzo-fused heterocyclic group" can comprise more than two ring systems with for example a ring system fused to the heterocyclic ring such as fused cycloalkyl or spirocyclic systems.

The expression **"benzo-fused oxygen containing heterocyclic group"** can refer to a polycyclic ring comprising a benzene ring which is fused to a heterocyclic ring containing at least one oxygen atom. These polycyclic ring systems can be fused, bridged or spirocyclic systems; They preferably consist in a 9 to 15 atom polycyclic ring system containing at least one oxygen atom and in which one ring is a phenyl. The heterocyclic ring can be five- to seven-membered, fully or partially saturated. Examples include dihydro benzodioxepine, tetrahydro-2-benzoxepine and dihydrobenzofuran. A "benzo-fused oxygen containing heterocyclic group" can comprise more than two ring systems with for example a ring system fused to the heterocyclic ring such-as 3-Oxabicyclo[4.1.0]heptane or 2-Oxabicyclo [3.1.0]hexane or a spirocyclic systems such as 5-Oxaspiro[2.4]heptane or 6-Oxaspiro[2.5]octane.

The expression **"benzo-fused heterocycloalkyl group"** refers to a polycyclic ring system formed by the fusion of a benzene ring with a heterocycloalkyl ring. The heterocycloalkyl portion is a saturated or partially saturated ring that contains one or more heteroatoms such as nitrogen, oxygen, or sulfur. This fused ring system can be saturated or partially saturated. Examples of benzo-fused heterocycloalkyl groups include dihydrobenzofuran (benzene fused to a partially saturated oxygen-containing ring).

The expression **"benzo-fused cycloalkyl group"** refers to a polycyclic ring system formed by the fusion of a benzene ring with a cycloalkyl ring. The cycloalkyl portion is a saturated or partially saturated cyclic hydrocarbon ring. This fused ring system can be saturated or partially saturated and includes both carbocyclic ring. Examples of benzo-fused cycloalkyl groups include indane (benzene fused to cyclopentane) and tetralin (benzene fused to cyclohexane).

The expression **"partially saturated"** within the meaning of the invention, can refer to a molecule, a molecular fragment, or a group that contains both single bonds and multiple bonds (such as double or triple bonds) within its structure. This means that while part of the molecule retains full saturation (i.e., single bonds between atoms), certain regions of the molecule exhibit unsaturation, often involving carbon-carbon or carbon-heteroatom double or triple bonds

The term **"unsaturated"** within the meaning of the invention, can refer to a group or functional group that contains one or more double or triple bonds between carbon atoms or between carbon and heteroatoms (such as nitrogen, oxygen, or sulfur) within its structure.

The term **"substituted"** within the meaning of the invention, can refer to a molecule, a molecular fragment or a group in which one or more hydrogen atoms have been replaced by other atoms or functional groups. The expression **"optionally substituted"** within the meaning of the invention, can refer to a molecule, a molecular fragment or a group that may or may not contain one or more substituent groups attached to its core structure.

The term **"solvate"** is used herein to describe a molecular complex comprising a compound described herein and one or more pharmaceutically acceptable solvent molecules, for example, ethanol.

### Compounds of the Invention

In some embodiments, a compound of the present disclosure is represented by general formula (I): 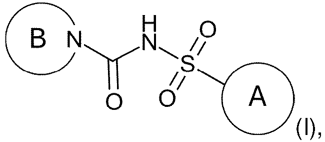 or a pharmaceutically acceptable salt thereof, wherein:
A represents an aryl group, such as a phenyl group or a benzo-fused cycloalkyl group, or a heteroaryl group, such as a benzo-fused heterocyclic group; wherein A is optionally substituted by one or more members selected from the group consisting of:
   - alkoxy groups, such as a C₁-C₄ alkoxy group;
   - alkyl groups, such as a C₁-C₄ alkyl group, optionally substituted by 1 to 3 fluorine atoms;
   - halogens;
   - unsubstituted or substituted amino groups, such as dialkylamino groups, such as a dimethylamino group (-N(CH₃)₂);
   - heterocyclyl groups, preferably a 4- to 6-member heterocycloalkyl group, such as azetidine or pyrrolidine, optionally substituted by one or more alkyl groups, oxo groups and/or halogens;
   - phenyl groups, optionally substituted by one or more alkyl groups and/or halogens; and
   - cycloalkyl group, optionally substituted by one or more alkyl groups and/or halogens,such as a C₃-C₅ cycloalkyl group optionally substituted by 1 to 2 fluorine atoms; and
B represents a partially saturated nitrogen containing bicyclic ring system, such as tetrahydro-isoquinoline or isoindoline, substituted by one or more from:
   - halogens;
   - dialkylamino groups, such as a dimethylamino group (-N(CH₃)₂);
   - cycloalkyl groups optionally substituted by one or more alkyl groups and/or halogens, such as a C₃-C₅ cycloalkyl group optionally substituted by 1 to 2 fluorine atoms;
   - alkoxy groups such as a C₁-C₄ alkoxy group;
   - 3- to 6-member cycloalkyloxy groups, such as cyclopropyloxy, optionally substituted by one or more alkyl groups and/or halogens;
   - 4- to 6-member heterocycloalkyl groups, optionally substituted by one or more alkyl groups and/or halogens, which is directly bonded to the bicyclic ring system via a covalent bond or linked through a spacer, preferably chosen from a C₁-C₄ alkylene chain, or a single oxygen atom; for example a 4- to 6-member heterocyclyl group, such as azetidine, optionally substituted by one or more alkyl groups and/or halogens;
   - 5- or 6-member heteroaryl groups, optionally substituted by one or more alkyl groups and/or halogens, which is directly bonded to the bicyclic ring system via a covalent bond or linked through a spacer, preferably chosen from a C₁-C₄ alkylene chain, or a single oxygen atom; for example a 5- or 6-member heteroaryl group, such as pyrazole, isoxazole, thiazole, pyridine or oxazole, optionally substituted by one or more alkyl groups and/or halogens; and
   - alkyl groups, such as a C₁-C₄ alkyl group, optionally substituted by 1 to 3 fluorine atoms.

In some embodiments, the invention relates to a compound of general formula (I), or a pharmaceutically acceptable salt thereof, wherein:
A represents an aryl group or a heteroaryl group, substituted by one or more alkoxy groups, such as a C₁-C₄ alkoxy group, and/or alkyl groups, such as a C₁-C₄ alkyl group, optionally substituted by 1 to 3 fluorine atoms;
wherein A is optionally further substituted by one or more members selected from the group consisting of:
- alkoxy groups, such as a C₁-C₄ alkoxy group;
- alkyl groups, such as a C₁-C₄ alkyl group, optionally substituted by 1 to 3 fluorine atoms;
- halogens;
- unsubstituted or substituted amino groups, such as dialkylamino groups, such as a dimethylamino group (-N(CH₃)₂);
- heterocyclyl groups, preferably a 4- to 6-member heterocycloalkyl group, such as azetidine or pyrrolidine, optionally substituted by one or more alkyl groups, oxo groups and/or halogens;
- phenyl groups, optionally substituted by one or more alkyl groups and/or halogens; and
- cycloalkyl groups optionally substituted by one or more alkyl groups and/or halogens, such as a C₃-C₅ cycloalkyl group optionally substituted by 1 to 2 fluorine atoms.

In preferred embodiments, the invention relates to a compound of general formula (I), or a pharmaceutically acceptable salt thereof, wherein:
A represents an aryl group or a heteroaryl group;
wherein A is optionally substituted by one or more members selected from the group consisting of:
- halogens;
- alkoxy groups, such as a C₁-C₄ alkoxy group;
- alkyl groups, such as a C₁-C₄ alkyl group, optionally substituted by 1 to 3 fluorine atoms;
- unsubstituted or substituted amino groups, such as dialkylamino groups, such as a dimethylamino group (-N(CH₃)₂);
- heterocyclyl groups, preferably a 4- to 6-member heterocycloalkyl group, such as azetidine or pyrrolidine, optionally substituted by one or more alkyl groups, oxo groups and/or halogens; and
- cycloalkyl groups optionally substituted by one or more alkyl groups and/or halogens, such as a C₃-C₅ cycloalkyl group optionally substituted by 1 to 2 fluorine atoms.

In more preferred embodiments, the invention relates to a compound of general formula (I) or formula (III), or a pharmaceutically acceptable salt thereof, wherein:
A represents a phenyl group substituted by an alkoxy group such as a C₁-C₄ alkoxy group;
wherein A is further substituted by one or more members selected from the group consisting of:
   - alkoxy groups, such as a C₁-C₄ alkoxy group;
   - alkyl groups, such as a C₁-C₄ alkyl group, optionally substituted by 1 to 3 fluorine atoms;
   - halogens; and
   - unsubstituted or substituted amino groups, such as dialkylamino groups, such as a dimethylamino group (-N(CH₃)₂).

In even more preferred embodiments, the invention relates to a compound of general formula (I) or formula (III), or a pharmaceutically acceptable salt thereof, wherein:
A represents a benzo-fused heterocyclic group, optionally substituted by an alkoxy group such as a C₁-C₄ alkoxy group;
wherein A is optionally substituted, or further substituted by one or more members selected from the group consisting of:
- alkoxy groups, such as a C₁-C₄ alkoxy group;
- alkyl groups, such as a C₁-C₄ alkyl group, optionally substituted by 1 to 3 fluorine atoms;
- 4- to 6-member heterocyclyl groups, such as azetidine or pyrrolidine, optionally substituted by one or more alkyl groups, oxo groups and/or halogens; and
- cycloalkyl groups optionally substituted by one or more alkyl groups and/or halogens, such as a C₃-C₅ cycloalkyl group optionally substituted by 1 to 2 fluorine atoms.

In some embodiments, the invention relates to a compound of general formula (I) or formula (II), or a pharmaceutically acceptable salt thereof, wherein:
B represents a partially saturated nitrogen containing bicyclic ring system, such as tetrahydro-isoquinoline or isoindoline, comprising an aryl part substituted by a 5- or 6-member heteroaryl group, which is directly bonded to the aryl part of the bicyclic ring system via a covalent bond or linked through a spacer, said spacer being preferably chosen from a C₁-C₄ alkylene chain or a single oxygen atom;
wherein B is further substituted by one or more members selected from the group consisting of:
   - halogens;
   - dialkylamino groups, such as a dimethylamino group (-N(CH₃)₂);
   - cycloalkyl groups optionally substituted by one or more alkyl groups and/or halogens, such as a C₃-C₅ cycloalkyl group optionally substituted by 1 to 2 fluorine atoms;
   - alkoxy groups such as a C₁-C₄ alkoxy group;
   - 3- to 6-member cycloalkyloxy groups, such as cyclopropyloxy, optionally substituted by one or more alkyl groups and/or halogens; and
   - alkyl groups, such as a C₁-C₄ alkyl group, optionally substituted by 1 to 3 fluorine atoms.

In preferred embodiments, the invention relates to a compound of general formula (I) or formula (II) or a pharmaceutically acceptable salt thereof, wherein:
B represents a partially saturated nitrogen containing heterocycloalkyl-fused aryl substituted by a 5- or 6-member heteroaryl group, which is directly bonded to the aryl part of the heterocycloalkyl-fused aryl through a covalent bond or linked through a spacer, said spacer being preferably chosen from a C₁-C₄ alkylene chain or a single oxygen atom;
wherein B is further substituted by one or more members selected from the group consisting of:
   - halogens;
   - dialkylamino groups, such as a dimethylamino group (-N(CH₃)₂);
   - cycloalkyl groups optionally substituted by one or more alkyl groups and/or halogens, such as a C₃-C₅ cycloalkyl group optionally substituted by 1 to 2 fluorine atoms;
   - alkoxy groups, such as a C₁-C₄ alkoxy group;
   - 3- to 6-member cycloalkyloxy groups, such as cyclopropyloxy, optionally substituted by one or more alkyl groups and/or halogens; and
   - alkyl groups, such as a C₁-C₄ alkyl group, optionally substituted by 1 to 3 fluorine atoms.

In more preferred embodiments, the invention relates to a compound of general formula (I) or formula (II), or a pharmaceutically acceptable salt thereof, wherein:
B represents a partially saturated nitrogen containing heterocycloalkyl-fused aryl substituted by a 5- or 6-member heteroaryl group, which is directly bonded to the aryl part of the heterocycloalkyl-fused aryl through a covalent bond;
wherein B is optionally further substituted by one or more members selected from the group consisting of:
   - halogens;
   - cycloalkyl groups optionally substituted by one or more alkyl groups and/or halogens, such as a C₃-C₅ cycloalkyl group optionally substituted by 1 to 2 fluorine atoms; and
   - alkoxy groups, such as a C₁-C₄ alkoxy group;

In even more preferred embodiments, the invention relates to a compound of general formula (I) or formula (II), or a pharmaceutically acceptable salt thereof, wherein:
B represents a partially saturated nitrogen containing heterocycloalkyl-fused aryl substituted by a pyrazole which is directly bonded to the aryl part of the heterocycloalkyl-fused aryl; wherein B is optionally further substituted by one or more members selected from the group consisting of:
- halogens;
- cycloalkyl groups optionally substituted by one or more alkyl groups and/or halogens, such as a C₃-C₅ cycloalkyl group optionally substituted by 1 to 2 fluorine; and
- alkoxy groups, such as a C₁-C₄ alkoxy group.

In some embodiments, the invention relates to a compound of general formula (I) or formula (II), or a pharmaceutically acceptable salt thereof, wherein:
B is a tetrahydro-isoquinoline substituted on its aryl part of the bicyclic ring system by a 5- or 6-member heteroaryl group, which is directly bonded to the aryl part via a covalent bond or linked through a spacer such as an alkyl chain;
wherein B is optionally further substituted by one or more members selected from the group consisting of:
   - halogens;
   - dialkylamino groups, such as a dimethylamino group (-N(CH₃)₂);
   - cycloalkyl groups optionally substituted by one or more alkyl groups and/or halogens, such as a C₃-C₅ cycloalkyl group optionally substituted by 1 to 2 fluorine atoms;
   - alkoxy groups, such as a C₁-C₄ alkoxy group;
   - 3- to 6-member cycloalkyloxy groups, such as cyclopropyloxy, optionally substituted by one or more alkyl groups and/or halogens; and
   - alkyl groups, such as a C₁-C₄ alkyl group, optionally substituted by 1 to 3 fluorine atoms.

In preferred embodiments, the invention relates to a compound of general formula (I), or a pharmaceutically acceptable salt thereof, wherein:
A represents an aryl group or a heteroaryl group;
wherein A is optionally substituted or, further substituted by one or more members selected from the group consisting of:
   - alkoxy groups, such as a C₁-C₄ alkoxy group;
   - alkyl groups, such as a C₁-C₄ alkyl group, optionally substituted by 1 to 3 fluorine atoms;
   - halogens;
   - unsubstituted or substituted amino groups, such as dialkylamino groups, such as a dimethylamino group (-N(CH₃)₂);
   - phenyl groups, optionally substituted by one or more alkyl groups and/or halogens;
   - heterocyclyl groups, preferably a 4- to 6-member heterocycloalkyl group, such as azetidine or pyrrolidine, optionally substituted by one or more alkyl groups, oxo groups and/or halogens; and
   - cycloalkyl groups optionally substituted by one or more alkyl groups and/or halogens, such as a C₃-C₅ cycloalkyl group optionally substituted by 1 to 2 fluorine atoms; and
B represents a partially saturated nitrogen containing heterocycloalkyl-fused aryl substituted by a 5- or 6-member heteroaryl group, which is directly bonded to the aryl part of the heterocycloalkyl-fused aryl via a covalent bond or linked through a spacer, said spacer being preferably chosen from a C₁-C₄ alkylene chain, or a single oxygen atom;
wherein B is optionally further substituted by one or more members selected from the group consisting of:
   - halogens;
   - dialkylamino groups, such as a dimethylamino group (-N(CH₃)₂);
   - cycloalkyl groups optionally substituted by one or more alkyl groups and/or halogens, such as a C₃-C₅ cycloalkyl group optionally substituted by 1 to 2 fluorine atoms;
   - alkoxy groups such as a C₁-C₄ alkoxy group;
   - 3- to 6-member cycloalkyloxy groups, such as cyclopropyloxy, optionally substituted by one or more alkyl groups and/or halogens; and
   - alkyl groups, such as a C₁-C₄ alkyl group, optionally substituted by 1 to 3 fluorine atoms.

In more preferred embodiments, the invention relates to a compound of general formula (I), or a pharmaceutically acceptable salt thereof, wherein:
A represents a phenyl group substituted by an alkoxy group such as a C₁-C₄ alkoxy group;
wherein A is further substituted by one or several from:
   - alkoxy groups, such as a C₁-C₄ alkoxy group;
   - alkyl groups, such as a C₁-C₄ alkyl group, optionally substituted by 1 to 3 fluorine atoms;
   - halogens; and
   - unsubstituted or substituted amino groups, such as dialkylamino groups, such as a dimethylamino group (-N(CH₃)₂); and
B represents a partially saturated nitrogen containing heterocycloalkyl-fused aryl substituted by a 5- or 6-member heteroaryl group, which is directly bonded to the aryl part of the heterocycloalkyl-fused aryl via a covalent bond;
wherein B is optionally further substituted by one or more members selected from the group consisting of:
   - halogens;
   - cycloalkyl groups optionally substituted by one or more alkyl groups and/or halogens, such as a C₃-C₅ cycloalkyl group optionally substituted by 1 to 2 fluorine atoms; and
   - alkoxy groups, such as a C₁-C₄ alkoxy group.

In even more preferred embodiments, the invention relates to a compound of general formula (I), or a pharmaceutically acceptable salt thereof, wherein:
A represents a phenyl group substituted by an alkoxy group such as a C₁-C₄ alkoxy group;
wherein A is further substituted by one or more members selected from the group consisting of:
   - alkoxy groups, such as a C₁-C₄ alkoxy group;
   - alkyl groups, such as a C₁-C₄ alkyl group, optionally substituted by 1 to 3 fluorine atoms;
   - 4- to 6-member heterocyclyl groups, such as azetidine or pyrrolidine, optionally substituted by one or more alkyl groups, oxo groups and/or halogens; and
   - cycloalkyl groups optionally substituted by one or more alkyl groups and/or halogens, such as a C₃-C₅ cycloalkyl group optionally substituted by 1 to 2 fluorine atoms; and
B represents a partially saturated nitrogen containing heterocycloalkyl-fused aryl substituted by a pyrazole which is directly bonded to the aryl part of the heterocycloalkyl-fused aryl;
wherein B is further substituted by one or more members selected from the group consisting of:
   - halogens;
   - cycloalkyl groups optionally substituted by one or more alkyl groups and/or halogens, such as a C₃-C₅ cycloalkyl group optionally substituted by 1 to 2 fluorine; and
   - alkoxy groups, such as a C₁-C₄ alkoxy group.

In some embodiments, a compound of Formula (I), or pharmaceutically acceptable salt thereof, is a compound of Formula (II): 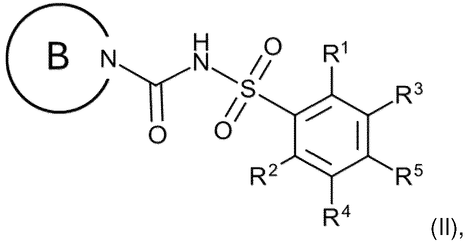 or a pharmaceutically acceptable salt thereof, wherein:
B is selected as previously described;
R¹, R², R³, R⁴, and R⁵ each independently represent H; an alkyl group, such as a C₁-C₄ alkyl group, optionally substituted by 1 to 3 fluorine atoms; a cycloalkyl group, optionally substituted by one or more alkyl groups and/or halogens; a heterocyclyl group, preferably a 4- to 6-member heterocycloalkyl group, optionally substituted by one or more alkyl groups, oxo groups and/or halogens; a halogen; an alkoxy group, such as a C₁-C₄ alkoxy group; a cycloakyloxy group, optionally substituted by one or more alkyl groups and/or halogens; a phenyl group, optionally substituted by one or more alkyl groups and/or halogens; or a substituted or unsubstituted amino groups, such as dialkylamino groups, such as a dimethylamino group (-N(CH₃)₂);
alternatively, any one pair selected from R¹ and R³, R² and R⁴, R⁴ and R⁵, or R³ and R⁵, taken together, form a 5- or 6-member heterocycloalkyl group or an heteroaromatic ring or a 5- or 6-member cycloalkyl group, optionally substituted by one or more alkyl groups and/or halogens, while the remaining R¹, R², R³, R⁴, and R⁵, each independently, represent H; an alkyl group, optionally substituted by 1 to 3 fluorine atoms; a cycloalkyl group, optionally substituted by one or more alkyl groups and/or halogens; a heterocyclyl group, preferably a 4- to 6-member heterocycloalkyl group, optionally substituted by one or more alkyl groups, oxo groups and/or halogens; a halogen; an alkoxy group, such as a C₁-C₄ alkoxy group; a cycloakyloxy group, optionally substituted by one or more alkyl groups and/or halogens; a phenyl group, optionally substituted by one or more alkyl groups and/or halogens; or a substituted or unsubstituted amino group, such as dialkylamino group, such as a dimethylamino group (-N(CH₃)₂).

In some embodiments, a compound of Formula (I), or pharmaceutically acceptable salt thereof, is a compound of **Formula (III):** 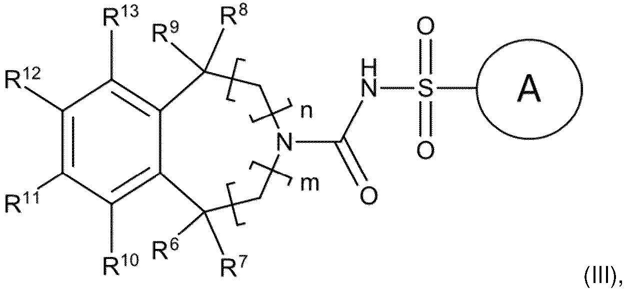 or a pharmaceutically acceptable salt thereof, wherein:
A is selected as previously described;
R⁶, R⁷, R⁸, and R⁹ each independently represent H; or an alkyl group;
n and m each independently represent 0 or 1;
R¹⁰ and R¹³ each independently represent H; a halogen; an alkoxy group; a 4- to 6-member heterocycloalkyl group or a 5- or 6-member heteroaryl group, optionally substituted by one or more alkyl groups and/or halogens; a cycloalkyl group, optionally substituted by one or more alkyl groups and/or halogens; or an alkyl group, optionally substituted by 1 to 3 fluorine atoms; and
R¹¹ and R¹² each independently represent H; a halogen; a dialkylamino group, such as a dimethylamino group (-N(CH₃)₂); a cycloalkyl group optionally substituted by one or more alkyl groups and/or halogens; a 4- to 6-member heterocycloalkyl group, optionally substituted by one or more alkyl groups and/or halogens, such as azetidine, optionally substituted by one or more alkyl groups and/or halogens; or a 5- or 6-member heteroaryl group, optionally substituted by one or more alkyl groups and/or halogens, which is directly bonded to the aryl via a covalent bond or linked through a spacer such as an alkyl chain; such as pyrazole or oxazole, optionally substituted by one or more alkyl groups and/or halogens.

In some embodiments, a compound of Formula (I), or pharmaceutically acceptable salt thereof, is a compound of Formula (IV): 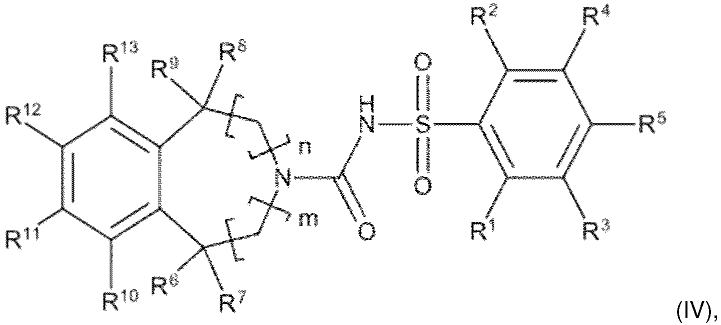 or a pharmaceutically acceptable salt thereof, wherein:
R¹, R², R³, R⁴, and R⁵ each independently represent H; an alkyl group, such as a C₁-C₄ alkyl group, optionally substituted by 1 to 3 fluorine atoms; a cycloalkyl group, optionally substituted by one or more alkyl groups and/or halogens; a heterocyclyl group, preferably a 4- to 6-member heterocycloalkyl group, optionally substituted by one or more alkyl groups, oxo groups and/or halogens; a halogen; an alkoxy group such as a C₁-C₄ alkoxy group; a cycloakyloxy group, optionally substituted by one or more alkyl groups and/or halogens; a phenyl group, optionally substituted by one or more alkyl groups and/or halogens; or a substituted or unsubstituted amino group, such as dialkylamino groups, such as a dimethylamino group (-N(CH₃)₂);
alternatively, any one pair selected from R¹ and R³, R² and R⁴, R⁴ and R⁵, or R³ and R⁵, taken together, form a 5- or 6-member heterocycloalkyl group or an heteroaromatic ring or a 5- or 6-member cycloalkyl group, optionally substituted by one or more alkyl groups and/or halogens, while the remaining R¹, R², R³, R⁴, and R⁵, each independently, represent H; an alkyl group, optionally substituted by 1 to 3 fluorine atoms; a cycloalkyl group, optionally substituted by one or more alkyl groups and/or halogens; a heterocyclyl group, preferably a 4- to 6-member heterocycloalkyl group, optionally substituted by one or more alkyl groups, oxo groups and/or halogens; a halogen; an alkoxy group such as a C₁-C₄ alkoxy group; a cycloakyloxy group, optionally substituted by one or more alkyl groups and/or halogens; a phenyl group optionally substituted by one or more alkyl groups and/or halogens; or a substituted or unsubstituted amino group, such as dialkylamino groups, such as a dimethylamino group (-N(CH₃)₂);
R⁶, R⁷, R⁸, and R⁹ each independently represent H; or an alkyl group;
n and m each independently represent 0 or 1;
R¹⁰ and R¹³ each independently represent H; a halogen; an alkoxy group; a 4- to 6-member heterocycloalkyl group or a 4- to 6-member heteroaryl group, optionally substituted by one or more alkyl groups and/or halogens; a cycloalkyl group, optionally substituted by one or more alkyl groups and/or halogens; or an alkyl group, optionally substituted by 1 to 3 fluorine atoms; and
R¹¹ and R¹² each independently represent H; a halogen; a dialkylamino group, such as a dimethylamino group (-N(CH₃)₂); a cycloalkyl group, optionally substituted by one or more alkyl groups and/or halogens; a 4- to 6-member heterocycloalkyl group, optionally substituted by one or more alkyl groups and/or halogens, such as azetidine, optionally substituted by one or more alkyl groups and/or halogens; or a 4- to 6-member heteroaryl group, optionally substituted by one or more alkyl groups and/or halogens, which is directly bonded to the aryl via a covalent bond or linked through a spacer such as an alkyl chain; such as pyrazole or oxazole, optionally substituted by one or more alkyl groups and/or halogens.

In some embodiments, the present invention provides compounds of formula (II) or (IV), supra, or a pharmaceutically acceptable salt thereof, in which R¹ is selected from a hydrogen atom; an alkyl group, such as a C₁-C₄ alkyl group, optionally substituted by 1 to 3 fluorine atoms; a cycloalkyl group, optionally substituted by one or more alkyl groups and/or halogens; a heterocyclyl group, preferably a 4- to 6-member heterocyclyl group, optionally substituted by one or more alkyl groups, oxo groups and/or halogens; a halogen; an alkoxy group, such as a C₁-C₄ alkoxy group; a cycloakyloxy group, optionally substituted by one or more alkyl groups and/or halogens; a phenyl group, optionally substituted by one or more alkyl groups and/or halogens; or a substituted or unsubstituted amino group, such as dialkylamino groups, such as a dimethylamino group (-N(CH₃)₂).

In some embodiments, the present invention provides compounds of formula (II) or (IV), supra, or a pharmaceutically acceptable salt thereof, in which R¹ is selected from a hydrogen atom; a halogen; an alkoxy group; a cycloakyloxy group, optionally substituted by one or more alkyl groups and/or halogens. Preferably, R¹ is selected from a hydrogen atom; a halogen; or an alkoxy group. More preferably, R¹ is selected from a hydrogen atom; or an alkoxy group. Even more preferably, R¹ is selected from an alkoxy group, such as a methoxy group or ethoxy group.

In some embodiments, the present invention provides compounds of formula (II) or (IV), supra, or a pharmaceutically acceptable salt thereof, in which R² is selected from a hydrogen atom; an alkyl group, such as a C₁-C₄ alkyl group, optionally substituted by 1 to 3 fluorine atoms; a cycloalkyl group, optionally substituted by one or more alkyl groups and/or halogens; a heterocyclyl group, preferably a 4- to 6-member heterocyclyl group, optionally substituted by one or more alkyl groups, oxo groups and/or halogens; a halogen; an alkoxy group, such as a C₁-C₄ alkoxy group; a cycloakyloxy group, optionally substituted by one or more alkyl groups and/or halogens; a phenyl group, optionally substituted by one or more alkyl groups and/or halogens; or a substituted or unsubstituted amino group, such as dialkylamino groups, such as a dimethylamino group (-N(CH₃)₂).

In some embodiments, the present invention provides compounds of formula (II) or (IV), supra, or a pharmaceutically acceptable salt thereof, in which R² is selected from a hydrogen atom; a halogen; an alkoxy group; or a cycloakyloxy group, optionally substituted by one or more alkyl groups and/or halogens. Preferably, R² is selected from a hydrogen atom; a halogen; or an alkoxy group. More preferably, R² is selected from a hydrogen atom; or an alkoxy group. Even more preferably, R² is selected from an alkoxy group, such as a methoxy group or ethoxy group.

In some embodiments, the present invention provides compounds of formula (II) or (IV), supra, or a pharmaceutically acceptable salt thereof, in which R¹ and R ² each independently represent an alkoxy group, such as a methoxy group or ethoxy group.

In some embodiments, the present invention provides compounds of formula (II) or (IV), supra, or a pharmaceutically acceptable salt thereof, in which R³ is selected from a hydrogen atom; an alkyl group, such as a C₁-C₄ alkyl group, optionally substituted by 1 to 3 fluorine atoms; a cycloalkyl group, optionally substituted by one or more alkyl groups and/or halogens; a heterocyclyl group, preferably a 4- to 6-member heterocyclyl group, optionally substituted by one or more alkyl groups, oxo groups and/or halogens; a halogen; an alkoxy group, such as a C₁-C₄ alkoxy group; a cycloakyloxy group, optionally substituted by one or more alkyl groups and/or halogens; a phenyl group, optionally substituted by one or more alkyl groups and/or halogens; or a substituted or unsubstituted amino group, such as dialkylamino groups, such as a dimethylamino group (-N(CH₃)₂).

In some embodiments, the present invention provides compounds of formula (II) or (IV), supra, or a pharmaceutically acceptable salt thereof, in which R³ is selected from a hydrogen atom; an alkyl group, such as a C₁-C₄ alkyl group, optionally substituted by 1 to 3 fluorine atoms; a cycloalkyl group, optionally substituted by one or more alkyl groups and/or halogens; a heterocyclyl group, preferably a 4- to 6-member heterocyclyl group, optionally substituted by one or more alkyl groups, oxo groups and/or halogens; a cycloakyloxy group, optionally substituted by one or more alkyl groups and/or halogens; a phenyl group, optionally substituted by one or more alkyl groups and/or halogens; or a substituted or unsubstituted amino group, such as dialkylamino group, such as a dimethylamino group (-N(CH₃)₂). Preferably, R³ is selected from a hydrogen atom and a C₁-C₄ alkyl group substituted by 1 to 3 fluorine atoms; a heterocyclyl group substituted by one or more alkyl groups and/or halogens. More preferably, R³ is selected from a hydrogen atom and a C₁-C₄ alkyl group substituted by 1 to 3 fluorine atoms. Even more preferably, R³ represents a hydrogen atom.

In some embodiments, the present invention provides compounds of formula (II) or (IV), supra, or a pharmaceutically acceptable salt thereof, in which R⁴ is selected from a hydrogen atom; an alkyl group, such as a C₁-C₄ alkyl group, optionally substituted by 1 to 3 fluorine atoms; a cycloalkyl group, optionally substituted by one or more alkyl groups and/or halogens; a heterocyclyl group, preferably a 4- to 6-member heterocyclyl group, optionally substituted by one or more alkyl groups, oxo groups and/or halogens; a halogen; an alkoxy group, such as a C₁-C₄ alkoxy group; a cycloakyloxy group, optionally substituted by one or more alkyl groups and/or halogens; a phenyl group, optionally substituted by one or more alkyl groups and/or halogens; or a substituted or unsubstituted amino group, such as dialkylamino groups, such as a dimethylamino group (-N(CH₃)₂).

In some embodiments, the present invention provides compounds of formula (II) or (IV), supra, or a pharmaceutically acceptable salt thereof, in which R⁴ is selected from a hydrogen atom; an alkyl group, such as a C₁-C₄ alkyl group, optionally substituted by 1 to 3 fluorine atoms; a cycloalkyl group, optionally substituted by one or more alkyl groups and/or halogens; a heterocyclyl group, preferably a 4- to 6-member heterocyclyl group, optionally substituted by one or more alkyl groups, oxo groups and/or halogens; a cycloakyloxy group, optionally substituted by one or more alkyl groups and/or halogens; a phenyl group, optionally substituted by one or more alkyl groups and/or halogens; or a substituted or unsubstituted amino group, such as dialkylamino groups, such as a dimethylamino group (-N(CH₃)₂). Preferably, R⁴ is selected from a hydrogen atom and a C₁-C₄ alkyl group substituted by 1 to 3 fluorine atoms; a heterocyclyl group substituted by one or more alkyl groups and/or halogens. More preferably, R⁴ is selected from a hydrogen atom and a C₁-C₄ alkyl group substituted by 1 to 3 fluorine atoms. Even more preferably, R⁴ represents a hydrogen atom.

In some embodiments, the present invention provides compounds of formula (II) or (IV), supra, or a pharmaceutically acceptable salt thereof, in which R⁵ is selected from a hydrogen atom; an alkyl group, such as a C₁-C₄ alkyl group, optionally substituted by 1 to 3 fluorine atoms; a cycloalkyl group, optionally substituted by one or more alkyl groups and/or halogens; a heterocyclyl group, preferably a 4- to 6-member heterocyclyl group, optionally substituted by one or more alkyl groups, oxo groups and/or halogens; a halogen; an alkoxy group, such as a C₁-C₄ alkoxy group; a cycloakyloxy group, optionally substituted by one or more alkyl groups and/or halogens; a phenyl group, optionally substituted by one or more alkyl groups and/or halogens; or a substituted or unsubstituted amino group, such as dialkylamino groups, such as a dimethylamino group (-N(CH₃)₂).

In some embodiments, the present invention provides compounds of formula (II) or (IV), supra, or a pharmaceutically acceptable salt thereof, in which R⁵ is selected from a hydrogen atom and a halogen atom. Preferably, R⁵ represents a hydrogen atom.

In some embodiments, the present invention provides compounds of formula (II) or (IV), supra, or a pharmaceutically acceptable salt thereof, in which R¹ and R³ taken together, form a 5- or 6-member heterocycloalkyl group or an heteroaromatic ring or a 5- or 6-member cycloalkyl group, optionally substituted by one or more alkyl groups and/or halogens while R², R⁴, and R⁵ each independently represent H; an alkyl group, optionally substituted by 1 to 3 fluorine atoms; a cycloalkyl group, optionally substituted by one or more alkyl groups and/or halogens; a heterocyclyl group, optionally substituted by one or more alkyl groups and/or halogens; a halogen; an alkoxy group; or a cycloakyloxy group, optionally substituted by one or more alkyl groups and/or halogens.

In some embodiments, the present invention provides compounds of formula (II) or (IV), supra, or a pharmaceutically acceptable salt thereof, in which R² and R⁴ taken together, form a 5- or 6-member heterocycloalkyl group or an heteroaromatic ring or a 5- or 6-member cycloalkyl group, optionally substituted by one or more alkyl groups and/or halogens while R¹, R³, and R⁵ each independently represent H; an alkyl group, optionally substituted by 1 to 3 fluorine atoms; a cycloalkyl group, optionally substituted by one or more alkyl groups and/or halogens; a heterocyclyl group, optionally substituted by one or more alkyl groups and/or halogens; a halogen; an alkoxy group; or a cycloakyloxy group, optionally substituted by one or more alkyl groups and/or halogens.

In some embodiments, the present invention provides compounds of formula (II) or (IV), supra, or a pharmaceutically acceptable salt thereof, in which R⁴ and R⁵ taken together, form a 5- or 6-member heterocycloalkyl group or an heteroaromatic ring or a 5- or 6-member cycloalkyl group, optionally substituted by one or more alkyl groups and/or halogens while R¹, R², and R³ each independently represent H; an alkyl group, optionally substituted by 1 to 3 fluorine atoms; a cycloalkyl group, optionally substituted by one or more alkyl groups and/or halogens; a heterocyclyl group, optionally substituted by one or more alkyl groups and/or halogens; a halogen; an alkoxy group; or a cycloakyloxy group, optionally substituted by one or more alkyl groups and/or halogens.

In some embodiments, the present invention provides compounds of formula (II) or (IV), supra, or a pharmaceutically acceptable salt thereof, in which R³ and R⁵ taken together, form a 5- or 6-member heterocycloalkyl group or an heteroaromatic ring or a 5- or 6-member cycloalkyl group, optionally substituted by one or more alkyl groups and/or halogens while R¹, R², and R⁴ each independently represent H; an alkyl group, optionally substituted by 1 to 3 fluorine atoms; a cycloalkyl group optionally substituted by one or more alkyl groups and/or halogens; a heterocyclyl group, optionally substituted by one or more alkyl groups and/or halogens; a halogen; an alkoxy group; or a cycloakyloxy group, optionally substituted by one or more alkyl groups and/or halogens.

In some embodiments, the present invention provides compounds of formula (III) or (IV), supra, or a pharmaceutically acceptable salt thereof, in which R⁶, R⁷, R⁸, and R⁹ each independently represent H; or an alkyl group; n represents 1 and m represents 0.

Preferably, R⁶ and R⁸ represent H and R⁷ and R⁹ each independently represent H; or an alkyl group; n represents 1 and m represents 0. More preferably, R⁶, R⁷, R⁸, and R⁹ represent H; n represents 1 and m represents 0.

In some embodiments, the present invention provides compounds of formula (III) or (IV), supra, or a pharmaceutically acceptable salt thereof, in which R¹⁰ represents H; a halogen; an alkoxy group; a 4- to 6-member heterocycloalkyl group or a 5- or 6-member heteroaryl group, optionally substituted by one or more alkyl groups and/or halogens; a cycloalkyl group, optionally substituted by one or more alkyl groups and/or halogens; or an alkyl group, optionally substituted by 1 to 3 fluorine atoms.

In some embodiments, the present invention provides compounds of formula (III) or (IV), supra, or a pharmaceutically acceptable salt thereof, in which R¹⁰ represents H; a halogen; an alkoxy group; a 4- to 6-member heterocycloalkyl group; a cycloalkyl group, optionally substituted by one or more alkyl groups and/or halogens; an alkyl group, optionally substituted by 1 to 3 fluorine atoms. Preferably, R¹⁰ represents H; a halogen; an alkoxy group; a 4- to 6-member heterocycloalkyl group; a cycloalkyl group, optionally substituted by one or more alkyl groups and/or halogens; or an alkyl group, substituted by 1 to 3 fluorine atoms. More preferably, R¹⁰ represents a halogen; an alkoxy group; a 4- to 6-member heterocycloalkyl group; a cycloalkyl group, optionally substituted by one or more alkyl groups and/or halogens. Even more preferably, R¹⁰ represents a halogen; an alkoxy group; or a cycloalkyl group.

In some embodiments, the present invention provides compounds of formula (III) or (IV), supra, or a pharmaceutically acceptable salt thereof, in which R¹¹ represent H; a halogen; a dialkylamino group, such as a dimethylamino group (-N(CH₃)₂); a cycloalkyl group, optionally substituted by one or more alkyl groups and/or halogens; a 4- to 6-member heterocycloalkyl group, optionally substituted by an alkyl group or a halogen, such as azetidine, optionally substituted by one or more alkyl groups and/or halogens; or a 4- to 6-member heteroaryl group, optionally substituted by one or more alkyl groups and/or halogens, which is directly bonded to the aryl via a covalent bond or linked through a spacer such as an alkyl chain; such as pyrazole or oxazole, optionally substituted by one or more alkyl groups and/or halogens. Preferably, R¹¹ represents H; a halogen; a dialkylamino group, such as a dimethylamino group (-N(CH₃)₂); a cycloalkyl group, optionally substituted by one or more alkyl groups and/or halogens. More preferably, R¹¹ represents H; a halogen; a dialkylamino group, such as a dimethylamino group (-N(CH₃)₂). Even more preferably, R¹¹ represents a hydrogen atom.

In some embodiments, the present invention provides compounds of formula (III) or (IV), supra, or a pharmaceutically acceptable salt thereof, in which R¹² represents H; a halogen; a dialkylamino group, such as a dimethylamino group (-N(CH₃)₂); a cycloalkyl group, optionally substituted by one or more alkyl groups and/or halogens; a 4- to 6-member heterocycloalkyl group, optionally substituted by one or more alkyl groups and/or halogens, such as azetidine, optionally substituted by one or more alkyl groups and/or halogens; or a 4- to 6-member heteroaryl group, optionally substituted by one or more alkyl groups and/or halogens, which is directly bonded to the aryl via a covalent bond or linked through a spacer such as an alkyl chain; such as pyrazole or oxazole, optionally substituted by one or more alkyl groups and/or halogens. Preferably, R¹² represent H; a halogen; a dialkylamino group, such as a dimethylamino group (-N(CH₃)₂); a cycloalkyl group optionally substituted by one or more alkyl groups and/or halogens. More preferably, R¹² represents H; a halogen; a dialkylamino group, such as a dimethylamino group (-N(CH₃)₂). Even more preferably, R¹² represents a hydrogen atom.

In some embodiments, the present invention provides compounds of formula (III) or (IV), supra, or a pharmaceutically acceptable salt thereof, in which R¹³ represents H; a halogen; an alkoxy group; a 4- to 6-member heterocycloalkyl group or a 5- or 6-member heteroaryl group, optionally substituted by one or more alkyl groups and/or halogens; a cycloalkyl group, optionally substituted by one or more alkyl groups and/or halogens; or an alkyl group, optionally substituted by 1 to 3 fluorine atoms.

In some embodiments, the present invention provides compounds of formula (III), or (IV), in which R¹³ represents H; a halogen; a 4- to 6-member heterocycloalkyl group or a 4- to 6-member heteroaryl group, optionally substituted by one or more alkyl groups and/or halogens; or a cycloalkyl group, optionally substituted by one or more alkyl groups and/or halogens. Preferably, R¹³ represents H; a halogen; a 4- to 6-member heteroaryl group, optionally substituted by one or more alkyl groups and/or halogens. More preferably, R¹³ a halogen or a 4- to 6-member heteroaryl group, optionally substituted by one or more alkyl groups and/or halogens. Even more preferably, R¹³ represent a 4- to 6-member heteroaryl group, such as a pyrazol.

In some embodiments, the present invention provides compounds of formula (III) or (IV), supra, or a pharmaceutically acceptable salt thereof, in which n and m each independently represent 0 or 1. For example, n represents 1 and m represents 0. Preferably, n represents 1 and m represents 1. More preferably, n represents 0 and m represents 0. Even more preferably, n represents 0 and m represents 1.

In some embodiments, a compound of Formula (I) or Formula (II), or pharmaceutically acceptable salt thereof, is a compound of Formula (IIa): 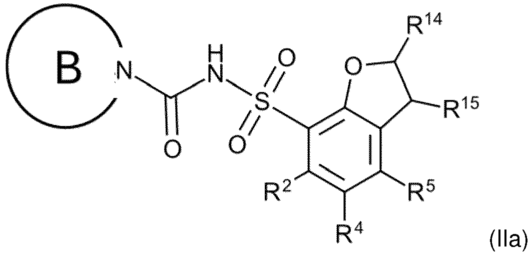 or a pharmaceutically acceptable salt thereof, wherein B, R², R⁴, and R⁵, are selected as previously described; and R¹⁴ and R¹⁵, each independently, represent H or an alkyl group, preferably R¹⁴ and R¹⁵ represent H.

In some embodiments, a compound of Formula (I) or Formula (II), or pharmaceutically acceptable salt thereof, is a compound of Formula (IIb): 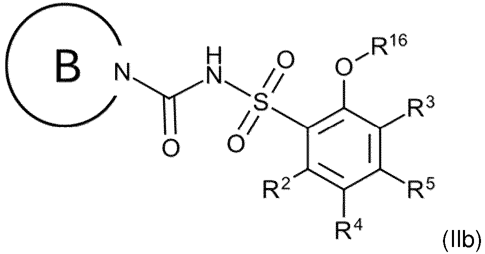 or a pharmaceutically acceptable salt thereof, wherein B, R², R³, R⁴, and R⁵, are selected as previously described; and wherein R¹⁶ represents an alkyl group or cycloalkyl group.

In some embodiments, a compound of Formula (I) or Formula (II), or pharmaceutically acceptable salt thereof, is a compound of Formula (llb-1): 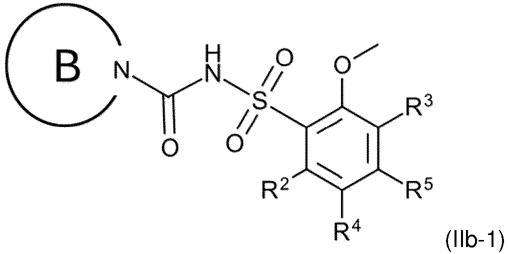 or a pharmaceutically acceptable salt thereof, wherein B, R², R³, R⁴, and R⁵, are selected as previously described.

In some embodiments, a compound of Formula (I) or Formula (II), or pharmaceutically acceptable salt thereof, is a compound of Formula (IIc): 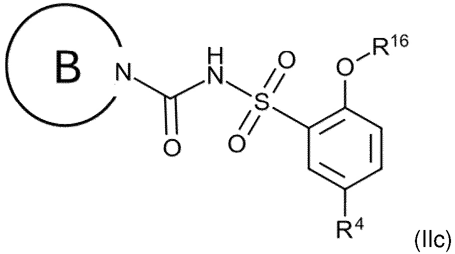 or a pharmaceutically acceptable salt thereof, wherein B and R⁴ are selected as previously described; and wherein R¹⁶ represents an alkyl group or a cycloalkyl group.

In some embodiments, a compound of Formula (I) or Formula (II), or pharmaceutically acceptable salt thereof, is a compound of Formula (llc-1): 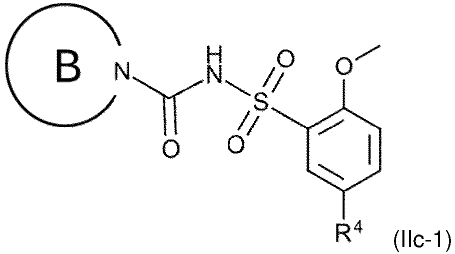 or a pharmaceutically acceptable salt thereof, wherein B and R⁴ are selected as previously described.

In some embodiments, a compound of Formula (I) or Formula (II), or pharmaceutically acceptable salt thereof, is a compound of Formula (Ild): 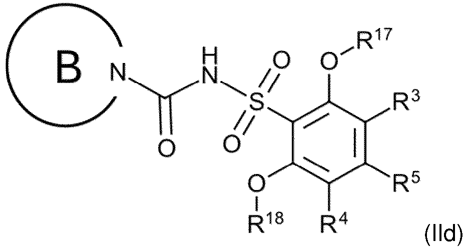 or a pharmaceutically acceptable salt thereof, wherein B, R³, R⁴, and R⁵, are selected as previously described; and wherein R¹⁷ and R¹⁸ represent, each independently, an alkyl group or a cycloalkyl group.

In some embodiments, a compound of Formula (I) or Formula (II), or pharmaceutically acceptable salt thereof, is a compound of Formula (IIe): 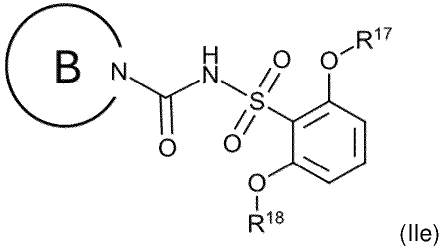 or a pharmaceutically acceptable salt thereof, wherein B is selected as previously described; and wherein R¹⁷ and R¹⁸ represent, each independently, an alkyl group or a cycloalkyl group.

In some embodiments, a compound of Formula (I) or Formula (II), or pharmaceutically acceptable salt thereof, is a compound of Formula (Ilf): 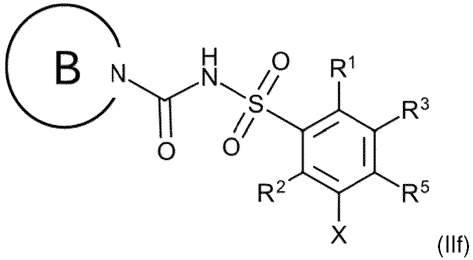 or a pharmaceutically acceptable salt thereof, wherein B, R¹, R², R³, and R⁵, are selected as previously described; and wherein X represents an amino group, such as substituted amino group, such as dialkylamino groups, such as a dimethylamino group (-N(CH₃)₂); or a heterocyclyl group, preferably a 4- to 6-member heterocycloalkyl group, such as azetidine or pyrrolidine, optionally substituted by one or more alkyl groups, oxo groups and/or halogens.

In some embodiments, a compound of Formula (I) or Formula (II), or pharmaceutically acceptable salt thereof, is a compound of Formula (llf-1): 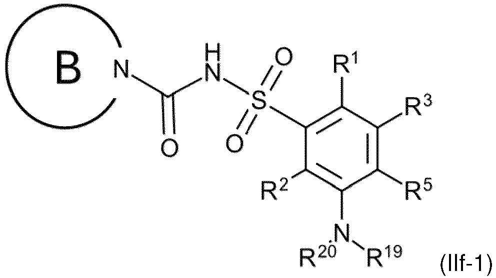 or a pharmaceutically acceptable salt thereof, wherein B, R¹, R², R³, and R⁵, are selected as previously described; and wherein R¹⁹ and R²⁰ represent, each independently, an alkyl group or taken together can form an azetidine or a pyrrolidine, substituted or not.

In some embodiments, a compound of Formula (I) or Formula (II), or pharmaceutically acceptable salt thereof, is a compound of Formula (IIf-2): 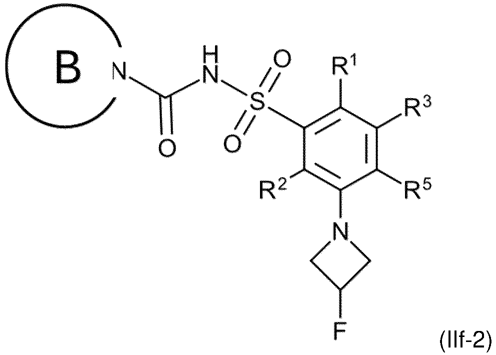 or a pharmaceutically acceptable salt thereof, wherein B, R¹, R², R³, and R⁵, are selected as previously described.

In some embodiments, a compound of Formula (I) or Formula (II), or pharmaceutically acceptable salt thereof, is a compound of Formula (IIf-3): 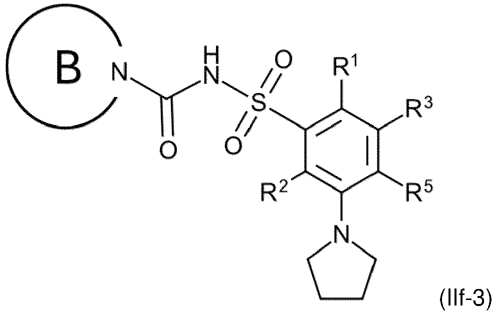 or a pharmaceutically acceptable salt thereof, wherein B, R¹, R², R³, and R⁵, are selected as previously described.

In some embodiments, a compound of Formula (I) or Formula (II), or pharmaceutically acceptable salt thereof, is a compound of Formula (IIf-4): 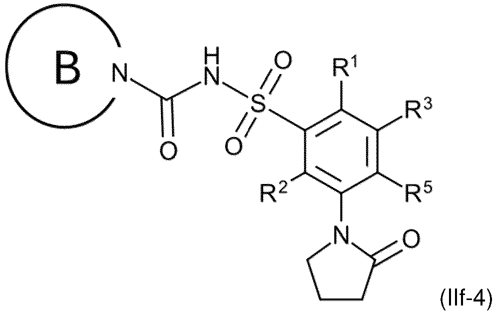 or a pharmaceutically acceptable salt thereof, wherein B, R¹, R², R³, and R⁵, are selected as previously described.

In some embodiments, a compound of Formula (I) or Formula (II), or pharmaceutically acceptable salt thereof, is a compound of Formula (IIf-5): 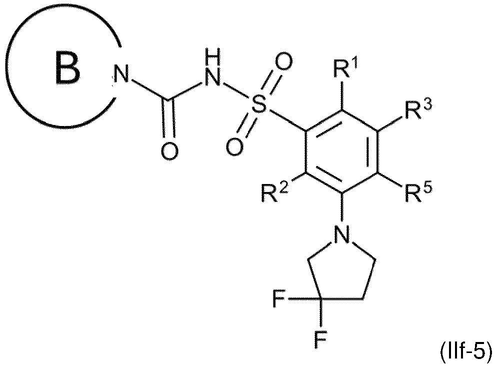 or a pharmaceutically acceptable salt thereof, wherein B, R¹, R², R³, and R⁵, are selected as previously described.

In some embodiments, a compound of Formula (I) or Formula (III), or pharmaceutically acceptable salt thereof, is a compound of Formula (Illa): 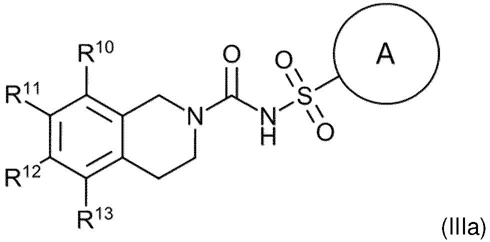 or a pharmaceutically acceptable salt thereof, wherein A, R¹⁰, R¹¹, R¹², and R¹³, are selected as previously described.

In some embodiments, a compound of Formula (I) or Formula (III), or pharmaceutically acceptable salt thereof, is a compound of Formula (Illb): 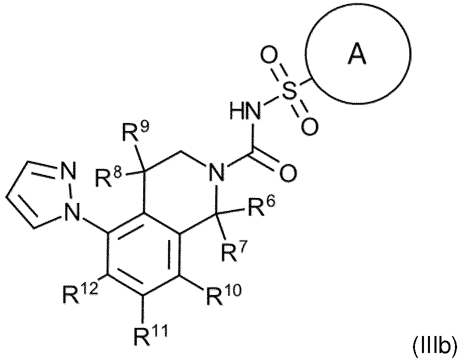 or a pharmaceutically acceptable salt thereof, wherein A, R⁶, R⁷, R⁸, R⁹, R¹⁰, R¹¹, and R¹², are selected as previously described.

In some embodiments, a compound of Formula (I) or Formula (III), or pharmaceutically acceptable salt thereof, is a compound of Formula (IIIc): 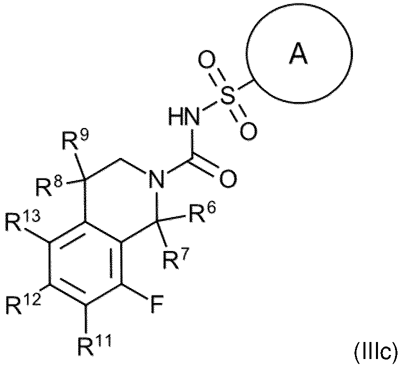 or a pharmaceutically acceptable salt thereof, wherein A, R⁶, R⁷, R⁸, R⁹, R¹¹, R¹², and R¹³, are selected as previously described.

In some embodiments, a compound of Formula (I) or Formula (III), or pharmaceutically acceptable salt thereof, is a compound of Formula (IIId): 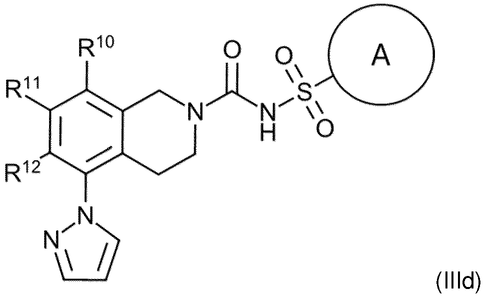 or a pharmaceutically acceptable salt thereof, wherein A, R¹⁰, R¹¹, and R¹², are selected as previously described.

In some embodiments, a compound of Formula (I) or Formula (III), or pharmaceutically acceptable salt thereof, is a compound of Formula (IIIe): 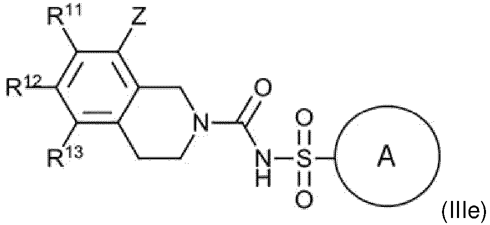 or a pharmaceutically acceptable salt thereof, wherein A, R¹¹, R¹², and R¹³, are selected as previously described; Z represents an halogen.

In some embodiments, a compound of Formula (I) or Formula (III), or pharmaceutically acceptable salt thereof, is a compound of Formula (IIIf): 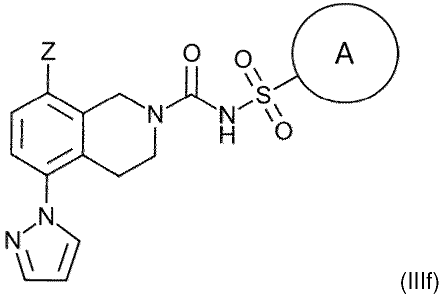 or a pharmaceutically acceptable salt thereof, wherein A, is selected as previously described; and wherein Z represents a halogen; an alkyl group optionally substituted by 1 to 3 fluorine atoms; an alkoxy group; or a cycloalkyl group optionally substituted by one or more alkyl groups and/or halogens.

In some embodiments, a compound of Formula (I) or Formula (III), or pharmaceutically acceptable salt thereof, is a compound of Formula (IIIf-1): 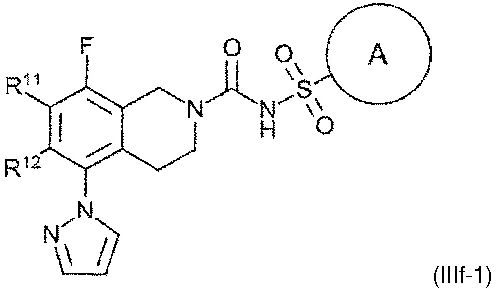 or a pharmaceutically acceptable salt thereof, wherein A, R¹¹ and R¹², are selected as previously described.

In some embodiments, a compound of Formula (I) or Formula (III), or pharmaceutically acceptable salt thereof, is a compound of Formula (IIIg): 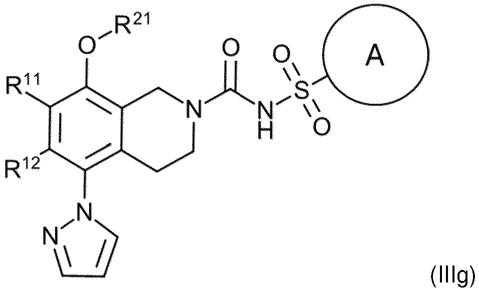 or a pharmaceutically acceptable salt thereof, wherein A, R¹¹ and R¹², are selected as previously described; and wherein R²¹ represents an alkyl group.

In some embodiments, a compound of Formula (I) or Formula (III), or pharmaceutically acceptable salt thereof, is a compound of Formula (IIIg-1): 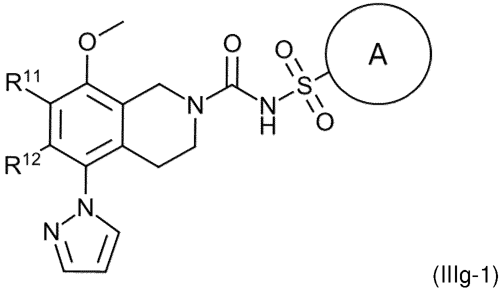 or a pharmaceutically acceptable salt thereof, wherein A, R¹¹ and R¹², are selected as previously described.

In some embodiments, a compound of Formula (I) or Formula (III), or pharmaceutically acceptable salt thereof, is a compound of Formula (IIIh): 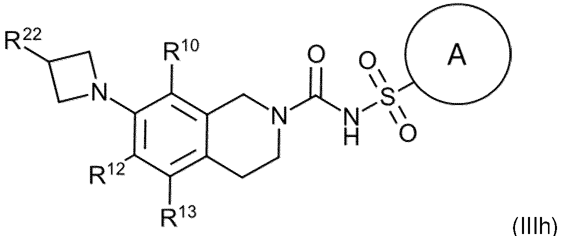 or a pharmaceutically acceptable salt thereof, wherein A, R¹⁰, R¹² and R¹³, are selected as previously described; and wherein R²² represents an alkyl group or a hydrogen atom or a halogen.

In some embodiments, a compound of Formula (I) or Formula (III), or pharmaceutically acceptable salt thereof, is a compound of Formula (IIIi): 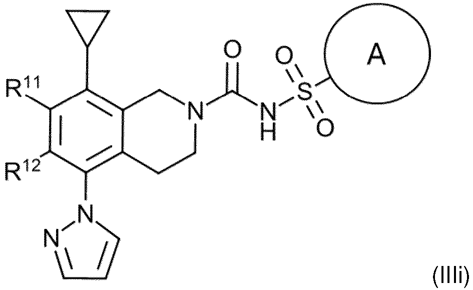 or a pharmaceutically acceptable salt thereof, wherein A, R¹¹ and R¹², are selected as previously described.

In some embodiments, a compound of Formula (I) or Formula (III), or pharmaceutically acceptable salt thereof, is a compound of Formula (IIIj): 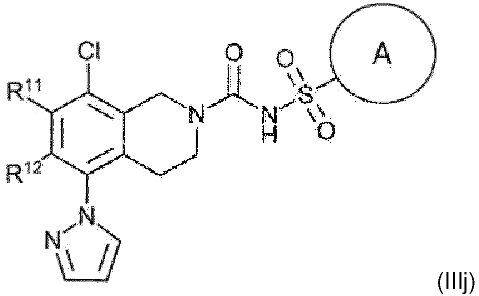 or a pharmaceutically acceptable salt thereof, wherein A, R¹¹ and R¹², are selected as previously described.

In some embodiments, a compound of Formula (I) or Formula (III), or pharmaceutically acceptable salt thereof, is a compound of Formula (IIIk): 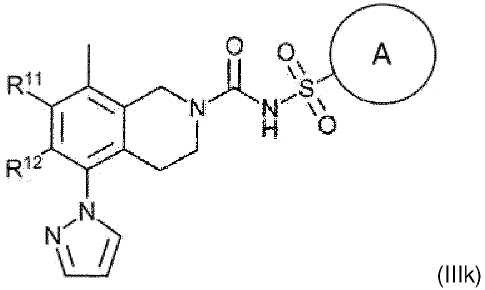 or a pharmaceutically acceptable salt thereof, wherein A, R¹¹ and R¹², are selected as previously described.

In some embodiments, a compound of Formula (I), (II), (III), and/or (IV), or a pharmaceutically acceptable salt thereof, is a compound wherein the compound has the structure of a compound shown in **Table 1**, or a pharmaceutically acceptable salt thereof.

**Table 1**

| **No.** | **Structure** |
|---|---|
| 1 | 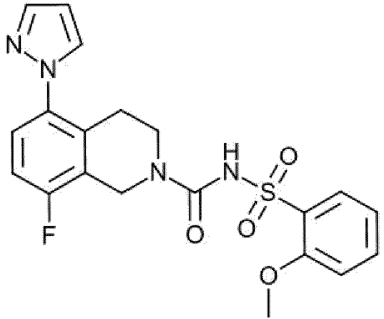 |
| 2 | 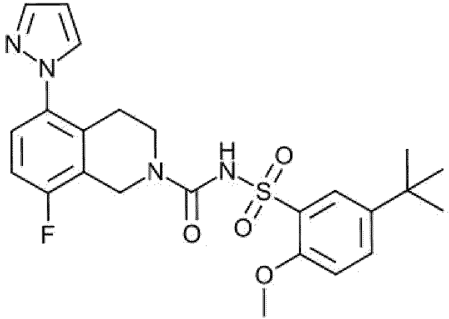 |
| 3 | 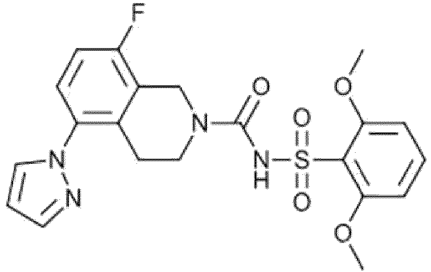 |
| 4 | 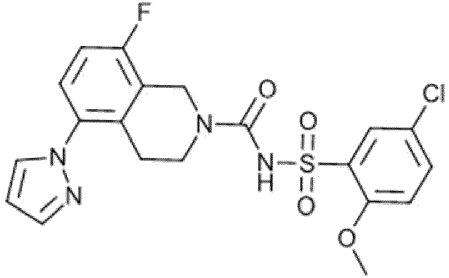 |
| 5 | 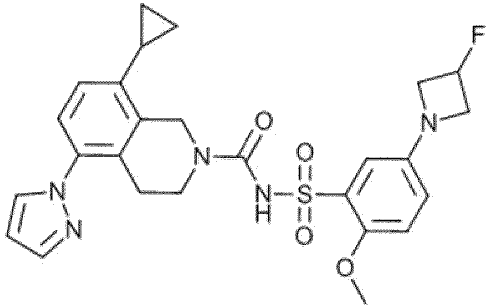 |
| 6 | 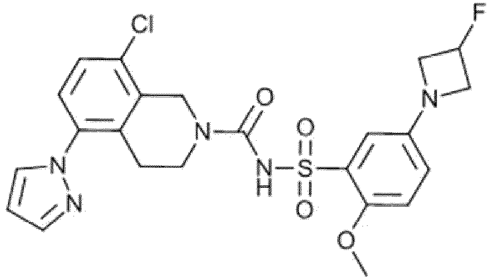 |
| 7 | 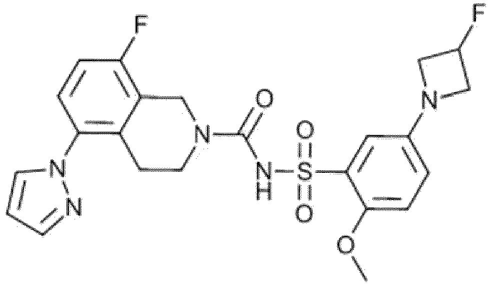 |
| 8 | 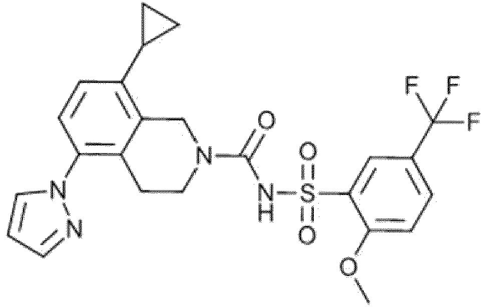 |
| 9 | 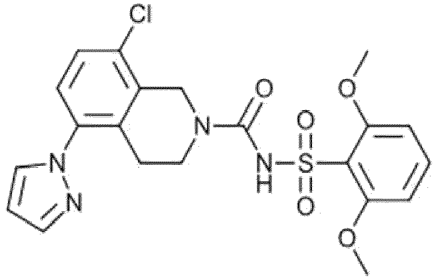 |
| 10 | 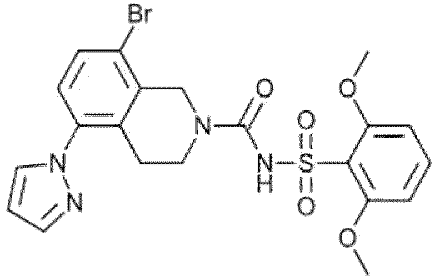 |
| 11 | 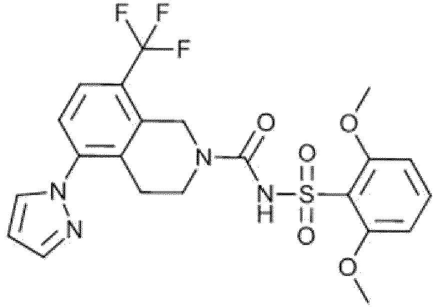 |
| 12 | 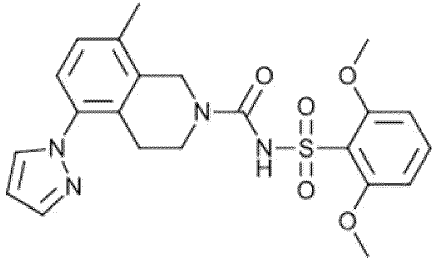 |
| 13 | 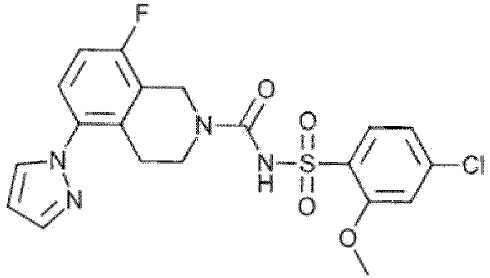 |
| 14 | 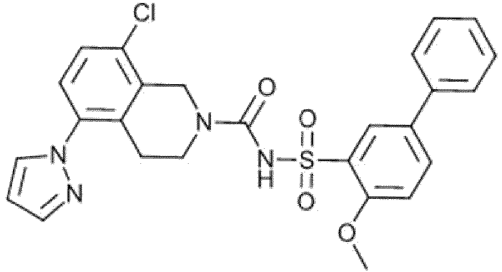 |
| 15 | 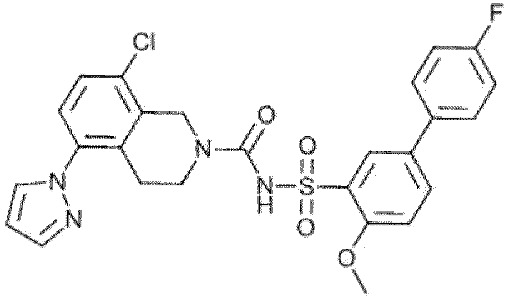 |
| 16 | 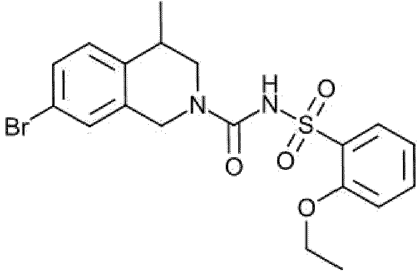 |
| 17 | 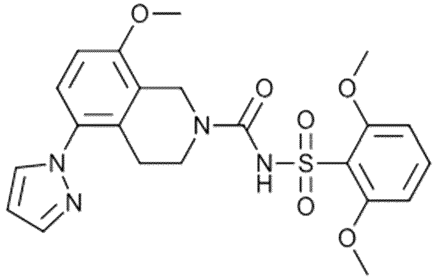 |
| 18 | 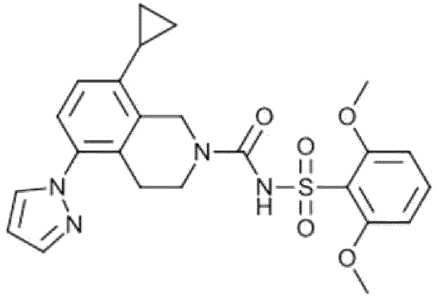 |
| 19 | 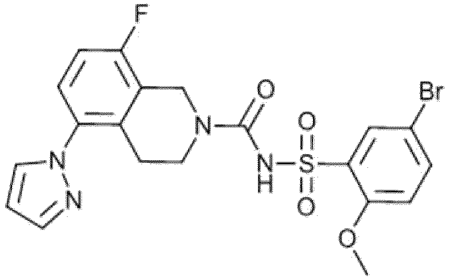 |
| 20 | 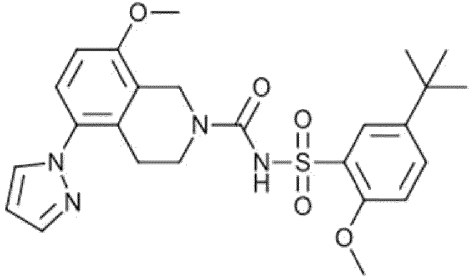 |
| 21 | 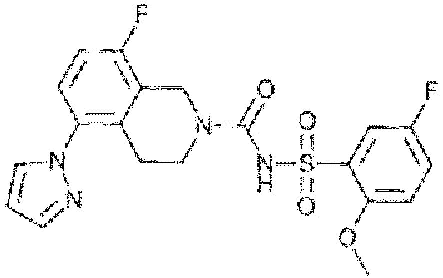 |
| 22 | 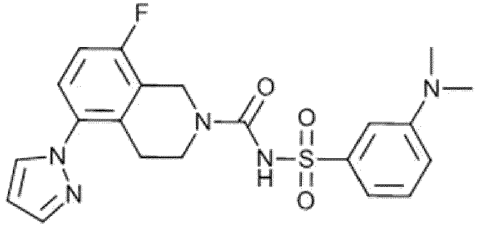 |
| 23 | 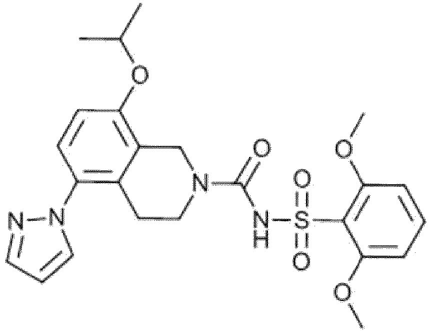 |
| 24 | 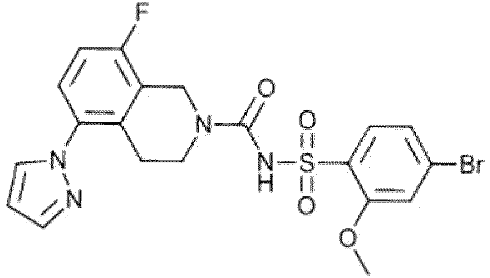 |
| 25 | 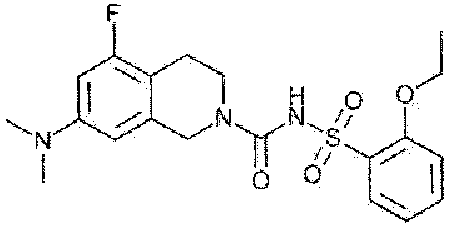 |
| 26 | 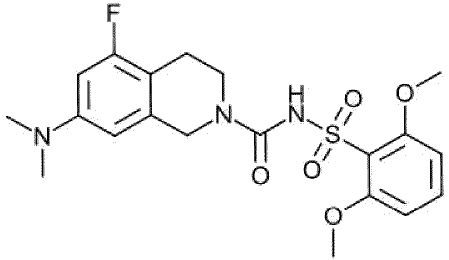 |
| 27 | 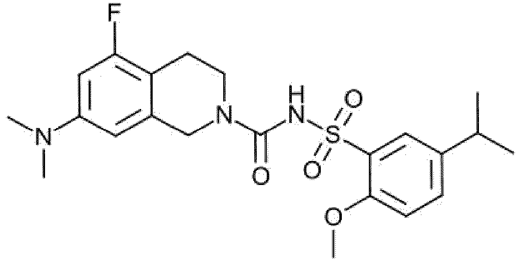 |
| 28 | 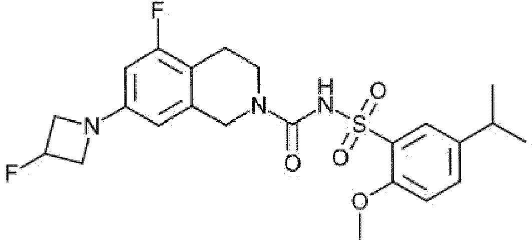 |
| 29 | 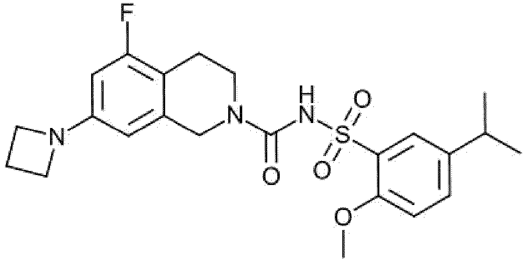 |
| 30 | 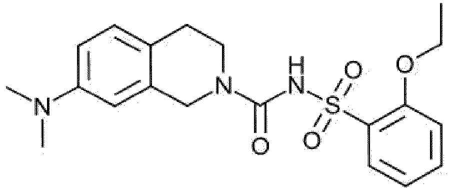 |
| 31 | 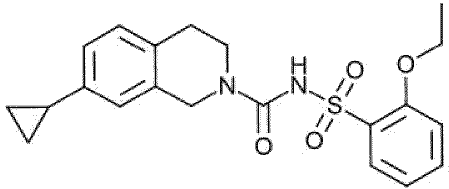 |
| 32 | 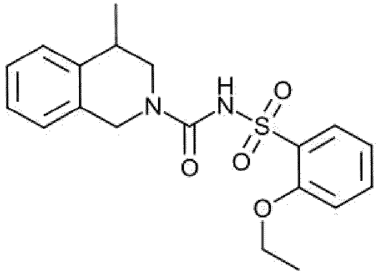 |
| 33 | 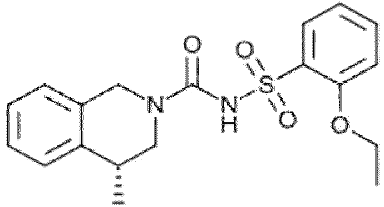 |
| 34 | 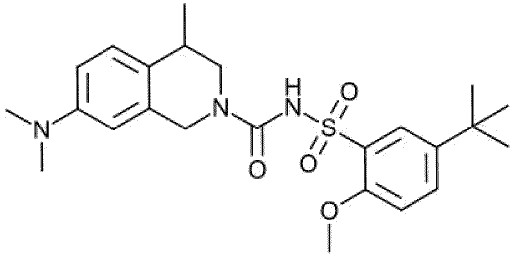 |
| 35 | 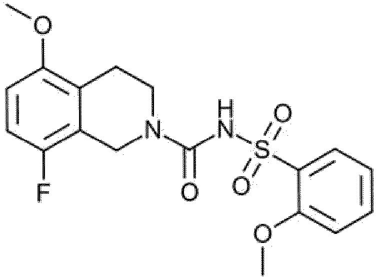 |
| 36 | 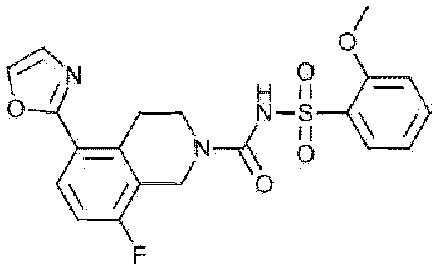 |
| 37 | 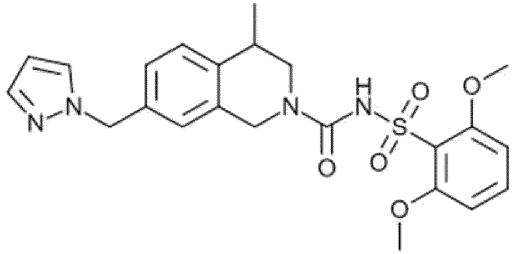 |
| 38 | 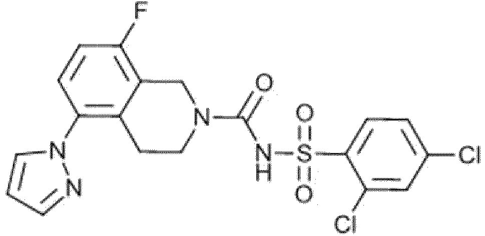 |
| 39 | 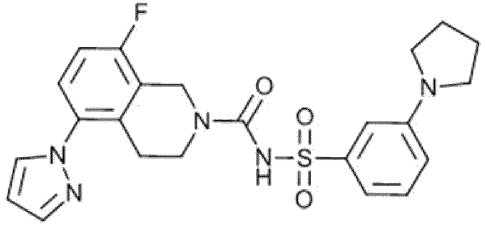 |
| 40 | 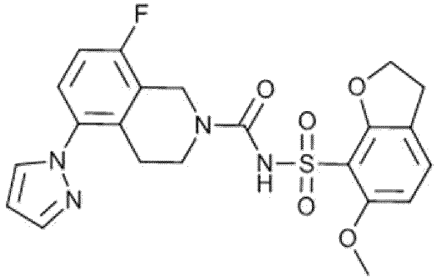 |
| 41 | 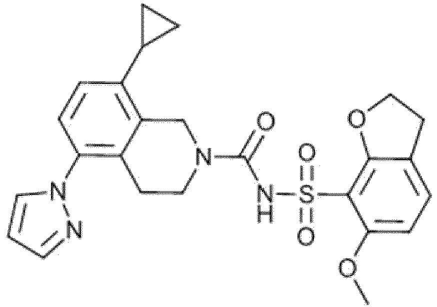 |
| 42 | 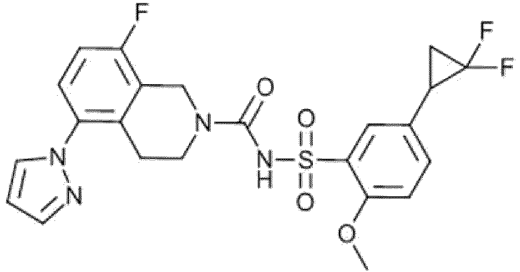 |
| 43 | 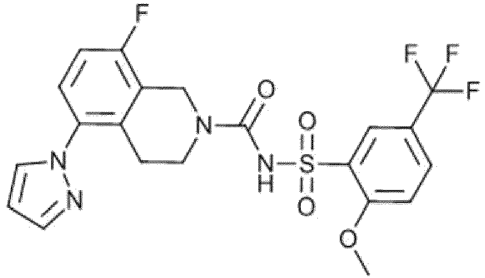 |
| 44 | 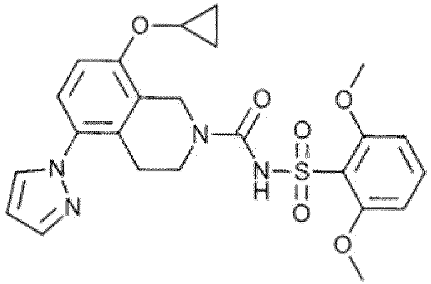 |
| 45 | 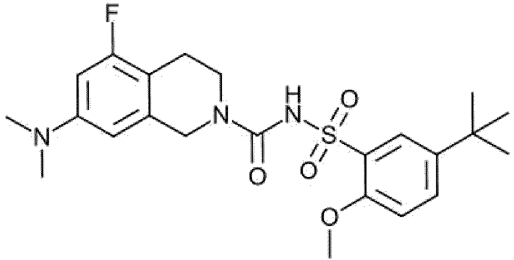 |
| 46 | 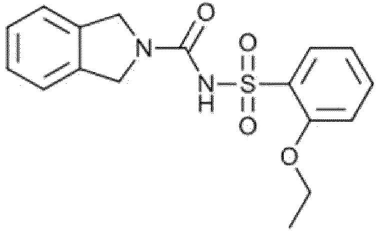 |
| 47 | 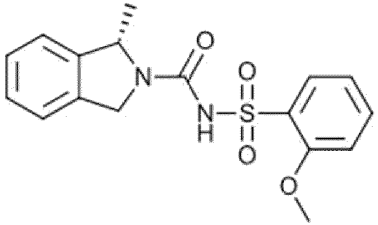 |
| 48 | 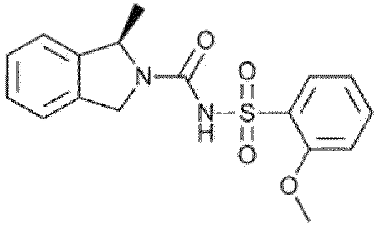 |
| 49 | 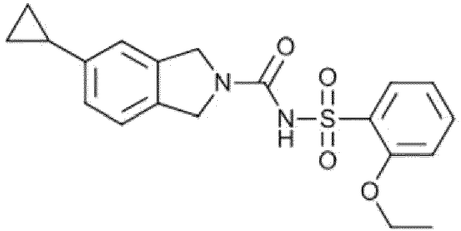 |
| 50 | 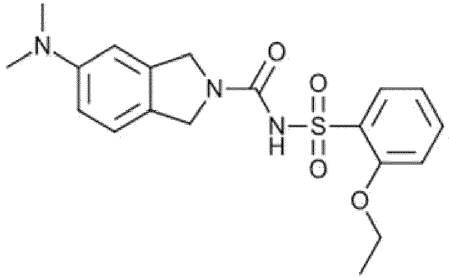 |
| 51 | 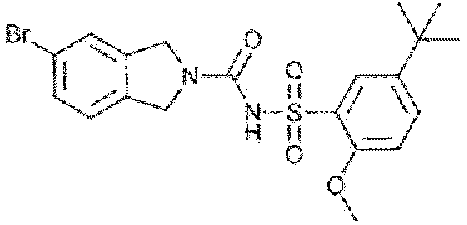 |
| 52 | 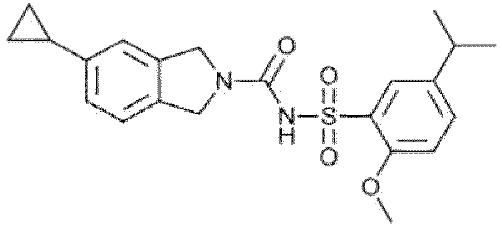 |
| 53 | 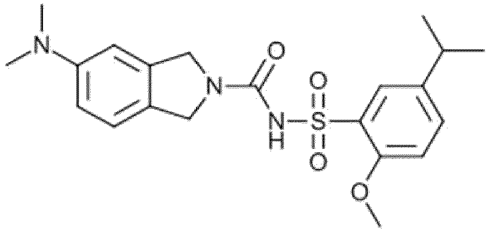 |
| 54 | 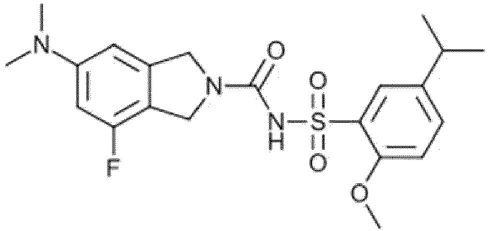 |
| 55 | 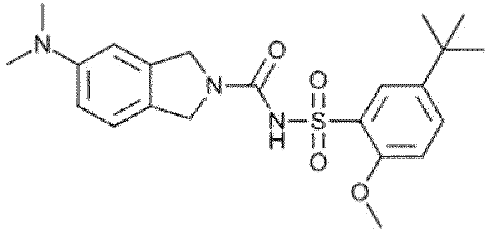 |
| 56 | 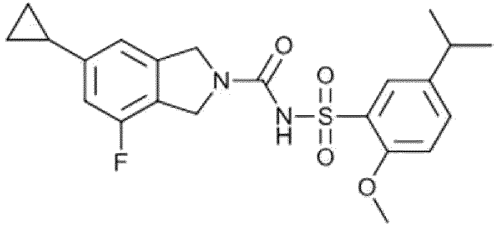 |
| 57 | 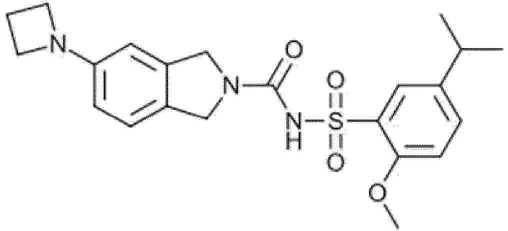 |
| 58 | 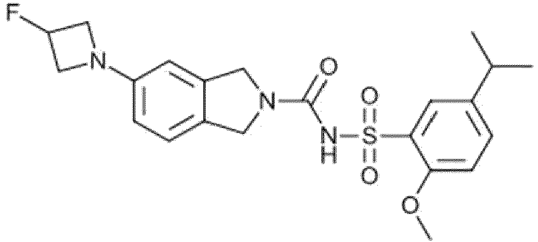 |
| 59 | 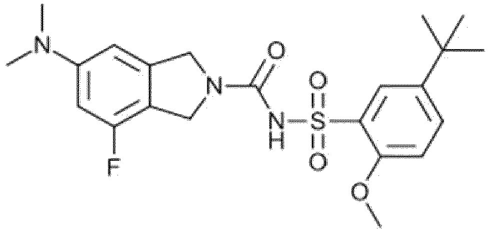 |
| 60 | 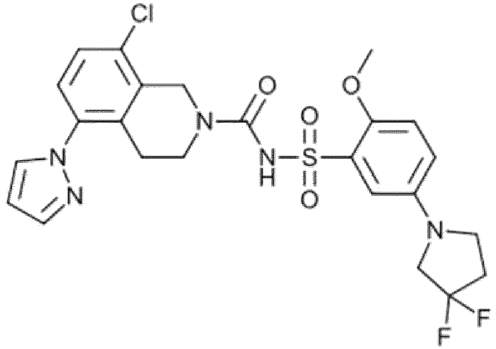 |
| 61 | 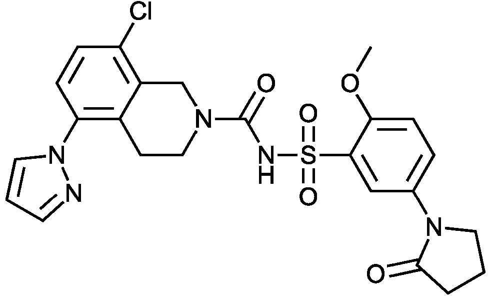 |
| 62 | 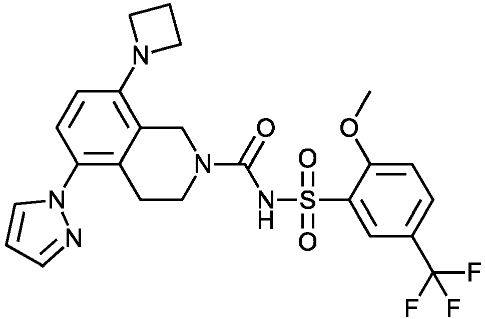 |
| 63 | 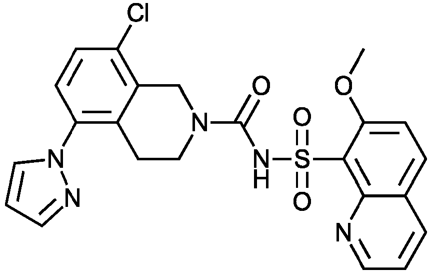 |
| 64 | 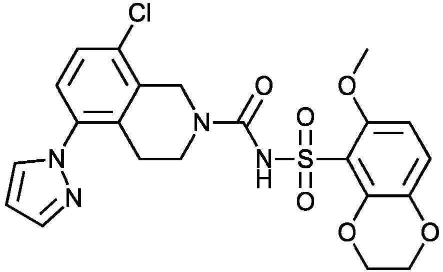 |
| 65 | 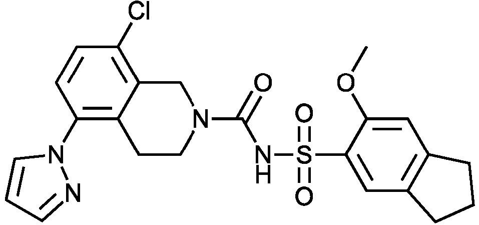 |
| 66 | 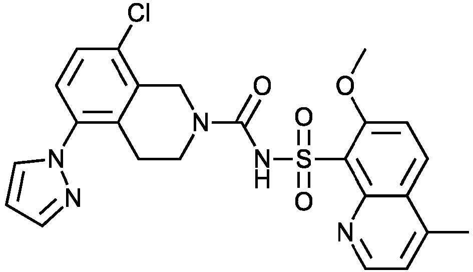 |
| 67 | 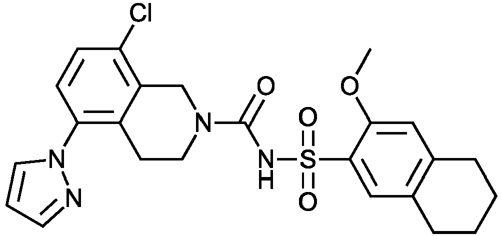 |
| 68 | 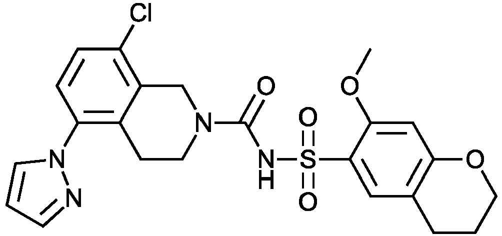 |
| 69 | 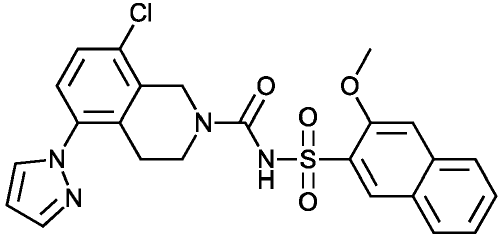 |
| 70 | 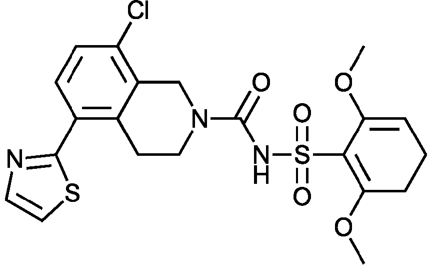 |
| 71 | 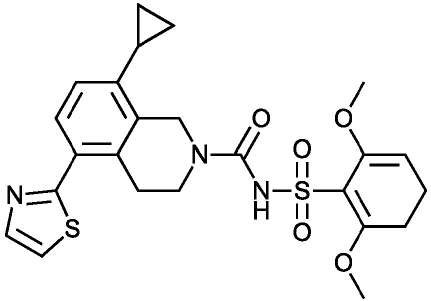 |
| 72 | 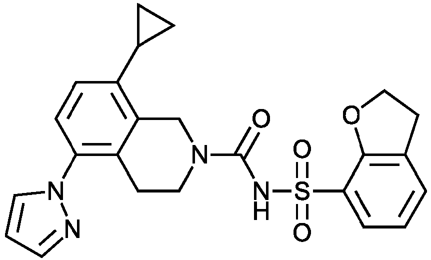 |

### Pharmaceutical Compositions

The present invention relates to pharmaceutical compositions comprising at least one active compound of Formula (I), (II), (IIa), (IIb), (IIb-1), (IIc), (IId), (IIe), (IIf), (IIf-1), (IIf-2), (IIf-3), (IIf-4), (IIf-5), (III), (IIIa), (IIIb), (IIIc), (IIId), (IIIe), (IIIf), (IIIf-1), (IIIg), (IIIg-1), (IIIh), (IIIi), (IIIj), (IIIk) and/or (IV), or a pharmaceutically acceptable salt thereof. These compositions are formulated with one or more pharmaceutically acceptable excipients suitable for human or animal use, aiming to provide effective treatment for cancer and related disorders.

The compounds of the present invention may be administered alone or in combination with various pharmaceutically acceptable carriers, adjuvants, diluents, fillers, buffers, stabilizers, preservatives, lubricants, or other materials commonly used in pharmaceutical formulations. These excipients are typically approved by regulatory authorities or are generally regarded as safe for human or animal use.

Pharmaceutically acceptable excipients include, but are not limited to, carriers such as lactose, dextrose, sucrose, mannitol, sorbitol, cellulose, and starch; diluents like water or other solvents; glidants and lubricants such as talc, magnesium stearate, and stearic acid; preservatives like methylparaben and propylparaben; buffering agents; chelating agents; polymers; gelling agents; viscosifying agents; and solvents. Auxiliary agents may also be included, such as wetting agents, suspending agents, sweetening agents, flavoring agents, colorants, and any combination thereof.

The pharmaceutical compositions can be prepared in various conventional forms, including tablets, capsules (soft or hard gelatin), dragees, troches, lozenges, solutions, suspensions, injectables, ointments, pastes, creams, lotions, powders, eye or ear drops, impregnated textiles, and products for topical application. These compositions may also be formulated to provide a desired release profile, such as immediate or controlled release.

In particular, the dosage forms for these compositions can take various conventional forms, including but not limited to: solid dosage forms, liquid dosage forms, injectable preparations, transdermal forms, rectal and vaginal formulations and powders and sprays.

Solid dosage forms can for example be selected among: tablets, capsules (soft or hard gelatin), dragees, troches, and lozenges. Tablets can be prepared by compression or molding techniques and may contain binders (e.g., povidone, gelatin), fillers or diluents (e.g., lactose, microcrystalline cellulose), lubricants (e.g., magnesium stearate), disintegrants (e.g., sodium starch glycolate), and preservatives. Capsules can be filled with the active compound in powder or pellet form along with appropriate excipients. The active compounds can also be formulated into microencapsulated forms with excipients. Solid dosage forms may have coatings such as enteric coatings or release-controlling coatings to modify the release profile of the active compound. Buffering agents may be included to maintain the stability of the active compound.

Liquid dosage forms can for example include emulsions, microemulsions, solutions, and suspensions designed for oral administration. These formulations may contain inert diluents like water, solubilizing agents, and emulsifiers such as ethyl alcohol, isopropyl alcohol, and various oils (e.g., cottonseed, olive, castor oils). Adjuvants like wetting agents, emulsifying and suspending agents, sweetening agents, flavoring agents, and perfuming agents may also be included

Injectable preparations such as sterile aqueous or oleaginous suspensions are generally formulated using dispersing, wetting, or suspending agents. Sterile injectable solutions, suspensions, or emulsions can be prepared in nontoxic, parenterally acceptable diluents or solvents like water, Ringer's solution, or isotonic sodium chloride solution. Fixed oils such as synthetic mono- or diglycerides and fatty acids like oleic acid may be used as solvents or suspending media.

Topical and transdermal forms comprise compositions such as ointments, pastes, creams, lotions, gels, solutions, sprays, and transdermal patches. These may be formulated with suitable fats, oils, waxes, or polymers to achieve desired topical or transdermal absorption rates. Patches may also employ rate-controlling membranes or polymer matrices to control the release of the active compounds.

Rectal and vaginal formulations may be in the form of suppositories or gels, incorporating the active compounds into bases such as cocoa butter, polyethylene glycol, or other suitable non-irritating materials.

Powders and Sprays, for topical or inhalation use, may be prepared as powders or aerosol sprays. Propellants like chlorofluorohydrocarbons may be used for spray formulations.

The formulation process for these compositions follows conventional pharmaceutical techniques, ensuring uniform mixing of the active compound with the chosen excipients. Manufacturing steps may include granulation, encapsulation, or coating, depending on the desired release characteristics of the final product. Controlled-release formulations can be achieved through the application of suitable coatings or matrix systems that modulate the release profile of the active ingredient.

### Administration and dosage

The compounds of the present invention, including their pharmaceutically acceptable salts, may be administered to patients using various routes, depending on the nature of the disease, the desired therapeutic effect, and the formulation of the pharmaceutical composition. The choice of administration route and dosage form ensures that the active compounds are delivered effectively to the intended site of action with an appropriate pharmacokinetic profile, taking into consideration factors like bioavailability, absorption rate, and patient compliance. Suitable routes of administration include oral, nasal, buccal, dermal, intradermal, transdermal, parenteral (including intravenous, intramuscular, and subcutaneous), rectal, intraurethral, and topical applications.

The compound described herein may be administered to a subject in an effective amount to achieve the desired therapeutic effect. The appropriate dosage and dosing regimen can vary based on several factors, including the activity of the compound, the route and timing of administration, the rate of excretion, the duration of treatment, any concurrent medications, the severity of the condition being treated, and characteristics of the patient such as species, sex, age, weight, general health, and medical history. Determining the optimal dosage involves balancing the therapeutic benefits against potential risks or side effects and is ultimately at the discretion of the treating physician.

Administration of the compound can be performed in a single dose or multiple doses, either continuously or intermittently throughout the course of treatment. The compound may be administered via any convenient route, whether systemically or at the site of desired action. Routes of administration include, but are not limited to, oral ingestion; topical application (such as transdermal, intranasal, ocular, buccal, and sublingual routes); pulmonary delivery via inhalation or insufflation therapy using an aerosol through the mouth or nose; rectal or vaginal administration; and parenteral routes, including various forms of injection such as subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, intravitreal, and intrasternal injections. Additionally, the compound may be administered through the implantation of a depot, for example, subcutaneously, intramuscularly, or intravitreally.

The dosing frequency may vary depending on the needs of the patient and the judgment of the physician. A single dose may be administered hourly, daily, or weekly. For instance, administration may occur once every 1 hour, 2 hours, 3 hours, 4 hours, 6 hours, 8 hours, 12 hours, 16 hours, or once every 24 hours. Alternatively, the compound may be administered once every 1 day, 2 days, 3 days, 4 days, 5 days, 6 days, or once every 7 days. In some cases, the compound may be administered once every week, every two weeks, every three weeks, or once every four weeks. In certain embodiments, administration may occur once every month. The dosage and frequency may be adjusted over the course of treatment based on the patient's response and any side effects experienced.

Therapeutically effective amounts of the compound generally range from about 0.00001 mg/kg to about 10 mg/kg of body weight per day. More specific dosage ranges include from about 0.0001 mg/kg to about 10 mg/kg per day, from about 0.001 mg/kg to about 1 mg/kg per day, from about 0.01 mg/kg to about 1 mg/kg per day, and from about 0.05 mg/kg to about 0.5 mg/kg per day. Alternatively, the compound may be administered in doses ranging from about 0.01 mg to about 1000 mg per dose. Examples of therapeutically effective amounts include doses from about 0.01 mg to about 100 mg, from about 0.1 mg to about 100 mg, from about 1 mg to about 100 mg, from about 1 mg to about 50 mg, and from about 1 mg to about 10 mg per dose. Specific dose amounts may be about 1 mg, 2 mg, 3 mg, 4 mg, 5 mg, 6 mg, 7 mg, 8 mg, 9 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, or about 100 mg per dose. Higher doses may include about 100 mg, 125 mg, 150 mg, 175 mg, 200 mg, 225 mg, 250 mg, 275 mg, 300 mg, 350 mg, 400 mg, 450 mg, or about 500 mg per dose.

In specific embodiments, the compound may be administered to a human patient according to the following dosage regimens: about 5 mg or about 10 mg administered three or four times daily; about 20 mg or about 40 mg administered three or four times daily; about 50 mg or about 75 mg administered three or four times daily; about 100 mg or about 125 mg administered twice daily; about 100 mg administered three times daily; about 150 mg administered twice daily; or about 200 mg administered twice daily.

The duration of treatment may vary depending on the condition being treated and the patient's response. Treatment may be administered for at least about one week, at least about two weeks, at least about three weeks, one month, at least about two months, at least about three months, at least about six months, at least about twelve months, or potentially extending for the lifetime of the individual. The dosage or dosing frequency may be adjusted over the course of treatment based on the patient's response and the judgment of the administering physician.

In some cases, the active compounds of the present invention may be administered in combination with other therapeutic agents, such as chemotherapeutic drugs, immunotherapy agents, or radiation therapy, for a synergistic effect in treating diseases such as cancer. The combination therapy may be administered concurrently, sequentially, or as a fixed-dose combination, with careful consideration given to potential interactions and the pharmacokinetics of the combined agents.

### Methods and uses

The present invention relates to methods and uses concerning the treatment of diseases and disorders, for example hyperproliferative disorders, particularly cancer, by administering therapeutically effective amounts of compounds of Formula (I), (II), (IIa), (IIb), (IIb-1), (IIc), (IId), (IIe), (IIf), (IIf-1), (IIf-2), (IIf-3), (IIf-4), (IIf-5), (III), (IIIa), (IIIb), (IIIc), (IIId), (IIIe), (IIIf), (IIIf-1), (IIIg), (IIIg-1), (IIIh), (IIIi), (IIIj), (IIIk) and/or (IV), or their pharmaceutically acceptable salts. These compounds are valuable as medicaments for treating conditions mediated by KAT6A, a histone acetyltransferase implicated in cancer progression.

The pharmaceutical compositions of the present invention are intended for use as medicaments for example in the treatment of hyperproliferative disorders, in particular in cancer and related disorders. The active compounds may be formulated to provide a therapeutically effective amount sufficient to achieve the desired therapeutic benefit without causing unwanted side effects. Dosages and modes of administration can be determined and adjusted by healthcare professionals based on factors like the type and severity of the disease, patient characteristics, and preliminary evidence from clinical studies.

The compounds of the present invention effectively inhibit KAT6A activity, thereby modulating pathological processes associated with KAT6A-mediated diseases. Inhibition of KAT6A can suppress cancer cell growth and proliferation, making these compounds significant therapeutic agents in oncology.

Cancers that can be treated using these compounds encompass a wide range, including but not limited to brain gliomas, glioblastomas, astrocytomas, multiforme, Bannayan-Zonana syndrome, Cowden disease, Lhermitte-Duclos disease, breast cancer (such as estrogen receptor-positive [ER+] breast cancer, ER+ HER2- breast cancer, and locally advanced or metastatic ER+ HER2- breast cancer), colon cancer, head and neck cancer, kidney cancer, liver cancer, lung cancer (including non-small cell lung cancer and locally advanced or metastatic non-small cell lung cancer), bone cancer, colorectal cancer, germ cell cancer, melanoma, ovarian cancer, pancreatic cancer, adenocarcinomas (including ductal and adenosquamous carcinoma), acinar cell carcinoma, glucagonoma, insulinoma, prostate cancer (including castration-resistant forms and locally advanced or metastatic cases), sarcoma, thyroid cancer, various leukemias (such as lymphoblastic T cell leukemia, chronic myelogenous leukemia, chronic lymphocytic leukemia, hairy-cell leukemia, acute lymphoblastic leukemia, acute myelogenous leukemia, chronic neutrophilic leukemia, plasmacytoma, mantle cell leukemia, megakaryoblastic leukemia, multiple myeloma, promyelocytic leukemia, erythroleukemia), malignant lymphomas (including Hodgkin's and non-Hodgkin's lymphoma, Burkitt's lymphoma, follicular lymphoma), neuroblastoma, bladder cancer, urothelial cancer, vulval cancer, uterine cancer, cervical cancer, endometrial cancer, renal cancer, mesothelioma, esophageal cancer, salivary gland cancer, hepatocellular cancer, gastric cancer, nasopharyngeal cancer, buccal cancer, cancers of the mouth, gastrointestinal stromal tumors (GIST), neuroendocrine cancers, testicular cancer, and virus-related cancers.

Moreover, the compounds may be used in treating other disorders associated with abnormal cell growth, such as benign proliferative diseases including psoriasis, benign prostatic hypertrophy, or restenosis. They may also be applied in methods of treating disorders associated with angiogenesis, such as age-related macular degeneration, proliferative diabetic retinopathy, rheumatoid arthritis, osteoporosis, Paget's disease, humoral hypercalcemia of malignancy, coronary restenosis, and certain microbial infections.

In more preferred embodiment, the compounds of the invention are used in treating or preventing a disease selected among: breast cancer(such as estrogen receptor-positive [ER+] breast cancer, ER+ HER2- breast cancer, and locally advanced or metastatic ER+ HER2- breast cancer), prostate cancer, ovarian cancer, cervical cancer, lung adenocarcinoma, colon and rectal adenocarcinomas, medulloblastoma, glioblastoma and blood cancer such as acute myeloid leukaemia. In more preferred embodiment, the invention relates to method for treating or prophylaxis of a disease selected from a KATs overexpressing cancer.

One embodiment of the invention provides a pharmaceutical composition comprising a compound of any of the aforementioned formulas, or a pharmaceutically acceptable salt thereof, for use in the treatment, management, or prophylaxis of a disease, disorder, or condition in a subject.

The subject is typically a human in need of such treatment. The diseases or disorders targeted include those mediated by KAT6A, with a particular focus on various forms of cancer.

One embodiment of the invention provides a method of treating, managing, or preventing a disease, disorder, or condition in a subject by administering a therapeutically effective amount of a compound of any of the aforementioned formulas, or a pharmaceutically acceptable salt thereof.

### Combination therapy

Compounds of Formula (I), (II), (Ila), (Ilb), (Ilb-1), (IIc), (Ild), (Ile), (Ilf), (Ilf-1), (IIf-2), (Ilf-3), (Ilf-4), (Ilf-5), (III), (Illa), (IIIb), (IIIc), (IIId), (IIIe), (IIIf), (IIIf-1), (IIIg), (IIIg-1), (IIIh), (IIIi), (IIIj), (IIIk) and/or (IV), described herein, or their pharmaceutically acceptable salts, may be used in combination with other therapeutic agents for the treatment of diseases such as cancer. These compounds can be administered alongside additional therapeutic agents, including chemotherapeutic agents, biological agents, targeted therapies, or agents that alleviate symptoms associated with the disease. The combination therapy may involve administering the compounds and additional agents either in a single pharmaceutical formulation or in separate formulations, delivered simultaneously or sequentially.

The compounds may act synergistically with chemotherapy, radiotherapy, or targeted therapies, enhancing the overall therapeutic effect. For instance, they can be combined with fibroblast growth factor receptor 1 (FGFR1) inhibitors, nuclear hormone receptor-targeting therapies, or immune checkpoint inhibitors. Notably, the compounds may be used in conjunction with bromodomain and extraterminal domain (BET) inhibitors, which reversibly bind to the bromodomains of BET proteins BRD2, BRD3, BRD4, and BRDT.

Inhibition of histone acetyltransferase (KAT) proteins of the MYST family by these compounds reduces lysine acetylation of histones and other nuclear proteins. This action sensitizes tumor cells to chemotherapy and radiotherapy by attenuating DNA damage repair processes, such as the repair of DNA double-strand breaks, thereby increasing the efficacy of cancer cell eradication induced by these therapies. Consequently, the compounds are expected to combine effectively with low-dose chemotherapy or radiotherapy.

The compounds may be administered in conjunction with radiotherapeutic or chemotherapeutic regimens. Suitable chemotherapeutic agents include, but are not limited to, platinum compounds (e.g., cisplatin, carboplatin, oxaliplatin), alkylating agents (e.g., cyclophosphamide, ifosfamide), antitumor antibiotics (e.g., doxorubicin, bleomycin), taxanes (e.g., paclitaxel, docetaxel), antimetabolites (e.g., 5-fluorouracil, methotrexate), nucleoside analogues (e.g., fludarabine), and topoisomerase inhibitors (e.g., irinotecan). Suitable biological agents include monoclonal antibodies (e.g., rituximab, trastuzumab, bevacizumab), enzymes (e.g., L-asparaginase), cytokines (e.g., interferons, interleukins), growth factors, cancer vaccines, and gene therapy vectors.

In scenarios where the compounds are used to abrogate regulatory T cell (Treg) suppression, they may be combined with immune checkpoint inhibitors, such as those targeting PD-1, PD-L1, or CTLA-4, to enhance anti-tumor immunity. Additionally, combining these compounds with radiotherapy may further diminish Treg function within tumors, potentially improving therapeutic outcomes.

These treatment methods may be applied to subjects for whom other treatments have failed or have had limited success, such as those with cancers refractory to standard-of-care treatments. The compounds can be administered prior to, simultaneously with, or after other therapeutic modalities, including chemotherapy, surgery, hormone therapy, or radiation. The combinations described are illustrative and not limiting, and the compounds may be used with one or more additional agents to achieve the desired therapeutic effect.

Combination therapies may involve co-administering the compounds with estrogen receptor antagonists or partial antagonists, aromatase inhibitors (e.g., letrozole), Selective Estrogen Receptor Modulators (SERMs) such as tamoxifen, endoxifene, raloxifene, toremifene, lasofoxifene, ospemifene, elacestrant, or bazedoxifene, and Selective Estrogen Receptor Degraders (SERDs) like fulvestrant, camizestrant, palazestrant, imlunestrant, elacestrant, or giredestrant. Complete Estrogen Receptor Antagonists (CERANs) such as fulvestrant or palazestrant can also be used in combination.

Additionally, the compounds may be combined with other anti-cancer agents, including HER2 inhibitors (e.g., tucatinib, trastuzumab, pertuzumab, ado-trastuzumab emtansine, trastuzumab deruxtecan, lapatinib, neratinib), mTOR inhibitors (e.g., everolimus, sirolimus, temsirolimus, LY3023414), CDK4/6 inhibitors (e.g., palbociclib, abemaciclib, ribociclib, lerociclib, trilaciclib, SHR6390), CDK2 inhibitors (e.g., PF-07104091), CDK4-selective inhibitors (e.g., PF-07220060), PI3 kinase inhibitors (e.g., perifosine, CAL101, BEZ235, XL147, XL765, GDC-0941, IPI-145), PIK3CA inhibitors (e.g., alpelisib, taselisib, LY3023414, inavolisib, STX-478, RLY-2608, LOXO-783, OKI-219, TOS-358), aromatase inhibitors (e.g., aminoglutethimide, testolactone, anastrozole, letrozole, exemestane, vorozole, formestane, fadrozole, 4-hydroxyandrostenedione, 1,4,6-androstatrien-3,17-dione, 4-androstene-3,6,17-trione), antibodies or inhibitors targeting PD-1, PD-L1, or CTLA-4, and inhibitors of EGFR, PGFR, or IGFR (e.g., erlotinib, gefitinib). USP1 inhibitors and AKT inhibitors such as capivasertib are also suitable for combination therapy.

The examples and preparations presented herein further illustrate and exemplify the compounds described, along with the methods for their preparation. It should be noted that the scope of the embodiments described is not limited in any way by these examples and preparations.

In the following examples, unless otherwise specified, molecules with a single chiral center are provided as racemic mixtures. Molecules with two or more chiral centers are also presented as racemic mixtures of diastereomers unless stated otherwise. Methods for obtaining single enantiomers or diastereomers are well-known to those skilled in the art.

In light of the descriptions provided herein, the compounds described herein can be prepared by processes known in the chemical arts. Specific processes for the manufacture of these compounds are included as additional features of the embodiments and are illustrated in the reaction schemes provided below and in the experimental section.

### EXAMPLES

The following examples are provided to illustrate specific embodiments of the invention and are not intended to limit the scope thereof. Variations and modifications will become apparent to those skilled in the art upon reading this specification. The full scope of the invention should be determined by the claims and their equivalents.

### I. Synthesis of sulfonyl isocyanate or sulfonyl carbamate Intermediates

### Intermediate INT_{A-1}: 2,6-dimethoxybenzenesulfonyl isocyanate (Intermediate INT_{A-1})

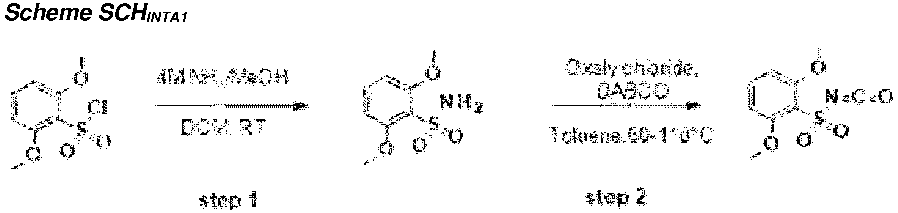

**Step 1:** To a solution of 2,6-dimethoxybenzenesulfonyl chloride (2.62 g, 11.07 mmol) in DCM (25 mL) at room temperature was added 4M ammonia in MeOH (27.68 mL, 110.7 mmol). The reaction mixture was stirred at room temperature for 30 min. The solvent was removed under reduced pressure and water was added. The resulting precipitate was filtered and washed with ACN. The solid was suspended in toluene and evaporated to dryness under reduced pressure to afford 2,6-dimethoxybenzenesulfonamide (1.80 g, 74 % yield) as a white solid. LCMS (ES, m/z): 218.1 [M+H]⁺.

**Step 2:** To a solution of 2,6-dimethoxybenzenesulfonamide (180 mg, 828 µmol) and oxalyl chloride (326 µL, 3.72 mmol) in toluene (3 mL) at room temperature under argon was added DABCO (9.29 mg, 82.85 µmol). The reaction mixture was stirred at 60°C for 1 h then at 110°C for 5 h. The reaction mixture was filtered and washed with toluene then concentrated to dryness under reduced pressure to afford 2,6-dimethoxybenzenesulfonyl isocyanate **(Intermediate INT_{A-1})** as an orange oil which was used without further purification. LCMS (ES, m/z): 276.1 [M+H]⁺. (Compound quenched with MeOH to follow carbamate formation)

### Intermediate INT_{A-2}: 5-isopropyl-2-methoxybenzenesulfonyl isocyanate (Intermediate INT_{A-2})

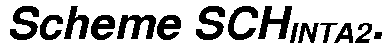

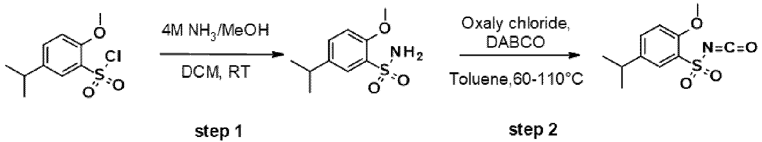

**Step 1:** To a mixture of 5-isopropyl-2-methoxybenzenesulfonyl chloride (1.00 g, 4.02 mmol) in DCM (15 mL) at room temperature was added 4M ammonia solution in MeOH (10.1 mL, 40.2 mmol). The reaction mixture was stirred at room temperature for 30 min. The reaction mixture was filtered and the precipitate was dried under reduced pressure to afford 5-isopropyl-2-methoxybenzenesulfonamide (980 mg, 96 % yield) as a white solid. LCMS (ES, m/z): 230.2 [M+H]⁺.

**Step 2:** To a mixture of 5-isopropyl-2-methoxybenzenesulfonamide (600 mg, 2.62 mmol) and oxalyl chloride (1.01 mL, 11.8 mmol) in toluene (6 mL) at room temperature was added DABCO (29.4 mg, 262 µmol). The reaction mixture was stirred at 60°C for 1 h then at 110°C for 4 h. The reaction mixture was filtered, rinsed with anhydrous toluene and concentrated under reduced pressure to dryness to afford 5-isopropyl-2-methoxybenzenesulfonyl isocyanate **(Intermediate INT_{A-2})** (660 mg, 74 % yield) as a brown oil which was used without further purification. LCMS (ES, m/z): 288.1 [M+H]⁺. (Compound quenched with MeOH to follow carbamate formation)

### Intermediate INT_{A-3}: 5-(tert-butyl)-2-methoxybenzenesulfonyl isocyanate (Intermediate INT_{A-3})

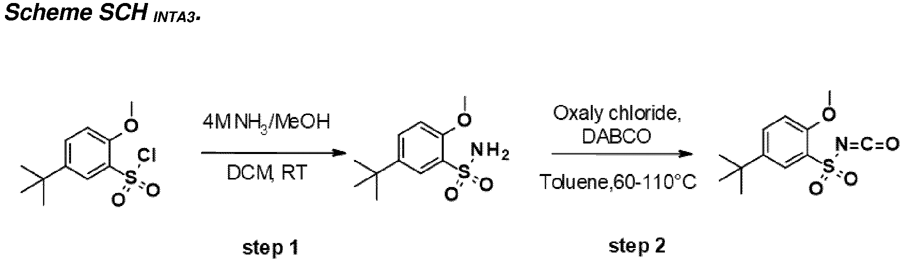

**Step 1:** To a mixture of 5-(tert-butyl)-2-methoxybenzenesulfonyl chloride (2.00 g, 7.61 mmol) in DCM (30 mL) at room temperature was added 4M ammonia solution in methanol (9.51 mL, 38.05 mmol). The reaction mixture was stirred at room temperature for 16h. The reaction mixture was filtered and washed with DCM. The filtrate was concentrated to dryness under reduced pressure to afford 5-(tert-butyl)-2-methoxybenzenesulfonamide (1.75 g, 90 % yield) as a white solid. LCMS (ES, m/z): 244.2 [M+H]⁺.

**Step 2:** To a mixture of 5-(*tert*-butyl)-2-methoxybenzenesulfonamide (227 mg, 932 µmol) and oxalyl chloride (0.36 mL, 4.19 mmol) in toluene (2 mL) at room temperature under argon was added DABCO (10.4 mg, 93 µmol). The reaction mixture was stirred at 60°C for 1 h then at 110°C for 3 h. The reaction mixture was filtered, rinsed with toluene and the filtrate was concentrated to dryness to afford 5-(*tert*-butyl)-2-methoxybenzenesulfonyl isocyanate **(Intermediate INT_{A-3})** as an orange oil which was used without further purification. LCMS (ES, m/z): 302.1 [M+H]⁺. (Compound quenched with MeOH to follow carbamate formation)

### Intermediate INT_{A-4}: 5-chloro-2-methoxybenzenesulfonyl isocyanate (Intermediate INT_{A-4})

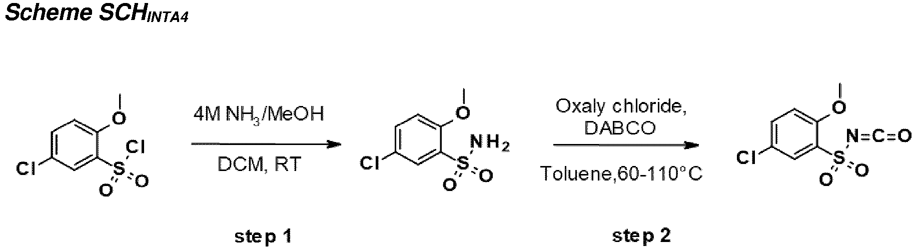

**Step 1:** To a mixture of 5-bromo-2-methoxybenzenesulfonyl chloride (4.83 g, 16.9 mmol) in DCM (84.6 mL) at room temperature was added 4M ammonia solution in MeOH (42.3 mL, 169 mmol). The reaction mixture was stirred at room temperature for 30 min. The reaction mixture was concentrated under reduced pressure then diluted with water. The suspension was stirred for 10 min then filtered and dried to afford 5-bromo-2-methoxybenzenesulfonamide (4.41 g, 93 % yield) as a white solid. LCMS (ES, m/z): 266.1-268.1 [M+H]⁺.

**Step** 1: To a mixture of 5-chloro-2-methoxybenzenesulfonyl chloride (1.5 g, 6.22 mmol) in DCM (10 mL) at room temperature was added 4M ammonia solution in MeOH (15.5 mL, 62.2 mmol). The reaction mixture was stirred at room temperature for 30 min. DCM was added and the resulting precipitate was filtered. The filtrate was concentrated to dryness under reduced pressure to afford 5-chloro-2-methoxybenzenesulfonamide (1.49 g, Qt.) as a white solid. LCMS (ES, m/z): 222.0 [M+H]⁺.

**Step 2:** To a solution of 5-chloro-2-methoxybenzenesulfonamide (227 mg, 1.02 mmol) and oxalyl chloride (0.39 mL, 4.60 mmol) in toluene (2 mL) at room temperature under argon was added DABCO (11.4 mg, 102.4 µmol). The reaction mixture was stirred at 60°C for 1 h then at 110°C for 3 h. The reaction mixture was filtered and rinsed with toluene. The filtrate was concentrated to dryness to afford 5-chloro-2-methoxybenzenesulfonyl isocyanate **(Intermediate INT_{A-4})** as an orange oil which was used without further purification. LCMS (ES, m/z): 280.0 [M+H]⁺. (Compound quenched with MeOH to follow carbamate formation).

### Intermediate INT_{A-5}: 5-fluoro-2-methoxybenzenesulfonyl isocyanate (Intermediate INT_{A-5})

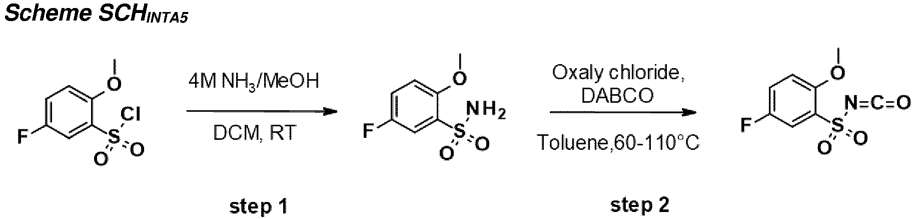

**Step 1:** To a solution of 5-chloro-2-methoxybenzenesulfonyl chloride (1.5 g, 6.22 mmol) in DCM (10 mL) at room temperature was added 4M ammonia in methanol (15.5 mL, 62.2 mmol). The reaction mixture was stirred at room temperature for 30 min. DCM was added and the resulting precipitate was filtered. The filtrate was concentrated to dryness under reduced pressure to afford 5-chloro-2-methoxybenzenesulfonamide (1.49 g, Qt. yield) as a white solid. LCMS (ES, m/z): 206.0 [M+H]⁺.

**Step 2:** To a solution of 5-fluoro-2-methoxybenzenesulfonamide (200 mg, 974 µmol) and oxalyl chloride (0.37 mL, 4.38 mmol) in toluene (2 mL) at room temperature under argon was added DABCO (10.9 mg, 97.4 µmol). The reaction mixture was stirred at 60°C for 1 h then at 110°C for 3 h. The solution was filtered, rinsed with toluene and the filtrate was concentrated to dryness under reduced pressure to afford 5-fluoro-2-methoxybenzenesulfonyl isocyanate **(Intermediate INT_{A-5})** as an orange oil which was used without further purification. LCMS (ES, m/z): 264.0 [M+H]⁺. (Compound quenched with MeOH to follow carbamate formation)

### Intermediate INT_{A-6}: 2-methoxybenzenesulfonyl isocyanate (Intermediate INT_{A-6})

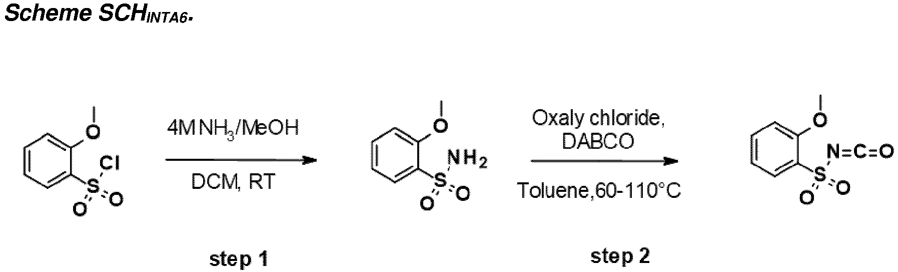

To a mixture of 2-methoxybenzenesulfonyl chloride (2.74 g, 13.3 mmol) in DCM (44.2 mL) at room temperature was added 4M ammonia solution in MeOH (33.1 mL, 133 mmol). The reaction mixture was stirred at room temperature for 30 min. The reaction mixture was filtered and the filtrate was evaporated to dryness under reduced pressure to afford 2-methoxybenzenesulfonamide hydrochloride (3.05 g, 98 % yield) as a white solid. LCMS (ES, m/z): 188.1 [M+H]⁺.

**Step 2:** To a mixture of 2-methoxybenzenesulfonamide (720 mg, 3.84 mmol) and oxalyl chloride (8.65 mL, 17.307 mmol) in toluene (8 mL) at room temperature was added DABCO (43 mg, 384 µmol). The reaction mixture was stirred at 60°C for 1 h then at 110°C for 3 h. The reaction mixture was filtered and rinsed with toluene. The filtrate was concentrated to dryness under reduced pressure to afford 2-methoxybenzenesulfonyl isocyanate **(Intermediate INT_{A-6})** as an orange oil which was used without further purification. LCMS (ES, m/z): 246.2 [M+H]⁺. (Compound quenched with MeOH to follow carbamate formation)

### Intermediate INT_{A-7}: 2-ethoxybenzenesulfonyl isocyanate (Intermediate INTA₋₇)

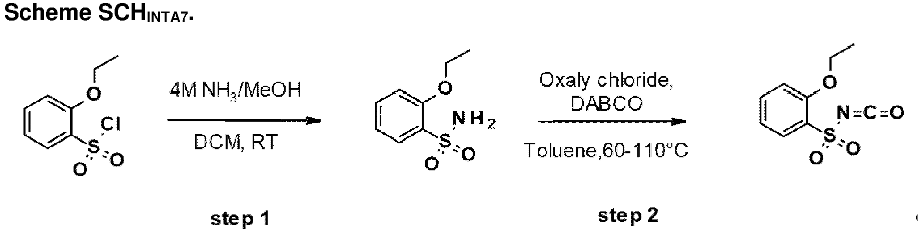

**Step 1:** To a mixture of 2-ethoxybenzenesulfonyl chloride (2.00 g, 9.06 mmol) in DCM (15 mL) at room temperature was added 4M ammonia solution in methanol (22.7 mL, 90.6 mmol). The reaction mixture was stirred at room temperature for 30 min. The reaction mixture was filtered and the filtrate was concentrated to dryness under reduced pressure to afford 2-ethoxybenzenesulfonamide (2.13 g, Qt. yield) as a white solid. LCMS (ES, m/z): 202.1 [M+H]⁺.

**Step 2:** To a mixture of 2-ethoxybenzenesulfonamide (200 mg, 994 µmol) and oxalyl chloride (384 µL, 4.47 mmol) in toluene (4 mL) at room temperature was added DABCO (11.1 mg, 99.4 µmol). The reaction mixture was stirred at 60°C for 1 h then at 110°C for 4 h. The solution was filtered, rinsed with toluene and concentrated under reduced pressure to dryness to afford 2-ethoxybenzenesulfonyl isocyanate **(Intermediate INT_{A-7})** (199 mg, 88 % yield) as a brown oil which was used without further purification. LCMS (ES, m/z): 258.1 [M+H]⁻. (Compound quenched with MeOH to follow carbamate formation).

### Intermediate INT_{A-8}: ethyl ((2,4-dichlorophenyl)sulfonyl)carbamate (Intermediate INT_{A-8})

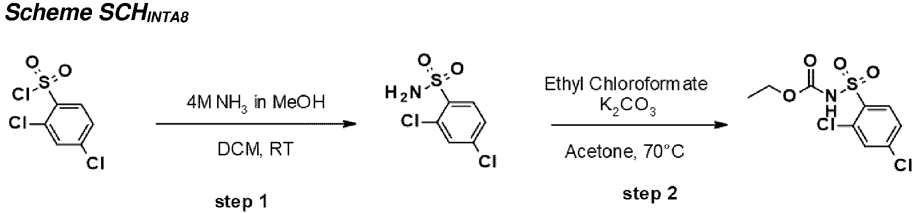

**Step 1:** To a mixture of 2,4-dichlorobenzenesulfonyl chloride (5.00 g, 20.4 mmol) in DCM (100 mL) at room temperature was added 4M ammonia solution in MeOH (50.9 mL, 204 mmol). The reaction mixture was stirred at room temperature for 30 min. The reaction mixture was filtered and the filtrate was concentrated to dryness under reduced pressure to afford 2,4-dichlorobenzenesulfonamide (4.94 g, 97 % yield) as a white solid. LCMS (ES, m/z): 224.0-226.0 [M+H]⁻.

**Step 2:** To a mixture of 2,4-dichlorobenzenesulfonamide (400 mg, 1.76 mmol) and potassium carbonate (635 mg, 4.60 mmol) in acetone (4.4 mL) at room temperature was added ethyl chloroformate (220 µL, 2.30 mmol). The reaction mixture was stirred at 70 °C for 16 h. The reaction mixture was filtered and rinsed with water. The filtrate was acidified with HCl 12N until pH=1 then diluted with ethyl acetate. After separation, the aqueous phase was extracted twice with ethyl acetate. The combined organic phases were dried over anhydrous sodium sulfate, filtered and concentrated under reduced pressure to afford ethyl ((2,4-dichlorophenyl)sulfonyl)carbamate **(Intermediate INT_{A-8})** (424 mg, 80 % yield) as a yellow solid. LCMS (ES, m/z): 296.2-298.2 [M+H]⁻.

### Intermediate INT_{A-9}: ethyl ((5-bromo-2-methoxyphenyl)sulfonyl)carbamate (Intermediate INT_{A-9})

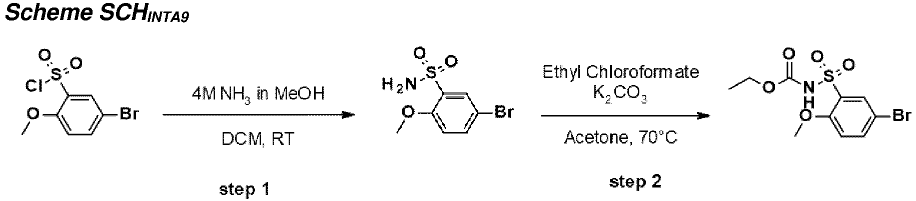

**Step 1:** To a mixture of 5-bromo-2-methoxybenzenesulfonyl chloride (5.00 g, 17.5 mmol) in DCM (87.6 mL) at room temperature was added 4M ammonia solution in MeOH (43.8 mL, 175 mmol). The reaction mixture was stirred at room temperature for 30 min. The reaction mixture was filtered and the filtrate was concentrated to dryness under reduced pressure to afford 5-bromo-2-methoxybenzenesulfonamide (4.80 g, 98 % yield) as a white solid. LCMS (ES, m/z): 265.0-267.1 [M+H]⁺.

**Step 2:** To a mixture of 5-bromo-2-methoxybenzenesulfonamide (200 mg, 751 µmol) and potassium carbonate (270 mg, 1.95 mmol) in acetone (7.5 mL) at room temperature was added ethyl chloroformate (93.8 µL, 977 µmol). The reaction mixture was stirred at 70 °C for 16 h. The reaction mixture was filtered and rinsed with water. The filtrate was acidified with HCl 12N until pH=1 then diluted with ethyl acetate. After separation, the aqueous phase was extracted twice with ethyl acetate. The combined organic phases were dried over anhydrous sodium sulfate, filtered and concentrated under reduced pressure to afford ethyl ((5-bromo-2-methoxyphenyl)sulfonyl)carbamate **(Intermediate INT_{A-9})** (220 mg, 76 % yield) as a yellow solid. LCMS (ES, m/z): 338.0-340.0 [M+H]⁺.

### Intermediate INT_{A-10}: ethyl ((4-chloro-2-methoxyphenyl)sulfonyl)carbamate (Intermediate INT_{A-10})

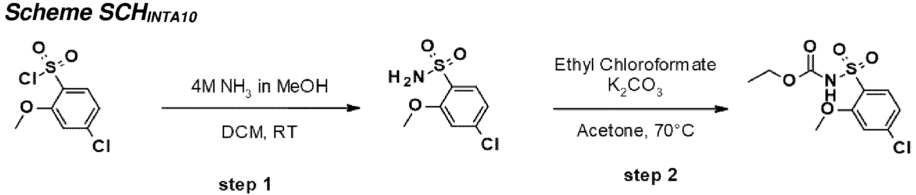

**Step 1:** To a mixture of 4-chloro-2-methoxybenzene-1-sulfonyl chloride (1.21 g, 5.04 mmol) in DCM (25.2 mL) at room temperature was added 4M ammonia solution in MeOH (12.6 mL, 50.4 mmol). The reaction mixture was stirred at room temperature for 30 min. The reaction mixture was filtered and the filtrate was concentrated to dryness under reduced pressure to afford 4-chloro-2-methoxybenzenesulfonamide (1.06 g, 94 % yield) as a white solid. LCMS (ES, m/z): 222.1 [M+H]⁺.

**Step 2:** To a mixture of 4-chloro-2-methoxybenzenesulfonamide (150 mg, 676 µmol) and potassium carbonate (243 mg, 1.75 mmol) in acetone (6.7 mL) at room temperature was added ethyl chloroformate (84 µL, 879 µmol). The reaction mixture was stirred at 70 °C for 16 h. The reaction mixture was filtered and rinsed with water. The filtrate was acidified with HCl 1N until pH=1 then diluted with ethyl acetate. After separation, the aqueous phase was extracted with ethyl acetate. The combined organic phases were dried over anhydrous sodium sulfate, filtered and concentrated under reduced pressure to afford ethyl ((4-chloro-2-methoxyphenyl)sulfonyl)carbamate **(Intermediate INT_{A-10})** (160 mg, 76 % yield) as a white solid. LCMS (ES, m/z): 294.0 [M+H]⁺.

### Intermediate INT_{A-11}: ethyl ((3-(dimethylamino)phenyl)sulfonyl)carbamate (Intermediate INT_{A-11})

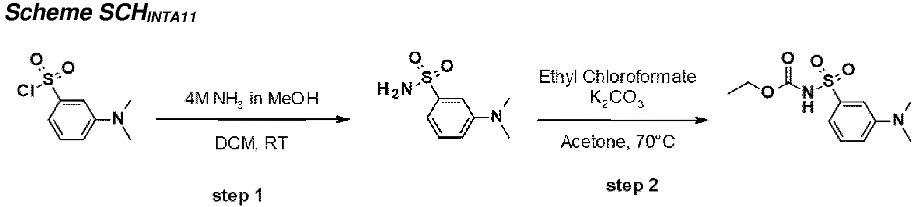

**Step 1:** To a mixture of 3-(dimethylamino)benzenesulfonyl chloride (1.00 g, 4.55 mmol) in DCM (20 mL) at room temperature was added 4M ammonia solution in MeOH (5.69 mL, 22.76 mmol). The reaction mixture was stirred at room temperature for 16 h. The reaction mixture was filtered, washed with DCM then water was added. After separation, the organic layer was concentrated under reduced pressure. The residue was purified by silica gel column chromatography eluted with cyclohexane/(ethyl acetate/EtOH 3:1) from 100/0 to 0/100 to afford 3-(dimethylamino)benzenesulfonamide (600 mg, 63 % yield) as a beige solid. LCMS (ES, m/z): 201.2 [M+H]⁺.

**Step 2:** To a mixture of 3-(dimethylamino)benzenesulfonamide (600 mg, 2.99 mmol) and potassium carbonate (1.07 g, 7.78 mmol) in acetone (20 mL) at room temperature was added ethyl chloroformate (374 µL, 3.89 mmol). The reaction mixture was stirred at 70 °C for 16 h. The reaction mixture was filtered and rinsed with acetone. The filtrate was poured into water then ethyl acetate was added. The aqueous phase was acidified with HCl 1N until pH=1 and ethyl acetate was added. The organic layer was washed with water then brine, dried over anhydrous sodium sulfate, filtered and concentrated under reduced pressure to afford ethyl ((3-(dimethylamino)phenyl)sulfonyl)carbamate **(Intermediate INT_{A-11})** (300 mg, 35 % yield) as a colorless oil. LCMS (ES, m/z): 273.2 [M+H]⁺.

### Intermediate INT_{A-12}: ethyl ((5-(3-fluoroazetidin-1-yl)-2-methoxyphenyl)sulfonyl)carbamate (Intermediate INT_{A-12})

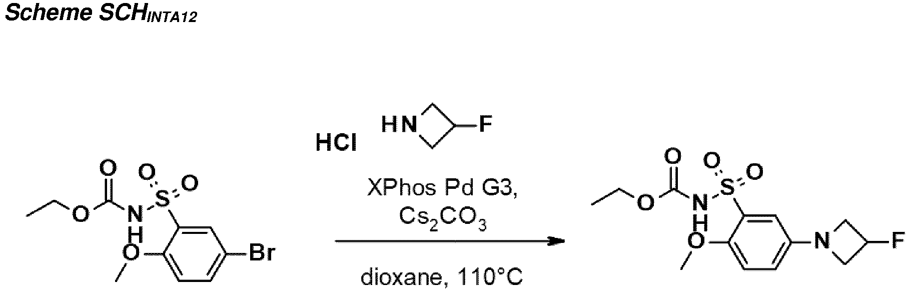

To a suspension of ethyl ((5-bromo-2-methoxyphenyl)sulfonyl)carbamate (280 mg, 827 µmol), 3-fluoroazetidine hydrochloride (369 mg, 3.31 mmol) and cesium carbonate (2.15 g, 6.62 mmol) at room temperature in dioxane (8.27 mL). The reaction mixture was degassed with argon for 5 min then XPhos Pd G3 (105 mg, 124.2 µmol) was added. The reaction mixture tube was sealed and stirred at 110 °C for 6 h under MW irradiations. The reaction mixture was filtered then evaporated under reduced pressure. The residue was purified by silica gel column chromatography eluted with cyclohexane/ethyl acetate from 100/0 to 50/50. The resulting product was suspended in saturated NH₄Cl aqueous solution and ethyl acetate and stirred at room temperature for 30 min. After separation, the aqueous phase was acidified with HCl 1N until pH=2. The aqueous phase was extracted twice with ethyl acetate. The combined organic phase was dried over anhydrous sodium sulfate, filtered and concentrated under reduced pressure to afford ethyl ((5-(3-fluoroazetidin-1-yl)-2-methoxyphenyl)sulfonyl)carbamate **(Intermediate INT_{A-12})** (140 mg, 48 % yield) as a yellow oil. LCMS (ES, m/z): 333.2 [M+H]⁺.

### Intermediate INT_{A-13}: ethyl ((4-bromo-2-methoxyphenyl)sulfonyl)carbamate (Intermediate INT_{A-13})

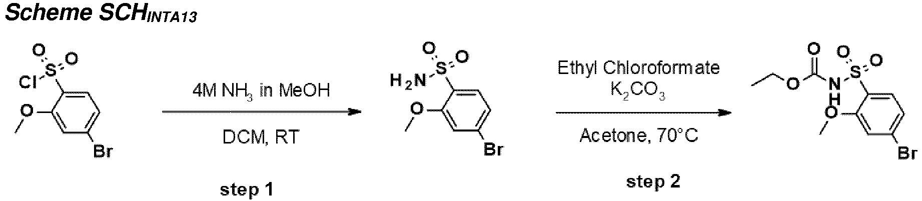

**Step 1:** To a mixture of 4-bromo-2-methoxybenzenesulfonyl chloride (2.00 g, 7.0 mmol) in DCM (35 mL) at room temperature was added 4M ammonia solution in MeOH (17.5 mL, 70 mmol). The reaction mixture was stirred at room temperature for 30 min. The reaction mixture was filtered and the filtrate was concentrated under reduced pressure. The residue was suspended in water and stirred for 10 min then filtered and dried to afford 4-bromo-2-methoxybenzenesulfonamide (1.46 g, 73 % yield) as a white solid. LCMS (ES, m/z): 266.0-268.0 [M+H]⁺.

**Step 2:** To a mixture of 4-bromo-2-methoxybenzenesulfonamide (150 mg, 564 µmol) and potassium carbonate (203 mg, 1.47 mmol) in acetone (5.6 mL) at room temperature was added ethyl chloroformate (70 µL, 733 µmol). The reaction mixture was stirred at 70 °C for 16 h. The reaction mixture was filtered and rinsed with water. The filtrate was acidified with HCl 12N until pH=1 then diluted with ethyl acetate. After separation, the aqueous phase was extracted with ethyl acetate. The combined organic phases were dried over anhydrous sodium sulfate, filtered and concentrated under reduced pressure to afford ethyl ((4-bromo-2-methoxyphenyl)sulfonyl)carbamate **(Intermediate INT_{A-13})** (130 mg, 56 % yield) as a white solid. LCMS (ES, m/z): 338.0-340.0 [M+H]⁺.

### Intermediate INT_{A-14}: ethyl ((3-(pyrrolidin-1-yl)phenyl)sulfonyl)carbamate (Intermediate INT_{A-14})

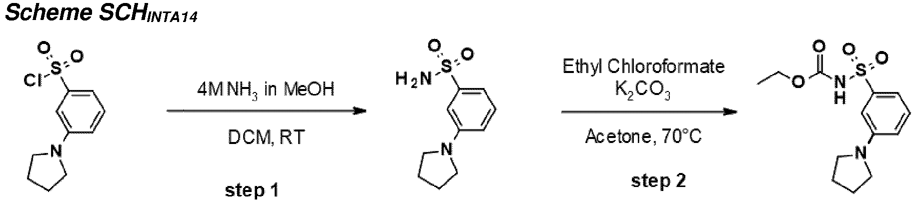

**Step 1:** To a mixture of 3-(pyrrolidin-1-yl)benzenesulfonyl chloride (1.00 g, 4.07 mmol) in DCM (20 mL) at room temperature was added 4M ammonia solution in MeOH (10.2 mL, 40.7 mmol). The reaction mixture was stirred at room temperature for 30 min. The reaction mixture was filtered and the filtrate was concentrated under reduced pressure to afford 3-(pyrrolidin-1-yl)benzenesulfonamide (960 mg, Qt. yield) as a white solid. LCMS (ES, m/z): 227.1 [M+H]⁺.

**Step 2:** To a mixture of 3-(pyrrolidin-1-yl)benzenesulfonamide (200 mg, 883 µmol) and potassium carbonate (317 mg, 2.29 mmol) in acetone (8.8 mL) at room temperature was added ethyl chloroformate (127 µL, 1.32 mmol). The reaction mixture was stirred at 70 °C for 16 h. The reaction mixture was filtered and rinsed with water. The filtrate was acidified with HCl 1N until pH=1 then diluted with ethyl acetate. After separation, the aqueous phase was extracted twice with ethyl acetate. The combined organic phases were dried over anhydrous sodium sulfate, filtered and concentrated under reduced pressure to afford ethyl ((3-(pyrrolidin-1-yl)phenyl)sulfonyl)carbamate **(Intermediate INT**_{**A-**14}) (226 mg, 60 % yield) as a clear oil. LCMS (ES, m/z): 299.2 [M+H]⁺.

### Intermediate INT_{A-15}: 2-methoxy-5-(trifluoromethyl)benzenesulfonyl isocyanate (Intermediate INT_{A-15})

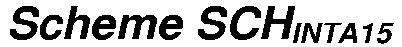

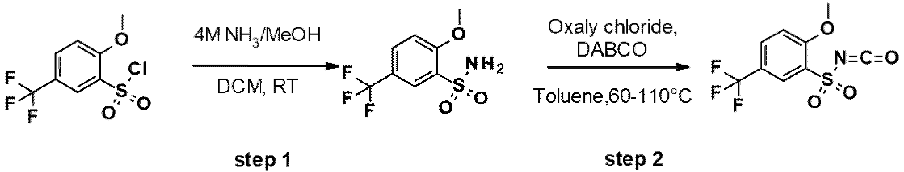

**Step** 1: To a mixture of 2-methoxy-5-(trifluoromethyl)benzenesulfonyl chloride (2.50 g, 9.10 mmol) in DCM (30 mL) at room temperature was added 4M ammonia solution in MeOH (22.8 mL, 91.0 mmol). The reaction mixture was stirred at room temperature for 1 h. The reaction mixture was concentrated to dryness under reduced pressure then diluted with water. The resulting suspension was stirred for 10 min then filtered and dried to afford 2-methoxy-5-(trifluoromethyl)benzenesulfonamide (2.0 g, 82 % yield) as a white solid. LCMS (ES, m/z): 255.0 [M+H]⁻.

**Step 2:** Under argon, to a mixture of 2-methoxy-5-(trifluoromethyl)benzenesulfonamide (170 mg, 666 µmol) and oxalyl chloride (262 µL, 2.99 mmol) in toluene (3 mL) at room temperature was added DABCO (7.47 mg, 66.6 µmol). The reaction mixture was stirred at 60 °C for 1 h then at 110 °C for 3 h. The reaction mixture was filtered, rinsed with anhydrous toluene and concentrated to dryness under reduced pressure to afford 2-methoxy-5-(trifluoromethyl)benzenesulfonyl isocyanate **(Intermediate INT_{A-15})** as an orange oil which was used without further purification. LCMS (ES, m/z): 314.1 [M+H]⁺. (Compound quenched with MeOH to follow carbamate formation)

### Intermediate INT_{A-16}: tert-butyl ((5-(3-fluoroazetidin-1-yl)-2-methoxyphenyl)sulfonyl)carbamate (Intermediate INT_{A-16})

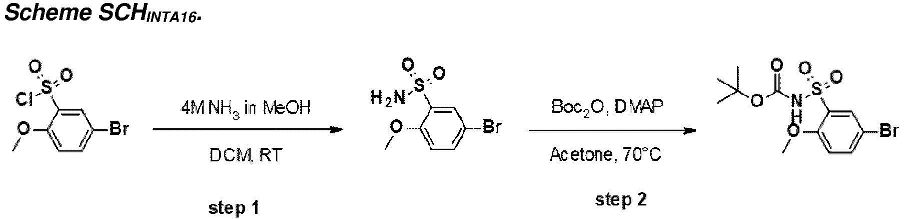

**Step 1:** To a mixture of 5-bromo-2-methoxybenzenesulfonyl chloride (4.83 g, 16.9 mmol) in DCM (84 mL) at room temperature was added 4M ammonia solution in MeOH (42.3 mL, 169 mmol). The reaction mixture was stirred at room temperature for 30 min. The reaction mixture was concentrated under reduced pressure. The residue was diluted in water, stirred 10 min at room temperature then filtered and dried to afford 5-bromo-2-methoxybenzenesulfonamide (4.41 g, 93 % yield) as a white solid.
LCMS (ES, m/z): 266.1-268.1 [M+H]⁺.

**Step 2:** To a solution of 5-bromo-2-methoxybenzenesulfonamide (500 mg, 1.87 mmol) in dichloromethane (18.8 mL) was added Et₃N (786 µL, 5.63 mmol) and Boc₂O (518 µL, 2.25 mmol). The reaction mixture was stirred at room temperature for 16 h. Water was added and the layers were separated. Then, the organic one was washed with brine, dried over anhydrous sodium sulfate, filtered and evaporated under reduced pressure to afford tert-butyl ((5-bromo-2-methoxyphenyl)sulfonyl)carbamate **(Intermediate INT_{A16})** (1.1 g, Qte yield) as a clear oil which was used in the next step without further purification.
LCMS (ES, m/z): 364.2-366.1 [M+H]⁺.

### Intermediate INT_{A-17}: tert-butyl ((5-(3-fluoroazetidin-1-yl)-2-methoxyphenyl)sulfonyl)carbamate (Intermediate INT_{A-17})

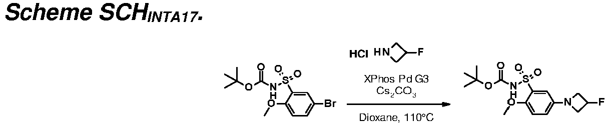

To a suspension of *tert*-butyl ((5-bromo-2-methoxyphenyl)sulfonyl)carbamate (1.1 g, 2.10 mmol), 3-fluoroazetidine hydrochloride (938 mg, 8.41 mmol) and cesium carbonate (5.48 g, 16.82 mmol) in dioxane (21 mL) at room temperature under argon was added XPhos Pd G3 (267 mg, 315 µmol). The reaction mixture was stirred at 110 °C for 6 h under MW irradiations. The reaction mixture was dissolved in saturated NH₄Cl aqueous solution and ethyl acetate and the resulting suspension was stirred at room temperature for 30 min. The pH of the aqueous phase was adjusted to 2 by addition of 1N HCl aqueous solution. After separation, the aqueous phase was extracted with ethyl acetate. The combined organic phases were dried over anhydrous sodium sulfate, filtered and evaporated under reduced pressure. The residue was purified by silica gel column chromatography eluted with cyclohexane/ethyl acetate from 100/0 to 50/50 to afford *tert-*butyl ((5-(3-fluoroazetidin-1-yl)-2-methoxyphenyl)sulfonyl)carbamate **(Intermediate INT**_{**A-**17}) (261 mg, 34 % yield) as a yellow solid.
LCMS (ES, m/z): 361.2 [M+H]⁺.

### Intermediate INT_{A-18}: tert-butyl ((4'-fluoro-4-methoxy-[1,1'-biphenyl]-3-yl)sulfonyl)carbamate (Intermediate INT_{A-18})

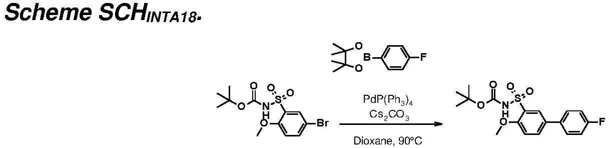

To a mixture of tert-butyl ((5-bromo-2-methoxyphenyl)sulfonyl)carbamate (INT_{A-16}) (415 mg, 906 µmol), 2-(4-fluorophenyl)-4,4,5,5-tetramethyl-1,3,2-dioxaborolane (241 mg, 1.08 mmol) and cesium carbonate (886 mg, 2.71 mmol) in DME (8 mL) and water (0.9 mL) under nitrogen at room temperature was added PdP(Ph₃)₄ (104 mg, 90.6 µmol). The reaction mixture was stirred at 90 °C for 16 h. The reaction mixture was poured into water and ethyl acetate then acetic acid (5 mL) was added. Organic layer was washed with water, dried over anhydrous sodium sulfate, filtered and evaporated under reduced pressure. The residue was purified by silica column chromatography eluted with cyclohexane/ethyl acetate from 100/0 to 70/30 to afford *tert*-butyl ((4'-fluoro-4-methoxy-[1,1'-biphenyl]-3-yl)sulfonyl)carbamate **(Intermediate INT_{A-18})** (170 mg, 59 % yield) as a beige solid.
LCMS (ES, m/z): 380.2 [M-H]⁺.

### Intermediate INT_{A-19}: 4-methoxy-[1,1'-biphenyl]-3-sulfonyl isocyanate (Intermediate INT_{A-19})

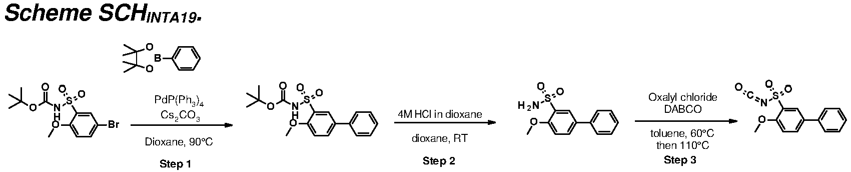

**Step** 1: To a mixture of *tert*-butyl ((5-bromo-2-methoxyphenyl)sulfonyl)carbamate (INT_{A-16}) (400 mg, 873 µmol), phenylboronic acid (127 mg, 1.04 mmol) and cesium carbonate (854 mg, 2.62 mmol) in DME (8 mL) and water (0.9 mL) under argon was added PdP(Ph₃)₄ (100 mg, 87 µmol). The reaction mixture was stirred at 90 °C for 16 h. The reaction mixture was dissolved in water and ethyl acetate. Then, acetic acid was added until pH=3. The layers were separated and the organic one was washed with brine, dried over anhydrous sodium sulfate, filtered and evaporated under reduced pressure. The residue was purified by silica gel column chromatography eluted with cyclohexane/ethyl acetate from 100/0 to 70/30 to afford *tert*-butyl ((4-methoxy-[1,1'-biphenyl]-3-yl)sulfonyl)carbamate (306 mg, 96% yield) as a brown solid.
LCMS (ES, m/z): 362.2 [M-H]⁺.

**Step 2:** To a solution of *tert*-butyl ((4-methoxy-[1,1'-biphenyl]-3-yl)sulfonyl)carbamate (306 mg, 749 µmol) in dioxane (2 mL) was added 4M hydrogen chloride solution in dioxane (1.87, 7.49 mmol). The reaction mixture was stirred at room temperature for 16 h. The solvent was evaporated under reduced pressure to afford 4-methoxy-[1,1'-biphenyl]-3-sulfonamide (180 mg, 80 % yield) as a yellowish solid.
LCMS (ES, m/z): 264.3 [M+H]⁺.

**Step 3:** To a mixture of 4-methoxy-[1,1'-biphenyl]-3-sulfonamide (180 mg, 683 µmol) and oxalyl chloride (269 µL, 3.07 mmol) in toluene (4 mL) at room temperature was added DABCO (7.66 mg, 68.3 µmol). The reaction mixture was stirred at 60 °C for 1 h then at 110 °C for 3 h. The solution was filtered, rinsed with anhydrous dichloromethane and concentrated to dryness under reduced pressure to afford 4-methoxy-[1,1'-biphenyl]-3-sulfonyl isocyanate **(Intermediate INT_{A-19})** (Purity 200 mg, 58 % yield) as an orange oil which was engaged in the next step of the synthesis without purification.
LCMS (ES, m/z): 264.3 [M+H]⁺. (quenched with methanol to observe carbamate mass)

### Intermediate INT_{A-20}: 2,3-dihydrobenzofuran-7-sulfonyl isocyanate (Intermediate INT_{A-20})

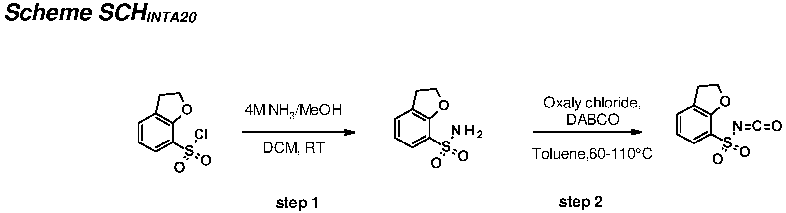

**Step 1:** To a mixture of 2,3-dihydrobenzofuran-7-sulfonyl chloride (2.00 g, 9.15 mmol) in DCM (15 mL) at room temperature was added 4M ammonia in MeOH (22.9 mL, 91.5 mmol). The reaction mixture was stirred at room temperature for 30 min. The solvent was removed under reduced pressure. The residue was purified by reverse phase C₁₈ column chromatography eluted with water/acetonitrile 100/0 to 20/80 to afford 2,3-dihydrobenzofuran-7-sulfonamide (1.46 g, 80 % yield) as a white solid.
LCMS (ES, m/z): 200.1 [M+H]⁺.

**Step 2:** To a mixture of 2,3-dihydrobenzofuran-7-sulfonamide (300 mg, 1.51 mmol) and oxalyl chloride (593 µL, 6.78 mmol) in toluene (4 mL) at room temperature under argon was added DABCO (16.9 mg, 151 µmol). The reaction mixture was stirred at 60 °C for 1 h then at 110 °C for 3 h. The solution was filtered, rinsed with anhydrous toluene and concentrated to dryness under reduced pressure to afford 2,3-dihydrobenzofuran-7-sulfonyl isocyanate **(Intermediate INT_{A-20})** (250 mg, 1.11 mmol, 73.7 %) as an yellow oil which was used in the next step without any further purification. LCMS (ES, m/z): 258.1 [M+H]⁺. (Compound quenched with MeOH to follow carbamate formation)

### Intermediate INT_{A-21}: tert-butyl ((7-methoxyquinolin-8-yl) sulfonyl)carbamate (Intermediate INT_{A-21})

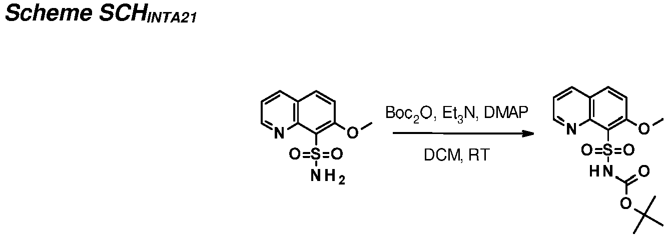

To a mixture of 7-methoxyquinoline-8-sulfonamide (500 mg, 2.10 mmol), triethylamine (877 µL, 6.30 mmol) and DMAP (25.6 mg, 210 µmol) in DCM (8.0 mL) was added Boc₂O (530 µL, 2.31 mmol). The reaction mixture was stirred at room temperature for 16 h. Water was added and the layers were separated. The organic phase was washed with brine, dried over anhydrous sodium sulfate, filtered and concentrated under reduced pressure to afford tert-butyl ((7-methoxyquinolin-8-yl) sulfonyl)carbamate **(Intermediate INT_{B-59})** (409 mg, 57 % yield) as a yellow solid.
LCMS (ES, m/z): 339.1 [M+H]⁺.

### Intermediates INT_{A-1} to INT_{A-21}

Intermediates INT_{A-1} to INT_{A-21}, as listed in Table 2, were synthesized by methods analogous to those described above for Intermediates INT_{A-1}, to INT_{A-21} utilizing suitable starting materials and standard procedures familiar to those skilled in the art. In some cases, commercially available sulfonyl chloride building blocks were employed directly.

**Table 2. Intermediates A**

| **Intermediate** | **Structure** |
|---|---|
| **INT_{A-1}** | 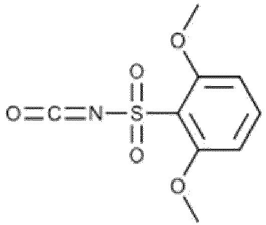 |
| **INT_{A-2}** | 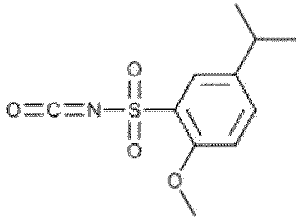 |
| **INT_{A-3}** | 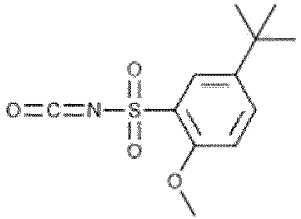 |
| **INT_{A-4}** | 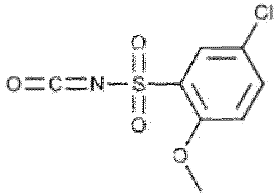 |
| **INT_{A-5}** | 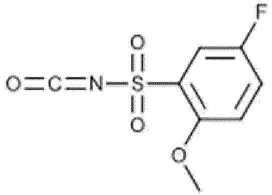 |
| **INT_{A-6}** | 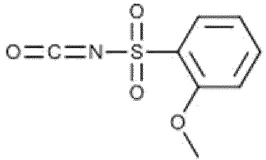 |
| **INT_{A-7}** | 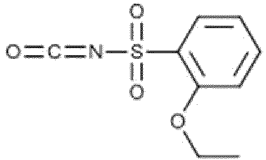 |
| **INT_{A-8}** | 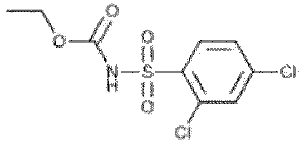 |
| **INT_{A-9}** | 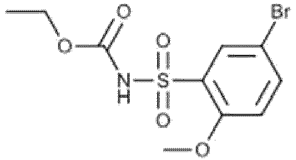 |
| **INT_{A-10}** | 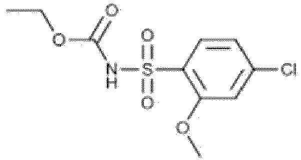 |
| **INT_{A11}** | 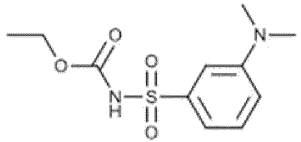 |
| **INT_{A-12}** | 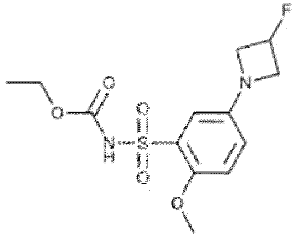 |
| **INT_{A-13}** | 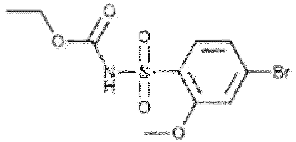 |
| **INT_{A-14}** | 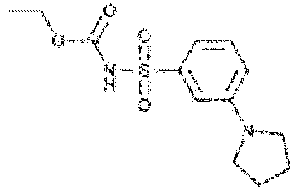 |
| **INT_{A-15}** | 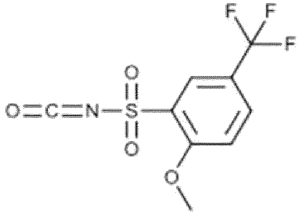 |
| **INT_{A-16}** | 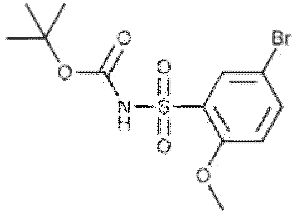 |
| **INT_{A-17}** | 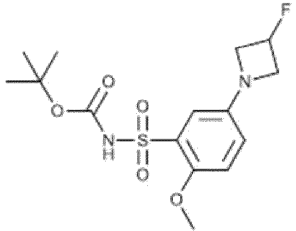 |
| **INT_{A-18}** | 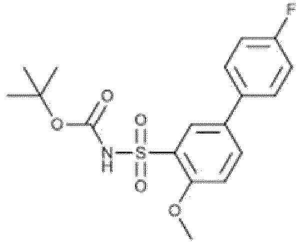 |
| **INT_{A-19}** | 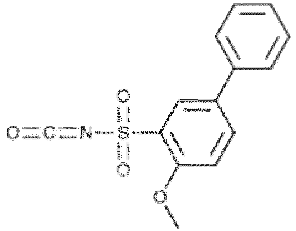 |
| **INT_{A-20}** | 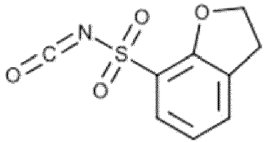 |
| **INT_{A-21}** | 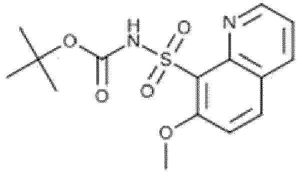 |

### II. Synthesis of isoquinoline Intermediates

### Intermediate INT_{B-1}: tert-butyl 8-methoxy-5-(1H-pyrazol-1-yl)-3,4-dihydroisoquinoline-2(1H)-carboxylate (Intermediate INT_{B-1})

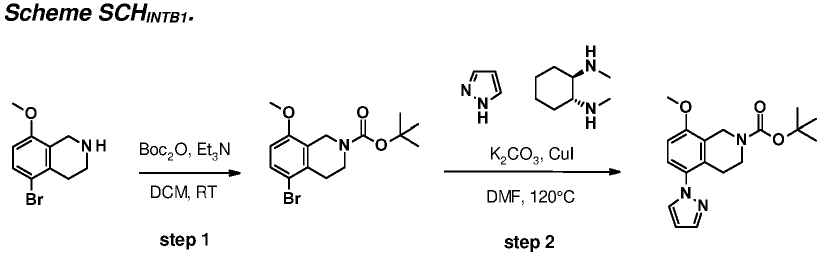

**Step 1:** To a solution of 5-bromo-8-methoxy-1,2,3,4-tetrahydroisoquinoline (946 mg, 3.90 mmol) in DCM (7 mL) was added Et₃N (1.63 mL, 11.7 mmol) and Boc₂O (1.08 mL, 4.68 mmol). The reaction mixture was stirred at room temperature for 16 h. Water was added and the layers were separated. The organic phase was washed with brine, dried over anhydrous sodium sulfate, filtered and concentrated under reduced pressure to afford *tert*-butyl 5-bromo-8-methoxy-3,4-dihydroisoquinoline-2(1H)-carboxylate (1.35 g, Qt. yield) which was used without further purification. LCMS (ES, m/z): 286.1-288.1 [M+H-*t*Bu]⁺.

**Step 2:** A mixture of 1H-pyrazole (537 mg, 7.88 mmol), *tert*-butyl 5-bromo-8-methoxy-3,4-dihydroisoquinoline-2(1H)-carboxylate (1.35 g, 3.94 mmol), potassium carbonate (1.63 g, 11.83 mmol) and trans-(1R,2R)-N1,N2-bismethyl-1,2-cyclohexanediamine (622 µL, 3.94 mmol) in DMF (10 mL) was stirred at room temperature under argon for 5 min then copper(I) iodide (751 mg, 3.94 mmol) was added and the reaction mixture was stirred at 120 °C for 48 h. Ethyl acetate (EtOAc) and a saturated NH₄Cl aqueous solution were added. The reaction mixture was filtered over a pad of Celite. After separation, the organic layer was washed with brine, dried over anhydrous sodium sulfate, filtered and concentrated under reduced pressure. The residue was purified by silica gel column chromatography eluted with cyclohexane/ Ethyl acetate from 100/0 to 50/50 to afford *tert*-butyl 8-methoxy-5-(1H-pyrazol-1-yl)-3,4-dihydroisoquinoline-2(1H)-carboxylate (Intermediate INT**_{B-1}**) (1.00 g, 73 % yield) as a dark oil. LCMS (ES, m/z): 330.1 [M+H]⁺.

### Intermediate INT_{B-2}: 8-methoxy-5-(1H-pyrazol-1-yl)-1,2,3,4-tetrahydroisoquinoline hydrochloride (Intermediate INT_{B-2})

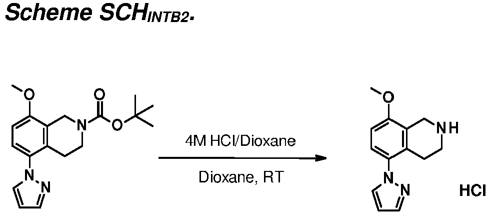

To a solution of *tert*-butyl 8-methoxy-5-(1H-pyrazol-1-yl)-3,4-dihydroisoquinoline-2(1H)-carboxylate **(Intermediate INT_{B-1})** (335 mg, 1.01 mmol) in dioxane (2 mL) was added 4M HCl solution in dioxane (3.81 mL, 15.2 mmol). The reaction mixture was stirred at room temperature for 16 h. The solvent was removed under reduced pressure and diethyl ether was added. The resulting precipitate was filtered and dried to afford 8-methoxy-5-(1H-pyrazol-1-yl)-1,2,3,4-tetrahydroisoquinoline hydrochloride **(Intermediate INT_{B-2})** (262 mg, 96 % yield) as a yellow solid. LCMS (ES, m/z): 230.3 [M+H]⁺.

### Intermediate INT_{B-3}: 8-cyclopropyl-5-(1H-pyrazol-1-yl)-1,2,3,4-tetrahydroisoquinoline hydrochloride (Intermediate INT_{B-3})

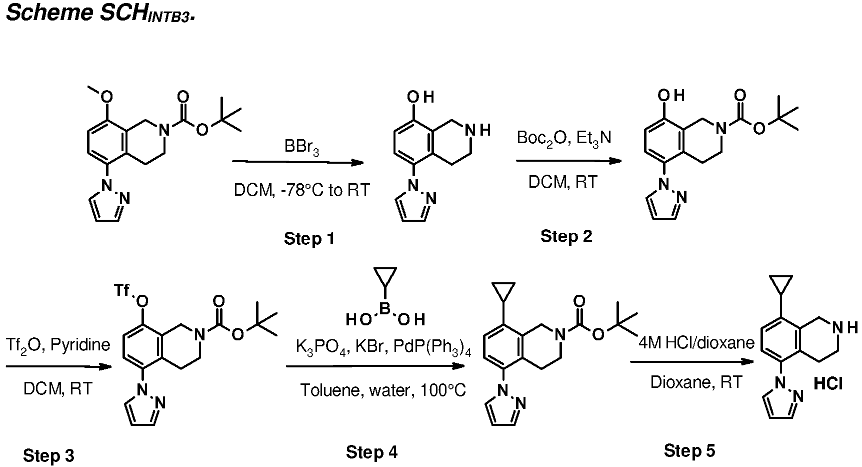

**Step 1:** To a mixture of *tert*-butyl 8-methoxy-5-(1H-pyrazol-1-yl)-3,4-dihydroisoquinoline-2(1H)-carboxylate **(Intermediate INT_{B-1})** (596 mg, 1.80 mmol) in DCM (10 mL) at -78°C was slowly added 1M BBr₃ solution in DCM (9.04 mL, 9.04 mmol). The reaction mixture was allowed to warm up at room temperature and stirred for 16 h. A saturated aqueous solution of NaHCO₃ was added to the reaction mixture until pH=8. The aqueous layer was extracted with a mixture of DCM/IPA (9/1). The organic layers were combined and washed with brine then dried over anhydrous sodium sulfate, filtered and concentrated under reduced pressure to afford 5-(1H-pyrazol-1-yl)-1,2,3,4-tetrahydroisoquinolin-8-ol (240 mg, 40 % yield) as a brown oil. LCMS (ES, m/z): 216.3 [M+H]⁺.

**Step 2:** To 5-(1H-pyrazol-1-yl)-1,2,3,4-tetrahydroisoquinolin-8-ol (240 mg, 1.11 mmol) in DCM (3 mL) was added Et₃N (466 µL, 3.34 mmol) and Boc₂O (307 µL, 1.33 mmol). The reaction mixture was stirred at room temperature for 16 h. Methanol (8 mL) and K₂CO₃ (231 mg, 1.67 mmol) were added. The reaction mixture was stirred at room temperature for 5 h. Water and AcOH were added until pH=5. The aqueous layer was extracted with ethyl acetate. The combined organic phases were washed with brine and dried over anhydrous sodium sulfate, filtered and concentrated under reduced pressure to afford *tert-*butyl 8-hydroxy-5-(1H-pyrazol-1-yl)-3,4-dihydroisoquinoline-2(1H)-carboxylate (274 mg, 77 % yield) as a brown oil. LCMS (ES, m/z): 316.2 [M+H]⁺.

**Step 3:** To *tert*-butyl 8-hydroxy-5-(1H-pyrazol-1-yl)-3,4-dihydroisoquinoline-2(1H)-carboxylate (454 mg, 1.43 mmol) in DCM (7 mL) was added trifluoromethanesulfonic anhydride (291 µL, 1.72 mmol) and pyridine (233 µL, 2.87 mmol). The reaction mixture was stirred at room temperature for 16 h. Water and ethyl acetate were added and the layers were separated. The organic phase was washed with brine, dried over anhydrous sodium sulfate, filtered and concentrated under reduced pressure. The residue was purified by silica gel column chromatography eluted with cyclohexane/ ethyl acetate from 100/0 to 70/30 to afford *tert*-butyl 5-(1H-pyrazol-1-yl)-8-(((trifluoromethyl)sulfonyl)oxy)-3,4-dihydroisoquinoline-2(1H)-carboxylate (492 mg, 76 % yield) as a clear oil. LCMS (ES, m/z): 392.1 [M+H-*t*Bu]⁺.

**Step 4:** To a mixture of *tert*-butyl 5-(1H-pyrazol-1-yl)-8-(((trifluoromethyl)sulfonyl)oxy)-3,4-dihydroisoquinoline-2(1H)-carboxylate (492 mg, 1.09 mmol), cyclopropylboronic acid (188 mg, 2.19 mmol), potassium phosphate tribasic (455 µL, 5.49 mmol) and potassium bromide (130 mg, 1.09 mmol) in toluene (5 mL) and water (0.6 mL) was degassed with argon for 10 min then PdP(Ph₃)₄ (127 mg, 109 µmol). The reaction mixture was stirred at 100 °C for 16 h. Water and ethyl acetate were added. After separation, the aqueous layer was extracted with ethyl acetate. The combined organic layers were washed with brine, dried over anhydrous sodium sulfate, filtered and concentrated under reduced pressure. The residue was purified by silica gel column chromatography eluted with cyclohexane/CPME from 100/0 to 30/70 to afford tert-butyl 8-cyclopropyl-5-(1H-pyrazol-1-yl)-3,4-dihydroisoquinoline-2(1H)-carboxylate (142 mg, 38 % yield) as a yellow pale oil. LCMS (ES, m/z): 338.2 [M+H]⁺.

**Step 5:** To a solution of *tert*-butyl 8-cyclopropyl-5-(1H-pyrazol-1-yl)-3,4-dihydroisoquinoline-2(1H)-carboxylate (142 mg, 418 µmol) in dioxane (1 mL) was added 4M HCl solution in dioxane (1.04 mL, 4.18 mmol). The reaction mixture was stirred at room temperature for 16 h. The solvent was evaporated under reduced pressure to afford 8-cyclopropyl-5-(1H-pyrazol-1-yl)-1,2,3,4-tetrahydroisoquinoline hydrochloride **(Intermediate INT_{B-3})** (114 mg, 98 % yield) as a brown solid. LCMS (ES, m/z): 240.2 [M+H]⁺.

### Intermediate INT_{B-4}: tert-butyl 7-bromo-5-fluoro-3,4-dihydroisoquinoline-2(1H)-carboxylate (Intermediate INT_{B-4})

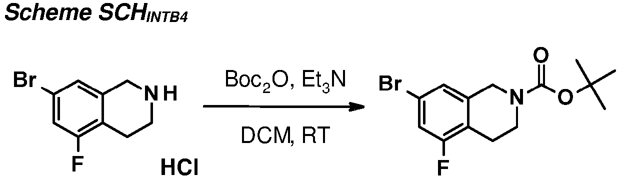

**Step 1:** To 7-bromo-5-fluoro-1,2,3,4-tetrahydroisoquinoline hydrochloride (520 mg, 1.95 mmol) in DCM (8.0 mL) was added Boc₂O (538 µL, 2.34 mmol) and Et₃N (816 µL, 5.85 mmol). The reaction mixture was stirred at room temperature for 16 h. Water was added and the layers were separated. The organic phase was dried over anhydrous sodium sulfate, filtered and concentrated under reduced pressure to afford *tert*-butyl 7-bromo-5-fluoro-3,4-dihydroisoquinoline-2(1H)-carboxylate **(Intermediate INT_{B-4})** (698 mg, Qt. yield) which was used without further purification. LCMS (ES, m/z): 273-9-275.9 [M+H-*t*Bu]⁺.

### Intermediate INT_{B-5}: 5-fluoro-N,N-dimethyl-1,2,3,4-tetrahydroisoquinolin-7-amine hydrochloride (Intermediate INT_{B-5})

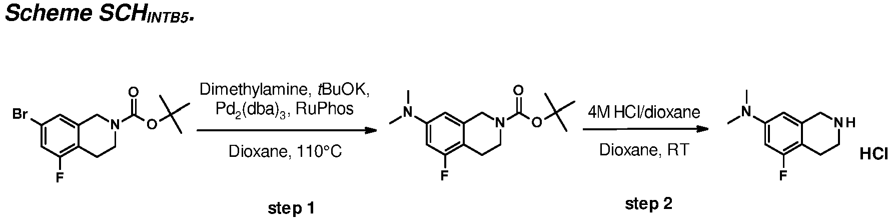

**Step 1:** To a mixture of *tert*-butyl 7-bromo-5-fluoro-3,4-dihydroisoquinoline-2(1H)-carboxylate (INT_{B-4}) (52 mg, 1.44 mmol), sodium *tert*-butoxide (691 mg, 7.2 mmol) and RuPhos (67 mg, 144 µmol) in dioxane (7 mL) at room temperature was stirred under argon for 5 min then 2M solution dimethylamine in MeOH (3.6 mL, 7.2 mmol) and Pd₂(dba)₃ (65 mg, 72 µmol) were added. The reaction mixture was stirred at 110°C for 2 h. The reaction mixture was directly absorbed with a pad of Celite and concentrated to dryness. The residue was purified by silica gel column chromatography eluted with cyclohexane/CPME from 100/0 to 50/50 to afford *tert*-butyl 7-(dimethylamino)-5-fluoro-3,4-dihydroisoquinoline-2(1H)-carboxylate (446 mg, 100 % yield) as a brown oil. LCMS (ES, m/z): 295.1 [M+H]⁺.

**Step 2:** To a solution of *tert*-butyl 7-(dimethylamino)-5-fluoro-3,4-dihydroisoquinoline-2(1H)-carboxylate (446 mg, 1.52 mmol) in dioxane (5 mL) was added 4M HCl solution in dioxane (3.79 mL, 15.2 mmol). The reaction mixture was stirred at room temperature for 16 h. The solvent was removed under reduced pressure. The resulting residue was suspended in diethyl ether, filtered and dried to afford 5-fluoro-N,N-dimethyl-1,2,3,4-tetrahydroisoquinolin-7-amine hydrochloride **(Intermediate INT_{B-5})** (378 mg, Qt. yield) as a brown solid. LCMS (ES, m/z): 295.1 [M+H]⁺.

### Intermediate INT_{B-6}: N,N-dimethyl-1,2,3,4-tetrahydroisoquinolin-7-amine hydrochloride (Intermediate INT_{B-6})

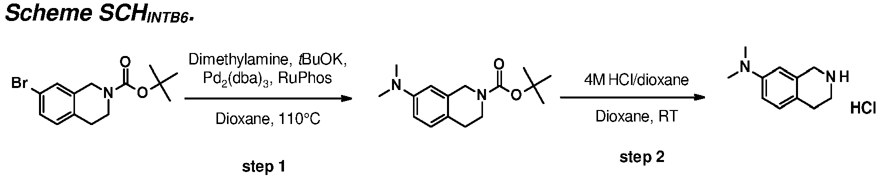

**Step 1:** In a sealed tube, a mixture of *tert*-butyl 7-bromo-3,4-dihydroisoquinoline-2(1H)-carboxylate hydrochloride (1.00 g, 2.86 mmol), sodium tert-butoxide (1.37 g, 14.3 mmol) and RuPhos (133 mg, 286 µmol) in dioxane (14 mL) at room temperature under argon was stirred for 5 min then 2M dimethylamine solution in MeOH (7.17 mL, 14.34 mmol) and Pd₂(dba)3 (131 mg, 143.4 µmol) were added. The reaction mixture was stirred at 110°C for 2 h. The reaction mixture was directly absorbed with a pad of Celite and concentrated to dryness. The residue was purified by silica gel column chromatography eluted with cyclohexane/CPME from 100/0 to 50/50 to afford *tert*-butyl 7-(dimethylamino)-3,4-dihydroisoquinoline-2(1H)-carboxylate (900 mg, Qt. yield) as a brown oil. LCMS (ES, m/z): 277.1 [M+H]⁺.

**Step 2:** To a solution of *tert*-butyl 7-(dimethylamino)-3,4-dihydroisoquinoline-2(1H)-carboxylate (900 mg, 3.26 mmol) in dioxane (8 mL) was added 4 HCl solution in dioxane (18.14 mL, 32.6 mmol). The reaction mixture was stirred at room temperature for 16 h. The solvent was removed under reduced pressure and diethyl ether was added. The resulting solid was filtered and dried to afford *N*,*N*-dimethyl-1,2,3,4-tetrahydroisoquinolin-7-amine hydrochloride **(Intermediate INT_{B-6})** (545 mg, 78 % yield) as a brown solid.

1H NMR (400 MHz, DMSO) δ9.46 (2H, br s), 7.24 - 6.96 (1H, m), 4.25 - 4.21 (2H, m), 4.19 - 3.93 (2H, m), 3.36 - 3.31 (2H, m), 2.99 - 2.94 (8H, br s).

### Intermediate INT_{B-7}: 7-cyclopropyl-1,2,3,4-tetrahydroisoquinoline hydrochloride (Intermediate INT_{B-7})

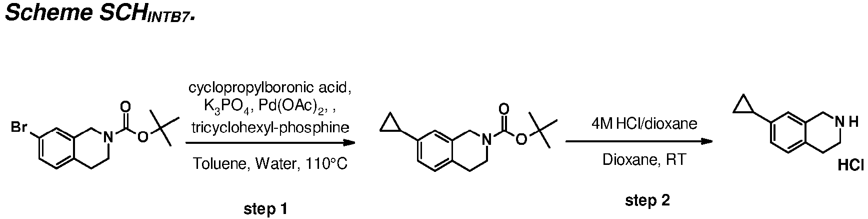

**Step 1:** In a sealed tube, to a mixture of *tert*-butyl 7-bromo-3,4-dihydroisoquinoline-2(1H)-carboxylate (1.00 g, 3.20 mmol) in toluene (12 mL) and water (1.2 mL) was added cyclopropylboronic acid (412 mg, 4.80 mmol), tricyclohexylphosphine, (100 µL, 320 µmol) and potassium phosphate, tribasic (795 µL, 9.60 mmol). The reaction mixture was flushed under argon for 5 min and palladium diacetate (35.95 mg, 160 µmol) was added. The reaction mixture was stirred at 100°C for 2 h. Water and ethyl acetate were added. The layers were separated and the organic phase was washed with brine, dried over anhydrous sodium sulfate, filtered and concentrated under reduced pressure. The residue was purified by silica gel column chromatography eluted with cyclohexane/CPME from 100/0 to 70/30 to afford *tert*-butyl 7-cyclopropyl-3,4-dihydroisoquinoline-2(1H)-carboxylate (830 mg, 90 % yield) as a brown oil. LCMS (ES, m/z): 218.1 [M+H-*t*Bu]⁺.

**Step 2:** To a solution of *tert*-butyl 7-cyclopropyl-3,4-dihydroisoquinoline-2(1H)-carboxylate (830 mg, 3.04 mmol) in dioxane (8 mL) was added 4M HCl solution in dioxane (7.59 mL, 30.4 mmol). The reaction mixture was stirred at room temperature for 16 h. The solvent was removed under reduced pressure and diethyl ether was added. The resulting solid was filtered and dried to afford 7-cyclopropyl-1,2,3,4-tetrahydroisoquinoline hydrochloride **(Intermediate INT_{B-7})** (600 mg, 90 % yield) as a white solid. LCMS (ES, m/z): 174.1 [M+H]⁺.

### Intermediate INT_{B-8}: 5-fluoro-7-(3-fluoroazetidin-1-yl)-1,2,3,4-tetrahydroisoquinoline (Intermediate INT_{B-8})

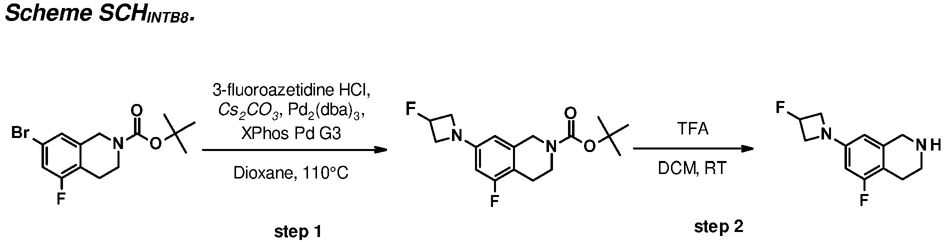

**Step 1:** To a solution of *tert*-butyl 7-bromo-5-fluoro-3,4-dihydroisoquinoline-2(1H)-carboxylate (INT_{B-4}) (48 mg, 145 µmol), 3-fluoroazetidine hydrochloride (81 mg, 726 µmol) and cesium carbonate (378 mg, 1.16 mmol) in dioxane (1 mL) under argon was added XPhos Pd G3 (12 mg, 4.54 µmol). The reaction mixture was stirred at 110 °C for 2 h. The reaction mixture was concentrated under reduced pressure. The residue was purified by silica gel column chromatography eluted with cyclohexane/CPME from 100/0 to 50/50 to afford tert-butyl 5-fluoro-7-(3-fluoroazetidin-1-yl)-3,4-dihydroisoquinoline-2(1H)-carboxylate (125 mg, 76 % yield) as a colorless oil. LCMS (ES, m/z): 325.2 [M+H]⁺.

**Step 2:** To a solution of *tert*-butyl 5-fluoro-7-(3-fluoroazetidin-1-yl)-3,4-dihydroisoquinoline-2(1H)-carboxylate (125 mg, 385 µmol) in DCM (2 mL) was added TFA (296 µL, 3.85 mmol). The reaction mixture was stirred at room temperature for 16 h. A saturated Na₂CO₃ aqueous solution was added until pH=11. The aqueous layer was extracted with a mixture of DCM/IPA. The combined organic extracts were washed with brine, dried over anhydrous sodium sulfate, filtered and concentrated under reduced pressure to afford 5-fluoro-7-(3-fluoroazetidin-1-yl)-1,2,3,4-tetrahydroisoquinoline **(Intermediate INT_{B-8})** (100 mg, Qt. yield) as a yellow oil. LCMS (ES, m/z): 225.2 [M+H]⁺.

### Intermediate INT_{B-9}: 7-(azetidin-1-yl)-5-fluoro-1,2,3,4-tetrahydroisoquinoline (Intermediate INT_{B-9})

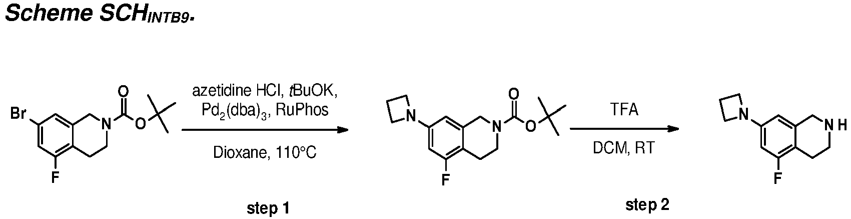

**Step 1:** In a sealed tube, a mixture of *tert*-butyl 7-bromo-5-fluoro-3,4-dihydroisoquinoline-2(1H)-carboxylate (INT_{B-4}) (740 mg, 2.24 mmol), sodium *tert*-butoxide (1.72 g, 17.9 mmol), azetidine hydrochloride (1.04 g, 11.20 mmol) and RuPhos (104.5 mg, 224 µmol) in dioxane (10 mL) at room temperature was stirred under argon for 5 min then Pd₂(dba)₃ (102 mg, 112 µmol) was added. The reaction mixture was stirred at 110°C for 4 h. The reaction mixture was absorbed with a pad of Celite and concentrated to dryness under reduced pressure. The residue was purified by silica gel column chromatography eluted with cyclohexane/CPME from 100/0 to 70/30 to afford *tert*-butyl 7-(azetidin-1-yl)-5-fluoro-3,4-dihydroisoquinoline-2(1H)-carboxylate (215 mg, 27 % yield) as a yellow oil. LCMS (ES, m/z): 307.2 [M+H]⁺.

**Step 2:** To a solution of *tert*-butyl 7-(azetidin-1-yl)-5-fluoro-3,4-dihydroisoquinoline-2(1H)-carboxylate (150 mg, 489.5 µmol) in DCM (3 mL) was added TFA (377 µL, 4.89 mmol). The reaction mixture was stirred at room temperature for 16 h. A saturated Na₂CO₃ aqueous solution of was added until pH=11. The aqueous layer was extracted with DCM/IPA. The combined organic extracts were washed with brine, dried over anhydrous sodium sulfate, filtered and concentrated under reduced pressure to afford 7-(azetidin-1-yl)-5-fluoro-1,2,3,4-tetrahydroisoquinoline **(Intermediate INT_{B-9})** (100 mg, 99 % yield) as a yellow oil. LCMS (ES, m/z): 207.2 [M+H]⁺.

### Intermediate INT_{B-10}: tert-butyl 7-bromo-4-methyl-3,4-dihydroisoquinoline-2(1H)-carboxylate (Intermediate INT_{B-10})

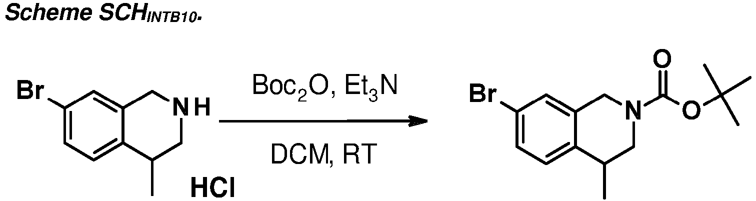

To 7-bromo-4-methyl-1,2,3,4-tetrahydroisoquinoline hydrochloride (1.62 g, 6.16 mmol) in DCM (15 mL) were added Boc₂O (1.7 mL, 7.40 mmol) and Et₃N (2.58 mL, 18.5 mmol). The reaction mixture was stirred at room temperature for 4 h. Water was added and the layers were separated. The organic phase was washed with brine, dried over anhydrous sodium sulfate, filtered and concentrated under reduced pressure to afford *tert-*butyl 7-bromo-4-methyl-3,4-dihydroisoquinoline-2(1H)-carboxylate **(Intermediate INT_{B-10})** (2.19 g, Qt. yield). LCMS (ES, m/z): 270.0-272.0 [M+H-*t*Bu]⁺.

### Intermediate INT_{B-11}: N,N,4-trimethyl-1,2,3,4-tetrahydroisoquinolin-7-amine hydrochloride (Intermediate INT_{B-11})

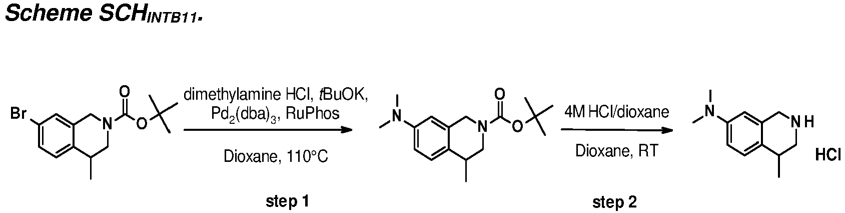

**Step 1:** In a sealed tube, a mixture of *tert*-butyl 7-bromo-4-methyl-3,4-dihydroisoquinoline-2(1H)-carboxylate (INT_{B-10}) (409 mg, 1.12 mmol), sodium tert-butoxide (650 mg, 6.76 mmol) and RuPhos (52 mg, 112 µmol) in dioxane (7 mL) at room temperature was stirred under argon for 5 min then 2M dimethylamine solution (1.69 mL, 3.38 mmol) and Pd₂(dba)₃ (51 mg, 56.3 µmol) were added. The reaction mixture was stirred at 110°C for 16 h. The reaction mixture was filtered and the filtrate was absorbed with a pad of Celite and concentrated under reduced pressure to dryness. The residue was purified by silica gel column chromatography eluted with cyclohexane/CPME from 100/0 to 80/20 to afford *tert*-butyl 7-(dimethylamino)-4-methyl-3,4-dihydroisoquinoline-2(1H)-carboxylate (186 mg, 50 % yield) as a colorless oil. LCMS (ES, m/z): 291.3 [M+H]⁺.

**Step 2:** To a solution of *tert*-butyl 7-(dimethylamino)-4-methyl-3,4-dihydroisoquinoline-2(1H)-carboxylate (180 mg, 620 µmol) in dioxane (1 mL) was added 4M HCl solution in dioxane (1.55 mL, 6.20 mmol). The reaction mixture was stirred at room temperature for 16 h. The solvent was removed under reduced pressure and diethyl ether was added. The resulting solid was filtered and dried to afford *N*,*N*,4-trimethyl-1,2,3,4-tetrahydroisoquinolin-7-amine hydrochloride **(Intermediate INT_{B-11})** (150 mg, Qt. yield) as a brown solid. LCMS (ES, m/z): 191.3 [M+H]⁺.

### Intermediate INT_{B-12}: 8-fluoro-5-methoxy-1,2,3,4-tetrahydroisoquinoline hydrochloride (Intermediate INT_{B-12})

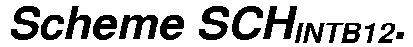

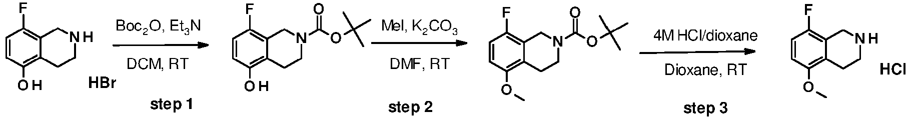

**Step 1:** To a solution of 8-fluoro-1,2,3,4-tetrahydroisoquinolin-5-ol hydrobromide (320 mg, 1.28 mmol) in DCM (3 mL) was added Boc₂O (356 µL, 1.54 mmol) and Et₃N (539 µL, 3.86 mmol). The reaction mixture was stirred at room temperature for 16 h. Water was added and the layers were separated. The organic phase was washed with brine, dried over anhydrous sodium sulfate, filtered and concentrated under reduced pressure to afford tert-butyl 8-fluoro-5-hydroxy-3,4-dihydroisoquinoline-2(1H)-carboxylate (370 mg, Qt. yield). LCMS (ES, m/z): 268.1 [M+H]⁺.

**Step 2:** To *tert*-butyl 8-fluoro-5-hydroxy-3,4-dihydroisoquinoline-2(1H)-carboxylate (255 mg, 953 µmol) and potassium carbonate (395 mg, 2.86 mmol) in DMF (5 mL) at room temperature was added iodomethane (89 µL, 1.43 mmol). The reaction mixture was stirred at room temperature for 4 h. The reaction mixture was directly absorbed with a pad of Celite and concentrated under reduced pressure to dryness. The residue was purified by silica gel column chromatography eluted with cyclohexane/ethyl acetate from 100/0 to 80/20 to afford tert-butyl 8-fluoro-5-methoxy-3,4-dihydroisoquinoline-2(1H)-carboxylate (234 mg, 83 % yield) as a white solid. LCMS (ES, m/z): 282.2 [M+H]⁺.

**Step 3:** To a solution of *tert*-butyl 8-fluoro-5-methoxy-3,4-dihydroisoquinoline-2(1H)-carboxylate (267 mg, 949 µmol) in dioxane (1 mL) was added 4M HCl solution in dioxane (346 mg, 2.37 mL, 9.49 mmol). The reaction mixture was stirred at room temperature for 16 h. The solvent was removed under reduced pressure and diethyl ether was added. The resulting solid was filtered and dried to afford 8-fluoro-5-methoxy-1,2,3,4-tetrahydroisoquinoline hydrochloride **(Intermediate INT_{B-12})** (202 mg, 97 % yield) as a white solid. LCMS (ES, m/z): 182.1 [M+H]⁺.

### Intermediate INT_{B-13}: 7-((1H-pyrazol-1-yl)methyl)-4-methyl-1,2,3,4-tetrahydroisoquinoline hydrochloride (Intermediate INT_{B-13})

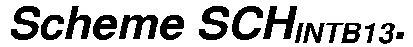

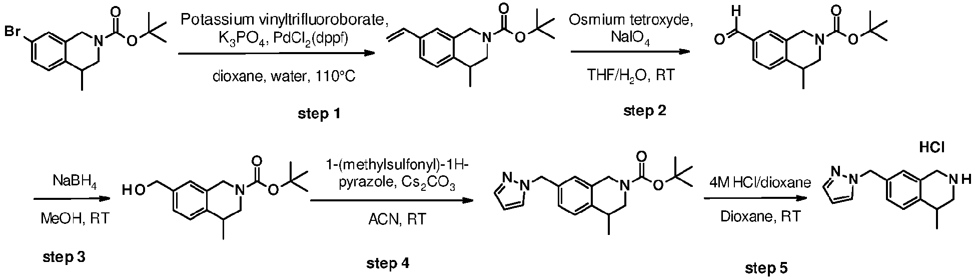

**Step 1:** To a mixture of *tert*-butyl 7-bromo-4-methyl-3,4-dihydroisoquinoline-2(1H)-carboxylate (INT_{B-10}) (1.00 g, 3.07 mmol), potassium trifluoro(vinyl)borate(1-) (821 mg, 6.13 mmol) and K₃PO₄ (826 µL, 9.2 mmol) in dioxane (8 mL) and water (0.9 mL) was stirred under argon for 10 min then was added [1,1'-Bis(diphenylphosphino)ferrocene]dichloropalladium(II) complex with DCM (125 mg, 153 µmol). The reaction mixture was stirred at 110°C for 16 h. Water and ethyl acetate were added. After separation, the aqueous layer was extracted with ethyl acetate. The combined organic layers were washed with brine, dried over anhydrous sodium sulfate, filtered and evaporated under reduced pressure. The residue was purified by silica gel column chromatography eluted with cyclohexane/CPME from 100/0 to 80/20 to afford *tert-*butyl 4-methyl-7-vinyl-3,4-dihydroisoquinoline-2(1H)-carboxylate (633 mg, 57 % yield) as a colorless oil. LCMS (ES, m/z): 274.2 [M+H]⁺.

**Step 2:** To *tert*-butyl 4-methyl-7-vinyl-3,4-dihydroisoquinoline-2(1H)-carboxylate (633 mg, 1.75 mmol) in THF (6 mL) and water (2 mL) at 0°C was added sodium metaperiodate (235 µL, 4.39 mmol) and osmium tetraoxide (35.19 µL, 87.98 µmol). The reaction mixture was allowed to warm up to room temperature and stirred for 1 h. Ethyl acetate and water were added. The layers were separated and the organic phase was dried over anhydrous sodium sulfate, filtered and concentrated under reduced pressure. The residue was purified by silica gel column chromatography eluted with cyclohexane/CPME from 100/0 to 70/30 to afford *tert*-butyl 7-formyl-4-methyl-3,4-dihydroisoquinoline-2(1H)-carboxylate (391 mg, 77 % yield) as a colorless oil. LCMS (ES, m/z): 176.1 [M+H-Boc]⁺.

**Step 3:** To a mixture of *tert*-butyl 7-formyl-4-methyl-3,4-dihydroisoquinoline-2(1H)-carboxylate (300 mg, 849.8 µmol) in MeOH (5 mL) at 0°C was slowly added NaBH₄ (64.3 mg, 1.69 mmol). The reaction mixture was allowed to warm up to room temperature and stirred for 2 h. The solvent was removed under reduced pressure. The crude material was dissolved in ethyl acetate and water. The layers were separated and the organic phase was dried over anhydrous sodium sulfate, filtered and concentrated under reduced pressure. The residue was purified by silica gel column chromatography eluted with cyclohexane/ethyl acetate from 100/0 to 50/50 to afford *tert*-butyl 7-(hydroxymethyl)-4-methyl-3,4-dihydroisoquinoline-2(1H)-carboxylate (325 mg, Qt. yield) as a colorless oil. LCMS (ES, m/z): 178.1 [M+H-Boc]⁺.

**Step 4:** To a mixture of *tert-butyl* 7-(hydroxymethyl)-4-methyl-3,4-dihydroisoquinoline-2(1H)-carboxylate (268 mg, 966.2 µmol) in ACN (5 mL) at room temperature was added 1-(methylsulfonyl)-1H-pyrazole (169 mg, 1.15 mmol) then Cs₂CO₃ (944 mg, 2.89 mmol). The reaction mixture was stirred at room temperature for 48 h. The reaction mixture material was dissolved in water and ethyl acetate then extracted with ethyl acetate. The layers were separated and the organic phase was dried over anhydrous sodium sulfate, filtered and concentrated under reduced pressure. The residue was purified by silica gel column chromatography eluted with cyclohexane/ethyl acetate 100/0 to 50/50 to afford *tert*-butyl 7-((1H-pyrazol-1-yl)methyl)-4-methyl-3,4-dihydroisoquinoline-2(1H)-carboxylate (248 mg, 78 % yield) as a colorless oil. LCMS (ES, m/z): 328.2 [M+H]⁺.

**Step 5:** To a solution of *tert*-butyl 7-((1H-pyrazol-1-yl)methyl)-4-methyl-3,4-dihydroisoquinoline-2(1H)-carboxylate (240 mg, 733 µmol) in dioxane (1 mL) was added 4M HCl solution in dioxane (1.83 mL, 7.33 mmol). The reaction mixture was stirred at room temperature for 16 h. The solvent was removed under reduced pressure. The residue was supspended in diethyl ether, filtered and dried to afford 7-((1H-pyrazol-1-yl)methyl)-4-methyl-1,2,3,4-tetrahydroisoquinoline hydrochloride **(Intermediate INT_{B-13})** (220 mg, 100 % yield) as a brown solid. LCMS (ES, m/z): 228.2 [M+H]⁺.

### Intermediate INT_{B-14}: 8-fluoro-5-(1H-pyrazol-1-yl)-1,2,3,4-tetrahydroisoquinoline hydrochloride (Intermediate INT_{B-14})

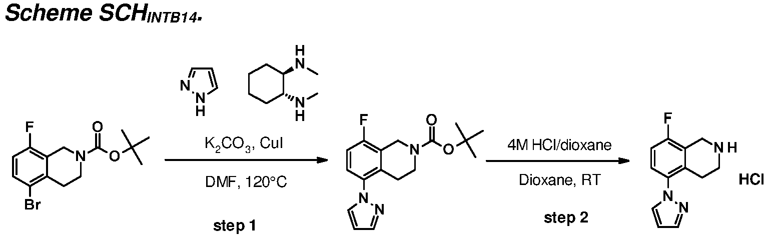

**Step 1:** A mixture of 1H-pyrazole (206 mg, 3.02 mmol) tert-butyl 5-bromo-8-fluoro-3,4-dihydroisoquinoline-2(1H)-carboxylate (500 mg, 1.51 mmol), potassium carbonate (627 mg, 4.54 mmol), trans-(1R,2R)-*N*,*N*'-bismethyl-1,2-cyclohexanediamine (239 µL, 1.51 mmol) in DMF (7 mL) was stirred under argon for 5 min then copper(I) iodide (288 mg, 1.51 mmol) was added. The reaction mixture was stirred at 120°C for 48 h then filtered over a pad of Celite. Water and ethyl acetate were added. After separation, the aqueous layer was extracted with ethyl acetate. The combined organic layers were washed with brine then dried over anhydrous sodium sulfate, filtered and evaporated under reduced pressure. The residue was purified by silica gel column chromatography eluted with cyclohexane/Ethyl acetate from 100/0 to 50/50 to afford *tert*-butyl 8-fluoro-5-(1H-pyrazol-1-yl)-3,4-dihydroisoquinoline-2(1H)-carboxylate (366 mg, 51 % yield) as a brown oil. LCMS (ES, m/z): 318.2 [M+H]⁺.

**Step 2:** To a solution of *tert*-butyl 8-fluoro-5-(1H-pyrazol-1-yl)-3,4-dihydroisoquinoline-2(1H)-carboxylate (366 mg, 1.15 mmol) in dioxane (2 mL) was added 4M HCl in dioxane (4.32 mL, 17.3 mmol). The reaction mixture was stirred at room temperature for 16 h. The solvent was evaporated under reduced pressure then diethyl ether was added. The resulting solid was filtered and dried to afford 8-fluoro-5-(1H-pyrazol-1-yl)-1,2,3,4-tetrahydroisoquinoline hydrochloride **(Intermediate INT_{B-14})** (293 mg, 100 % yield) as a brown solid. LCMS (ES, m/z): 218.1 [M+H]⁺.

### Intermediate INT_{B-15}: 2-(8-fluoro-1,2,3,4-tetrahydroisoquinolin-5-yl)oxazole hydrochloride (Intermediate INT_{B-15})

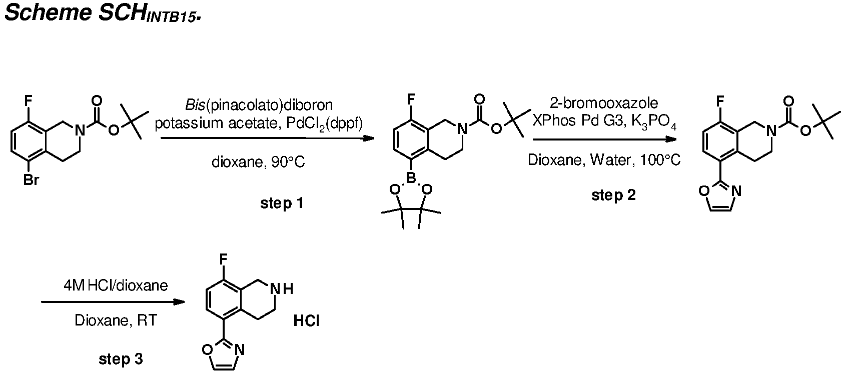

**Step 1:** To a solution of *tert*-butyl 5-bromo-8-fluoro-3,4-dihydroisoquinoline-2(1H)-carboxylate (365 mg, 1.10 mmol) in dioxane (6 mL) was added potassium acetate (207 µL, 3.31 mmol) and Bis(pinacolato)diboron (421 mg, 1.65 mmol). This reaction mixture was degassed with argon for 10 min then PdCl₂(dppf) (40.4 mg, 55.2 µmol) was added and the reaction mixture was stirred at 90°C for 18 h under argon. The reaction mixture was filtered over a Celite pad and water and ethyl acetate were added. After separation, the aqueous layer was extracted with ethyl acetate. The combined organic layers were washed with brine, dried over anhydrous sodium sulfate, filtered and concentrated under reduced pressure to afford *tert*-butyl 8-fluoro-5-(4,4,5,5-tetramethyl-1,3,2-dioxaborolan-2-yl)-3,4-dihydroisoquinoline-2(1H)-carboxylate (637 mg, Qt. yield) as a dark oil. LCMS (ES, m/z): 378.2 [M+H]⁺.

**Step 2:** In a sealed tube, *tert*-butyl 8-fluoro-5-(4,4,5,5-tetramethyl-1,3,2-dioxaborolan-2-yl)-3,4-dihydroisoquinoline-2(1H)-carboxylate (525 mg, 1.39 mmol), 2-bromooxazole (308 mg, 2.08 mmol) and K₃PO₄ (345.6 µL, 4.17 mmol) in dioxane (10 mL) and water (3 mL) was stirred under argon for 10 min then XPhos Pd G3 (58.8 mg, 69.5 µmol) was added. The reaction mixture was stirred at 100°C for 16 h. Water and Ethyl acetate were added. The layers were separated and the organic phase was washed with brine, dried over anhydrous sodium sulfate, filtered and concentrated under reduced pressure. The residue was purified by silica gel column chromatography eluted with cyclohexane/Ethyl acetate from 100/0 to 50/50 to afford *tert*-butyl 8-fluoro-5-(oxazol-2-yl)-3,4-dihydroisoquinoline-2(1H)-carboxylate (295 mg, 63 % yield) as a white solid. LCMS (ES, m/z): 319.2 [M+H]⁺.

**Step 3:** To a solution of *tert*-butyl 8-fluoro-5-(oxazol-2-yl)-3,4-dihydroisoquinoline-2(1H)-carboxylate (295 mg, 927 µmol) in dioxane (1 mL) was added 4M HCl in dioxane (2.32 mL, 9.27 mmol). The reaction mixture was stirred at room temperature for 16 h. The solvent was evaporated under reduced pressure and diethyl ether was added. The resulting solid was filtered and dried to afford 2-(8-fluoro-1,2,3,4-tetrahydroisoquinolin-5-yl)oxazole hydrochloride **(Intermediate INT_{B-15})** (250 mg, Qt. yield) as a brown solid. LCMS (ES, m/z): 219.2 [M+H]⁺.

### Intermediate INT_{B-16}: 8-methyl-5-(1H-pyrazol-1-yl)-1,2,3,4-tetrahydroisoquinoline hydrochloride (Intermediate INT_{B-16})

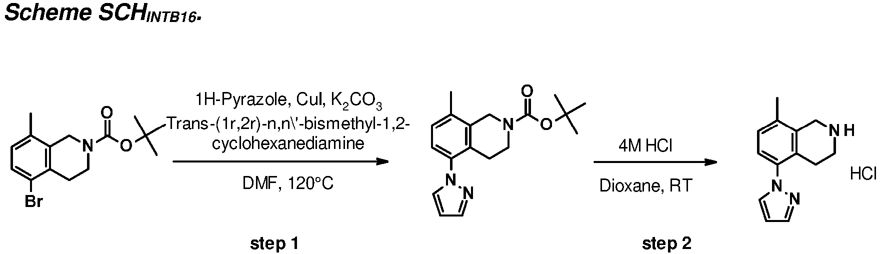

**Step 1:** A mixture of 1H-pyrazole (417 mg, 6.13 mmol) tert-butyl 5-bromo-8-methyl-3,4-dihydroisoquinoline-2(1H)-carboxylate (1.00 g, 3.06 mmol), potassium carbonate (1.27 g, 9.19 mmol), *Trans*-(1R,2R)-*N*,*N*'-bismethyl-1,2-cyclohexanediamine (483 µL, 3.06 mmol) in DMF (8 mL) was degassed under argon for 5 min then copper(I) iodide (583 mg, 3.06 mmol) was added. The reaction mixture was stirred at 120 °C for 48 h. The reaction mixture was filtered over a pad of Celite. Water and Ethyl acetate were added. After separation, the aqueous layer was extracted with Ethyl acetate. The combined organic layers were washed with brine, dried over anhydrous sodium sulfate, filtered and evaporated under reduced pressure. The residue was purified by silica gel column chromatography eluted with cyclohexane/Ethyl acetate from 100/0 to 50/50 to afford *tert*-butyl 8-methyl-5-(1H-pyrazol-1-yl)-3,4-dihydroisoquinoline-2(1H)-carboxylate (870 mg, 90 % yield) as a colorless oil. LCMS (ES, m/z): 314.2 [M+H]⁺.

**Step 2:** To a solution of *tert*-butyl 8-methyl-5-(1H-pyrazol-1-yl)-3,4-dihydroisoquinoline-2(1H)-carboxylate (870 mg, 2.77 mmol) in dioxane (5 mL) was added 4M hydrogen chloride in dioxane (6.94 mL, 27.7 mmol). The reaction mixture was stirred at room temperature for 16 h. The solvent was evaporated under reduced pressure then diethyl ether was added. The resulting solid was filtered and dried to afford 8-methyl-5-(1H-pyrazol-1-yl)-1,2,3,4-tetrahydroisoquinoline hydrochloride **(Intermediate INT_{B-16})** (618 mg, 89 % yield) as a white solid. LCMS (ES, m/z): 214.3 [M+H]⁺.

### Intermediate INT _{B-17}: tert-butyl 8-amino-5-(1H-pyrazol-1-yl)-3,4-dihydroisoquinoline-2(1H)-carboxylate (Intermediate INT_{B-17})

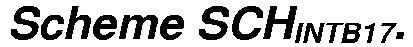

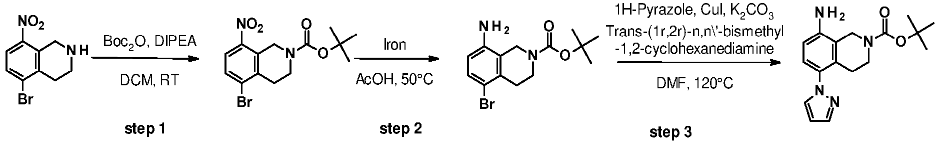

**Step 1:** To a mixture of 5-bromo-8-nitro-1,2,3,4-tetrahydroisoquinoline (1.50 g, 5.83 mmol) in DCM (20 mL) was added diisopropylethylamine (3.0 mL, 17.50 mmol) and Boc₂O (1.61 mL, 7.00 mmol). The reaction mixture was stirred at room temperature for 16 h. The reaction mixture was quenched with water and extracted with Ethyl acetate. The organic phase was dried over anhydrous magnesium sulfate, filtered, evaporated under reduced pressure. The residue was purified by silica gel column chromatography eluted with cyclohexane/Ethyl acetate from 100/0 to 80/20 to afford *tert*-butyl 5-bromo-8-nitro-3,4-dihydroisoquinoline-2(1H)-carboxylate (1.67 g, 80 % yield) as a yellow solid. LCMS (ES, m/z): 357.1-359.1 [M+H]⁺.

**Step 2:** To a solution of *tert*-butyl 5-bromo-8-nitro-3,4-dihydroisoquinoline-2(1H)-carboxylate (1.00 g, 2.80 mmol) in acetic Acid (20 mL) was added iron (1.25 g, 22.4 mmol). The reaction mixture was stirred at 50 °C for 6 h. The reaction mixture was filtered on a pad of Celite and the filtrate was concentrated under reduced pressure. Water was added and pH was adjusted to 8 using a solution of NaHCOs then extracted with ethyl acetate. The organic phase was dried over anhydrous sodium sulfate, filtered and evaporated under reduced pressure. The residue was purified by silica gel column chromatography eluted with cyclohexane/ ethyl acetate from 100/0 to 60/40 to afford *tert-*butyl 8-amino-5-bromo-3,4-dihydroisoquinoline-2(1H)-carboxylate (673 mg, 73 % yield) as a pale-yellow solid. LCMS (ES, m/z): 327.1-329.1 [M+H]⁺.

**Step 3:** A mixture of 1H-pyrazole (499 mg, 7.33 mmol) *tert*-butyl 8-amino-5-bromo-3,4-dihydroisoquinoline-2(1H)-carboxylate (600 mg, 1.83 mmol), potassium carbonate (760 mg, 5.50 mmol), *Trans*-(1R,2R)-*N*,*N*'-bismethyl-1,2-cyclohexanediamine (289 µL, 1.83 mmol) in DMF (8 mL) was degassed under argon for 5 min then copper(I) iodide (349 mg, 1.83 mmol) was added and the mixture was stirred at 120 °C for 16 h. The reaction mixture was filtered over a pad of Celite. Then, water and ethyl acetate were added. After separation, the aqueous layer was extracted with ethyl acetate. The combined organic layers were washed with brine then dried over anhydrous sodium sulfate, filtered and evaporated under reduced pressure. The residue was purified by silica gel column chromatography eluted with cyclohexane/ethyl acetate from 100/0 to 40/60 to afford *tert-*butyl 8-amino-5-(1H-pyrazol-1-yl)-3,4-dihydroisoquinoline-2(1H)-carboxylate **(Intermediate INT_{B-17})** (430 mg, 74 % yield) as a yellow oil. LCMS (ES, m/z): 315.3 [M+H]⁺.

### Intermediate INT_{B-18}: 8-bromo-5-(1H-pyrazol-1-yl)-1,2,3,4-tetrahydroisoquinoline hydrochloride (Intermediate INT_{B-18})

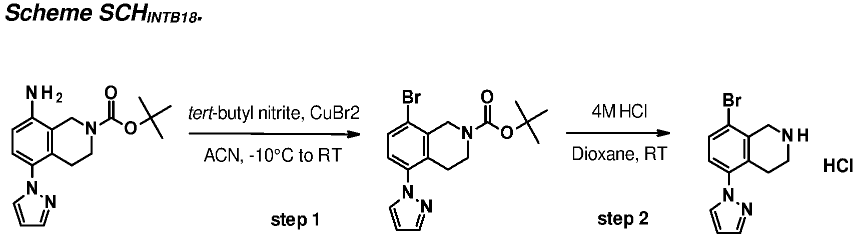

**Step 1:** In a three-necked round bottom flask under N₂ atmosphere, *tert*-butyl 8-amino-5-(1H-pyrazol-1-yl)-3,4-dihydroisoquinoline-2(1H)-carboxylate (400 mg, 1.27 mmol) and copper(ll) bromide (341 mg, 1.53 mmol) were suspended in acetonitrile (10 mL). The reaction mixture was cooled at -10°C then *tert*-butyl nitrite (227 µL, 1.91 mmol) was slowly added over a period of 5 min maintaining the internal temperature at -10°C. At the end of the addition, the reaction mixture was allowed to warm up to room temperature and stirred for 3 h. The reaction mixture was concentrated under reduced pressure and the residue was purified by silica gel column chromatography eluted with cyclohexane/AcOEt from 100/0 to 90/10 to afford *tert*-butyl 8-bromo-5-(1H-pyrazol-1-yl)-3,4-dihydroisoquinoline-2(1H)-carboxylate (162 mg, 33 % yield) as a light brown oil. LCMS (ES, m/z): 378.1-380.1 [M+H]⁺.

**Step 2:** To a solution of *tert*-butyl 8-bromo-5-(1H-pyrazol-1-yl)-3,4-dihydroisoquinoline-2(1H)-carboxylate (160 mg, 423 µmol) in dioxane (2 mL) was added 4M hydrogen chloride in dioxane (1.06 mL, 4.23 mmol). The reaction mixture was stirred at room temperature for 16 h. The solvent was removed under reduced pressure to afford 8-bromo-5-(1H-pyrazol-1-yl)-1,2,3,4-tetrahydroisoquinoline hydrochloride **(Intermediate INT_{B-18})** (150 mg, 100 % yield) as a brown solid. LCMS (ES, m/z): 278.0-280.0 [M+H]⁺.

### Intermediate INT_{B-19}: 8-chloro-5-(1H-pyrazol-1-yl)-1,2,3,4-tetrahydroisoquinoline hydrochloride (Intermediate INT _{B-19})

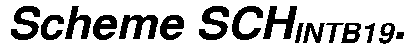

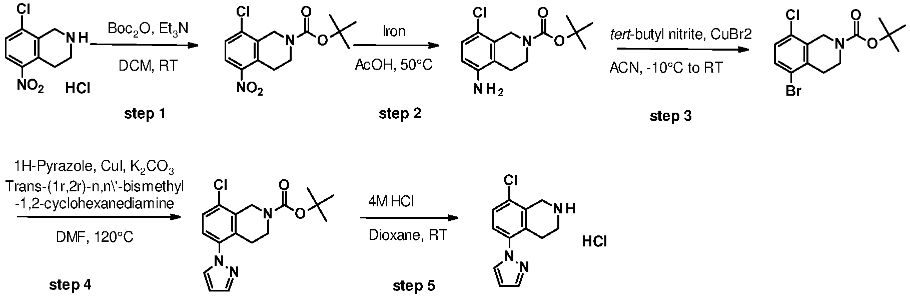

**Step 1:** To a mixture of 8-chloro-5-nitro-1,2,3,4-tetrahydroisoquinoline hydrochloride (1.00 g, 4.01 mmol) in DCM (20 mL) was added Et₃N (2.24 mL16.1 mmol) and Boc₂O (1.11 mL, 4.82 mmol). The reaction mixture was stirred at room temperature for 16 h. The reaction mixture was quenched with water and extracted with ethyl acetate. The organic phase was dried over anhydrous magnesium sulfate, filtered, concentrated under reduced pressure. The residue was purified by silica gel column chromatography eluted with cyclohexane/ethyl acetate from 100/0 to 80/20 to afford *tert*-butyl 8-chloro-5-nitro-3,4-dihydroisoquinoline-2(1H)-carboxylate (1.20 g, 96 % yield) as a yellow solid. LCMS (ES, m/z): 313.1 [M+H]⁺.

**Step 2:** To a solution of *tert*-butyl 8-chloro-5-nitro-3,4-dihydroisoquinoline-2(1H)-carboxylate (1.20 g, 3.84 mmol) in AcOH (20 mL) was added iron (1.71 g, 30.7 mmol) . The reaction mixture was stirred at 50 °C for 6 h. The reaction mixture was filtered over a pad of Celite and the filtrate was concentrated under reduced pressure. Water was added and the pH was adjusted to 8 using a solution of NaHCOs then extracted with ethyl acetate. The solvent was evaporated under reduced pressure and the residue was purified by silica gel column chromatography eluted with cyclohexane/ethyl acetate from 100/0 to 60/40 to afford *tert*-butyl 5-amino-8-chloro-3,4-dihydroisoquinoline-2(1H)-carboxylate (870 mg, 80 % yield) as a pale-yellow solid. LCMS (ES, m/z): 283.1 [M+H]⁺.

**Step 3:** To a suspension of *tert*-butyl 5-amino-8-chloro-3,4-dihydroisoquinoline-2(1H)-carboxylate (780 mg, 2.76 mmol) and copper(II) bromide (739 mg, 3.31 mmol) were suspended in acetonitrile (10 mL) under N₂ atmosphere. The reaction mixture was cooled to -10°C then *tert*-butyl nitrite (492 µL, 4.14 mmol) was slowly added over a period of 5 min maintaining the internal temperature at -10°C. At the end of the addition, the reaction mixture was allowed to warm to room temperature and stirred for 2 h. Water and ethyl acetate were added. After the separation, the organic phase was extracted with ethyl acetate. The organic phase was dried over anhydrous sodium sulfate, filtered and concentrated under reduced pressure. The residue was purified by reverse phase C₁₈ column chromatography eluted with water/ACN 95/5 to 40/60 to afford *tert*-butyl 5-bromo-8-chloro-3,4-dihydro-1H-isoquinoline-2-carboxylate (465 mg, 46 % yield) as a pale brown oil. LCMS (ES, m/z): 346.1-348.1 [M+H]⁺.

**Step 4:** A mixture of 1H-pyrazole (440 mg, 6.46 mmol) *tert*-butyl 5-bromo-8-chloro-3,4-dihydroisoquinoline-2(1H)-carboxylate (560 mg, 1.62 mmol), potassium carbonate (670 mg, 4.85 mmol), *Trans*-(1R,2R)-*N*,*N*'-bismethyl-1,2-cyclohexanediamine (255 µL, 1.62 mmol) in DMF (6 mL) was degassed under argon for 5 min then copper(I) iodide (308 mg, 1.62 mmol) was added and the reaction mixture was stirred at 120 °C for 16 h. The mixture was filtrated over a pad of Celite. Water and ethyl acetate were added. After separation, the aqueous layer was extracted with ethyl acetate. The combined organic layers were washed with brine, dried over anhydrous sodium sulfate, filtered and concentrated under reduced pressure. The residue was purified by silica gel column chromatography eluted with cyclohexane/ethyl acetate from 100/0 to 80/20 to afford *tert-*butyl 8-chloro-5-(1H-pyrazol-1-yl)-3,4-dihydroisoquinoline-2(1H)-carboxylate (183 mg, 33 % yield) as a yellow oil. LCMS (ES, m/z): 334.2 [M+H]⁺.

**Step 5:** To a solution of *tert*-butyl 8-chloro-5-(1H-pyrazol-1-yl)-3,4-dihydroisoquinoline-2(1H)-carboxylate (180 mg, 539 µmol) in dioxane (2 mL) was added 4M hydrogen chloride in dioxane (1.35 mL, 5.39 mmol). The reaction mixture was stirred at room temperature for 16 h. The solvent was removed under reduced pressure to afford 8-chloro-5-(1H-pyrazol-1-yl)-1,2,3,4-tetrahydroisoquinoline hydrochloride **(Intermediate INT_{B-19})** (127 mg, 87 % yield) as a pale-yellow solid. LCMS (ES, m/z): 234.3 [M+H]⁺.

### Intermediate INT_{B-20}: 5-(1H-pyrazol-1-yl)-8-(trifluoromethyl)-1,2,3,4-tetrahydroisoquinoline hydrochloride (Intermediate INT_{B-20})

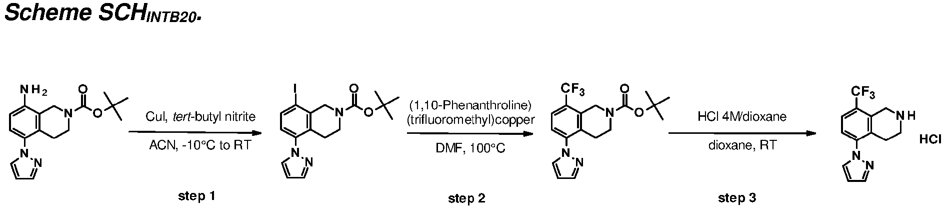

**Step 1:** In a three-necked round bottom flask under N₂ atmosphere at room temperature, *tert*-butyl 8-amino-5-(1H-pyrazol-1-yl)-3,4-dihydroisoquinoline-2(1H)-carboxylate (INT_{B-17}) (810 mg, 2.58 mmol) and *tert*-butyl nitrite (460 µL, 3.86 mmol) were suspended in acetonitrile (10 mL) . The reaction mixture was cooled at -10 °C then CuI (638 mg, 3.35 mmol) was slowly added maintaining the internal temperature at -10 °C. At the end of the addition, the reaction mixture was allowed to warm at room teperature and strirred for 1 h. Water was added followed by an extraction with ethyl acetate. The organic layer was evaporated under reduced pressure. The residue was purified by silica gel column chromatography eluted with cyclohexane/ethyl acetate from 100/0 to 50/50 to afford *tert*-butyl 8-iodo-5-(1H-pyrazol-1-yl)-3,4-dihydroisoquinoline-2(1H)-carboxylate (330 mg, 30 % yield) as a brown oil. LCMS (ES, m/z): 426.2 [M-H]⁺.

**Step 2:** To a mixture of *tert*-butyl 8-iodo-5-(1H-pyrazol-1-yl)-3,4-dihydroisoquinoline-2(1H)-carboxylate (150 mg, 353 µmol) in DMF (1.5 mL) was added (1,10-phenanthroline)(trifluoromethyl) copper (331 mg, 1.06 mmol). The reaction mixture was stirred at 100 °C for 72 h. The solvent was removed under reduced pressure and the residue was purified by silica gel column chromatography eluted with cyclohexane/ethyl acetate 100/0 to 50/50 to afford *tert*-butyl 5-(1H-pyrazol-1-yl)-8-(trifluoromethyl)-3,4-dihydroisoquinoline-2(1H)-carboxylate (120 mg, 92 % yield) as a colorless oil. LCMS (ES, m/z): 366.3 [M-H]⁺.

**Step 3:** To a solution of *tert*-butyl 5-(1H-pyrazol-1-yl)-8-(trifluoromethyl)-3,4-dihydroisoquinoline-2(1H)-carboxylate (120 mg, 327 µmol) in dioxane (2 mL) was added 4M hydrogen chloride solution in dioxane (817 µL, 3.27 mmol). The reaction mixture was stirred at room temperature for 16 h. The solvent was removed under reduced pressure to afford 5-(1H-pyrazol-1-yl)-8-(trifluoromethyl)-1,2,3,4-tetrahydroisoquinoline hydrochloride **(Intermediate INT_{B-20})** (99 mg, 100 % yield) as a pale-yellow solid. LCMS (ES, m/z): 268.1 [M+H]⁺.

### Intermediate INT_{B-21}: 8-isopropoxy-5-(1H-pyrazol-1-yl)-1,2,3,4-tetrahydroisoquinoline hydrochloride (Intermediate INT_{B-21})

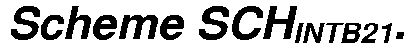

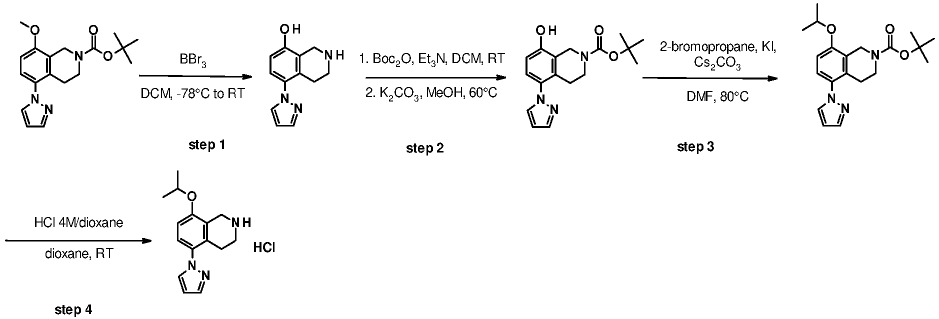

**Step 1:** To a mixture of *tert*-butyl 8-methoxy-5-(1H-pyrazol-1-yl)-3,4-dihydroisoquinoline-2(1H)-carboxylate **(Intermediate INT_{B-1})** (7.7 g, 23.3 mmol) in DCM (80 mL) at -78°C was slowly added BBr₃ (70.1 mL, 70.1 mmol). The reaction mixture was allowed to warm up at room temperature and stirred for 16 h. A saturated NaHCOs aqueous solution was added until pH=8. The aqueous layer was extracted with a mix of DCM/IPA (9/1). The organic layers were combined, washed with brine and dried over anhydrous sodium sulfate, filtered and concentrated under reduced pressure to afford 5-(1H-pyrazol-1-yl)-1,2,3,4-tetrahydroisoquinolin-8-ol (15 g, Qt. yield) as a brown solid which was used without further purification. LCMS (ES, m/z): 216.3 [M+H]⁺.

**Step 2:** To 5-(1H-pyrazol-1-yl)-1,2,3,4-tetrahydroisoquinolin-8-ol (5.07 g, 23.6 mmol) in DCM (50 mL) was added Boc₂O (6.49 mL, 28.3 mmol) and triethylamine (16.4 mL, 118 mmol). The reaction mixture was stirred at room temperature for 16 h. MeOH (10 mL) and potassium carbonate (4.88 g, 35.3 mmol) were added. The reaction mixture was stirred at 60 °C for 24 h. Water and acetic acid were added until pH=5. The aqueous layer was extracted with ethyl acetate. The organic layers were combined, washed with brine and dried over anhydrous sodium sulfate, filtered and concentrated under reduced pressure. The residue was purified by silica gel column chromatography eluted with cyclohexane/ethyl acetate from 100/0 to 70/30 to afford tert-butyl 8-hydroxy-5-(1H-pyrazol-1-yl)-3,4-dihydroisoquinoline-2(1H)-carboxylate (3.94 g, 53 % yield) as a white solid. LCMS (ES, m/z): 316.2 [M+H]⁺.

**Step 3:** To *tert*-butyl 8-hydroxy-5-(1H-pyrazol-1-yl)-3,4-dihydroisoquinoline-2(1H)-carboxylate (253 mg, 802 µmol) in DMF (6 mL) at room temperature was added 2-bromopropane (226 µL, 2.40 mmol), potassium iodide (13.3 mg, 80.2 µmol) and cesium carbonate (1.30 g, 4.01 mmol). The reaction mixture was stirred at 80 °C for 2 h. The reaction mixture was filtered and the filtrate was concentrated under reduced pressure. The residue was purified by silica gel column chromatography eluted with cyclohexane/CPME from 100/0 to 70/30 to afford *tert*-butyl 8-isopropoxy-5-(1H-pyrazol-1-yl)-3,4-dihydroisoquinoline-2(1H)-carboxylate (328 mg, 85 % yield) as a colorless oil. LCMS (ES, m/z): 302.3 [M+H-*t*Bu]⁺.**Step 4:** To a solution of *tert*-butyl 8-isopropoxy-5-(1H-pyrazol-1-yl)-3,4-dihydroisoquinoline-2(1H)-carboxylate (328 mg, 844 µmol) in dioxane (2 mL) was added 4M hydrogen chloride solution in dioxane (2.11 mL, 8.44 mmol). The reaction mixture was stirred at room temperature for 16 h. The solvent was removed under reduced pressure then diethyl ether was added and the resulting precipitate was filtered and dried to afford 8-isopropoxy-5-(1H-pyrazol-1-yl)-1,2,3,4-tetrahydroisoquinoline hydrochloride **(Intermediate INT_{B-21})** (279 mg, 84 % yield) as a yellow solid. LCMS (ES, m/z): 258.3 [M+H]⁺.

### III. Synthesis of isoindoline Intermediates

### Intermediate INT_{B-51}: tert-butyl 5-bromoisoindoline-2-carboxylate (Intermediate INT_{B-51})

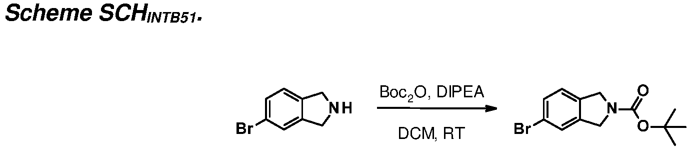

To a mixture of 5-bromoisoindoline (1.0 g, 5.04 mmol) and *N,N-*diisopropylethylamine (2.6 mL, 15.15 mmol) in dichloromethane (20 mL) was added di-tert-butyldicarbonate (1.32 g, 6.05 mmol). The reaction mixture was stirred at room temperature for 3 h. The reaction mixture was quenched with water and extracted with dichloromethane. The organic phase was dried over anhydrous sodium sulfate, filtered and concentrated under reduced pressure. The residue was purified by silica gel column chromatography eluted with cyclohexane/ethyl acetate from 100/0 to 70/30 to afford *tert-*butyl 5-bromoisoindoline-2-carboxylate **(Intermediate INT_{B-51})** (1.22 g, 81 % yield) as a colorless oil. LCMS (ES, m/z): 242.1-244.1 [M+H-*t*Bu]⁺.

### Intermediate INT_{B-52}: tert-butyl 6-bromo-4-fluoroisoindoline-2-carboxylate (Intermediate INT_{B-52})

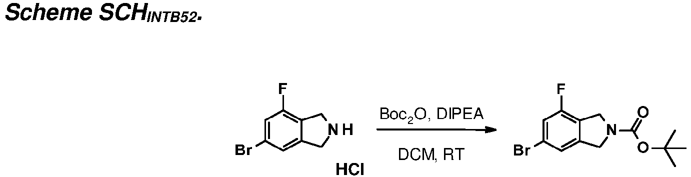

To a mixture of 6-bromo-4-fluoroisoindoline hydrochloride (500 mg, 1.98 mmol) and *N*,*N-*diisopropylethylamine (1.0 mL, 5.94 mmol) in dichloromethane (20 mL) was added di-*tert*-butyl dicarbonate (519 mg, 2.38 mmol). The reaction mixture was stirred at room temperature for 2 h. The reaction mixture was quenched with water and extracted with dichloromethane. The organic phase was dried over anhydrous sodium sulfate, filtered and concentrated under reduced pressure. The residue was purified by silica gel column chromatography eluted with cyclohexane/ethyl acetate from 100/0 to 70/30 to afford *tert*-butyl 6-bromo-4-fluoroisoindoline-2-carboxylate **(Intermediate INT_{B-52})** (660 mg, 100 % yield) as a colorless oil. LCMS (ES, m/z): 259.9-261.9 [M+H-*t*Bu]⁺.

### Intermediate INT_{B-53}: N,N-dimethylisoindolin-5-amine hydrochloride (Intermediate INT_{B-53})

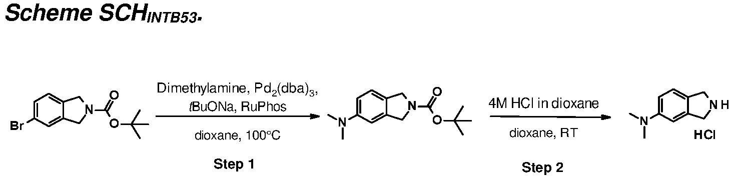

**Step 1:** To a mixture of *tert*-butyl 5-bromoisoindoline-2-carboxylate (INT_{B-51}) (300 mg, 1.01 mmol), sodium *tert*-butoxide (483 mg, 5.03 mmol), RuPhos (46.9 mg, 100 µmol) in dioxane (3 mL) at room temperature under argon was added dimethylamine (2.51 mL, 5.03 mmol) and Pd₂(dba)₃ (46 mg, 50.3 µmol). The reaction mixture was stirred at 100°C for 16 h. The solvent was removed under reduced pressure and the residue was purified by silica gel column chromatography eluted with cyclohexane/ethyl acetate from 100/0 to 70/30 to afford *tert*-butyl 5-(dimethylamino)isoindoline-2-carboxylate (227 mg, 82 % yield) as a white solid. LCMS (ES, m/z): 263.1 [M+H]⁺.

**Step 2:** To a mixture of *tert*-butyl 5-(dimethylamino)isoindoline-2-carboxylate (227 mg, 865 µmol) in dioxane (2 mL) was added a solution of 4M HCl in dioxane (2.16 mL, 8.65 mmol) . The reaction mixture was stirred at room temperature for 16 h. The reaction mixture was concentrated to dryness under reduced pressure to afford *N,N-*dimethylisoindolin-5-amine hydrochloride **(Intermediate INT_{B-53})** (170 mg, 94 % yield) as a brown solid which was used in the next step without any further purification.
LCMS (ES, m/z): 163.2 [M+H]⁺.

### Intermediate INT_{B-54}: 6-cyclopropyl-4-fluoroisoindoline hydrochloride (Intermediate INT_{B-54})

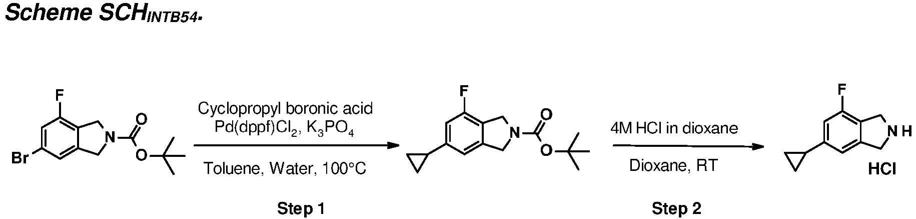

**Step 1:** To a mixture of *tert*-butyl 6-bromo-4-fluoroisoindoline-2-carboxylate (INT_{B-52}) (300 mg, 948 µmol) in toluene (4.7 mL) and water (200 µL) was added cyclopropylboronic acid (407 mg, 4.74 mmol), Pd(dppf)Cl₂ (69.4 mg, 94.8 µmol) and K₃PO₄ (235 µL, 2.84 mmol). The reaction mixture was stirred at 100 °C for 8 h. The solvent was removed under reduced pressure and the residue was purified by silica gel column chromatography eluted with cyclohexane/ethyl acetate from 100/0 to 70/30 to afford *tert*-butyl 6-cyclopropyl-4-fluoroisoindoline-2-carboxylate (140 mg, 51 % yield) as an off-white solid. LCMS (ES, m/z): 278.2 [M+H]⁺.

**Step 2:** To a mixture of *tert*-butyl 6-cyclopropyl-4-fluoroisoindoline-2-carboxylate (140 mg, 504 µmol) in dioxane (3 mL) was added a solution of 4M HCl in dioxane (1.26 mL, 4 molar, 5.04 mmol). The reaction mixture was stirred at room temperature for 16 h. The reaction mixture was concentrated to dryness under reduced pressure to afford 6-cyclopropyl-4-fluoroisoindoline hydrochloride **(Intermediate INT_{B-54})** (105 mg, 97 % yield) as a brown solid which was used in the next step without any further purification. LCMS (ES, m/z): 178.2 [M+H]⁺.

### Intermediate INT_{B-55}: 7-fluoro-N,N-dimethylisoindolin-5-amine hydrochloride (Intermediate INT_{B-55})

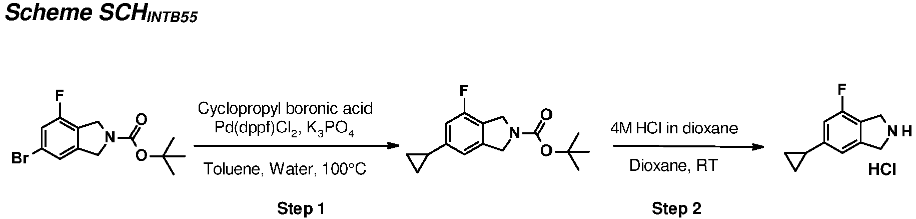

**Step** 1: To a mixture of *tert*-butyl 6-bromo-4-fluoroisoindoline-2-carboxylate (300 mg, 948 µmοl), sodium *tert*-butoxide (455 mg, 4.74 mmol), Pd₂(dba)₃ (43.4 mg, 47.4 µmol) in dioxane (4 mL) at room temperature under argon was added dimethylamine (2.37 mL, 4.74 mmol) and RuPhos (44.2 mg, 94.8 µmol). The reaction mixture was stirred at 100°C for 16 h. The solvent was removed under reduced pressure. The residue was purified by silica gel column chromatography eluted with cyclohexane/ethyl acetate from 100/0 to 70/30 to afford tert-butyl 6-(dimethylamino)-4-fluoroisoindoline-2-carboxylate (209 mg, 75 % yield) as a white solid. LCMS (ES, m/z): 281.2 [M+H]⁺.

**Step 2:** To a mixture of *tert*-butyl 6-(dimethylamino)-4-fluoroisoindoline-2-carboxylate (209 mg, 746 µmol) in dioxane (4 mL) was added a solution 4M HCl in dioxane (1.86 mL, 7.46 mmol). The reaction mixture was stirred at room temperature for 16 h. The reaction mixture was concentrated to dryness under reduced pressure to afford 7-fluoro-*N*,*N*-dimethylisoindolin-5-amine hydrochloride **(Intermediate INT_{B-55})** (134 mg, 82 % yield) as a brown solid which was used in the next step without any further purification. LCMS (ES, m/z): 176.4 [M+H]⁺.
1H NMR (400 MHz, DMSO) δ 10.06 (s, 1H), 6.62 (s, 1H), 6.57 (d, J = 13.0 Hz, 1H), 4.89 (bs, 4H), 2.93 (s, 6H).

### Intermediate INT_{B-56}: 5-(azetidin-1-yl)isoindoline (Intermediate INT_{B-56})

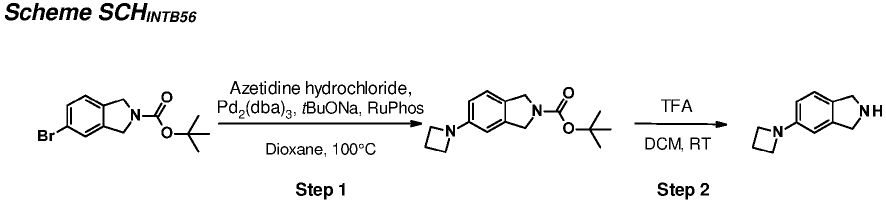

Step 1: To a mixture of tert-butyl 5-bromoisoindoline-2-carboxylate (INT_{B-51}) (400 mg, 1.34 mmol), sodium tert-butoxide (644 mg, 6.70 mmol), RuPhos (62.6 mg, 134 µmol) in dioxane (6 mL) at room temperature under argon was added azetidine hydrochloride (2.34 mL, 4.69 mmol) and Pd2(dba)3 (61.4 mg, 67.1 µmol). The reaction mixture was stirred at 100°C for 16 h. The solvent was removed under reduced pressure and the residue was purified by silica gel column chromatography eluted with cyclohexane/ethyl acetate from 100/0 to 70/30 to afford tert-butyl 5-(azetidin-1-yl)isoindoline-2-carboxylate (113 mg, 30 % yield) as a yellow solid. LCMS (ES, m/z): 275.2 [M+H]+.

Step 2: To a solution of tert-butyl 5-(azetidin-1-yl)isoindoline-2-carboxylate (50 mg, 182 µmol) in DCM (0.5 mL) was added TFA (140 µL, 1.82 mmol). The reaction mixture was stirred at room temperature for 16 h. The reaction mixture was diluted with NaHCOs aqueous solution until pH = 8 and ethyl acetate was added. After separation, the solvent was removed under reduced pressure and the residue was purified by silica gel column chromatography eluted with DCM/MeOH from 95/5 to 90/10 to afford 5-(azetidin-1-yl)isoindoline **(Intermediate INT_{B-56})** (35 mg, 99 % yield) as a pale brown oil. LCMS (ES, m/z): 175.3 [M+H]⁺.

### Intermediate INT_{B-57}: 5-(3-fluoroazetidin-1-yl)isoindoline (Intermediate INT_{B-57})

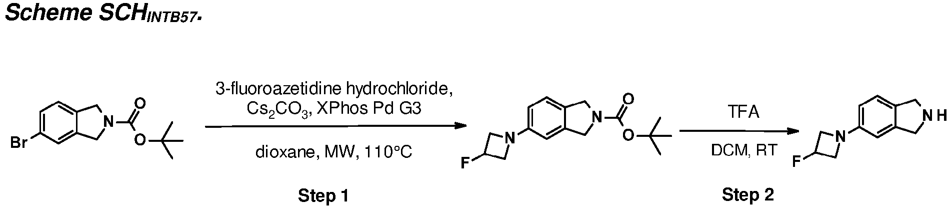

**Step 1:** To a suspension of *tert*-butyl 5-bromoisoindoline-2-carboxylate (INT_{B-51}) (250 mg, 838 µmol), cesium carbonate (2.18 g, 6.70 mmol) in dioxane (4 mL) under argon was added 3-fluoroazetidine hydrochloride (467 mg, 4.19 mmol) and XPhos Pd G3 (70.9 mg, 83.8 µmol). The reaction mixture was sealed and stirred at 110°C for 2 h under MW irradiations. The reaction mixture was concentrated under reduced pressure and the residue was purified by silica gel column chromatography eluted with cyclohexane/(ethyl acetate/ethanol (3:1)) from 100/0 to 60/40 to afford *tert*-butyl 5-(3-fluoroazetidin-1-yl)isoindoline-2-carboxylate (170 mg, 69 % yield) as a brown solid. LCMS (ES, m/z): 293.3 [M+H]⁺.

**Step 2:** To a solution of *tert*-butyl 5-(3-fluoroazetidin-1-yl)isoindoline-2-carboxylate (216 mg, 739 µmol) in DCM (1 mL) was added TFA (569 µL, 7.39 mmol). The reaction mixture was stirred at room temperature for 16 h. The reaction mixture was diluted with NaHCOs aqueous solution until pH = 8 and ethyl acetate was added. After separation, the solvent was removed under reduced pressure and the residue was purified by silica gel column chromatography eluted with dichloromethane/methanol from 95/5 to 90/10 to afford 5-(3-fluoroazetidin-1-yl)isoindoline **(Intermediate INT_{B-57})** (120 mg, 84.5 % yield) as a pale brown oil. LCMS (ES, m/z): 193.1 [M+H]⁺.

### Intermediates INT_{B-1} to INT_{B-21} and INT_{B-51} to INT_{B-57}

The following intermediates from Intermediate **INT_{B-1}** to Intermediate **INT_{B-21}** and Intermediate **INT_{B-51}** to Intermediate **INT_{B-57}** listed in Table 3 were prepared by following similar procedures described above for using appropriate reagents with suitable modifications known to the one skilled in the art.

**Table 3. Intermediates B**

| **Intermediate** | **Structure** |
|---|---|
| **INT_{B-1}** | 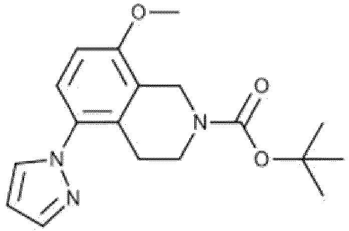 |
| **INT_{B-2}** | 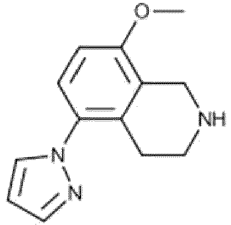 |
| **INT_{B-3}** | 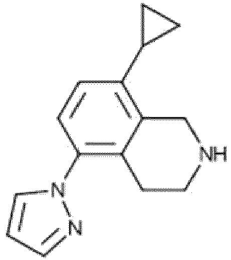 |
| **INT_{B-4}** | 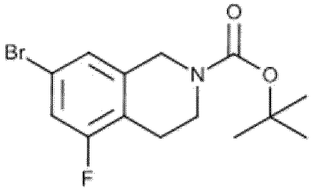 |
| **INT_{B-5}** | 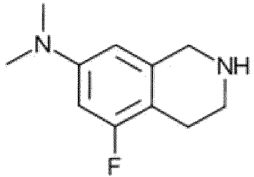 |
| **INT_{B-6}** | 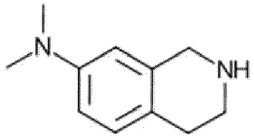 |
| **INT_{B-7}** | 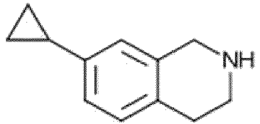 |
| **INT_{B-8}** | 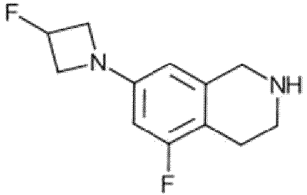 |
| **INT_{B-9}** | 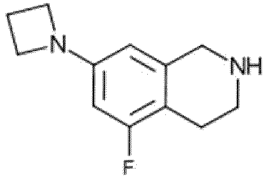 |
| **INT_{B-10}** | 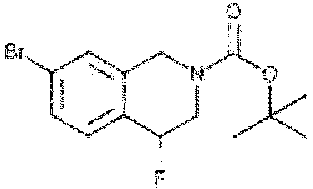 |
| **INT_{B-11}** | 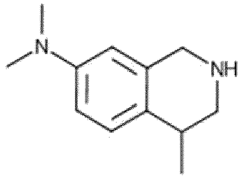 |
| **INT_{B-12}** | 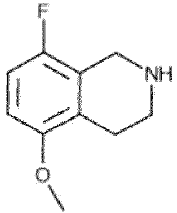 |
| **INT_{B-13}** | 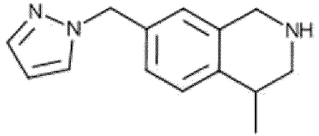 |
| **INT_{B-14}** | 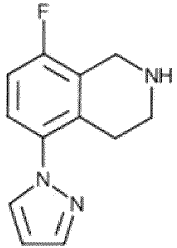 |
| **INT_{B-15}** | 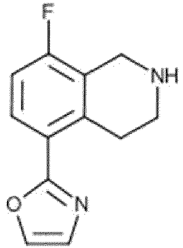 |
| **INT_{B-16}** | 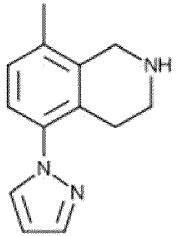 |
| **INT_{B-17}** | 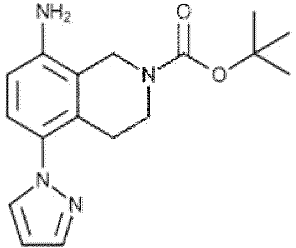 |
| **INT_{B-18}** | 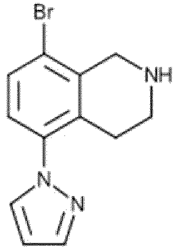 |
| **INT_{B-19}** | 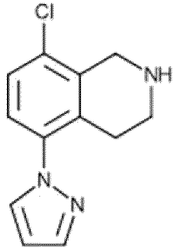 |
| **INT_{B-20}** | 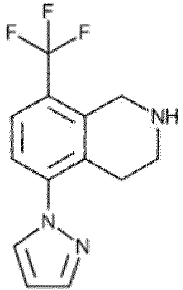 |
| **INT_{B-21}** | 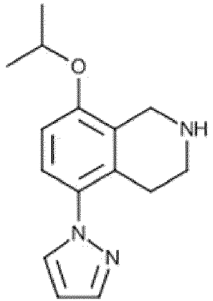 |
| **INT_{B-51}** | 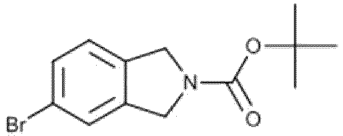 |
| **INT_{B-52}** | 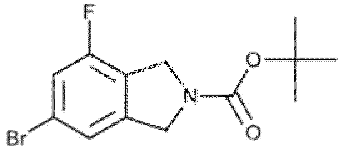 |
| **INT_{B-53}** | 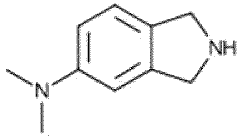 |
| **INT_{B-54}** | 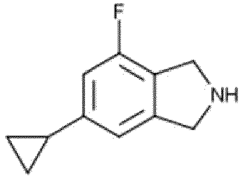 |
| **INT_{B-55}** | 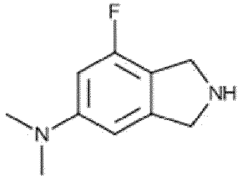 |
| **INT_{B-56}** | 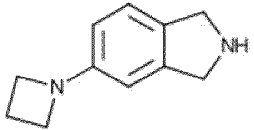 |
| **INT_{B-57}** | 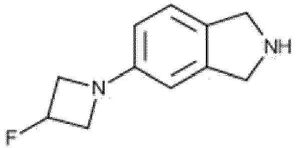 |

Compounds of the present invention can be synthesized following the processes outlined here after.

### Example Compound A - Comparative example: N-((2-ethoxyphenyl)sulfonyl)-3,4-dihydroisoquinoline-2(1H)-carboxamide (Example Compound A - Comparative example)

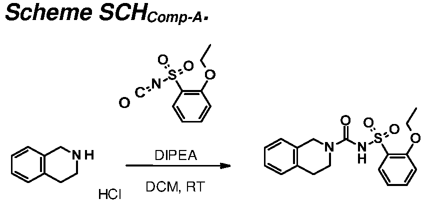

To a mixture of 1,2,3,4-tetrahydroisoquinoline hydrochloride (150 mg, 884 µmol) and DIPEA (1.54 mL, 8.84 mmol) in DCM (2 mL) at 0°C was added slowly a solution of 2-ethoxybenzenesulfonyl isocyanate **(Intermediate INT_{A-7})** (341 mg, 1.50 mmol) in DCM (2 mL). The reaction mixture was stirred at room temperature for 1 h. The reaction mixture was directly purified by silica gel column chromatography eluted with cyclohexane/ethyl acetate from 100/0 to 0/100 to afford *N*-((2-ethoxyphenyl)sulfonyl)-3,4-dihydroisoquinoline-2(1H)-carboxamide **(Example Compound A** - **Comparative example)** (101 mg, 30 % yield) as a white solid.
LCMS (ES, m/z): 361.3 [M+H]⁺.
¹H NMR (400 MHz, DMSO) δ 7.76 (d, J = 8.6 Hz, 1H), 7.45 (s, 1H), 7.19-7.00 (m, 5H), 6.94 (d, J = 22.8 Hz, 1H), 4.52 (s, 2H), 4.09 (dd, J = 13.6, 6.7 Hz, 2H), 3.60 (s, 2H), 2.73 (s, 2H), 1.31-1.15 (m, 3H). *Note:* NH not visible.

### Example EXA₁: 8-fluoro-N-((2-methoxyphenyl)sulfonyl)-5-(1H-pyrazol-1-yl)-3,4-dihydroisoquinoline-2(1H)-carboxamide (Example EXA₁)

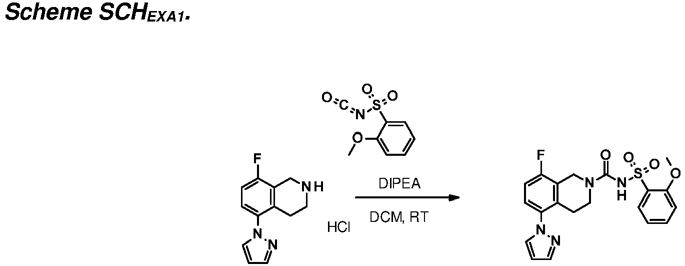

To a mixture of 8-fluoro-5-(1H-pyrazol-1-yl)-1,2,3,4-tetrahydroisoquinoline hydrochloride **(Intermediate INT_{B-14})** (100 mg, 394 µmol) and DIPEA (687 µL, 3.94 mmol) in DCM (2 mL) at room temperature was added dropwise a solution of 2-methoxybenzenesulfonyl isocyanate **(Intermediate INT_{A-6})** (142 mg, 670 µmol). The reaction mixture was stirred at room temperature for 30 min. The reaction mixture was directly absorbed with a Celite pad and concentrated to dryness. The residue was purified by silica gel column chromatography eluted with cyclohexane/acetone from 100/0 to 30/70. The resulting product was triturated in diethyl ether, filtered and dried to afford 8-fluoro-*N-*((2-methoxyphenyl)sulfonyl)-5-(1H-pyrazol-1-yl)-3,4-dihydroisoquinoline-2(1H)-carboxamide **(Example EXA₁)** (63 mg, 35 % yield) as a white solid.
LCMS (ES, m/z): 431.2 [M+H]⁺.
¹H NMR (400 MHz, DMSO) δ 8.00 (1H, d, J=2.3 Hz), 7.77 - 7.74 (1H, m), 7.72 (1H, d, J=1.5 Hz), 7.59 - 7.39 (1H, m), 7.33 - 7.28 (1H, m), 7.21 (1H, t, J=8.9 Hz), 7.13 - 6.97 (2H, m), 6.50 (1H, t, J=2.0 Hz), 4.60 (2H, br s), 3.79 (3H, br s), 3.66 - 3.59 (1H, m), 3.53 (2H, br s), 3.18 - 3.12 (1H, m). *Note:* NH sulfonylurea signal non-visible.

### Example EXA₂: N-((5-(tert-butyl)-2-methoxyphenyl)sulfonyl)-8-fluoro-5-(1H-pyrazol-1-yl)-3,4-dihydroisoquinoline-2(1H)-carboxamide (Example EXA₂)

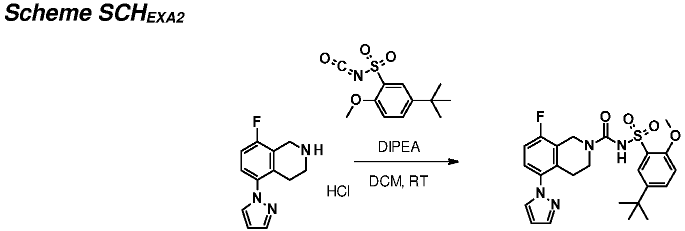

To a mixture of 8-fluoro-5-(1H-pyrazol-1-yl)-1,2,3,4-tetrahydroisoquinoline hydrochloride **(Intermediate INT_{B-14})** (98 mg, 386 µmol) and DIPEA (673 µL, 3.86 mmol) in DCM (2 mL) at room temperature was added dropwise a solution of 5-(tert-butyl)-2-methoxybenzenesulfonyl isocyanate **(Intermediate INT_{A-3})** (176 mg, 656 µmol). The reaction mixture was stirred at room temperature for 30 min. The reaction mixture was directly absorbed with a Celite pad and concentrated to dryness. The residue was purified by silica gel column chromatography eluted with cyclohexane/acetone from 100/0 to 30/70. The resulting product was triturated in diethyl ether, filtered and dried to afford N-((5-(*tert*-butyl)-2-methoxyphenyl)sulfonyl)-8-fluoro-5-(1H-pyrazol-1-yl)-3,4-dihydroisoquinoline-2(1H)-carboxamide **(Example EXA₂)** (20 mg, 10 % yield) as a white solid.
LCMS (ES, m/z): 487.1 [M+H]⁺.
¹H NMR (400 MHz, DMSO) 8.00 (1H, s), 7.75 (1H, d, J=2.3 Hz), 7.72 (1H, d, J=1.5 Hz), 7.61 - 7.39 (1H, m), 7.33 - 7.28 (1H, m), 7.23 - 7.19 (1H, m), 7.10 - 6.93 (1H, m), 6.51 - 6.49 (1H, m), 4.61 (2H, s), 3.76 (3H, s), 3.63 (1H, s), 3.53 (2H, s), 3.14 (1H, s), 1.25 (9H, s). NH sulfonylurea signal not visible.

### Example EXA₃: N-((2,6-dimethoxyphenyl)sulfonyl)-8-fluoro-5-(1H-pyrazol-1-yl)-3,4-dihydroisoquinoline-2(1H)-carboxamide (Example EXA₃)

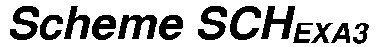

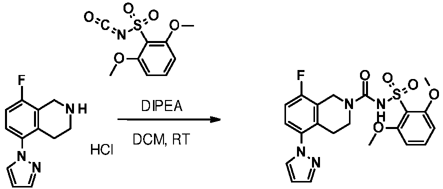

To a mixture of 8-fluoro-5-(1H-pyrazol-1-yl)-1,2,3,4-tetrahydroisoquinoline hydrochloride **(Intermediate INT_{B-14})** (130 mg, 512 µmol) and DIPEA (893 µL, 5.12 mmol) in DCM (2 mL) at room temperature was added dropwise a solution of 2,6-dimethoxybenzenesulfonyl isocyanate **(Intermediate INT_{A-1})** (211 mg, 871 µmol). The reaction mixture was stirred at room temperature for 30 min. The reaction mixture was directly absorbed with a Celite pad and concentrated to dryness. The residue was purified by silica gel column chromatography eluted with cyclohexane/acetone from 100/0 to 50/50 to afford *N*-((2,6-dimethoxyphenyl)sulfonyl)-8-fluoro-5-(1H-pyrazol-1-yl)-3,4-dihydroisoquinoline-2(1H)-carboxamide **(Example EXA₃)** (8 mg, 3 % yield) as a white solid.
LCMS (ES, m/z): 461.0 [M+H]⁺.
¹H NMR (400 MHz, DMSO) δ 10.81 (1H, br s), 8.03 (1H, dd, J=0.7, 2.3 Hz), 7.74 (1H, dd, J=0.6, 1.7 Hz), 7.47 (1H, t, J=8.4 Hz), 7.35 (1H, dd, J=5.3, 8.7 Hz), 7.25 (1H, t, J=9.0 Hz), 6.77 (2H, d, J=8.5 Hz), 6.52 - 6.50 (1H, m), 4.63 (2H, br s), 3.78 (6H, s), 3.59 - 3.56 (2H, m), 2.60 (2H, t, J=5.7 Hz).

### Example EXA₄: N-((5-chloro-2-methoxyphenyl)sulfonyl)-8-fluoro-5-(1H-pyrazol-1-yl)-3,4-dihydroisoquinoline-2(1H)-carboxamide (Example EXA₄)

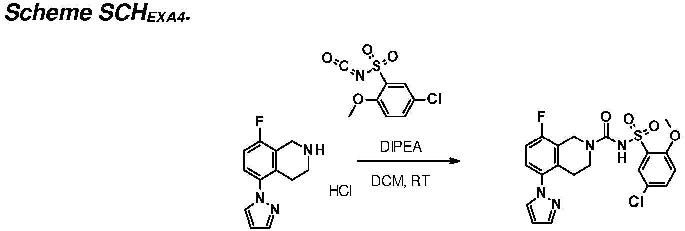

To a mixture of 8-fluoro-5-(1H-pyrazol-1-yl)-1,2,3,4-tetrahydroisoquinoline hydrochloride **(Intermediate INT_{B-14})** (90 mg, 333 µmol) and DIPEA (580 µL, 3.33 mmol) in DCM (1.5 mL) at room temperature was added dropwise a solution of 5-chloro-2-methoxybenzenesulfonyl isocyanate **(Intermediate INT_{A-4})** (140 mg, 566 µmol). The reaction mixture was stirred at room temperature for 30 min. The reaction mixture was directly absorbed with a pad of Celite and concentrated to dryness. The residue was purified by silica gel column chromatography eluted with cyclohexane/acetone from 100/0 to 40/60 to afford *N*-((5-chloro-2-methoxyphenyl)sulfonyl)-8-fluoro-5-(1H-pyrazol-1-yl)-3,4-dihydroisoquinoline-2(1H)-carboxamide **(Example EXA₄)** (46 mg, 28 % yield) as a white solid.
LCMS (ES, m/z): 465.1 [M+H]⁺.
¹H NMR (400 MHz, DMSO) δ 7.98 (1H, d, J=2.3 Hz), 7.72 - 7.69 (2H, m), 7.40 (1H, br s), 7.30 - 7.25 (1H, m), 7.19 (1H, t, J=8.9 Hz), 7.02 (1H, br s), 6.49 (1H, t, J=2.1 Hz), 4.60 (2H, br s), 3.73 (3H, br s), 3.50 (2H, br s). *Note:* one CH₂ under DMSO signal and NH sulfonamide non-visible.

### Example EXA₅: 8-cyclopropyl-N-((5-(3-fluoroazetidin-1-yl)-2-methoxyphenyl)sulfonyl)-5-(1H-pyrazol-1-yl)-3,4-dihydroisoquinoline-2(1H)-carboxamide (Example EXA₅)

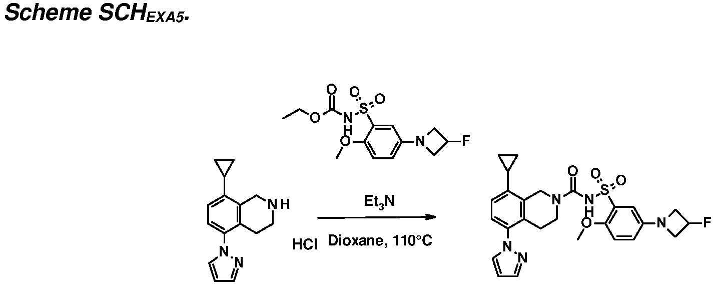

To a mixture of ethyl ((5-(3-fluoroazetidin-1-yl)-2-methoxyphenyl)sulfonyl)carbamate **(Intermediate INT_{A-12})** (130 mg, 360 µmol) and triethylamine (181 µL, 1.08 mmol) in dioxane (3.60 mL) at room temperature was added 8-cyclopropyl-5-(1H-pyrazol-1-yl)-1,2,3,4-tetrahydroisoquinoline hydrochloride **(Intermediate INT_{B-3})** (129 mg, 468 µmol). The reaction mixture was stirred at 110°C for 16 h. The residue was purified by silica gel column chromatography eluted with cyclohexane/acetone from 100/0 to 50/50 to afford 8-cyclopropyl-*N*-((5-(3-fluoroazetidin-1-yl)-2-methoxyphenyl)sulfonyl)-5-(1H-pyrazol-1-yl)-3,4-dihydroisoquinoline-2(1H)-carboxamide **(Example EXA₅)** (26 mg, 13 % yield) as a beige solid.
LCMS (ES, m/z): 526.2 [M+H]⁺
¹H NMR (400 MHz, CDCl₃) δ 7.72 - 7.70 (1H, m), 7.56 - 7.54 (1H, m), 7.19 - 7.15 (2H, m), 7.06 - 6.93 (2H, m), 6.67 - 6.62 (1H, m), 6.46 - 6.44 (1H, m), 5.52 - 5.46 (0.5H, m), 5.38 - 5.32 (0.5H, m), 4.78 (2H, s), 4.25 - 4.16 (2H, m), 4.02 - 3.99 (2H, m), 3.93 (3H, s), 3.57 (2H, t, J=5.7 Hz), 2.70 - 2.65 (2H, m), 0.95 - 0.89 (2H, m), 0.64 (2H, q, J=5.2 Hz).
Note : NH not visible

### Example EXA₆: 8-chloro-N-((5-(3-fluoroazetidin-1-yl)-2-methoxyphenyl)sulfonyl)-5-(1H-pyrazol-1-yl)-3,4-dihydroisoquinoline-2(1H)-carboxamide (Example EXA₆)

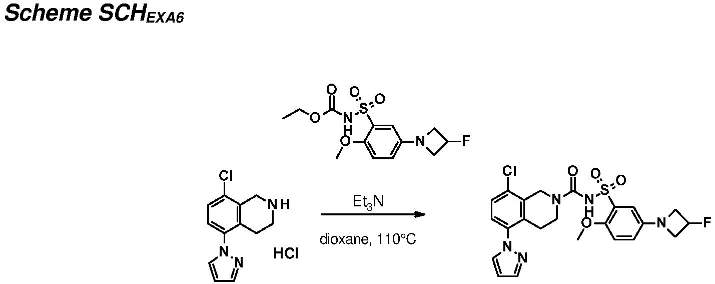

To a mixture of ethyl ((5-(3-fluoroazetidin-1-yl)-2-methoxyphenyl)sulfonyl)carbamate **(Intermediate INT_{A-12})** (260 mg, 721 µmol) and triethylamine (484 µL, 2.89 mmol) in dioxane (7.2 mL) at room temperature was added 8-chloro-5-(1H-pyrazol-1-yl)-1,2,3,4-tetrahydroisoquinoline hydrochloride **(Intermediate INT_{B-19})** (253 mg, 938 µmol). The reaction mixture was stirred at 110°C for 16 h. The solvent was evaporated under reduced pressure and the residue was purified by silica gel column chromatography eluted with cyclohexane/acetone from 100/0 to 70/30 to afford 8-chloro-*N-*((5-(3-fluoroazetidin-1-yl)-2-methoxyphenyl)sulfonyl)-5-(1H-pyrazol-1-yl)-3,4-dihydroisoquinoline-2(1H)-carboxamide **(Example EXA₆)** (15.8 mg, 4.2 % yield) as a grey solid.
LCMS (ES, m/z): 520.3 [M+H]⁺
1H NMR (400 MHz, DMSO) δ 8.01 (d, J = 1.9 Hz, 1H), 7.73 (d, J = 1.4 Hz, 1H), 7.47 (d, J = 8.4 Hz, 1H), 7.29 (d, J = 8.2 Hz, 1H), 6.94 (bs, 1H), 6.88 (d, J = 2.4 Hz, 1H), 6.56 (bs, 1H), 6.51 (t, J = 2.0 Hz, 1H), 5.46 (d, J = 56.3 Hz, 1H), 4.60 (s, 2H), 4.18 - 3.94 (m, 2H), 3.86 - 3.72 (m, 2H), 3.67 (s, 3H), 3.52 (s, 2H), 2.57 - 2.52 (m, 2H).
Note: NH not visible

### Example EXA₇: 8-fluoro-N-((5-(3-fluoroazetidin-1-yl)-2-methoxyphenyl)sulfonyl)-5-(1H-pyrazol-1-yl)-3,4-dihydroisoquinoline-2(1H)-carboxamide (Example EXA₇)

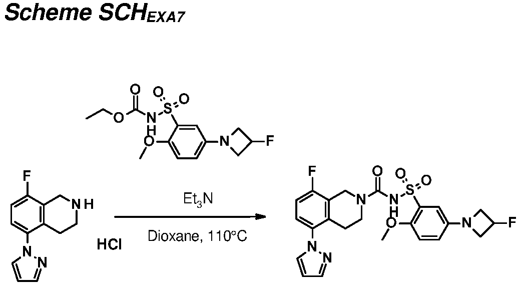

To a mixture of 8-fluoro-5-(1H-pyrazol-1-yl)-1,2,3,4-tetrahydroisoquinoline hydrochloride **(Intermediate INT_{B-14})** (139 mg, 548 µmol) and triethylamine (212 µL, 1.26 mmol) in dioxane (4.2 mL) at room temperature was added ethyl *N*-[5-(3-fluoroazetidin-1-yl)-2-methoxy-phenyl] sulfonylcarbamate **(Intermediate INT_{A-12})** (139 mg, 548 µmol). The reaction mixture was stirred at 110°C for 16 h. The solvent was evaporated under reduced pressure and the residue was purified by silica gel column chromatography eluted with cyclohexane/acetone from 100/0 to 50/50 to afford 8-fluoro-*N-*((5-(3-fluoroazetidin-1-yl)-2-methoxyphenyl)sulfonyl)-5-(1H-pyrazol-1-yl)-3,4-dihydroisoquinoline-2(1H)-carboxamide **(Example EXA₇)** (32 mg, 14 % yield) as a yellow solid.
LCMS (ES, m/z): 504.3 [M+H]⁺
¹H NMR (400 MHz, DMSO) δ 7.91 - 7.89 (1H, m), 7.70 (1H, d, J=1.7 Hz), 7.33 - 7.27 (1H, m), 7.17 (1H, q, J=6.0 Hz), 7.10 (0.3H, d, J=8.9 Hz), 7.02 - 6.99 (0.7H, m), 6.93 - 6.87 (1H, m), 6.71 - 6.62 (1H, m), 6.48 (1H, t, J=2.1 Hz), 5.56 - 5.51 (0.5H, m), 5.41 - 5.37 (0.5H, m), 4.64 (2H, s), 4.20 - 4.08 (2H, m), 3.94 - 3.80 (2H, m), 3.74 (3H, s), 3.58 (2H, t, J=5.8 Hz), 2.62 - 2.57 (2H, m). *Note:* NH not visible.

### Example EXA₈: 8-cyclopropyl-N-((2-methoxy-5-(trifluoromethyl)phenyl)sulfonyl)-5-(1H-pyrazol-1-yl)-3,4-dihydroisoquinoline-2(1H)-carboxamide (Example EXA₈)

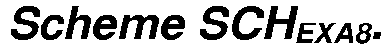

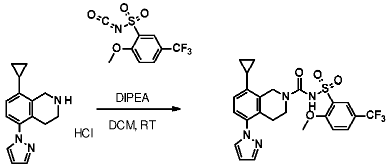

To a mixture of 8-cyclopropyl-5-(1H-pyrazol-1-yl)-1,2,3,4-tetrahydroisoquinoline hydrochloride **(Intermediate INT_{B-3})** (95 mg, 344 µmol) and DIPEA (600 µL, 3.44 mmol) in DCM (2 mL) at room temperature was added dropwise a solution of 2-methoxy-5-(trifluoromethyl)benzenesulfonyl isocyanate **(Intermediate INT_{A-15})** (164 mg, 585 µmol). The reaction mixture was stirred at room temperature for 30 min. The reaction mixture was directly absorbed with a Celite pad and concentrated to dryness under reduced pressure. The residue was purified by silica gel column chromatography eluted with cyclohexane/acetone from 100/0 to 50/50. The resulting solid was triturated in diethyl ether, filtered and dried to afford 8-cyclopropyl-*N*-((2-methoxy-5-(trifluoromethyl)phenyl)sulfonyl)-5-(1H-pyrazol-1-yl)-3,4-dihydroisoquinoline-2(1H)-carboxamide **(Example EXA₈)** (6.9 mg, 3.7 % yield) as a white solid.
LCMS (ES, m/z): 521.3 [M+H]⁺
¹H NMR (400 MHz, DMSO) δ 11.38 (1H, br s), 8.04 (1H, d, J=2.3 Hz), 8.03 - 7.97 (1H, m), 7.96 (1H, d, J=2.3 Hz), 7.70 (1H, d, J=1.3 Hz), 7.43 (1H, br s), 7.16 (1H, d, J=8.1 Hz), 7.02 (1H, d, J=8.5 Hz), 6.48 (1H, t, J=2.0 Hz), 4.72 (2H, br s), 3.97 (3H, br s), 3.54 (2H, br s), 1.90 (1H, br s), 0.98 - 0.96 (2H, m), 0.67 - 0.66 (2H, m).

### Example EXA₉: 8-chloro-N-((2,6-dimethoxyphenyl)sulfonyl)-5-(1H-pyrazol-1-yl)-3,4-dihydroisoquinoline-2(1H)-carboxamide (Example EXA₉)

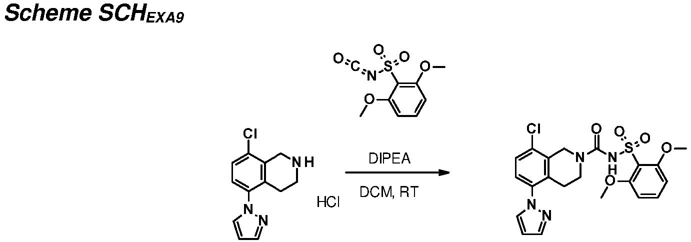

To a mixture of 8-chloro-5-(1H-pyrazol-1-yl)-1,2,3,4-tetrahydroisoquinoline hydrochloride **(Intermediate INT_{B-19})** (127 mg, 470 µmol) and DIPEA (819 µL, 4.70 mmol) in DCM (2 mL) at room temperature was dropwise 2,6-dimethoxybenzenesulfonyl isocyanate **(Intermediate INT_{A-1})** (194 mg, 799 µmol). The reaction mixture was stirred at room temperature for 16 h. The reaction mixture was directly absorbed with a pad of Celite and concentrated to dryness. The residue was purified by silica gel column chromatography eluted with cyclohexane/acetone from 100/0 to 0/100 to afford 8-chloro-*N*-((2,6-dimethoxyphenyl)sulfonyl)-5-(1H-pyrazol-1-yl)-3,4-dihydroisoquinoline-2(1H)-carboxamide **(Example EXA₉)** (15 mg, 6.4 % yield) as a white solid.
LCMS (ES, m/z): 477.3 [M+H]⁺.
¹H NMR (400 MHz, DMSO) δ 10.86 (s, 1H), 8.08 - 8.03 (dd, J = 2.4, 0.5 Hz 1H), 7.76 (dd, J = 1.8, 0.6 Hz, 1H), 7.51 (d, J = 8.5 Hz, 1H), 7.46 (t, J = 8.2 Hz, 1H), 7.34 (d, J = 8.6 Hz, 1H), 6.77 (d, J = 8.5 Hz, 2H), 6.53 (dd, J = 2.4, 1.9 Hz, 1H), 4.61 (s, 2H), 3.78 (s, 6H), 3.57 (t, J = 5.1 Hz, 2H), 2.63 (t, J = 5.7 Hz, 2H).

### Example EXA₁₀: 8-bromo-N-((2,6-dimethoxyphenyl)sulfonyl)-5-(1H-pyrazol-1-yl)-3,4-dihydroisoquinoline-2(1H)-carboxamide (Example EXA₁₀)

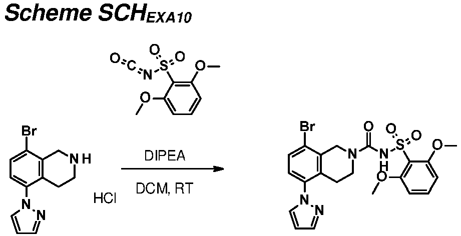

To a mixture of 8-bromo-5-(1H-pyrazol-1-yl)-1,2,3,4-tetrahydroisoquinoline hydrochloride **(Intermediate INT_{B-18})** (150 mg, 477 µmol) and DIPEA (831 µL, 4.77 mmol) in DCM (2 mL) at room temperature was added dropwise of 2,6-dimethoxybenzenesulfonyl isocyanate **(Intermediate INT_{A-1})** (197 mg, 811 µmol). The reaction mixture was stirred at room temperature for 16 h. The reaction mixture was directly absorbed with a Celite pad and concentrated to dryness. The residue was purified by silica gel column chromatography eluted with cyclohexane/acetone from 100/0 to 0/100. The resulting product was triturated with acetone then filtered and washed with dichloromethane, acetonitrile and ethyl acetate to afford 8-bromo-*N*-((2,6-dimethoxyphenyl)sulfonyl)-5-(1H-pyrazol-1-yl)-3,4-dihydroisoquinoline-2(1H)-carboxamide **(Example EXA₁₀)** (24 mg, 9.2 % yield) as a white solid.
LCMS (ES, m/z): 521.2-523.2 [M+H]⁺.
¹H NMR (400 MHz, DMSO) δ 10.89 (s, 1H), 8.06 (d, J = 2.2 Hz, 1H), 7.76 (s, 1H), 7.67 (d, J = 8.6 Hz, 1H), 7.48 (t, J = 8.4 Hz, 1H), 7.27 (d, J = 8.4 Hz, 1H), 6.77 (d, J = 8.5 Hz, 2H), 6.53 (s, 1H), 4.55 (s, 2H), 3.79 (s, 6H), 3.57 (s, 2H), 2.63 (s, 2H).

### Example EXA₁₁: N-((2,6-dimethoxyphenyl)sulfonyl)-5-(1H-pyrazol-1-yl)-8-(trifluoromethyl)-3,4-dihydroisoquinoline-2(1H)-carboxamide (Example EXA₁₁)

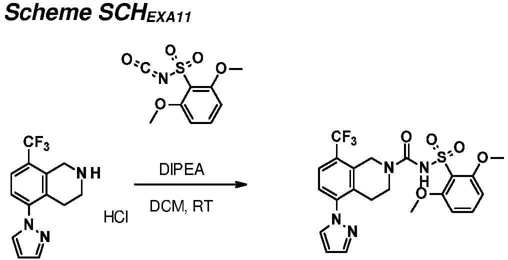

To a mixture of 5-(1H-pyrazol-1-yl)-8-(trifluoromethyl)-1,2,3,4-tetrahydroisoquinoline hydrochloride **(Intermediate INT_{B-20})** (100 mg, 329 µmol) and DIPEA (574 µL, 3.29 mmol) in DCM (2 mL) at room temperature was added dropwise a solution of 2,6-dimethoxybenzenesulfonyl isocyanate **(Intermediate INT_{A-1})** (136 mg, 560 µmol). The reaction mixture was stirred at room temperature for 16 h. The reaction mixture was directly absorbed with a Celite pad and concentrated to dryness under reduced pressure. The residue was purified by silica gel column chromatography eluted with cyclohexane/acetone from 100/0 to 50/50 to afford of *N-*((2,6-dimethoxyphenyl)sulfonyl)-5-(1H-pyrazol-1-yl)-8-(trifluoromethyl)-3,4-dihydroisoquinoline-2(1H)-carboxamide **(Example EXA₁₁)** (9.1 mg, 5.% yield) as a white solid
LCMS (ES, m/z): 511.3 [M+H]⁺
¹H NMR (400 MHz, DMSO) δ 1H NMR (400 MHz, DMSO) δ 10.84 (s, 1H), 8.17 - 8.13 (dd, J = 2.5, 0.6 Hz, 1H), 7.84 - 7.80 (dd, J = 1.8, 0.5 Hz, 1H), 7.78 (d, J = 8.3 Hz, 1H), 7.55 - 7.43 (m, 2H), 6.77 (d, J = 8.5 Hz, 2H), 6.58 (dd, J = 2.4, 1.8 Hz, 1H), 4.77 (s, 2H), 3.78 (s, 6H), 3.63 (t, J = 5.9 Hz, 2H), 2.78 (t, J = 5.9 Hz, 2H).

### Example EXA₁₂: N-((2,6-dimethoxyphenyl)sulfonyl)-8-methyl-5-(1H-pyrazol-1-yl)-3,4-dihydroisoquinoline-2(1H)-carboxamide (Example EXA₁₂)

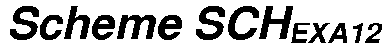

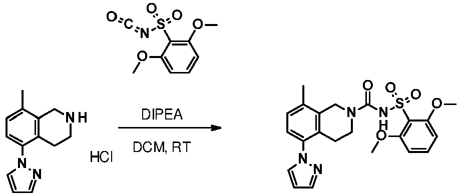

To a mixture of 8-methyl-5-(1H-pyrazol-1-yl)-1,2,3,4-tetrahydroisoquinoline hydrochloride **(Intermediate INT_{B-16})** (100 mg, 400 µmol) and DIPEA (697 µL, 4.00 mmol) in DCM (2 mL) at room temperature was added dropwise a solution of 2,6-dimethoxybenzenesulfonyl isocyanate **(Intermediate INT_{A-1})** (165 mg, 680 µmol). The reaction mixture was stirred at room temperature for 30 min. The reaction mixture was directly absorbed with a pad of Celite and concentrated to dryness. The residue was purified by silica gel column chromatography eluted with cyclohexane/acetone from 100/0 to 50/50 to afford *N*-((2,6-dimethoxyphenyl)sulfonyl)-8-methyl-5-(1H-pyrazol-1-yl)-3,4-dihydroisoquinoline-2(1H)-carboxamide **(Example EXA₁₂)** (27 mg, 14 % yield) as a white solid.
LCMS (ES, m/z): 457.4 [M+H]⁺.
¹H NMR (400 MHz, DMSO) δ 10.73 (1H, s), 7.97 (1H, d, J=1.9 Hz), 7.71 (1H, d, J=0.9 Hz), 7.47 (1H, t, J=8.7 Hz), 7.19 (2H, q, J=7.7 Hz), 6.77 (2H, d, J=8.8 Hz), 6.49 (1H, t, J=2.0 Hz), 4.52 (2H, s), 3.79 (6H, s), 3.54 - 3.51 (2H, m), 2.29 (3H, s).

### Example EXA₁₃: N-((4-chloro-2-methoxyphenyl)sulfonyl)-8-fluoro-5-(1H-pyrazol-1-yl)-3,4-dihydroisoquinoline-2(1H)-carboxamide (Example EXA₁₃)

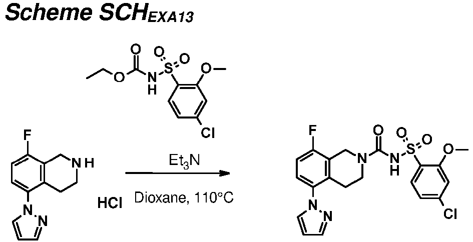

To a mixture of 8-fluoro-5-(1H-pyrazol-1-yl)-1,2,3,4-tetrahydroisoquinoline hydrochloride **(Intermediate INT_{B-14})** (179 mg, 708 µmol) and triethylamine (274 µL, 1.63 mmol) in dioxane (5.4 mL) at room temperature was added ethyl ((4-chloro-2-methoxyphenyl)sulfonyl)carbamate **(Intermediate INT_{A-10})** (160 mg, 544 µmol). The reaction mixture was stirred at 110°C for 16 h. The solvent was evaporated under reduced pressure and the residue was purified by silica gel column chromatography eluting with cyclohexane/acetone from 100/0 to 50/50 to afford *N*-((4-chloro-2-methoxyphenyl)sulfonyl)-8-fluoro-5-(1H-pyrazol-1-yl)-3,4-dihydroisoquinoline-2(1H)-carboxamide **(Example EXA₁₃)** (41 mg, 15 % yield) as a white foam.
LCMS (ES, m/z): 465.2 [M+H]⁺.
¹H NMR (400 MHz, DMSO) δ 11.22 (1H, s), 8.00 (1H, d, J=2.1 Hz), 7.76 (1H, d, J=8.9 Hz), 7.72 (1H, d, J=1.7Hz), 7.32 (1H, dd, J=5.2, 8.6 Hz), 7.22 (2H, t, J=9.1 Hz), 7.11 (1H, s), 6.51 - 6.49 (1H, m), 4.64 - 4.60 (2H, m), 3.85 (3H, s), 3.58 - 3.53 (2H, m), 2.58 - 2.53 (2H, m).

### Example EXA₁₄: 8-chloro-N-((4-methoxy-[1,1'-biphenyl]-3-yl)sulfonyl)-5-(1H-pyrazol-1-yl)-3,4-dihydroisoquinoline-2(1H)-carboxamide (Example EXA₁₄)

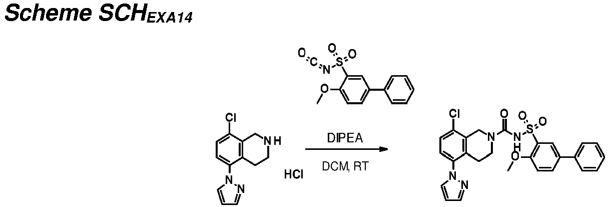

To a mixture of 8-chloro-5-(1H-pyrazol-1-yl)-1,2,3,4-tetrahydroisoquinoline hydrochloride **(Intermediate INT_{B-19})** (130 mg, 394 µmol) and DIPEA (687 µL, 3.94 mmol) in DCM (2 mL) at room temperature was added dropwise a solution of 4-methoxy-[1,1'-biphenyl]-3-sulfonyl isocyanate **(Intermediate INT_{A-19})** (194 mg, 670 µmol). The reaction mixture was stirred at room temperature for 30 min. The solvent was evaporated under reduced pressure. The residue was purified by silica gel column chromatography eluted with cyclohexane/acetone from 100/0 to 50/50 to afford 8-chloro-*N*-((4-methoxy-[1,1'-biphenyl]-3-yl)sulfonyl)-5-(1H-pyrazol-1-yl)-3,4-dihydroisoquinoline-2(1H)-carboxamide **(Example EXA₁₄)** (20 mg, 9.2 % yield) as a white solid.
LCMS (ES, m/z): 523.2 [M+H]⁺
¹H NMR (400 MHz, DMSO) δ 8.04 - 7.99 (2H, m), 7.75 - 7.73 (2H, m), 7.60 - 7.56 (2H, m), 7.49 - 7.43 (3H, m), 7.36 - 7.28 (2H, m), 7.21 - 7.08 (1H, m), 6.52 - 6.50 (1H, m), 4.60 (2H, s), 3.82 (3H, s), 3.53 (2H, s).

### Example EXA₁₅: 8-chloro-N-((4'-fluoro-4-methoxy-[1,1'-biphenyl]-3-yl)sulfonyl)-5-(1H-pyrazol-1-yl)-3,4-dihydroisoquinoline-2(1H)-carboxamide (Example EXA₁₅)

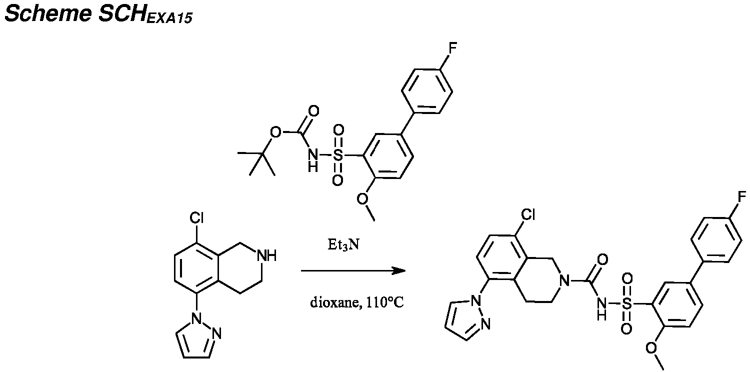

To a mixture of *tert*-butyl ((4'-fluoro-4-methoxy-[1,1'-biphenyl]-3-yl)sulfonyl)carbamate **(Intermediate INT_{A-18})** (150 mg, 393 µmol) and triethylamine (198 µL, 1.18 mmol) in dioxane (2.0 mL) at room temperature was added 8-chloro-5-(1H-pyrazol-1-yl)-1,2,3,4-tetrahydroisoquinoline hydrochloride **(Intermediate INT_{B-19})** (138 mg, 511 µmol). The reaction mixture was stirred at 110°C for 16 h. The crude was purified by silica gel column chromatography eluted with cyclohexane/acetone from 100/0 to 0/100 to afford 8-chloro-*N*-((4'-fluoro-4-methoxy-[1,1'-biphenyl]-3-yl)sulfonyl)-5-(1H-pyrazol-1-yl)-3,4-dihydroisoquinoline-2(1H)-carboxamide **(Example EXA₁₅)** (24 mg, 11 % yield) as a beige foam.
LCMS (ES, m/z): 541.3 [M+H]⁺
¹H NMR (400 MHz, DMSO) δ 11.27 (1H, s), 8.04 (1H, d, J=2.1 Hz), 7.99 (1H, d, J=2.5 Hz), 7.87 - 7.87 (1H, m), 7.75 (1H, dd, J=0.6, 1.9 Hz), 7.66 (2H, dd, J=5.4, 8.6 Hz), 7.50 (1H, d, J=8.4 Hz), 7.35 - 7.27 (4H, m), 6.52 (1H, dd, J=1.8, 2.4 Hz), 4.61 - 4.57 (2H, m), 3.90 (3H, s), 3.60 - 3.55 (2H, m), 2.60 - 2.56 (2H, m).

### Example EXA₁₆: 7-bromo-N-((2-ethoxyphenyl)sulfonyl)-4-methyl-3,4-dihydroisoquinoline-2(1H)-carboxamide (Example EXA₁₆)

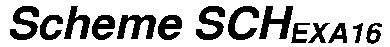

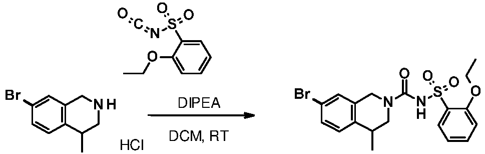

To a mixture of 7-bromo-4-methyl-1,2,3,4-tetrahydroisoquinoline hydrochloride (100 mg, 380 µmol) and DIPEA (663 µL, 3.80 mmol) in DCM (2 mL) at room temperature was added dropwise a solution of 2-ethoxybenzenesulfonyl isocyanate **(Intermediate INT_{A-7})** (129 mg, 571 µmol). The reaction mixture was stirred at room temperature for 30 min. The reaction mixture was directly absorbed with a Celite pad and concentrated to dryness. The residue was purified by silica gel column chromatography eluted with cyclohexane/acetone from 100/0 to 80/20. The resulting solid was triturated in diethyl, filtered and dried to afford 7-bromo-*N*-((2-ethoxyphenyl)sulfonyl)-4-methyl-3,4-dihydroisoquinoline-2(1H)-carboxamide **(Example EXA₁₆)** (74 mg, 41 % yield) as a white solid.
LCMS (ES, m/z): 453.0-455.0 [M+H]⁺.
¹H NMR (400 MHz, DMSO) δ 10.80 (1H, s), 7.79 (1H, dd, J=1.5, 7.9 Hz), 7.59 - 7.53 (1H, m), 7.38 (1H, dd, J=2.1, 8.2 Hz), 7.32 (1H, d, J=2.1 Hz), 7.21 (1H, d, J=8.4 Hz), 7.17 (1H, d, J=8.0 Hz), 7.06 (1H, t, J=7.1 Hz), 4.65 - 4.57 (1H, m), 4.46 (1H, d, J=17.5 Hz), 4.15 (2H, q, J=7.0 Hz), 3.60 (1H, br s), 3.41 (1H, dd, J=6.8, 13.3 Hz), 2.89 (1H, q, J=6.3 Hz), 1.26 (3H, t, J=6.9 Hz), 1.14 (3H, d, J=6.8 Hz).

### Example EXA₁₇: N-((2,6-dimethoxyphenyl)sulfonyl)-8-methoxy-5-(1H-pyrazol-1-yl)-3,4-dihydroisoquinoline-2(1H)-carboxamide (Example EXA₁₇)

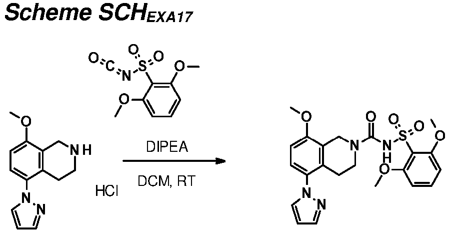

To a mixture of 8-methoxy-5-(1H-pyrazol-1-yl)-1,2,3,4-tetrahydroisoquinoline hydrochloride **(Intermediate INT_{B-2})** (120 mg, 451 µmol) and DIPEA (787 µL, 4.51 mmol) in DCM (2 mL) at room temperature was dropwise a solution of 2,6-dimethoxybenzenesulfonyl isocyanate **(Intermediate INT_{A-1})** (186 mg, 767 µmol). The reaction mixture was stirred at room temperature for 30 min. The reaction mixture was directly absorbed with a Celite pad and concentrated to dryness. The residue was purified by silica gel column chromatography eluted with cyclohexane/acetone from 100/0 to 50/50 to afford *N*-((2,6-dimethoxyphenyl)sulfonyl)-8-methoxy-5-(1H-pyrazol-1-yl)-3,4-dihydroisoquinoline-2(1H)-carboxamide **(Example EXA₁₇)** (21 mg, 9.3 % yield) as a brown solid.
LCMS (ES, m/z): 473.1 [M+H]⁺.
¹H NMR (400 MHz, DMSO) δ 10.71 (1H, br s), 7.93 (1H, dd, J=0.6, 2.3 Hz), 7.69 (1H, dd, J=0.5, 1.8 Hz), 7.46 (1H, t, J=8.5 Hz), 7.25 (1H, d, J=8.7 Hz), 6.99 (1H, d, J=8.8 Hz), 6.75 (2H, d, J=8.5 Hz), 6.47 (1H, t, J=2.1 Hz), 4.48 (2H, br s), 3.88 (3H, s), 3.77 (6H, s), 3.54 - 3.51 (2H, m).

### Example EXA₁₈: 8-cyclopropyl-N-((2,6-dimethoxyphenyl)sulfonyl)-5-(1H-pyrazol-1-yl)-3,4-dihydroisoquinoline-2(1H)-carboxamide (Example EXA₁₈)

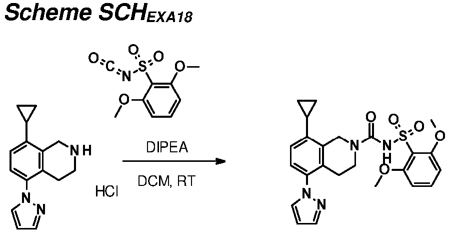

To a mixture of 8-cyclopropyl-5-(1H-pyrazol-1-yl)-1,2,3,4-tetrahydroisoquinoline hydrochloride **(Intermediate INT_{B-3})** (115 mg, 417 µmol) and DIPEA (726 µL, 4.17 mmol) in DCM (2 mL) at room temperature was added dropwise a solution of 2,6-dimethoxybenzenesulfonyl isocyanate **(Intermediate INT_{A-1})** (172 mg, 708 µmol). The reaction mixture was stirred at room temperature for 30 min. The reaction mixture was directly absorbed with a pad of Celite and concentrated to dryness. The residue was purified by silica gel column chromatography eluted with cyclohexane/acetone from 100/0 to 40/60 to afford 8-cyclopropyl-*N*-((2,6-dimethoxyphenyl)sulfonyl)-5-(1H-pyrazol-1-yl)-3,4-dihydro isoquinoline-2(1H)-carboxamide **(Example EXA₁₈)** (24 mg, 11 % yield) as a grey solid.
LCMS (ES, m/z): 483.3 [M+H]⁺.
¹H NMR (400 MHz, DMSO) δ 10.75 (1H, s), 7.97 (1H, dd, J=0.7, 2.4 Hz), 7.71 (1H, dd, J=0.7, 1.8 Hz), 7.48 (1H, t, J=8.4 Hz), 7.17 (1H, d, J=8.1 Hz), 7.03 (1H, d, J=8.5 Hz), 6.78 (2H, d, J=8.6 Hz), 6.49 - 6.48 (1H, m), 4.74 (2H, s), 3.79 (6H, s), 3.58 - 3.54 (2H, m), 2.57 (2H, t, J=5.6 Hz), 1.95 - 1.91 (1H, m), 1.01 - 0.95 (2H, m), 0.70 - 0.65 (2H, m).

### Example EXA₁₉: N-((5-bromo-2-methoxyphenyl)sulfonyl)-8-fluoro-5-(1H-pyrazol-1-yl)-3,4-dihydroisoquinoline-2(1H)-carboxamide (Example EXA₁₉)

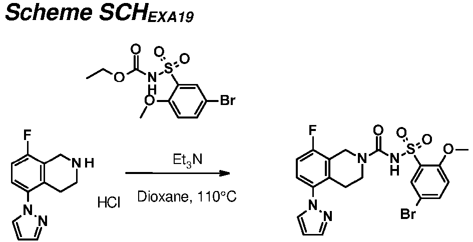

To a mixture of 8-fluoro-5-(1H-pyrazol-1-yl)-1,2,3,4-tetrahydroisoquinoline hydrochloride **(Intermediate INT_{B-14})** (218 mg, 859 µmol) and triethylamine (288 µL, 1.72 mmol) in dioxane (5.7 mL) at room temperature was added ethyl ((5-bromo-2-methoxyphenyl)sulfonyl)carbamate **(Intermediate INT_{A-9})** (220 mg, 572 µmol). The reaction mixture was stirred at 110°C for 16 h. The solvent was evaporated under reduced pressure and the residue was purified by silica gel column chromatography eluting with cyclohexane/acetone from 100/0 to 50/50 to afford *N*-((5-bromo-2-methoxyphenyl)sulfonyl)-8-fluoro-5-(1H-pyrazol-1-yl)-3,4-dihydroisoquinoline-2(1H)-carboxamide **(Example EXA₁₉)** (74 mg, 24 % yield) as a yellow powder.
LCMS (ES, m/z): 511.2 [M+H]⁺.
¹H NMR (400 MHz, DMSO) δ 7.94 - 7.93 (0.2H, m), 7.92 (0.8H, dd, J=0.8, 2.4 Hz), 7.87 (0.8H, d, J=2.5 Hz), 7.83 (0.2H, d, J=2.5 Hz), 7.74 - 7.71 (0.3H, m), 7.70 (0.7H, dd, J=0.6, 1.9 Hz), 7.63 - 7.58 (0.8H, m), 7.40 - 7.35 (0.2H, m), 7.31 - 7.24 (1H, m), 7.22 - 7.14 (1H, m), 7.10 - 7.03 (1H, m), 6.52 - 6.50 (0.2H, m), 6.48 (0.8H, dd, J=1.8, 2.4 Hz), 4.63 (1.6H, s), 4.24 (0.4H, s), 3.93 (0.5H, s), 3.80 (2.5H, s), 3.56 (1.6H, t, J=6.4 Hz), 3.23 - 3.19 (0.4H, m), 2.78 - 2.72 (0.4H, m), 2.56 (1.6H, t, J=6.6 Hz). *Note :* NH not visible

### Example EXA₂₀: N-((5-(tert-butyl)-2-methoxyphenyl)sulfonyl)-8-methoxy-5-(1H-pyrazol-1-yl)-3,4-dihydroisoquinoline-2(1H)-carboxamide (Example EXA₂₀)

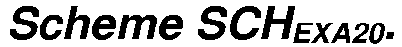

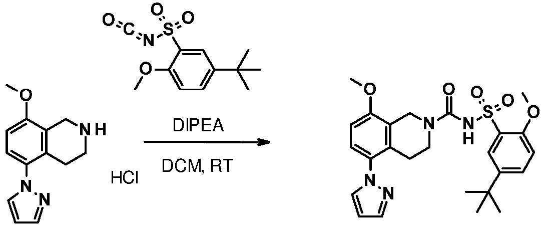

To a mixture of 8-methoxy-5-(1H-pyrazol-1-yl)-1,2,3,4-tetrahydroisoquinoline hydrochloride **(Intermediate INT_{B-2})** (135 mg, 508 µmol) and DIPEA (885 µL, 5.08 mmol) in DCM (2 mL) at room temperature was added dropwise a solution of 5-(*tert*-butyl)-2-methoxybenzenesulfonyl isocyanate **(Intermediate INT_{A-3})** (232 mg, 863 µmol). The reaction mixture was stirred at room temperature for 30 min. The reaction mixture was directly absorbed with a Celite pad and concentrated to dryness. The residue was purified by silica gel column chromatography eluted with cyclohexane/acetone from 100/0 to 50/50 to afford *N-*((5-(*tert*-butyl)-2-methoxyphenyl)sulfonyl)-8-methoxy-5-(1H-pyrazol-1-yl)-3,4-dihydroisoquinoline-2(1H)-carboxamide **(Example EXA₂₀)** (81 mg, 30 % yield) as a white solid.
LCMS (ES, m/z): 499.2 [M+H]⁺.
¹H NMR (400 MHz, DMSO) δ 11.01 (1H, s), 7.92 (1H, d, J=2.1 Hz), 7.75 (1H, d, J=2.5 Hz), 7.69 (1H, d, J=1.3 Hz), 7.66 - 7.63 (1H, m), 7.25 (1H, d, J=8.7 Hz), 7.13 (1H, d, J=8.7 Hz), 6.99 (1H, d, J=9.0 Hz), 6.47 (1H, t, J=2.1 Hz), 4.45 (2H, br s), 3.87 (3H, s), 3.83 (3H, s), 3.52 (2H, br s), 1.28 (9H, s). 2 protons under DMSO signal.

### Example EXA₂₁: 8-fluoro-N-((5-fluoro-2-methoxyphenyl)sulfonyl)-5-(1H-pyrazol-1-yl)-3,4-dihydroisoquinoline-2(1H)-carboxamide (Example EXA₂₁)

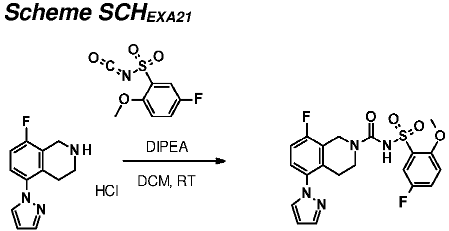

To a mixture of 8-fluoro-5-(1H-pyrazol-1-yl)-1,2,3,4-tetrahydroisoquinoline hydrochloride **(Intermediate INT_{B-14})** (90 mg, 354 µmol) and DIPEA (618 µL, 3.54 mmol) in DCM (1.5 mL) at room temperature was added dropwise a solution of 5-fluoro-2-methoxybenzenesulfonyl isocyanate **(Intermediate INT_{A-5})** (139 mg, 603 µmol). The reaction mixture was stirred at room temperature for 30 min. The reaction mixture was directly absorbed with a pad of Celite and concentrated to dryness. The residue was purified by silica gel column chromatography eluted with cyclohexane/acetone from 100/0 to 40/60. The resulting product was triturated in diethyl ether, filtered and dried to afford 8-fluoro-*N*-((5-fluoro-2-methoxyphenyl)sulfonyl)-5-(1H-pyrazol-1-yl)-3,4-dihydroisoquinoline-2(1H)-carboxamide **(Example EXA₂₁)** (43 mg, 26 % yield) as a white solid.
LCMS (ES, m/z): 449.1 [M+H]⁺.
¹H NMR (400 MHz, DMSO) δ 7.98 (1H, d, J=1.9 Hz), 7.71 (1H, d, J=1.3 Hz), 7.51 - 7.47 (1H, m), 7.31 - 7.26 (1H, m), 7.22 - 7.17 (2H, m), 7.02 (1H, br s), 6.49 (1H, t, J=2.1 Hz), 4.61 (2H, br s), 3.71 (3H, br s), 3.51 (2H, br s). *Note:* one CH₂ under DMSO signal and NH sulfonamide non-visible.

### Example EXA₂₂: N-((3-(dimethylamino)phenyl)sulfonyl)-8-fluoro-5-(1H-pyrazol-1-yl)-3,4-dihydroisoquinoline-2(1H)-carboxamide (Example EXA₂₂)

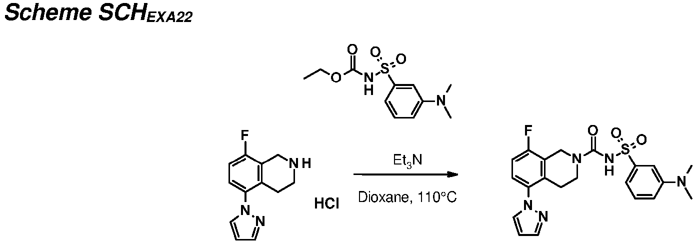

To a mixture of 8-fluoro-5-(1H-pyrazol-1-yl)-1,2,3,4-tetrahydroisoquinoline hydrochloride **(Intermediate INT_{B-14})** (383 mg, 1.51 mmol) and triethylamine (554 µL, 3.30 mmol) in dioxane (11 mL) at room temperature was added ethyl ((3-(dimethylamino)phenyl)sulfonyl)carbamate **(Intermediate INT_{A-11})** (300 mg, 1.10 mmol). The reaction mixture was stirred at 110°C for 16 h. The solvent was evaporated under reduced pressure and the residue was purified by silica gel column chromatography eluted with cyclohexane/acetone from 100/0 to 0/100 to afford N-((3-(dimethylamino)phenyl)sulfonyl)-8-fluoro-5-(1H-pyrazol-1-yl)-3,4-dihydroisoquinoline-2(1H)-carboxamide **(Example EXA₂₂)** (28 mg, 5.4 % yield) as a beige solid.
LCMS (ES, m/z): 444.3 [M+H]⁺.
¹H NMR (400 MHz, DMSO) δ 7.90 (1H, d, J=1.9 Hz), 7.69 (1H, dd, J=0.6, 1.9 Hz), 7.27 (1H, dd, J=6.1, 8.3 Hz), 7.21 - 7.10 (4H, m), 6.76 (1H, dd, J=2.0, 8.3 Hz), 6.47 (1H, dd, J=2.1, 2.1 Hz), 4.65 (2H, s), 3.57 - 3.54 (2H, m), 2.90 (6H, s), 2.56 - 2.53 (2H, m). *Note:* NH sulfonamide not visible

### Example EXA₂₃: N-((2,6-dimethoxyphenyl)sulfonyl)-8-isopropoxy-5-(1H-pyrazol-1-yl)-3,4-dihydroisoquinoline-2(1H)-carboxamide (Example EXA₂₃)

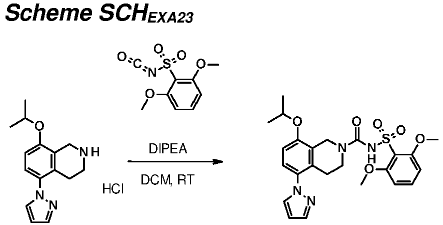

To a mixture of 8-isopropoxy-5-(1H-pyrazol-1-yl)-1,2,3,4-tetrahydroisoquinoline hydrochloride **(Intermediate INT_{B-21})** (120 mg, 408 µmol) and DIPEA (711 µL, 4.08 mmol) in DCM (2 mL) at room temperature was added dropwise a solution of 2,6-dimethoxybenzenesulfonyl isocyanate **(Intermediate INT_{A-1})** (168 mg, 694 µmol). The reaction mixture was stirred at room temperature for 30 min. The reaction mixture was directly absorbed with a Celite pad and concentrated to dryness under reduced pressure. The residue was purified by silica gel column chromatography eluted with cyclohexane/acetone from 100/0 to 50/50. The resulting solid was triturated in diethyl ether, filtered and dried to afford *N*-((2,6-dimethoxyphenyl)sulfonyl)-8-isopropoxy-5-(1H-pyrazol-1-yl)-3,4-dihydro isoquinoline-2(1H)-carboxamide **(Example EXA₂₃)** (34 mg, 16 % yield) as a white solid.
LCMS (ES, m/z): 501.4 [M+H]⁺
¹H NMR (400 MHz, DMSO) δ 10.73 (1H, s), 7.93 (1H, d, J=1.7 Hz), 7.68 (1H, d, J=1.3 Hz), 7.49 - 7.42 (1H, m), 7.21 (1H, d, J=8.4 Hz), 7.00 (1H, d, J=8.6 Hz), 6.77 - 6.75 (2H, m), 6.47 (1H, t, J=2.1 Hz), 4.75 - 4.68 (1H, m), 4.43 (2H, s), 3.78 (6H, s), 3.55 - 3.51 (2H, m), 1.32 (6H, d, J=6.1 Hz).

### Example EXA₂₄: N-((4-bromo-2-methoxyphenyl)sulfonyl)-8-fluoro-5-(1H-pyrazol-1-yl)-3,4-dihydroisoquinoline-2(1H)-carboxamide (Example EXA₂₄)

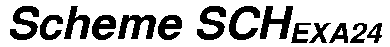

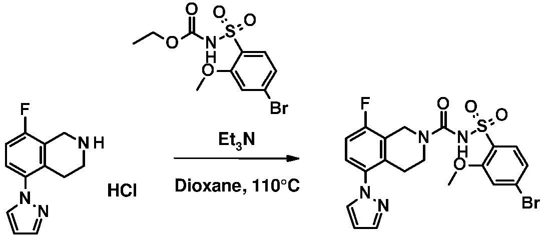

To a mixture of ethyl ((4-bromo-2-methoxyphenyl)sulfonyl)carbamate **(Intermediate INT_{A-13})** (130 mg, 384 µmol) and triethylamine (193 µL, 1.15 mmol) in 1,4-dioxane (3.84 mL) at room temperature was added 8-fluoro-5-(1H-pyrazol-1-yl)-1,2,3,4-tetrahydroisoquinoline hydrochloride **(Intermediate INT_{B-14})** (130 mg, 512 µmol). The reaction mixture was stirred at 110°C for 16 h. The reaction mixture was purified by silica gel column chromatography eluted with cyclohexane/acetone from 100/0 to 50/ 50 to afford *N*-((4-bromo-2-methoxyphenyl)sulfonyl)-8-fluoro-5-(1H-pyrazol-1-yl)-3,4-dihydroisoquinoline-2(1H)-carboxamide **(Example EXA₂₄)** (10.6 mg, 5.1 % yield) as a yellow solid.
LCMS (ES, m/z): 509.2-511.2 [M+H]⁺
¹H NMR (400 MHz, DMSO) δ 7.91 (1H, d, J=1.7 Hz), 7.72 - 7.69 (2H, m), 7.30 - 7.27 (2H, m), 7.21 - 7.13 (2H, m), 6.48 (1H, t, J=2.1 Hz), 4.63 (1.6H, s), 4.13 (0.4H, s), 3.96 (0.4H, s), 3.83 (2.6H, s), 3.56 - 3.55 (1.6H, m), 3.10 (0.4H, s), 2.66 (0.4H, s), 2.59 - 2.55 (1.6H, m).
Note: NH not visible.

### Example EXA₂₅: 7-(dimethylamino)-N-((2-ethoxyphenyl)sulfonyl)-5-fluoro-3,4-dihydro isoquinoline-2(1H)-carboxamide (Example EXA₂₅)

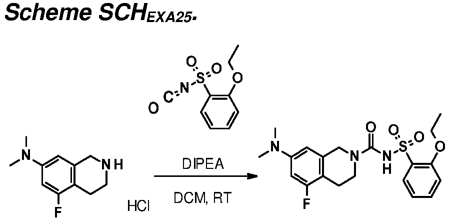

To a mixture of 5-fluoro-*N,N*-dimethyl-1,2,3,4-tetrahydroisoquinolin-7-amine hydrochloride **(Intermediate INT_{B-5})** (90 mg, 390 µmol) and DIPEA (680 µL, 3.901 mmol) in DCM (2 mL) at room temperature was added dropwise a solution of 2-ethoxybenzenesulfonyl isocyanate **(Intermediate INT_{A-7})** (150 mg, 663 µmol). The reaction mixture was stirred at room temperature for 30 min. The reaction mixture was directly absorbed with Celite pad and concentrated to dryness then purified by silica gel column chromatography eluted with cyclohexane/ethyl acetate from 100/0 to 0/100. The resulting product was triturated in diethyl ether, filtered and dried to afford 7-(dimethylamino)-*N-*((2-ethoxyphenyl)sulfonyl)-5-fluoro-3,4-dihydroisoquinoline-2(1H)-carboxamide **(Example EXA₂₅)** (88 mg, 51 % yield) as a brown solid.
LCMS (ES, m/z): 422.2 [M+H]⁺.
¹H NMR (400 MHz, DMSO) δ 7.75 (1H, d, J=7.6 Hz), 7.44 (1H, br s), 7.07 (1H, br s), 6.97 (1H, br s), 6.38 (1H, dd, J=1.9, 13.1 Hz), 6.28 (1H, s), 4.48 (2H, br s), 4.12 - 4.03 (2H, m), 3.60 (2H, br s), 2.85 (6H, s), 2.54 (2H, br s), 1.25 (3H, t, J=6.4 Hz). *Note:* NH proton non-visible.

### Example EXA₂₆: N-((2,6-dimethoxyphenyl)sulfonyl)-7-(dimethylamino)-5-fluoro-3,4-dihydro isoquinoline-2(1H)-carboxamide (Example EXA₂₆)

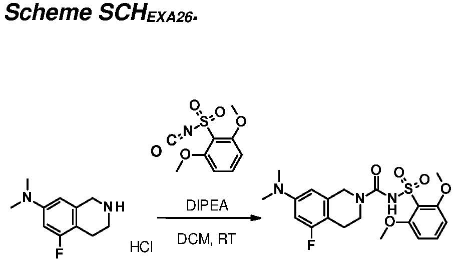

To a mixture of 5-fluoro-*N,N*-dimethyl-1,2,3,4-tetrahydroisoquinolin-7-amine hydrochloride **(Intermediate INT_{B-5})** (100mg, 433 µmol) and DIPEA (755 µL, 4.33 mmol) in DCM (2 mL) at room temperature was added slowly a solution of 2,6-dimethoxybenzenesulfonyl isocyanate **(Intermediate INT_{A-1})** (179.2 mg, 736.8 µmol). The reaction mixture was stirred at room temperature for 30 min. The reaction mixture was directly absorbed with a Celite pad and concentrated to dryness then purified by silica gel column chromatography eluted with cyclohexane/ethyl acetate from 100/0 to 0/100 to afford *N*-((2,6-dimethoxyphenyl)sulfonyl)-7-(dimethylamino)-5-fluoro-3,4-dihydroisoquinoline-2(1H)-carboxamide **(Example EXA₂₆)** (20 mg, 10 % yield) as a white solid.
LCMS (ES, m/z): 438.2 [M+H]⁺.
¹H NMR (400 MHz, CDCl₃) δ 8.04 (1H, br s), 7.45 (1H, t, J=8.4 Hz), 6.67 (2H, d, J=8.7 Hz), 6.41 (1H, br s), 6.26 (1H, br s), 4.56 (2H, s), 3.94 (6H, s), 3.65 (2H, t, J=5.9 Hz), 2.94 (6H, s), 2.76 (2H, t, J=6.2 Hz).

### Example EXA₂₇: 7-(dimethylamino)-5-fluoro-N-((5-isopropyl-2-methoxyphenyl)sulfonyl)-3,4-dihydroisoquinoline-2(1H)-carboxamide (Example EXA₂₇)

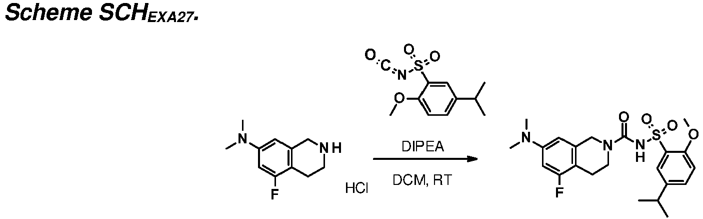

To a mixture of 5-fluoro-*N,N*-dimethyl-1,2,3,4-tetrahydroisoquinolin-7-amine hydrochloride **(Intermediate INT_{B-5})** (90 mg, 390 µmol) and DIPEA (680 µL, 3.901 mmol) in DCM (2 mL) at room temperature was added slowly a solution of 5-isopropyl-2-methoxybenzenesulfonyl isocyanate **(Intermediate INT_{A-2})** (in toluene) (169 mg, 663 µmol). The reaction mixture was stirred at room temperature for 30 min. The reaction mixture was directly absorbed with a Celite pad and concentrated to dryness then purified by silica gel column chromatography eluted with cyclohexane/ethyl acetate from 100/0 to 0/100. The resulting product was triturated in diethyl ether, filtered and dried to afford 7-(dimethylamino)-5-fluoro-*N*-((5-isopropyl-2-methoxyphenyl)sulfonyl)-3,4-dihydroisoquinoline-2(1H)-carboxamide **(Example EXA₂₇)** (5 mg, 3 % yield) as a brown solid.
LCMS (ES, m/z): 432.3 [M+H]⁺.
¹H NMR (400 MHz, CDCl₃) δ 7.93 (1H, br s), 7.43 (1H, br s), 6.95 (1H, br s), 6.31 (1H, d, J=13.4 Hz), 6.14 (1H, br s), 4.55 (2H, br s), 3.92 (3H, br s), 3.63 (2H, br s), 2.94 (1H, br s), 2.90 (6H, br s), 2.74 (2H, br s), 1.29 (6H, br s). *Note:* NH proton non-visible

### Example EXA₂₈: 5-fluoro-7-(3-fluoroazetidin-1-yl)-N-((5-isopropyl-2-methoxyphenyl)sulfonyl)-3,4-dihydroisoquinoline-2(1H)-carboxamide (Example EXA₂₈)

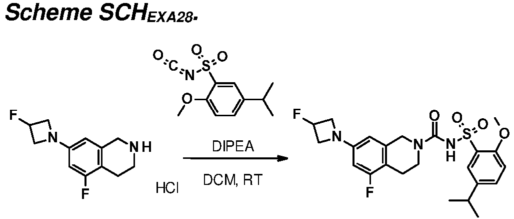

To a mixture of 5-fluoro-7-(3-fluoroazetidin-1-yl)-1,2,3,4-tetrahydroisoquinoline hydrochloride **(Intermediate INT_{B-8})** (100 mg, 383 µmol) and DIPEA (334 µL, 1.91 mmol) in DCM (2 mL) at room temperature was added dropwise a solution of 5-isopropyl-2-methoxybenzenesulfonyl isocyanate **(Intermediate INT_{A-2})** (166 mg, 652 µmol). The reaction mixture was stirred at room temperature for 30 min. The reaction mixture was directly absorbed with a Celite pad and concentrated to dryness. The residue was purified by silica gel column chromatography eluted with cyclohexane/acetone from 100/0 to 50/50. The resulting product was triturated in diethyl ether, filtered and dried to afford 5-fluoro-7-(3-fluoroazetidin-1-yl)-*N*-((5-isopropyl-2-methoxyphenyl)sulfonyl)-3,4-dihydroisoquinoline-2(1H)-carboxamide **(Example EXA₂₈)** (53 mg, 27 % yield) as a white solid. LCMS (ES, m/z): 480.2 [M+H]⁺.
1H NMR (400 MHz, DMSO) δ 10.88 (1H, s), 7.60 (1H, d, J=2.1 Hz), 7.45 (1H, s), 7.09 (1H, s), 6.21 (1H, dd, J=1.9, 11.4 Hz), 6.07 (1H, d, J=1.7 Hz), 5.56 - 5.37 (1H, d sept, J=57.5 Hz, 3.1 Hz), 4.45 (2H, s), 4.16 - 4.06 (2H, m), 3.84 (2H, dt, J=3.0, 11.9 Hz), 3.79 (3H, s), 3.61 (2H, s), 2.94 - 2.87 (1H, m), 2.58 (2H, t, J=6.1 Hz), 1.19 (6H, d, J=7.1 Hz).

### Example EXA₂₉: 7-(azetidin-1-yl)-5-fluoro-N-((5-isopropyl-2-methoxyphenyl)sulfonyl)-3,4-dihydroisoquinoline-2(1H)-carboxamide (Example EXA₂₉)

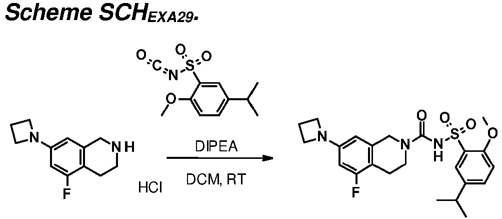

To a mixture of 7-(azetidin-1-yl)-5-fluoro-1,2,3,4-tetrahydroisoquinoline hydrochloride **(Intermediate INT_{B-9})** (120 mg, 494 µmol) and DIPEA (431 µL, 2.47 mmol) in DCM (2 mL) at room temperature was added dropwise a solution of 5-isopropyl-2-methoxybenzenesulfonyl isocyanate **(Intermediate INT_{A-2})** (214 mg, 840 µmol). The reaction mixture was stirred at room temperature for 30 min. The reaction mixture was directly absorbed with a Celite pad and concentrated to dryness. The residue was purified by silica gel column chromatography eluted with cyclohexane/acetone from 100/0 to 70/30. The resulting product was triturated in acetonitrile, filtered and dried to afford 7-(azetidin-1-yl)-5-fluoro-*N*-((5-isopropyl-2-methoxyphenyl)sulfonyl)-3,4-dihydroisoquinoline-2(1H)-carboxamide **(Example EXA₂₉)** (13 mg, 5.4 % yield) as a white solid.
LCMS (ES, m/z): 462.2 [M+H]⁺.
¹H NMR (400 MHz, DMSO) δ 10.87 (1H, s), 7.61 (1H, d, J=2.3 Hz), 7.48 (1H, dd, J=1.9, 8.4 Hz), 7.11 (1H, d, J=8.3 Hz), 6.10 (1H, dd, J=1.7, 11.8 Hz), 5.99 (1H, s), 4.44 (2H, s), 3.80 (3H, s), 3.75 (4H, t, J=7.2 Hz), 3.61 (2H, t, J=5.5 Hz), 2.91 (1H, quint), 2.58 (2H, t, J=5.7 Hz), 2.27 (2H, quint), 1.19 (6H, d, J=6.7 Hz).

### Example EXA₃₀: 7-(dimethylamino)-N-((2-ethoxyphenyl)sulfonyl)-3,4-dihydroisoquinoline-2(1H)-carboxamide (Example EXA₅)

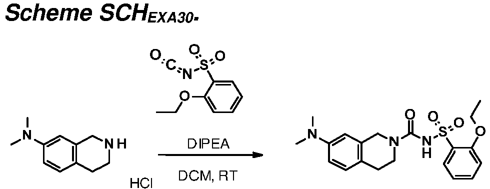

To a mixture of *N,N*-dimethyl-1,2,3,4-tetrahydroisoquinolin-7-amine hydrochloride **(Intermediate INT_{B-6})** (90 mg, 423 µmol) and DIPEA (737 µL, 4.23 mmol) in DCM (2 mL) at room temperature was added dropwise a solution of 2-ethoxybenzenesulfonyl isocyanate **(Intermediate INT_{A-7})** (163 mg, 719 µmol). The reaction mixture was stirred at room temperature for 30 min. The reaction mixture was directly absorbed with a Celite pad and concentrated to dryness. The residue was purified by silica gel column chromatography eluted with cyclohexane/acetone from 100/0 to 50/50. The resulting product was triturated in diethyl ether filtered and dried to afford 7-(dimethylamino)-*N*-((2-ethoxyphenyl)sulfonyl)-3,4-dihydroisoquinoline-2(1H)-carboxamide **(Example EXA₃₀)** (45 mg, 25 % yield) as a brown solid.
LCMS (ES, m/z): 404.2 [M+H]⁺.
¹H NMR (400 MHz, DMSO) δ 10.71 (1H, br s), 7.78 (1H, dd, J=1.7, 7.8 Hz), 7.57 (1H, t, J=7.6 Hz), 7.18 (1H, d, J=8.4 Hz), 7.06 (1H, t, J=7.2 Hz), 6.96 (1H, d, J=8.7 Hz), 6.59 (1H, dd, J=2.5, 8.4 Hz), 6.43 (1H, d, J=2.3 Hz), 4.45 (2H, br s), 4.17 (2H, q, J=7.0 Hz), 3.58 (2H, br s), 2.84 (6H, s), 2.64 (2H, t, J=6.0 Hz), 1.28 (3H, t, J=6.9 Hz).

### Example EXA₃₁: 7- cyclopropyl-N-((2-ethoxyphenyl)sulfonyl)-3,4-dihydroisoquinoline-2(1H)-carboxamide (Example EXA₃₁)

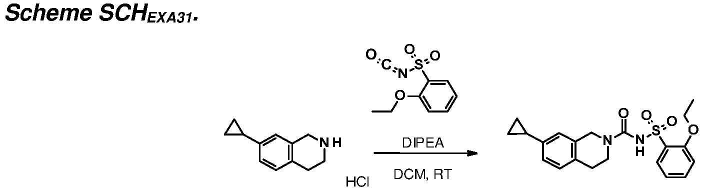

To a mixture of 7-cyclopropyl-1,2,3,4-tetrahydroisoquinoline hydrochloride **(Intermediate INT_{B-7})** (90 mg, 429 µmol) and DIPEA (748 µL, 4.29 mmol) in DCM (2 mL) at room temperature was added dropwise a solution of 2-ethoxybenzenesulfonyl isocyanate **(Intermediate INT_{A-7})** (165 mg, 729 µmol). The reaction mixture was stirred at room temperature for 30 min. The reaction mixture was directly absorbed with a Celite pad and concentrated to dryness. The residue was purified by silica column chromatography eluted with DCM/ethyl acetate from 100/0 to 90/10. The resulting product was triturated in diethyl ether, filtered and dried to afford 7-cyclopropyl-*N*-((2-ethoxyphenyl)sulfonyl)-3,4-dihydroisoquinoline-2(1H)-carboxamide **(Example EXA₃₁)** (75 mg, 41 % yield) as a white solid.
LCMS (ES, m/z): 401.1 [M+H]⁺.
¹H NMR (400 MHz, DMSO) δ 10.77 (1H, s), 7.78 (1H, dd, J=1.7, 7.8 Hz), 7.57 (1H, ddd, J=7.9, 7.9, 1.4 Hz), 7.18 (1H, d, J=8.3 Hz), 7.07 (1H, t, J=7.6 Hz), 7.02 (1H, d, J=7.8 Hz), 6.88 (1H, dd, J=1.8, 7.9 Hz), 6.80 (1H, s), 4.46 (2H, br s), 4.16 (2H, q, J=6.9 Hz), 3.58 (2H, br s), 2.71 (2H, t, J=5.8 Hz), 1.90 - 1.82 (1H, m), 1.27 (3H, t, J=6.9 Hz), 0.93 - 0.87 (2H, m), 0.63 - 0.59 (2H, m).

### Example EXA₃₂: N-((2-ethoxyphenyl)sulfonyl)-4-methyl-3,4-dihydroisoquinoline-2(1H)-carboxamide (Example EXA₃₂)

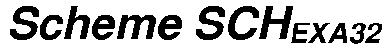

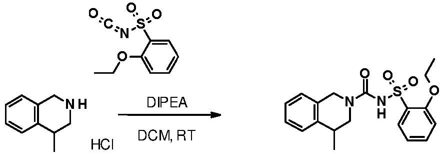

To a mixture of 4-methyl-1,2,3,4-tetrahydroisoquinoline hydrochloride (119 mg, 647 µmol) and DIPEA (564 µL, 3.23 mmol) in DCM (2 mL) at room temperature was added dropwise a solution of 2-ethoxybenzenesulfonyl isocyanate **(Intermediate INT_{A-7})** (220 mg, 971 µmol). The reaction mixture was stirred at room temperature for 30 min. The reaction mixture was directly absorbed with a Celite pad and concentrated to dryness. The residue was purified by silica gel column chromatography eluted with DCM/ethyl acetate from 100/0 to 90/10. The resulting product was triturated in diethyl ether, filtered and dried to afford *N*-((2-ethoxyphenyl)sulfonyl)-4-methyl-3,4-dihydroisoquinoline-2(1H)-carboxamide **(Example EXA₃₂)** (89 mg, 35 % yield) as a white solid.
LCMS (ES, m/z): 375.1 [M+H]⁺.
¹H NMR (400 MHz, DMSO) δ 10.74 (1H, s), 7.80 (1H, dd, J=1.7, 8.0 Hz), 7.60 - 7.54 (1H, m), 7.26 - 7.21 (1H, m), 7.21 - 7.15 (3H, m), 7.11 - 7.04 (2H, m), 4.65 - 4.61 (1H, m), 4.50 - 4.43 (1H, m), 4.19 - 4.12 (2H, m), 3.60 (1H, br s), 3.42 (1H, dd, J=7.1, 13.0 Hz), 2.93 (1H, q, J=6.2 Hz), 1.27 (3H, t, J=7.0 Hz), 1.16 (3H, d, J=6.8 Hz).

### Example EXA₃₃: (R)-N-((2-ethoxyphenyl)sulfonyl)-4-methyl-3,4-dihydroisoquinoline-2(1H)-carboxamide (Example EXA₃₃)

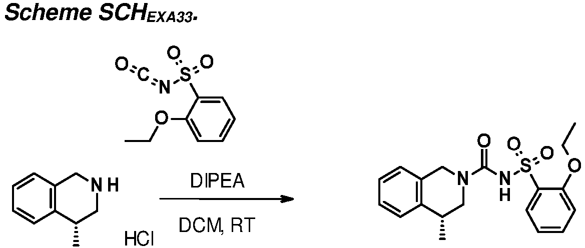

To a mixture of (R)-4-methyl-1,2,3,4-tetrahydroisoquinoline hydrochloride (117 mg, 636 µmol) and DIPEA (555 µL, 3.18 mmol) in DCM (2 mL) at room temperature was added dropwise a solution of 2-ethoxybenzenesulfonyl isocyanate **(Intermediate INT_{A-7})** (246 mg, 1.08 mmol). The reaction mixture was stirred at room temperature for 30 min. The reaction mixture was directly absorbed with a Celite pad and concentrated to dryness. The residue was purified by silica gel column chromatography eluted with cyclohexane/acetone from 100/0 to 50/50 to afford (R)-*N*-((2-ethoxyphenyl)sulfonyl)-4-methyl-3,4-dihydroisoquinoline-2(1H)-carboxamide **(Example EXA₃₃)** (74 mg, 29 % yield) as a white solid.
LCMS (ES, m/z): 375.1 [M+H]⁺.
¹H NMR (400 MHz, DMSO) δ 10.75 (1H, s), 7.79 (1H, dd, J=1.7, 7.8 Hz), 7.57 (1H, t, J=7.6 Hz), 7.26 - 7.21 (1H, m), 7.21 - 7.15 (3H, m), 7.11 - 7.03 (2H, m), 4.65 - 4.61 (1H, m), 4.49 - 4.42 (1H, m), 4.14 (2H, q, J=6.8 Hz), 3.60 (1H, br s), 3.43 (1H, dd, J=6.6, 12.9 Hz), 2.92 (1H, q, J=5.9 Hz), 1.26 (3H, t, J=7.0 Hz), 1.16 (3H, d, J=6.7 Hz).

### Example EXA₃₄: N-((5-(tert-butyl)-2-methoxyphenyl)sulfonyl)-7-(dimethylamino)-4-methyl-3,4-dihydroisoquinoline-2(1H)-carboxamide (Example EXA₃₄)

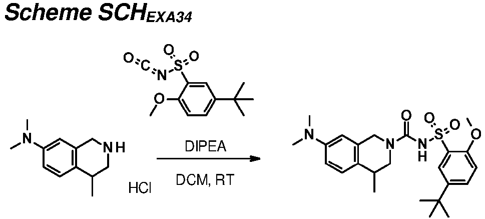

To a mixture of *N,N*,4-trimethyl-1,2,3,4-tetrahydroisoquinolin-7-amine hydrochloride **(Intermediate INT_{B-11})** (100 mg, 441µmol) and DIPEA (768 µL, 4.41 mmol) in DCM (2 mL) at room temperature was added dropwise a solution of 5-(*tert*-butyl)-2-methoxybenzenesulfonyl isocyanate **(Intermediate INT_{A-3})** (201 mg, 749 µmol). The reaction mixture was stirred at room temperature for 30 min. The reaction mixture was directly absorbed with a Celite pad and concentrated to dryness. The residue was purified by silica gel column chromatography eluted with DCM/ethyl acetate from 100/0 to 60/40. The resulting product was triturated in diethyl ether, filtered and dried to afford *N*-((5-(tert-butyl)-2-methoxyphenyl)sulfonyl)-7-(dimethylamino)-4-methyl-3,4-dihydroisoquinoline-2(1H)-carboxamide **(Example EXA₃₄)** (54 mg, 25 % yield) as a white solid.
LCMS (ES, m/z): 460.3 [M+H]⁺.
¹H NMR (400 MHz, DMSO) δ 10.75 (1H, s), 7.75 (1H, d, J=2.5 Hz), 7.63 (1H, dd, J=2.3, 8.9 Hz), 7.08 (2H, dd, J=8.8, 26.5 Hz), 6.61 (1H, dd, J=2.7, 8.5 Hz), 6.42 (1H, d, J=2.7 Hz), 4.55 - 4.50 (1H, m), 4.43 - 4.39 (1H, m), 3.81 (3H, s), 3.62 (1H, br s), 3.32 - 3.29 (1H, m), 2.84 (6H, s), 2.82 - 2.79 (1H, m), 1.28 (9H, s), 1.12 (3H, d, J=6.7 Hz).

### Example EXA₃₅: 8-fluoro-5-methoxy-N-((2-methoxyphenyl)sulfonyl)-3,4-dihydroisoquinoline-2(1H)-carboxamide (Example EXA₃₅)

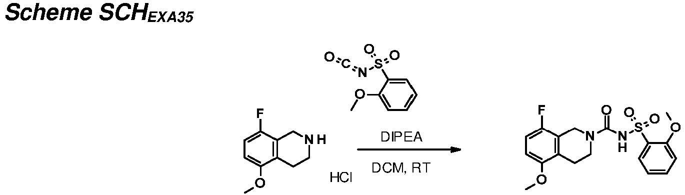

To a mixture of 8-fluoro-5-methoxy-1,2,3,4-tetrahydroisoquinoline hydrochloride **(Intermediate INT_{B-12})** (100 mg, 459 µmol) and DIPEA (800 µL, 4.59 mmol) in DCM (2 mL) at room temperature was added dropwise a solution of 2-methoxybenzenesulfonyl isocyanate **(Intermediate INT_{A-6})** (166 mg, 781 µmol). The reaction mixture was stirred at room temperature for 30 min. The reaction mixture was directly absorbed with a Celite pad and concentrated to dryness. The residue was purified by silica gel column chromatography eluted with DCM/ethyl acetate from 100/0 to 0/100. The resulting product was triturated in diethyl ether, filtered and dried to afford 8-fluoro-5-methoxy-*N*-((2-methoxyphenyl)sulfonyl)-3,4-dihydroisoquinoline-2(1H)-carboxamide **(Example EXA₃₅)** (141 mg, 74 % yied) as a white solid.
LCMS (ES, m/z): 395.1 [M+H]⁺.
¹H NMR (400 MHz, DMSO) δ 11.07 (1H, s), 7.76 (1H, d, J=7.9 Hz), 7.55 (1H, br s), 7.14 (1H, br s), 7.01 (2H, t, J=8.8 Hz), 6.83 (1H, dd, J=4.1, 9.1 Hz), 4.41 (2H, br s), 3.81 (3H, br s), 3.78 (3H, br s), 3.60 (2H, s), 2.65 (2H, br s).

### Example EXA₃₆: 8-fluoro-N-((2-methoxyphenyl)sulfonyl)-5-(oxazol-2-yl)-3,4-dihydro isoquinoline-2(1H)-carboxamide (Example EXA₃₆)

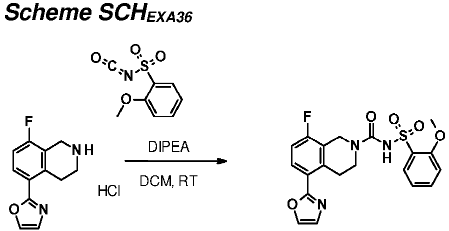

To a mixture of 2-(8-fluoro-1,2,3,4-tetrahydroisoquinolin-5-yl)oxazole hydrochloride **(Intermediate INT_{B-15})** (140 mg, 549 µmol) and DIPEA (958 µL, 5.49 mmol) in DCM (2 mL) at room temperature was added dropwise a solution of 2-methoxybenzenesulfonyl isocyanate **(Intermediate INT_{A-6})** (199 mg, 9 934 µmol). The reaction mixture was stirred at room temperature for 30 min. The reaction mixture was directly absorbed with a Celite pad and concentrated to dryness. The residue was purified by silica gel column chromatography eluted with cyclohexane/acetone from 100/0 to 50/50 to afford 8-fluoro-*N-*((2-methoxyphenyl)sulfonyl)-5-(oxazol-2-yl)-3,4-dihydro isoquinoline-2(1H)-carboxamide **(Example EXA₃₆)** (73 mg, 29 % yield) as a white solid.
LCMS (ES, m/z): 432.1 [M+H]⁺.
¹H NMR (400 MHz, DMSO) δ 11.14 (1H, s), 8.26 (1H, s), 7.90 - 7.86 (1H, m), 7.79 (1H, d, J=6.6 Hz), 7.61 (1H, t, J=7.4 Hz), 7.44 (1H, s), 7.26 (1H, t, J=8.8 Hz), 7.18 (1H, d, J=8.7 Hz), 7.09 (1H, t, J=7.8 Hz), 4.59 (2H, br s), 3.85 (3H, s), 3.66 (2H, br s), 3.21 - 3.16 (2H, m).

### Example EXA₃₇: 7-((1H-pyrazol-1-yl)methyl)-N-((2,6-dimethoxyphenyl)sulfonyl)-4-methyl-3,4-dihydroisoquinoline-2(1H)-carboxamide (Example EXA₃₇)

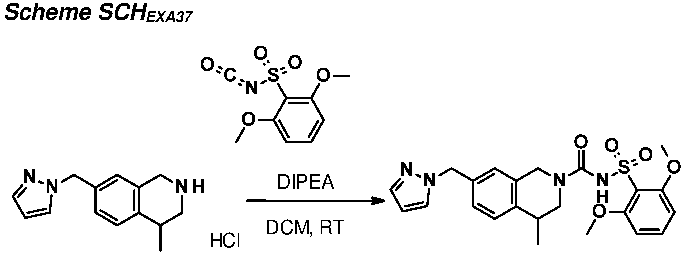

To a mixture of 7-((1H-pyrazol-1-yl)methyl)-4-methyl-1,2,3,4-tetrahydroisoquinoline hydrochloride **(Intermediate INT_{B-13})** (190 mg, 720 µmol) and DIPEA (1 mL, 5.76 mmol) in DCM (1 mL) at room temperature added was slowly a solution of 2,6-dimethoxybenzenesulfonyl isocyanate **(Intermediate INT_{A-1})** (298 mg, 1.22 mmol) in DCM (2 mL). The reaction mixture was stirred at room temperature for 1 h. The reaction mixture was evaporated to dryness and purified by silica gel column chromatography eluted with cyclohexane/acetone from 100/0 to 50/50 to afford 7-((1H-pyrazol-1-yl)methyl)-*N*-((2,6-dimethoxyphenyl)sulfonyl)-4-methyl-3,4-dihydroisoquinoline-2(1H)-carboxamide **(Example EXA₃₇)** (92 mg, 26 % yield) as a white solid. LCMS (ES, m/z): 471.3 [M+H]⁺.
¹H NMR (400 MHz, DMSO) δ 10.55 (1H, s), 7.81 (1H, dd, J=0.7, 2.2 Hz), 7.48 - 7.43 (2H, m), 7.22 (1H, d, J=8.1 Hz), 7.07 (1H, dd, J=1.5, 8.0 Hz), 6.95 (1H, s), 6.74 (2H, d, J=8.6 Hz), 6.26 (1H, t, J=2.1 Hz), 5.28 (2H, s), 4.59 (1H, d, J=16.7 Hz), 4.46 (1H, d, J=16.7 Hz), 3.73 (6H, s), 3.59 (1H, d, J=11.4 Hz), 3.38 (1H, dd, J=6.6, 13.1 Hz), 2.91 (1H, sext, J=6.0 Hz), 1.15 (3H, d, J=6.8 Hz).

### Example EXA₃₈: N-((2,4-dichlorophenyl)sulfonyl)-8-fluoro-5-(1H-pyrazol-1-yl)-3,4-dihydroisoquinoline-2(1H)-carboxamide (Example EXA₃₈)

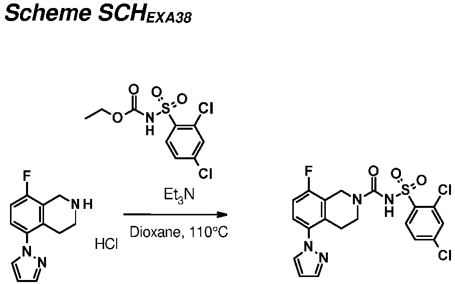

To a mixture of 8-fluoro-5-(1H-pyrazol-1-yl)-1,2,3,4-tetrahydroisoquinoline hydrochloride **(Intermediate INT_{B-14})** (320 mg, 1.26 mmol) and triethylamine (422 µL, 2.52 mmol) in dioxane (8.4 mL) at room temperature was added ethyl ((2,4-dichlorophenyl)sulfonyl)carbamate **(Intermediate INT_{A-8})** (250 mg, 0.839 mmol). The reaction mixture was stirred at 110°C for 16 h. The solvent was evaporated under reduced pressure and the residue was purified by silica gel column chromatography eluting with cyclohexane/acetone from 100/0 to 50/50 to afford *N*-((2,4-dichlorophenyl)sulfonyl)-8-fluoro-5-(1H-pyrazol-1-yl)-3,4-dihydroisoquinoline-2(1H)-carboxamide **(Example EXA₃₈)** (85 mg, 21 % yield) as a yellow solid.
LCMS (ES, m/z): 469.1-471.1 [M+H]⁺.
¹H NMR (400 MHz, DMSO) δ 8.00 (0.3H, d, J= 8.5 Hz), 7.97 (0.7H, d, J= 8.5 Hz), 7.95 (0.2H, dd ,J= 2.4, 0.6 Hz), 7.91 (0.8H, dd, J= 2.4, 0.6 Hz), 7.77 (0.2H, d, J= 2.1 Hz), 7.74 (0.3H, dd, J=1.9, 0.7 Hz), 7.74 (0.7H, dd, J=1.9, 0.6 Hz), 7.61 (0.2H, dd, J= 8.4, 2.1 Hz), 7.51 - 7.49 (0.8H, m), 7.44 - 7.40 (1H, m), 7.32 - 7.24 (1H, m), 7.15 (0.8H, t, J= 9 Hz), 6.52 (0.2H, dd, J=2.4, 1.9 Hz), 6.47 (0.8H, t, J=2.4, 1.9 Hz), 4.61 (1.6H, s), 4.34 (0.4H, s), 3.54 (1.6H, t, J= 6.1 Hz), 3.32 (0.4H, t, J= 6.0 Hz), 2.84 (0.4H, t, J= 6.0 Hz), 2.55 - 2.52 (1.6H, m). *Note:* NH not visible

### Example EXA₃₉: 8-fluoro-5-(1H-pyrazol-1-yl)-N-((3-(pyrrolidin-1-yl)phenyl)sulfonyl)-3,4-dihydroisoquinoline-2(1H)-carboxamide (Example EXA₃₉)

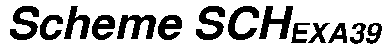

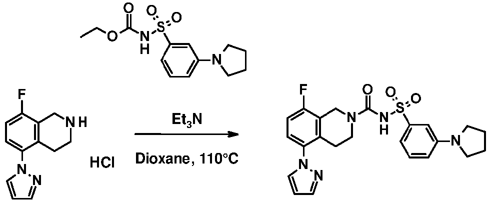

To a mixture of ethyl ((3-(pyrrolidin-1-yl)phenyl)sulfonyl)carbamate **(Intermediate INT_{A-14})** (226 mg, 530 µmol) and triethylamine (267 µL, 1.59 mmol) in 1,4-dioxane (5.3 mL) at room temperature was added 8-fluoro-5-(1H-pyrazol-1-yl)-1,2,3,4-tetrahydroisoquinoline hydrochloride **(Intermediate INT_{B-14})** (250 mg, 985 µmol). The reaction mixture was stirred at 110°C for 16 h. The reaction mixture was purified by silica gel column chromatography eluted with cyclohexane/acetone from 100/0 to 50/50 to afford 8-fluoro-5-(1H-pyrazol-1-yl)-N-((3-(pyrrolidin-1-yl)phenyl)sulfonyl)-3,4-dihydroisoquinoline-2(1H)-carboxamide **(Example EXA₃₉)** (47 mg, 18 %, yield) as a beige powder.
LCMS (ES, m/z): 470.3 [M+H]⁺
¹H NMR (400 MHz, DMSO) δ 7.91 - 7.89 (1H, m), 7.71 - 7.68 (1H, m), 7.31 - 7.00 (5H, m), 6.66 - 6.62 (1H, m), 6.49 - 6.46 (1H, m), 4.64 (1.6H, s), 4.10 (0.4H, s), 3.57 (1.6H, t, J=5.57 Hz), 3.25 - 3.19 (4H, m), 3.09 - 3.03 (0.4H, m), 2.63 (0.4H, t, J=6.2 Hz), 2.57 (1.6H, t, J=6.0 Hz), 2.01 - 1.95 (4H, m).
Note: NH not visible

### Example EXA₄₅: N-((5-(tert-butyl)-2-methoxyphenyl)sulfonyl)-7-(dimethylamino)-5-fluoro-3,4-dihydroisoquinoline-2(1H)-carboxamide (Example EXA₄₅)

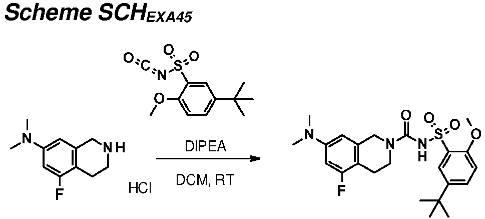

To a mixture of 5-fluoro-*N,N*-dimethyl-1,2,3,4-tetrahydroisoquinolin-7-amine hydrochloride **(Intermediate INT_{B-5})** (94 mg, 0.41 mmol) and DIPEA (0.71 mL, 4.1 mmol) in DCM (1 mL) was added slowly a solution of 5-(*tert*-butyl)-2-methoxybenzenesulfonyl isocyanate **(Intermediate INT_{A-3})** (190 mg, 0.69 mmol) in DCM (2.3 mL). The reaction mixture was stirred at room temperature for 1 h. The reaction mixture was purified by silica gel column chromatography eluted with cyclohexane/ethyl acetate 100/0 to 0/100. The resulting product was triturated in diethyl ether, filtered and dried to afford *N*-((5-(*tert-*butyl)-2-methoxyphenyl)sulfonyl)-7-(dimethylamino)-5-fluoro-3,4-dihydroisoquinoline-2(1H)-carboxamide (60 mg, 30 % yield) as a white solid.
LCMS (ES, m/z): 464.2 [M+H]⁺.
1H NMR (400 MHz, DMSO) δ 10.88 (1H, s), 7.75 - 7.74 (1H, d, J = 2.4 Hz), 7.64 - 7.61 (1H, d, J = 8.8 Hz), 7.12 - 7.10 (1H, d, J = 8.4 Hz), 6.42 - 6.39 (1H, dd, J = 1.6 Hz, J = 12.8 Hz), 6.282 - 6.278 (1H, d, J = 1.6 Hz), 4.46 (2H, s), 3.81 (3H, s), 3.62 (2H, t, J = 5.6 Hz), 2.85 (6H, s), 2.60 - 2.571 (2H, t, J = 12.4 Hz), 1.27 (9H, s).

### Example EXA₄₆: N-((2-ethoxyphenyl)sulfonyl)isoindoline-2-carboxamide (Example EXA₄₂)

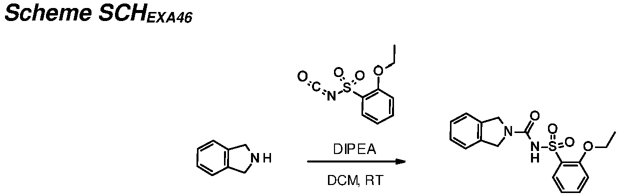

To a mixture of isoindoline (45 mg, 377 µmol) and DIPEA (658 µL, 3.77 mmol) in DCM (2 mL) at 0°C was slowly added a solution of 2-ethoxybenzenesulfonyl isocyanate **(Intermediate INT_{A-7})** (145 mg, 641 µmol) in DCM (2 mL). The reaction mixture was stirred at room temperature for 1 h. The reaction mixture was purified by silica gel column chromatography eluted with dichloromethane/methanol 100/0 to 95/5 to afford N ((2-ethoxyphenyl)sulfonyl)isoindoline-2-carboxamide **(Example EXA₄₆)** (20.2 mg, 15 % yield) as a white powder.
LCMS (ES, m/z): 347.3 [M+H]⁺
1H NMR (400 MHz, DMSO) δ 10.77 (s, 1H), 7.83 (dd, J = 7.9, 1.7 Hz, 1H), 7.60 (ddd, J = 8.4, 7.4, 1.8 Hz, 1H), 7.36 - 7.26 (m, 4H), 7.21 (d, J = 7.8 Hz, 1H), 7.14 - 7.04 (m, 1H), 4.78 (s, 2H), 4.54 (s, 2H), 4.21 (q, J = 7.0 Hz, 2H), 1.31 (t, J = 7.0 Hz, 3H).

### Example EXA₄₇: (S)-N-((2-methoxyphenyl)sulfonyl)-1-methylisoindoline-2-carboxamide (Example EXA₄₇)

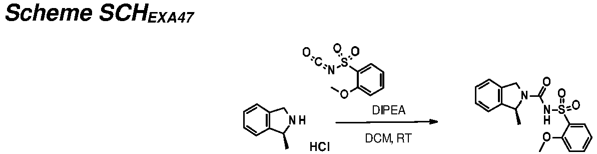

To a mixture of (1S)-1-methyl-2,3-dihydro-1H-isoindole hydrochloride (75 mg, 442 µmol) and DIPEA (385 µL, 2.21 mmol) in DCM (2 mL) at room temperature was added dropwise a solution of 2-methoxybenzenesulfonyl isocyanate **(Intermediate INT_{A-6})** (151 mg, 707 µmol). The reaction mixture was stirred at room temperature for 30 min. The solvent was evaporated under reduced pressure and the residue was purified by silica gel column chromatography eluted with cyclohexane/ethyl acetate from 100/0 to 80/20 to afford (S)-N-((2-methoxyphenyl)sulfonyl)-1-methylisoindoline-2-carboxamide **(Example EXA₄₇)** (32 mg, 20 % yield) as a brown powder.
LCMS (ES, m/z): 347.1 [M+H]⁺
¹H NMR (400 MHz, DMSO) δ 7.80 - 7.74 (1H, m), 7.47 (1H, s), 7.29 - 7.24 (4H, m), 7.10 (1H, s), 6.99 - 6.95 (1H, m), 5.10 - 4.90 (1H, m), 4.63 (2H, s), 3.81 - 3.79 (3H, m), 1.41 - 1.36 (3H, m). Note: NH not visible

### Example EXA₄₈: (R)-N-((2-methoxyphenyl)sulfonyl)-1-methylisoindoline-2-carboxamide (Example EXA₄₈)

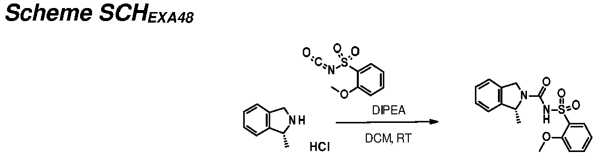

To a mixture of (1R)-1-methyl-2,3-dihydro-1H-isoindole hydrochloride (75 mg, 442 µmol) and DIPEA (385 µL, 2.21 mmol) in DCM (2 mL) at room temperature was dropwise a solution of 2-methoxybenzenesulfonyl isocyanate **(Intermediate INT_{A-6})** (151 mg, 707 µmol). The reaction mixture was stirred at room temperature for 30 min. The solvent was evaporated under reduced pressure and the residue was purified by silica gel column chromatography eluted with dichloromethane/ethyl acetate from 100/0 to 70/30. The resulting product was triturated in cyclohexane, filtered and dried to afford (R)-*N*-((2-methoxyphenyl)sulfonyl)-1-methylisoindoline-2-carboxamide **(Example EXA₄₈**) (11 mg, 7 % yield) as a brown powder.
LCMS (ES, m/z): 347.2 [M+H]⁺
¹H NMR (400 MHz, DMSO) δ 7.79 - 7.75 (1H, m), 7.41 - 7.37 (1H, m), 7.24 (4H, s), 7.07 - 6.91 (2H, m), 4.98 (1H,s), 4.63 (1H, s), 4.41 (1H, s), 3.76 (3H, s), 1.37 - 1.24 (3H, m).

### Example EXA₄₉: 5-cyclopropyl-N-((2-ethoxyphenyl)sulfonyl)isoindoline-2-carboxamide (Example EXA₄₉)

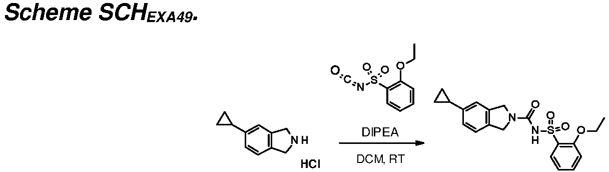

To a mixture of 5-cyclopropylisoindoline hydrochloride (100 mg, 511 µmol) and DIPEA (890 µL, 5.11 mmol) in DCM (2 mL) at 0°C was slowly added a solution of 2-ethoxybenzenesulfonyl isocyanate **(Intermediate INT_{A-7})** (174 mg, 766 µmol) in DCM (2 mL). The reaction mixture was stirred at room temperature for 1 h. The reaction mixture was purified by silica gel column chromatography eluted with cyclohexane/ethyl acetate from 30/70 to 0/100. The resulting product was triturated in acetonitrile, filtered and dried to afford 5-cyclopropyl-*N*-((2-ethoxyphenyl)sulfonyl) isoindoline-2-carboxamide **(Example EXA₄₉)** (26 mg, 13 % yield) as a white powder.
LCMS (ES, m/z): 387.2 [M+H]⁺
1H NMR (400 MHz, DMSO) δ 10.73 (s, 1H), 7.82 (dd, J = 7.9, 1.7 Hz, 1H), 7.66 - 7.55 (m, 1H), 7.19 (dd, J = 12.1, 8.1 Hz, 2H), 7.12 - 7.05 (m, 1H), 7.01 (s, 2H), 4.60 (d, J = 101.2 Hz, 4H), 4.21 (q, J = 6.9 Hz, 2H), 1.92 (td, J = 7.3, 4.6 Hz, 1H), 1.31 (t, J = 7.0 Hz, 3H), 0.98 - 0.87 (m, 2H), 0.66 (d, J = 3.6 Hz, 2H).

### Example EXA₅₀: 5-(dimethylamino)-N-((2-ethoxyphenyl)sulfonyl)isoindoline-2-carboxamide (Example EXA₅₀)

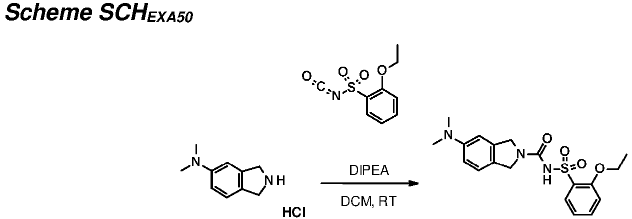

To a mixture of *N,N*-dimethylisoindolin-5-amine hydrochloride **(Intermediate INT_{B-53})** (60 mg, 302 µmol) and DIPEA (526 µL, 3.02 mmol) in DCM (1 mL) at 0°C was slowly added a solution of 2-ethoxybenzenesulfonyl isocyanate **(Intermediate INT_{A-7})** (103 mg, 453 µmol) in DCM (2 mL). The reaction mixture was stirred at room temperature for 1 h. The reaction mixture was purified by silica gel column chromatography eluted with cyclohexane/(ethyl acetate/ethanol 3:1) from 100/0 to 50/50. The resulting product was triturated in acetonitrile, filtered and dried to afford 5-(dimethylamino)-*N*-((2-ethoxyphenyl)sulfonyl)isoindoline-2-carboxamide **(Example EXA₅₀)** (15 mg, 12 % yield) as a white powder.
LCMS (ES, m/z): 390.2 [M+H]⁺
1H NMR (400 MHz, DMSO) δ 10.69 (s, 1H), 7.82 (d, J = 6.3 Hz, 1H), 7.60 (t, J = 7.8 Hz, 1H), 7.21 (d, J = 8.3 Hz, 1H), 7.09 (dd, J = 13.7, 7.9 Hz, 2H), 6.73 - 6.60 (m, 2H), 4.55 (d, J = 82.0 Hz, 4H), 4.21 (q, J = 7.0 Hz, 2H), 2.88 (s, 6H), 1.31 (t, J = 7.0 Hz, 3H).

### Example EXA₅₁: 5-bromo-N-((5-(tert-butyl)-2-methoxyphenyl)sulfonyl)isoindoline-2-carboxamide (Example EXA₅₁)

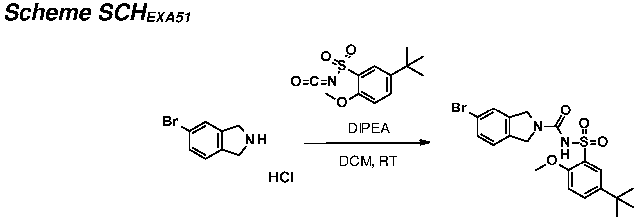

To a mixture of 5-bromoisoindoline hydrochloride (200 mg, 852 µmol) and DIPEA (1.19 mL, 6.82 mmol) in DCM (1 mL) at 0°C was slowly added a solution of 5-(tert-butyl)-2-methoxybenzenesulfonyl isocyanate **(Intermediate INT_{A-3})** (390 mg, 1.45 mmol) in DCM (2 mL). The reaction mixture was stirred at room temperature for 1 h. The reaction mixture was purified by silica gel column chromatography eluted with cyclohexane/(ethyl acetate/ethanol (3/1)) from 100/0 to 50/50 to afford 5-bromo-*N*-((5-(tert-butyl)-2-methoxyphenyl)sulfonyl)isoindoline-2-carboxamide **(Example EXA₅₁**) (31 mg, 7.4 % yield) as an off-white solid.
LCMS (ES, m/z): 467.1-469.1 [M+H]⁺
1H NMR (400 MHz, DMSO) δ 10.75 (s, 1H), 7.78 (d, J = 2.5 Hz, 1H), 7.58 - 7.47 (m, 2H), 7.45 (d, J = 8.1 Hz, 1H), 7.28 (d, J = 8.0 Hz, 1H), 7.05 (d, J = 8.7 Hz, 1H), 4.53 (m, 4H), 3.79 (s, 3H), 1.27 (s, 9H).

### Example EXA₅₂: 5-cyclopropyl-N-((5-isopropyl-2-methoxyphenyl)sulfonyl)isoindoline-2-carboxamide (Example EXA₅₂)

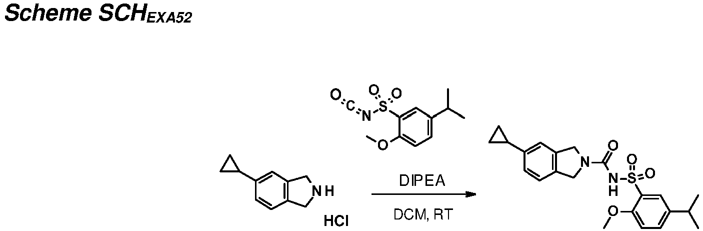

To a mixture of 5-cyclopropylisoindoline hydrochloride (100 mg, 511 µmol) and DIPEA (890 µL, 5.11 mmol) in DCM (2 mL) at 0°C was slowly added a solution of 5-isopropyl-2-methoxybenzenesulfonyl isocyanate **(Intermediate INT_{A-2})** (221 mg, 868 µmol) in DCM (2 mL). The reaction mixture was stirred at room temperature for 1 h. The reaction mixture was purified by silica gel column chromatography eluted with cyclohexane/(ethyl acetate/ethanol 3:1) from 100/0 to 50/50. The resulting product was triturated in acetonitrile, filtered and dried to afford 5-cyclopropyl-*N*-((5-isopropyl-2-methoxyphenyl)sulfonyl)isoindoline-2-carboxamide **(Example EXA₅₂**) (26 mg, 12 % yield) as a white powder.
LCMS (ES, m/z): 415.2 [M+H]⁺
1H NMR (400 MHz, DMSO) δ 10.76 (s, 1H), 7.65 (d, J = 2.3 Hz, 1H), 7.50 (dd, J = 8.6, 2.2 Hz, 1H), 7.16 (dd, J = 13.2, 8.5 Hz, 2H), 7.01 (s, 2H), 4.60 (d, J = 85.7 Hz, 4H), 3.86 (s, 3H), 2.94 (dt, J = 13.5, 6.8 Hz, 1H), 1.93 (s, 1H), 1.21 (d, J = 6.9 Hz, 6H), 0.98 - 0.88 (m, 2H), 0.65 (d, J = 5.0 Hz, 2H).

### Example EXA₅₃: 5-(dimethylamino)-N-((5-isopropyl-2-methoxyphenyl)sulfonyl)isoindoline-2-carboxamide (Example EXA₅₃)

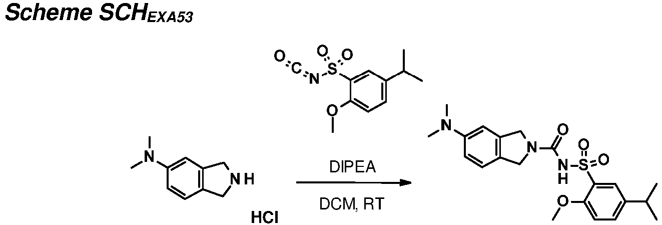

To a mixture of *N,N*-dimethylisoindolin-5-amine hydrochloride **(Intermediate INT_{B-53})** (60 mg, 302 µmol) and DIPEA (526 µL, 3.02 mmol) in DCM (1 mL) at 0°C was slowly added a solution of 5-isopropyl-2-methoxybenzenesulfonyl isocyanate **(Intermediate INT_{A-2})** (131 mg, 513 µmol) in DCM (2 mL). The reaction mixture was stirred at room temperature for 1 h. The reaction mixture was purified by silica gel column chromatography eluted cyclohexane/(ethyl acetate/ethanol) from 100/0 to 50/50. The resulting product was triturated in acetonitrile, filtered and dried to afford 5-(dimethylamino)-*N*-((5-isopropyl-2-methoxyphenyl)sulfonyl)isoindoline-2-carboxamide **(Example EXA₅₃)** (60 mg, 45 % yield) as a white powder.
LCMS (ES, m/z): 418.2 [M+H]⁺
1H NMR (400 MHz, DMSO) δ 10.73 (s, 1H), 7.65 (d, J = 2.4 Hz, 1H), 7.50 (dd, J = 8.7, 2.4 Hz, 1H), 7.14 (d, J = 8.7 Hz, 1H), 7.10 (d, J = 8.6 Hz, 1H), 6.68 (dd, J = 8.5, 2.4 Hz, 1H), 6.63 (s, 1H), 4.56 (d, J = 83.6 Hz, 4H), 3.86 (s, 3H), 2.94 (dt, J = 13.7, 6.9 Hz, 1H), 2.88 (s, 6H), 1.21 (d, J = 6.9 Hz, 6H).

### Example EXA₅₄: 6-(dimethylamino)-4-fluoro-N-((5-isopropyl-2-methoxyphenyl)sulfonyl) isoindoline-2-carboxamide (Example EXA₅₄)

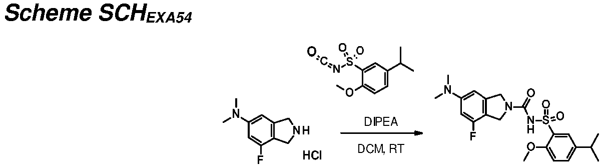

To a mixture of 7-fluoro-*N,N*-dimethylisoindolin-5-amine hydrochloride **(Intermediate INT_{B-55})** (180 mg, 830 µmol) and DIPEA (1.45 mL, 8.30 mmol) in DCM (1 mL) at 0°C was slowly added a solution of 5-isopropyl-2-methoxybenzenesulfonyl isocyanate **(Intermediate INT_{A-2})** (296 mg, 1.16 mmol) in DCM (2 mL). The reaction mixture was stirred at room temperature for 1 h. The reaction mixture was purified by silica gel column chromatography eluted with cyclohexane/(ethyl acetate/ethanol 3:1) from 100/0 to 50/50. The resulting product was triturated in acetonitrile, filtered and dried to afford 6-(dimethylamino)-4-fluoro-*N*-((5-isopropyl-2-methoxyphenyl)sulfonyl) isoindoline-2-carboxamide **(Example EXA₅₄)** (35 mg, 9 % yield) as a white powder.
LCMS (ES, m/z): 436.1 [M+H]⁺
1H NMR (400 MHz, DMSO) δ 10.81 (s, 1H), 7.65 (d, J = 2.4 Hz, 1H), 7.51 (dd, J = 8.4, 2.4 Hz, 1H), 7.15 (d, J = 8.7 Hz, 1H), 6.44 (d, J = 13.2 Hz, 2H), 4.60 (d, J = 86.8 Hz, 4H), 3.86 (s, 3H), 2.95 (q, 6.6 Hz, 1H), 2.90 (s, 6H), 1.21 (d, J = 6.9 Hz, 6H).

### Example EXA₅₅: N-((5-(tert-butyl)-2-methoxyphenyl)sulfonyl)-5-(dimethylamino)isoindoline-2-carboxamide (Example EXA₅₅)

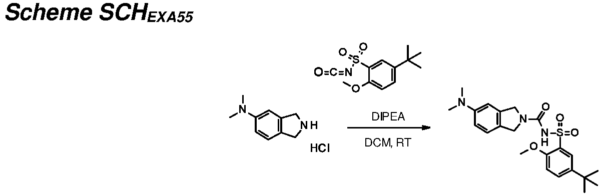

To a mixture of *N,N*-dimethylisoindolin-5-amine hydrochloride **(Intermediate INT**_{**B-**53}) (100 mg, 503 µmol) and DIPEA (877 µL, 5.03 mmol) in DCM (1 mL) was slowly added a solution of 5-(tert-butyl)-2-methoxybenzenesulfonyl isocyanate **(Intermediate INT_{A-3})** (230 mg, 855 µmol) in DCM (2.85 mL). The reaction mixture was stirred at room temperature for 1 h. The reaction mixture was purified by silica gel column chromatography eluted with cyclohexane/ethyl acetate from 100/0 to 0/100. The resulting product was triturated in acetonitrile, filtered and dried to afford *N*-((5-(*tert*-butyl)-2-methoxyphenyl)sulfonyl)-5-(dimethylamino)isoindoline-2-carboxamide **(Example EXA₅₅)** (144 mg, 63 % yield) as a white powder.
LCMS (ES, m/z): 432.2 [M+H]⁺
1H NMR (400 MHz, DMSO) δ 10.71 (1H, s), 7.79 - 7.78 (1H, d, J = 2.4 Hz), 7.58 (1H, s), 7.11 - 7.09 (2H, d, J = 8.4 Hz), 6.68 - 6.66 (1H, dd, J = 2.4 Hz, J = 8.8 Hz), 6.64 (1H, s), 4.61 - 4.46 (4H, d), 3.84 (3H, s), 2.88 (6H, s), 1.29 (9H, s).

### Example EXA₅₆: 6-cyclopropyl-4-fluoro-N-((5-isopropyl-2-methoxyphenyl) sulfonyl)isoindoline-2-carboxamide (Example EXA₅₆)

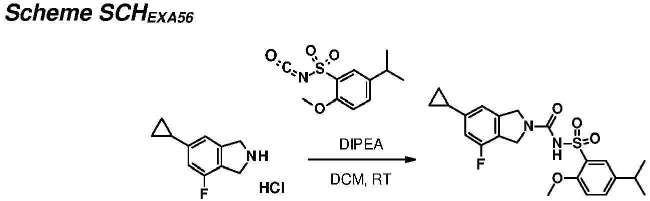

To a mixture of 6-cyclopropyl-4-fluoroisoindoline hydrochloride **(Intermediate INT_{B-54})** (105 mg, 491 µmol) and DIPEA (856 µL, 4.91 mmol) in DCM (1 mL) at 0°C was slowly added a solution of 5-isopropyl-2-methoxybenzenesulfonyl isocyanate **(Intermediate INT_{A-2})** (175 mg, 687 µmol) in DCM (2 mL). The reaction mixture was purified by silica gel column chromatography eluted with cyclohexane/(ethyl acetate/ethanol 3:1) from 100/0 to 50/50. The resulting product was triturated in diethyl ether, filtered and dried to afford 6-cyclopropyl-4-fluoro-*N*-((5-isopropyl-2-methoxyphenyl) sulfonyl)isoindoline-2-carboxamide **(Example EXA₅₆)** (15 mg, 6 % yield) as a pale pink powder.
LCMS (ES, m/z): 433.1 [M+H]⁺
1H NMR (400 MHz, DMSO) δ 10.85 (s, 1H), 7.65 (d, J = 2.4 Hz, 1H), 7.51 (dd, J = 8.6, 2.3 Hz, 1H), 7.14 (d, J = 8.7 Hz, 1H), 6.90 (s, 1H), 6.84 (d, J - 10.8 Hz, 1H), 4.65 (d, J = 92.7 Hz, 4H), 3.86 (s, 3H), 2.94 (dt, J = 13.9, 6.9 Hz, 1H), 1.96 (m, 1H), 1.21 (d, J = 6.9 Hz, 6H), 0.99 - 0.91 (m, 2H), 0.70 (d, J = 5.0 Hz, 2H).

### Example EXA₅₇: 5-(azetidin-1-yl)-N-((5-isopropyl-2-methoxyphenyl)sulfonyl)isoindoline-2-carboxamide (Example EXA₅₇)

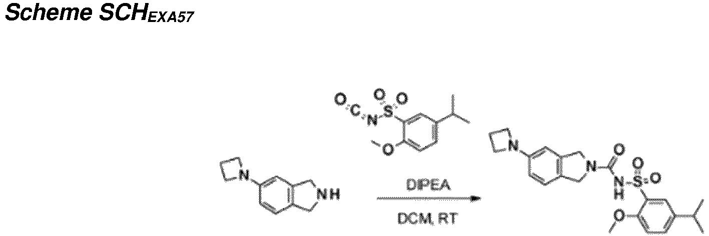

To a mixture of 5-(azetidin-1-yl)isoindoline **(Intermediate INT_{B-56})** (70 mg, 402 µmol) and DIPEA (700 µL, 4.02 mmol) in DCM (1 mL) at 0°C was slowly added a solution of 5-isopropyl-2-methoxybenzenesulfonyl isocyanate **(Intermediate INT_{A-2})** (174 mg, 683 µmol) in DCM (2 mL). The reaction mixture was stirred at room temperature for 1 h. The reaction mixture was purified by silica gel column chromatography eluted with cyclohexane/(ethyl acetate/ethanol 3:1) from 100/0 to 50/50 to afford 5-(azetidin-1-yl)-*N-*((5-isopropyl-2-methoxyphenyl)sulfonyl)isoindoline-2-carboxamide **(Example EXA₅₇)** (27 mg, 15 % yield) as an off-white powder.
LCMS (ES, m/z): 430.2 [M+H]+
1H NMR (400 MHz, DMSO) δ 10.72 (s, 1H), 7.65 (d, J = 2.4 Hz, 1H), 7.48 (d, J = 7.3 Hz, 1H), 7.11 (dd, J = 15.8, 8.2 Hz, 2H), 6.33 (d, J = 7.3 Hz, 2H), 4.52 (d, J = 93.2 Hz, 4H), 3.85 (s, 3H), 3.77 (t, J = 7.2 Hz, 4H), 2.93 (dt, J = 13.6, 6.8 Hz, 1H), 2.34 - 2.24 (m, 2H), 1.20 (d, J = 6.9 Hz, 6H).

### Example EXA₅₈: 5-(3-fluoroazetidin-1-yl)-N-((5-isopropyl-2-methoxyphenyl)sulfonyl)isoindoline-2-carboxamide (Example EXA₅₈)

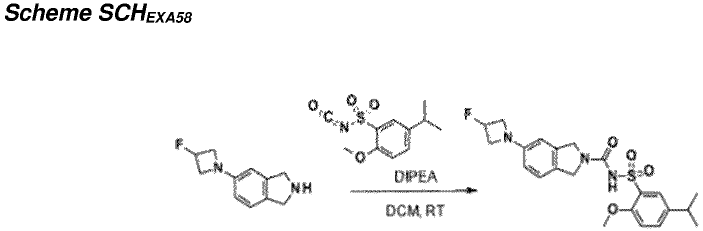

To a mixture of 5-(3-fluoroazetidin-1-yl)isoindoline **(Intermediate INT_{B-57})** (75 mg, 390 µmol) and DIPEA (680 µL, 3.90 mmol) in DCM (1 mL) at 0°C was slowly added a solution of 5-isopropyl-2-methoxybenzenesulfonyl isocyanate **(Intermediate INT_{A-2})** (169 mg, 663 µmol) in DCM (2 mL). The reaction mixture was stirred at room temperature for 1 h. The reaction mixture was purified by silica gel column chromatography eluted with cyclohexane/(ethyl acetate/ethanol 3:1) from 100/0 to 70/30. The resulting product was triturated in diethylether, filtered and dried to afford 5-(3-fluoroazetidin-1-yl)-*N*-((5-isopropyl-2-methoxyphenyl)sulfonyl)isoindoline-2-carboxamide **(Example EXA₅₈)** (2.3 mg, 1.3 % yield) as an off-white powder.
LCMS (ES, m/z): 448.2 [M+H]⁺
1H NMR (400 MHz, DMSO) δ 10.76 (s, 1H), 7.64 (d, J = 1.9 Hz, 1H), 7.45 (s, 1H), 7.12 (d, J = 8.0 Hz, 2H), 6.42 (s, 2H), 5.48 (d, J = 57.3 Hz, 1H), 4.52 (m, 4H), 4.13 (m, 2H), 3.96 - 3.79 (m, 5H), 3.00 - 2.82 (m, 1H), 1.20 (d, J = 6.9 Hz, 6H).

### Example EXA₅₉: N-((5-(tert-butyl)-2-methoxyphenyl)sulfonyl)-6-(dimethylamino)-4-fluoroiso indoline-2-carboxamide (Example EXA₅₉)

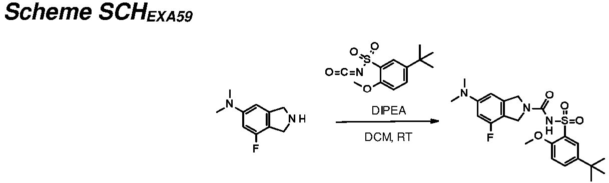

To a mixture of 7-fluoro-*N,N*-dimethylisoindolin-5-amine **(Intermediate INT_{B-55})** (109 mg, 604 µmol) and DIPEA (1.05 mL, 6.05 mmol) in DCM (1 mL) at 0°C was slowly added a solution of 5-(tert-butyl)-2-methoxybenzenesulfonyl isocyanate **(Intermediate INT_{A-3})** (276 mg, 1.02 mmol) in DCM (2 mL). The reaction mixture was stirred at room temperature for 1 h. The reaction mixture was purified by silica gel column chromatography eluted with cyclohexane/(ethyl acetate/ethanol 3:1) from 100/0 to 50/50 to afford *N-*((5-(*tert*-butyl)-2-methoxyphenyl)sulfonyl)-6-(dimethylamino)-4-fluoroiso indoline-2-carboxamide **(Example EXA₅₉)** (16 mg, 4 5 % yield) as pink powder.
LCMS (ES, m/z): 450.2 [M+H]⁺
1H NMR (400 MHz, DMSO) δ 10.80 (s, 1H), 7.78 (d, J = 2.6 Hz, 1H), 7.60 (s, 1H), 7.11 (d, J = 8.7 Hz, 1H), 6.47 (s, 1H), 6.44 (d, J = 13.2 Hz, 1H), 4.58 (d, J = 68.9 Hz, 4H), 3.84 (s, 3H), 2.90 (s, 6H), 1.29 (s, 9H).

### Example EXA₆₃: 8-chloro-N-((7-methoxyquinolin-8-yl)sulfonyl)-5-(1H-pyrazol-1-yl)-3,4-dihydroisoquinoline-2(1H)-carboxamide (Example EXA₆₃)

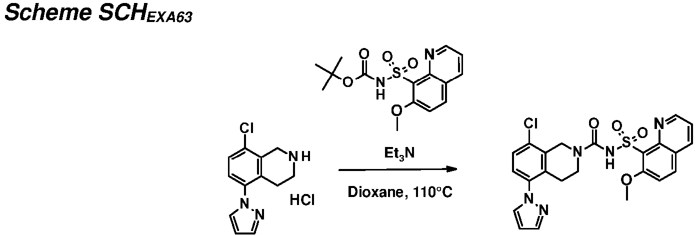

To a mixture of tert-butyl ((7-methoxyquinolin-8-yl)sulfonyl)carbamate **(Intermediate INT_{A-21})** (200 mg, 531 µmol) and triethylamine (267 µL, 1.59 mmol) in dioxane (3 mL) at room temperature was added 8-chloro-5-(1H-pyrazol-1-yl)-1,2,3,4-tetrahydroisoquinoline hydrochloride **(Intermediate INT_{B-19})** (186 mg, 691 µmol). The reaction mixture was stirred at 110°C for 16 h. The reaction mixture was directly absorbed with a Celite pad and concentrated to dryness under reduced pressure. The residue was purified by silica gel column chromatography eluted with dichloromethane/acetone from 90/10 to 0/100 to afford 8-chloro-*N*-((7-methoxyquinolin-8-yl)sulfonyl)-5-(1H-pyrazol-1-yl)-3,4-dihydroisoquinoline-2(1H)-carboxamide **(Example EXA₆₃)** (6 mg, 2.2 % yield) as a yellow solid. LCMS (ES, m/z): 498.2 [M+H]⁺
¹H NMR (400 MHz, DMSO) δ 8.88 (m, 1H), 8.44 (m, 1H), 8.18 (m, 1H), 7.92 - 7.73 (m, 1H), 7.68 (m, 1H), 7.61 - 7.45 (m, 1H), 7.43 - 7.34 (m, 2H), 7.25 (m, 1H), 6.51 - 6.38 (m, 1H), 4.65 (s, 2H), 4.10 -3.98 (m, 3H), 3.56 (t, J = 5.6 Hz, 2H), 2.57 - 2.52 (m, 2H) (Rotamers). NH not visible.

### Example EXA₇₂: 8-cyclopropyl-N-((2,3-dihydrobenzofuran-7-yl) sulfonyl)-5-(1H-pyrazol-1-yl)-3,4-dihydroisoquinoline-2(1H)-carboxamide (Example EXA₇₂)

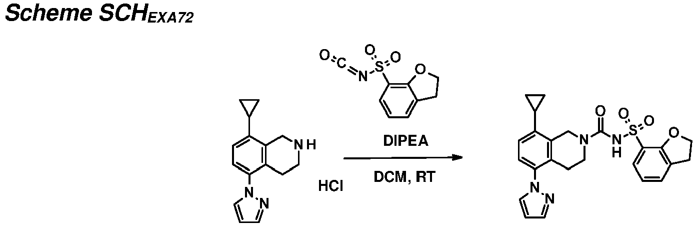

To a mixture of 8-cyclopropyl-5-(1H-pyrazol-1-yl)-1,2,3,4-tetrahydroisoquinoline hydrochloride **(Intermediate INT_{B-3})** (100 mg, 363 µmol) and DIPEA (632 µL, 3.63 mmol) in DCM (2 mL) at room temperature was added dropwise a solution of 2,3-dihydrobenzofuran-7-sulfonyl isocyanate **(Intermediate INT_{A-20})** (139 mg, 616 µmol). The reaction mixture was stirred at room temperature for 16 h. The reaction mixture was directly absorbed with a Celite pad and concentrated to dryness under reduced pressure. The residue was purified by silica gel column chromatography eluted with cyclohexane/acetone from 100/0 to 50/50 to afford 8-cyclopropyl-N-((2,3-dihydrobenzofuran-7-yl)sulfonyl)-5-(1H-pyrazol-1-yl)-3,4-dihydroisoquinoline-2(1H)-carboxamide **(Example EXA₇₂)** (11 mg, 6 % yield) as a white solid.
LCMS (ES, m/z): 465.4 [M+H]⁺
¹H NMR (400 MHz, DMSO) δ 7.93 (s, 1H), 7.69 (d, J = 1.5 Hz, 1H), 7.46 (d, J = 7.9 Hz, 1H), 7.28 (s, 1H), 7.12 (d, J = 8.1 Hz, 1H), 6.99 (d, J = 7.6 Hz, 1H), 6.82 (s, 1H), 6.47 (t, J = 2.1 Hz, 1H), 4.71 (s, 2H), 4.55 (s, 2H), 3.52 (s, 2H), 3.17 (t, J = 8.2 Hz, 2H), 2.48 (s, 2H), 1.87 (s, 1H), 0.97 (m, 2H), 0.66 (m, 2H). NH not visible.

The examples and preparations presented herein further illustrate and exemplify the compounds described, along with the methods for their preparation. It should be noted that the scope of the embodiments described is not limited in any way by these examples and preparations.

In the following examples, unless otherwise specified, molecules with a single chiral center are provided as racemic mixtures. Molecules with two or more chiral centers are also presented as racemic mixtures of diastereomers unless stated otherwise. Methods for obtaining single enantiomers or diastereomers are well-known to those skilled in the art.

In light of the descriptions provided herein, the compounds described herein can be prepared by processes known in the chemical arts. Specific processes for the manufacture of these compounds are included as additional features of the embodiments and are illustrated in the reaction schemes provided below and in the experimental section.

### II. Biological Examples

### Abbreviations:

Acetyl coenzyme A : AcetylCoA or AC-Coa
Bovine serum albumin : BSA
Dithiothreitol : DTT
Ethylenediaminetetraacetic acid : EDTA
Trizma Hydrochloride : Tris-HCL

### KAT6A, KAT7 - Enzyme Activity Assay (AlphaScreen Method)

### Materials and Instruments

Active KAT6A/MOZ protein: Active motif # 81223 _ lot. 2122003
Active KAT7: Active motif # 31489_lot. 24321003
H4 peptide: Upstate Biotechnology # 12-405 lot 3743857 - 100 µg
AcetylCoA: Sigma # A2056 lot 0000303137 - 5 mg
Anti-Histone H4 (acetyl K8) antibody:
   Cell Signaling # 2594S lot 11 (a-H4AcK8-1) 73 µg/mL
   Abcam # ab15823 lot 1029174-1 (a-H4AcK8-2) 0.9 mg/mL
Trizma Hydrochloride solution pH8 (Sigma # T3038 lot SLBL2857V) 100 mM
NaCl (Sigma # S5150 lot SLBH4802V) 15 mM
EDTA (Sigma # E7889 lot SLBD5434V) 1 mM
Tween-20 (Sigma # P7949 lot SZBC2920V ) 0.01%
BSA (Jackson v 001-000-173) 0.02% (= 0.2 mg/mL)
DTT (Sigma # 43816) 1mM
384-well plate: AlphaPlate (PerkinElmer # 6005350)
TopSeal-A PLUS (PerkinElmer # 6050185)
AlphaLISA beads :
   Protein A acceptor (PerkinElmer # AL101Clot 3178960)
   Streptavidin donor beads (PerkinElmer # 6760002S lot 3082282)

### Assay preparation:

Assay Buffer with DTT: Composed of 100 mM Tris-HCl (pH 8), 15 mM NaCl, 1 mM EDTA, 0.01% Tween-20, 0.02% BSA, 1 mM DTT.

Compounds Solutions: Compounds solutions 3x were prepared in the assay buffer with DTT from solution comprising different concentrations of compounds in DMSO.

KAT6A Enzyme Solution 3x: Prepared a 60 nM for a use at a final concentration of 20 nM in the 3× assay buffer with DTT.

KAT7 Enzyme Solution 3x: Prepared a 60 nM for a use at a final concentration of 20 nM in the 3× assay buffer with DTT.

H4 peptide, antibodies, Ac-Coa 3X: Contains 150nM H4 peptide; 3 µM Ac-CoA and 1/1167 Cell signaling # 2594S and 1/1000 Abcam # ab15823 in the 1× assay buffer with DTT.

Detection Reagent AlphaLisa beads 2.2X: Consists of AlphaScreen Protein A Acceptor Beads and AlphaScreen Streptavidin Donor Beads (2.93 or 8.8 µg/mL (final concentration, all prepared in the 1× assay buffer.

### Assay Procedure

The prepared enzyme solution was added to a 384-well plate distribute of 4µL compounds 3x or DMSO and 4 µL KAT6A or KAT7 enzyme 3x, then vortexed and incubated 15 minutes at 30°C.

4 µL Mix antibodies + AcetylCoA + H4 peptide 3x or 4 µL (Mix antibodies + H4 peptide) 3x (condition w/o AcetylCoA) is added, then the plate is vertically centrifugated, vortexed and incubated 1h at 30°C with a film.

10 µL mix AlphaLISA beads 2.2 × in Assay buffer with DTT is added, and the plate is vortexed and incubated 5h at 23 °C protected from light before reading results with Envison.

### Cellular Assay ZR75-1 H3K23ac

### Materials and reagents:

Cell line: ZR-75-1 (Human breast ductal carcinoma, ECACC #87012601)
Cell line passage number: P'13
Medium: RPMI 1640 (Gibco #41965062) supplemented with:
   o 10% Fetal Bovine Serum (FBS, Pan Biotech #P30-3306)
   o 1% Glutamax (Gibco #35050038)
   o 1% Sodium pyruvate (Gibco #11360-039)
   o 4.5 g/L glucose
Plates: 96-well Nunc^{™} Edge^{™} Nunclon Delta surface plates (ThermoFisher #167425)
Histone H3 Monoclonal Antibody (6D3B9), ThermoFisher # MA5-31759 lot XG3638535
Acetyl-Histone H3 (Lys23) (D6Y7M) rabbit mAb, Cell Signaling # 14932
Glo Substrate Reagent Pack DY993: R&D Systems lot P392320
HRP secondary antibodies Jackson Immuno Research Laboratories HRP DONKEY IGG ANTI RABBIT IGG (H+(Interchim # 711-035-152)
Envision luminometer in luminescent reading mode

### Cell Seeding

ZR-75-1 cells (passage 13) are seeded into two 96-well plates. Each well receives 25,000 cells in 200 µL of RPMI 1640 medium supplemented with 10% FBS, 1% Glutamax, 1% sodium pyruvate, and 4.5 g/L glucose. The plates are incubated for 24 hours at 37°C and 5% CO₂ to allow the cells to adhere and reach an appropriate confluence for treatment.

### Compound Treatment

The compounds are prepared for treatment. Stock solutions of the compounds are diluted in DMSO. The final working concentrations for treatment are achieved by diluting the compounds dilutions in the medium. After aspirating the culture medium from each well, 180 µL of fresh incubation medium is added. Then, 20 µL of the compound solutions are added to each well, bringing the final volume to 200 µL per well. The plates are incubated for another 24 hours under the same conditions (37°C, 5% CO₂).

### Cell Lysis and Preparation for ELISA

After 24 hours of compound incubation, the medium is aspirated from the wells, and the cells are lysed. The lysis buffer is prepared by combining 0.4 N HCl with 2 mM sodium butyrate. Each well receives 100 µL of this lysis buffer and is then agitated for 1 hour at 4°C. After lysis, the lysates are neutralized by adding 75 µL of neutralization buffer (1 N sodium phosphate dibasic pH 12.5 + 2 mM sodium butyrate + 1 protease inhibitor tablet). The plate is then agitated for 5 minutes at room temperature, and the entire plate is frozen at -80°C for 1 hour before further processing.

### ELISA Assay for Histone H3 Acetylation (Lys23) - ELISA plate preparation

The ELISA procedure begins with coating the ELISA plate. A half-area 96-well white OptiPlate is coated with Histone H3 monoclonal antibody (6D3B9) at 2 µg/mL in coating buffer (10 mM Tris-HCl pH 8.0, 10 mM NaCl). The antibody is diluted from its stock solution and distributed at 25 µL per well in the ELISA plate. The plate is sealed and incubated overnight at 4°C.

After overnight incubation, the ELISA plate is washed three times with 200 µL of PBS + 0.05% Tween20. The plate is then blocked by adding 100 µL per well of blocking buffer (PBS + 0.05% Tween20 + 1% BSA), and it is incubated for 1 hour at room temperature. After blocking, the plate is washed three more times with 200 µL of the wash buffer.

### ELISA Assay for Histone H3 Acetylation (Lys23) - HRP Substrate Addition and Plate Reading

The previously prepared cell lysates are thawed on ice and added to the ELISA plate. Each well receives 50 µL of cell lysate, and the plate is incubated for 1.5 hours at room temperature. In parallel, blanks are prepared using hydrochloric acid and sodium phosphate dibasic buffers containing sodium butyrate. After incubation, the plate is washed three times with the wash buffer.

Next, the primary antibody, Acetyl-Histone H3 (Lys23) rabbit mAb (D6Y7M), is diluted to 0.1 µg/mL in the blocking buffer and 25 µL is added to each well. The plate is sealed and incubated for 1.5 hours at room temperature. The plate is washed again, and then a secondary HRP-conjugated antibody (Donkey IgG anti-Rabbit IgG, diluted to 0.4 µg/mL in blocking buffer) is added at 50 µL per well. The plate is incubated for 1 hour at room temperature, followed by three washes with the wash buffer.

The HRP substrate is prepared by mixing Glo Reagent A and Glo Reagent B in a 1:2 ratio to produce 7 mL of luminescent substrate. Fifty microliters of this substrate mixture are added to each well, and the plate is incubated at room temperature for 10 minutes in the dark.

Finally, the luminescence is measured using the Envision luminometer in luminescent reading mode. The luminescence readings provide a quantitative measure of histone acetylation at Lys23, which is used to assess the inhibition of KAT6A activity. The IC50 values for each compound are determined by plotting the luminescence data against the compound concentrations and fitting the data to a four-parameter logistic model to calculate the concentration at which 50% inhibition of KAT6A activity occurs.

### Comparative compounds

The compounds of the invention have been compared to two well-known inhibitors of KAT6A activity: CTX-3648 and PF07248144.

The compounds of the invention have been also compared to a compound close to the invention.

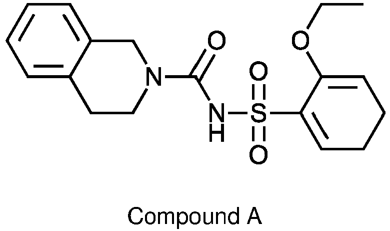

### Results on the efficacy of the compounds of the invention on KAT6A inhibition

The results from the inhibition of the enzymatic activity of Human KAT6A by the comparative compounds CTX-3648, PF07248144, and Compound A and the compounds of the invention are reported in the following table 4.

inhibitory effects were assessed using both enzymatic and cellular assays, with results expressed in micromolar (µM). The data provides a comparative analysis of the inhibition efficacy across these two assay formats.

**Table 4: IC₅₀ Values for the inhibition of the enzymatic activity of Human KAT6A by the compounds of the invention and control compounds CTX-3648, PF07248144 and compound A using both an enzymatic assay and a cellular assay. Values are reported in µM.**

| **Compound ID** | **KAT6A Mean enzymatic Assay (µM)** | **Cellular assay ZR75-1 H3K23ac (µM)** |
|---|---|---|
| CTX-3648 (Comparative compound) | 0.030 | 0.009 |
| PF07248144 | 0.040 | 0.010 |
| (Comparative compound) | | |
| Compound A - (Comparative compound) | 105.024 | nd |
| 1 | 0.247 | 2.559 |
| 2 | 0.049 | 0.208 |
| 3 | 0.056 | 0.857 |
| 4 | 0.261 | 2.046 |
| 5 | 0.014 | 0.198 |
| 6 | 0.054 | 0.363 |
| 7 | 0.116 | 1.929 |
| 8 | 0.074 | 1.072 |
| 9 | 0.016 | 0.385 |
| 10 | 0.017 | 0.515 |
| 11 | 0.049 | 0.851 |
| 12 | 0.045 | 0.395 |
| 13 | 0.385 | 4.536 |
| 14 | 0.214 | 1.249 |
| 15 | 0.090 | 1.347 |

Table 4 shows that the compounds of the invention can inhibit KAT6A activity at low concentrations whether in enzymatic assay or in cellular assay.

These results confirm the potency of the compound within a complex cellular environment, accounting for factors such as membrane permeability, intracellular compound concentration, and the biological context in which KAT6A operates.

### Results on the selectivity of the compounds of the invention on KAT6A / KAT7 inhibition

The results from the inhibition of the enzymatic activity of Human KAT6A and Human KAT7 by the comparative compounds CTX-3648, PF07248144, and Compound A and the compounds of the invention are reported in the following table 5.

inhibitory effects were assessed using enzymatic assay and were used to compute a Selectivity Fold Change KAT6A/KAT7.

**Table 5: IC₅₀ Values for the inhibition of the enzymatic activity of Human KAT6A and KAT7 by the compounds of the invention and control compounds CTX-3648, PF07248144 and compound A ; using both an enzymatic assay. Values are reported in µM.**

| **Compound ID** | **KAT6A Mean enzymatic Assay (µM)** | **KAT7 Mean enzymatic Assay (µM)** | **Selectivity Fold Change KAT6A/KAT7** |
|---|---|---|---|
| CTX-3648 (Comparative compound) | 0.030 | 0.940 | 313 |
| PF07248144 (Comparative compound) | 0.040 | 0.907 | 239 |
| 1 | 0.247 | 30.167 | 122 |
| 2 | 0.049 | 20.662 | 421 |
| 3 | 0.056 | 155.335 | 2790 |
| 4 | 0.261 | 11.312 | 43 |
| 5 | 0.014 | 1.775 | 128 |
| 6 | 0.054 | 1.902 | 35 |
| 7 | 0.116 | 52.486 | 451 |
| 8 | 0.074 | 0.815 | 11 |
| 9 | 0.016 | 5.037 | 315 |
| 10 | 0.017 | 0.980 | 57 |
| 11 | 0.049 | 6.217 | 126 |
| 12 | 0.045 | 2.234 | 50 |
| 13 | 0.385 | 19.388 | 50 |
| 14 | 0.214 | 6.574 | 31 |
| 15 | 0.090 | 6.317 | 70 |
| 16 | 0.927 | 37.186 | 40 |
| 17 | 0.068 | 9.648 | 143 |
| 18 | 0.012 | 0.623 | 52 |
| 19 | 0.284 | 10.466 | 37 |
| 20 | 0.043 | 3.386 | 78 |
| 21 | 0.393 | 10.436 | 27 |
| 22 | 0.898 | 57.450 | 64 |
| 23 | 0.203 | 3.050 | 15 |
| 24 | 0.285 | 19.023 | 67 |

Table 5 shows that the compounds of the invention are more selective for KAT6A over KAT7 as they require a higher concentration to inhibit KAT7.

Moreover, several compounds of the invention exhibit high selectivity for KAT6A. Such compounds may be more suitable for therapeutic applications where selective inhibition of KAT6A is important to minimize off-target effects on KAT7.

Such specificity for KAT6A compared to KAT7 is a further asset for the molecules according to the present invention and suggests a therapeutic potential superior to the molecules of the state of the technique.

### Radiometric evaluation for KAT6A, KAT6B, KAT8 and KAT5

Histone acetyltransferase assays performed at Reaction Biology are radiometric activity assays using tritiated acetyl-Coenzyme A as a cofactor.

### Materials and reagents:

KAT5 (Sino Biological Cat# K314-380G)
Recombinant full length human KAT5 protein (accession number NM_006388) was expressed in a baculovirus expression system Sf9 insect cells using an N-terminal GST tag. The molecular weight of the protein is 88 kDa. MYST3/KAT6A (Active Motif Cat# 81223) Recombinant human KAT6A protein that includes amino acids 488-778 (accession number NP_006757.2) was expressed in a baculovirus expression system and contains an N-terminal FLAG tag. The molecular weight of the protein is 35.5 kDa. This product is the catalytic domain of KAT6A protein.
MYST4/KAT6B (Sino Biological Cat# K315-31 BG) Recombinant human KAT6B protein that includes amino acids 657-1069 (accession number NM_012330) was expressed in a baculovirus expression system and contains an N-terminal GST tag. The molecular weight of the protein is 76 kDa. This product is the catalytic domain of KAT6B protein.
MYST2/KAT7 (Sino Biological Cat# K315-31BG) Recombinant full length human KAT7 protein (accession number NM_007067) was expressed in a baculovirus expression system S9 insect cells and contains an N-terminal GST tag. The molecular weight of the protein is 110 kDa.
MYST1/KAT8 (RBC Cat# RBC-MYST1/KAT8) Recombinant human KAT8 protein that includes amino acids 2-458 (accession number NP_006757.2) was expressed in a baculovirus expression system and contains an N-terminal GST-TEV tag. The molecular weight of the protein is 79.2 kDa. This product is the catalytic domain of KAT6A protein.

Substrates: H3, H2A, or H4 (all full length)
KAT5: 25 nM + 1.25 µM Histone H2A
MYST3/KAT6A: 10 nM + 5 µM Histone H3
MYST4/KAT6B: 10 nM + 2.5 µM Histone H4
MYST2/KAT7: 25 nM + 2.5 µM Histone H3
MYST1/KAT8-GST: 5 nM + 2.5 µM Histone H4

- [³H]-acetyl-CoA concentrations:
   KAT5: 1.5 µM
   KAT6A: 0.5 µM
   KAT6B: 0.5 µM
   KAT7: 0.5 µM
   KAT8: 3 µM
- Assay buffer: 50 mM Tris-HCl, pH 8.0, 100 mM NaCl, 0.1 mM EDTA, 0.1 mM DTT, 10% glycerol.
- Liquid scintillation counter or microplate scintillation reader.

### Procedure

The enzymes are diluted in an appropriate assay buffer.

The tritiated acetyl-CoA ([³H]-acetyl-CoA) are prepared at a final concentration of 0.5 µM for KAT6A/B and KAT7, 1.5 µM for KAT5 and 3 µM for KAT8

Test compounds are prepared by serial dilution to create 10-dose IC50 curves, starting from 10 µM. Each reaction well in the plate receives histone substrate, enzyme, and the test compound or control. The reaction is initiated by adding the acetyl-CoA.

For reaction conditions, the assay is incubated at 30°C to 37°C for 30-60 minutes. Upon completion, the reaction is stopped by adding a stop solution, such as 2% SDS or acetic acid.

Filtration and detection involve transferring the reaction mixture to GF/B filter plates for radioisotope-based filtration. The unincorporated acetyl-CoA is removed by washing the plates three to five times with a cold buffer, leaving only the acetylated histone bound to the filter. The filter plates are then air-dried, and scintillation fluid is added to each well for detection. A scintillation counter measures the incorporation of the radioactive acetyl group into the histone substrate, with counts per minute (cpm) serving as the readout.

Data analysis starts by calculating the percentage inhibition of acetylation for each test compound relative to a vehicle control (DMSO). The IC50 is determined by plotting the percentage inhibition against the compound concentration on a logarithmic scale. A four-parameter logistic curve is fitted to the data to calculate the concentration of the compound required to inhibit 50% of the enzyme activity.

### Result

The results for the inhibition of the enzymatic activity of Human KAT6A and KAT6B by the compounds of the invention and the control compound PF07248144 are presented in Table 6. As for the results of the inhibition of the enzymatic activity of Human KAT5, KAT7 and KAT8 by the compounds of the invention and the control compound PF07248144, they are presented in Table 7. The inhibitory effects were determined using a radiometric assay, with IC₅₀ values reported for each compound.

**Table 6: IC₅₀ Values for the inhibition of Human KAT6A and KAT6B by the compounds of the invention and control compound PF07248144 in radiometric assay**

| **ID** | **KAT6A (µM) Radiometric** | **KAT6B (µM) Radiometric** | **KAT6A/KAT6B ratio** |
|---|---|---|---|
| PF07248144 (Comparative compound) | 0.0005 | 0.049 | 98 |
| 18 | 0.0039 | 0.675 | 174 |
| 9 | 0.023 | 9.7 | 412 |

**Table 7: IC₅₀ Values for the inhibition of Human KAT5, KAT7 and KAT8 by the compounds of the invention and control compound PF07248144 in radiometric assay**

| **ID** | **KAT5 (µM) Radiometric** | **KAT8 (µM) Radiometric** | **KAT7 (µM) Radiometric** |
|---|---|---|---|
| PF07248144 (Comparative compound) | 3.92 | 15.2 | 3.1 |
| 18 | 45 | 49.1 | 3.3 |
| 9 | > 100 | >100 | 29.5 |

As demonstrated by the results in Table 6, the compounds described in this invention exhibit potent inhibitory activity against KAT6A, as evidenced by their low IC₅₀ values in the radiometric assay.

Moreover, they exhibit a high specificity for KAT6A compared to other enzymes of the same family such as KAT6B. For example, compounds 9 and 18 have a KAT6A/KAT6B ratio higher than 170 whereas comparative compound PF07248144 has a KAT6A/KAT6B ratio lower than 100.

Furthermore, they exhibit a high specificity for KAT6A compared to other enzymes of the same family such as KAT5 and KAT8 as shown in Table 7.

This high degree of selectivity for KAT6A suggests a reduced likelihood of off-target effects, which enhances their therapeutic potential. The ability to selectively target KAT6A, while minimizing activity against KAT6B and KAT7 (as well as KAT5 and KAT8), positions the compounds of the invention as promising candidate family for therapeutic intervention with a reduced risk of adverse side effects.

### REFERENCES

Bergamasco, M.I., Ranathunga, N., Abeysekera, W., et al. The histone acetyltransferase KAT6B is required for hematopoietic stem cell development and function. Stem Cell Reports. 2024 Apr 9;19(4):469-485. doi: 10.1016/j.stemcr.2024.02.005. Epub 2024 Mar 21. PMID: 38518784; PMCID: PMC11096436.
Coenen, Eva A et al. Pediatric acute myeloid leukemia with t(8;16)(p11;p13), a distinct clinical and biological entity: a collaborative study by the International-Berlin-Frankfurt-Munster AML-study group. Blood vol. 122,15 (2013): 2704-13
Hu, Z. et al. Genomic characterization of genes encoding histone acetylation modulator proteins identifies therapeutic targets for cancer treatment. Nat Commun 10, 733 (2019). Lv, D., et al. "Histone Acetyltransferase KAT6A Upregulates PI3K/AKT Signaling through TRIM24 Binding." Cancer Research, vol. 77 (22) (2017): 6190-6201Mukohara T. et al. inhibition of lysine acetyltransferase KAT6 in ER+HER2- metastatic breast cancer: a phase 1 trial. Nat Med 30, 2242-2250 (2024).
Mukohara, T., Park, Y.H., Sommerhalder, D. et al. inhibition of lysine acetyltransferase KAT6 in ER+HER2- metastatic breast cancer: a phase 1 trial. Nat Med 30, 2242-2250 (2024).
Sharma S. et al., Discovery of a highly potent, selective, orally bioavailable inhibitor of KAT6A/B histone acetyltransferases with efficacy against KAT6A-high ER+ breast cancer. Cell Chemical Biology, Volume 30, Issue 10, 2023, 1191-1210.e20
Stahl, P. H., & Wermuth, C. G. (Eds.). (2011). Handbook of Pharmaceutical Salts: Properties, Selection, and Use.
Turner-ivey B. et al. KAT6A, a Chromatin Modifier from the 8p11-p12 Amplicon is a Candidate Oncogene in Luminal Breast Cancer. Neoplasia, 16 (2014), pp. 644-655 Weber L.M. et al. The Histone Acetyltransferase KAT6A is Recruited to Unmethylated CpG Islands via a DNA Binding Winged Helix Domain. Nucleic Acids Research, vol. 51, no. 2, 25 Jan. 2023, pp. 574-594

## Claims

1. A compound of general formula (I) 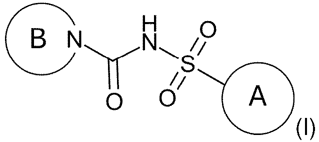 or a pharmaceutically acceptable salt thereof, wherein:
A represents an aryl group, such as a phenyl group or a benzo fused cycloalkyl group, or a heteroaryl group, such as a benzo-fused heterocyclic group;
wherein A is optionally substituted by one or more members selected from the group consisting of:
- alkoxy groups, such as C₁-C₄ alkoxy groups;
- alkyl groups, such as C₁-C₄ alkyl groups, optionally substituted by 1 to 3 fluorine atoms;
- halogens;
- unsubstituted or substituted amino groups, such as dialkylamino groups, such as a dimethylamino group (-N(CH₃)₂);
- heterocyclyl groups, preferably a 4- to 6-member heterocycloalkyl group, such as azetidine, or pyrrolidine optionally substituted by one or more alkyl groups, oxo groups and/or halogens;
- phenyl groups, optionally substituted by one or more alkyl groups and/or halogens; and
- cycloalkyl groups optionally substituted by one or more alkyl groups and/or halogens, such as a C₃-C₅ cycloalkyl group optionally substituted by 1 to 2 fluorine atoms;
and
B represents a partially saturated nitrogen containing bicyclic ring system, such as tetrahydro-isoquinoline or isoindoline, substituted by one or more members selected from the group consisting of:
- halogens;
- dialkylamino groups, such as a dimethylamino group (-N(CH₃)₂);
- cycloalkyl groups optionally substituted by one or more alkyl groups and/or halogens, such as a C₃-C₅ cycloalkyl group optionally substituted by 1 to 2 fluorine atoms;
- alkoxy groups, such as a C₁-C₄ alkoxy group;
- 3- to 6-member cycloalkyloxy groups, such as cyclopropyloxy group, optionally substituted by one or more alkyl groups and/or halogens;
- 4- to 6-member heterocycloalkyl groups, optionally substituted by one or more alkyl groups and/or halogens, which is directly bonded to the bicyclic ring system via a covalent bond or linked through a spacer, preferably chosen from a C₁-C₄ alkylene chain, or a single oxygen atom; for example a 4- to 6-member heterocyclyl group, such as azetidine, optionally substituted by one or more alkyl groups and/or halogens;
- 5- or 6-member heteroaryl groups, optionally substituted by one or more alkyl groups and/or halogens, which is directly bonded to the bicyclic ring system via a covalent bond or linked through a spacer, preferably chosen from a C₁-C₄ alkylene chain, or a single oxygen atom; for example a 5- or 6-member heteroaryl group, such as pyrazole, isoxazole, thiazole, pyridine or oxazole, optionally substituted by one or more alkyl groups and/or halogens; and
- alkyl groups, such as a C₁-C₄ alkyl group, optionally substituted by 1 to 3 fluorine atoms.

2. The compound according to claim 1, or a pharmaceutically acceptable salt thereof, wherein:
A represents an aryl group, such as a phenyl group or a benzo-fused cycloalkyl group, or a heteroaryl group, such as a benzo-fused heterocyclic group;
substituted by one or more alkoxy groups, such as a C₁-C₄ alkoxy group,
and/or alkyl groups, such as a C₁-C₄ alkyl group, optionally substituted by 1 to 3 fluorine atoms;
wherein A is optionally further substituted by one or more members selected from the group consisting of:
- alkoxy groups, such as a C₁-C₄ alkoxy group;
- alkyl groups, such as a C₁-C₄ alkyl group, optionally substituted by 1 to 3 fluorine atoms;
- halogens;
- unsubstituted or substituted amino groups, such as dialkylamino groups, such as a dimethylamino group (-N(CH₃)₂);
- heterocyclyl groups, preferably a 4- to 6-member heterocycloalkyl group, such as azetidine, or pyrrolidine optionally substituted by one or more alkyl groups, oxo groups and/or halogens;
- phenyl groups, optionally substituted by one or more alkyl groups and/or halogens; and
- cycloalkyl groups optionally substituted by one or more alkyl groups and/or halogens, such as a C₃-C₅ cycloalkyl group optionally substituted by 1 to 2 fluorine atoms.

3. The compound according to any one of claim 1 or 2, or a pharmaceutically acceptable salt thereof, wherein:
B represents a partially saturated nitrogen containing bicyclic ring system, such as tetrahydro-isoquinoline or isoindoline, comprising an aryl part substituted by a 5- or 6-member heteroaryl group, which is directly bonded to the aryl part of the bicyclic ring system via a covalent bond or linked through a spacer, said spacer being preferably chosen from a C₁-C₄ alkylene chain or a single oxygen atom;
wherein B is optionally further substituted by one or more members selected from the group consisting of:
- halogens;
- dialkylamino groups, such as a dimethylamino group (-N(CH₃)₂);
- cycloalkyl groups optionally substituted by one or more alkyl groups and/or halogens, such as a C₃-C₅ cycloalkyl group optionally substituted by 1 to 2 fluorine atoms;
- alkoxy groups, such as a C₁-C₄ alkoxy group;
- 3- to 6-member cycloalkyloxy groups, such as cyclopropyloxy, optionally substituted by one or more alkyl groups and/or halogens; and
- alkyl groups, such as a C₁-C₄ alkyl group, optionally substituted by 1 to 3 fluorine atoms.

4. The compound according to any one of claim 1 to 3, wherein the compound is of formula (II): 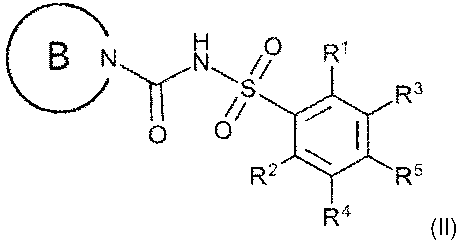 or a pharmaceutically acceptable salt thereof, wherein:
R¹, R², R³, R⁴, and R⁵ each independently represent H; an alkyl group, such as a C₁-C₄ alkyl group, optionally substituted by 1 to 3 fluorine atoms; a cycloalkyl group, optionally substituted by one or more alkyl groups and/or halogens; a heterocyclyl group, preferably a 4- to 6-member heterocyclyl group, optionally substituted by one or more alkyl groups, oxo groups and/or halogens; a halogen;
an alkoxy group, such as a C₁-C₄ alkoxy group; a cycloakyloxy group, optionally substituted by one or more alkyl groups and/or halogens; a phenyl group,
optionally substituted by one or more alkyl groups and/or halogens; or a substituted or unsubstituted amino group, such as dialkylamino group, such as a dimethylamino group (-N(CH₃)₂);
alternatively, any one pair selected from R¹ and R³, R² and R⁴, R⁴ and R⁵, or R³ and R⁵, taken together, form a 5- or 6-member heterocycloalkyl group or an heteroaromatic ring or a 5- or 6-member cycloalkyl group, optionally substituted by one or more alkyl groups and/or halogens, while the remaining R¹, R², R³, R⁴,
and R⁵, each independently, represent H; an alkyl group, optionally substituted by 1 to 3 fluorine atoms; a cycloalkyl group, optionally substituted by one or more alkyl groups and/or halogens; a heterocyclyl group, preferably a 4- to 6-member heterocyclyl group, optionally substituted by one or more alkyl groups, oxo groups and/or halogens; a halogen; an alkoxy group, such as a C₁-C₄ alkoxy group; a cycloakyloxy group, optionally substituted by one or more alkyl groups and/or halogens; a phenyl group, optionally substituted by one or more alkyl groups and/or halogens; or a substituted or unsubstituted amino group, such as dialkylamino groups, such as a dimethylamino group (-N(CH₃)₂).

5. The compound according to any one of claim 1 or 2, wherein the compound is of formula (III): 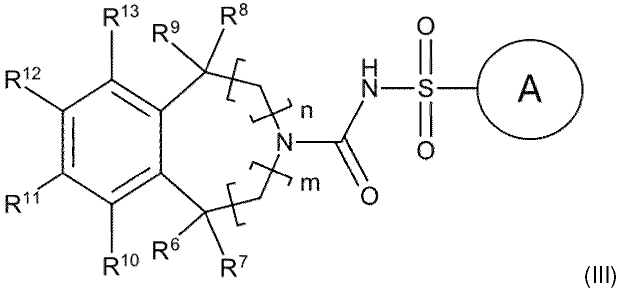 or a pharmaceutically acceptable salt thereof, wherein:
R⁶, R⁷, R⁸, and R⁹ each independently represent H; or an alkyl group;
n and m each independently represent 0 or 1;
R¹⁰ and R¹³ each independently represent H; a halogen; an alkoxy group; a 4- to 6-member heterocycloalkyl group or a 5- or 6-member heteroaryl group,
optionally substituted by one or more alkyl groups and/or halogens; a cycloalkyl group, optionally substituted by one or more alkyl groups and/or halogens; or an alkyl group, optionally substituted by 1 to 3 fluorine atoms; and
R¹¹ and R¹² each independently represent H; a halogen; a dialkylamino group, such as a dimethylamino group (-N(CH₃)₂); a cycloalkyl group, optionally substituted by one or more alkyl groups and/or halogens; a 4- to 6-member heterocycloalkyl group, optionally substituted by one or more alkyl groups and/or halogens, such as azetidine, optionally substituted by one or more alkyl groups and/or halogens; or a 4- to 6-member heteroaryl group, optionally substituted by one or more alkyl groups and/or halogens, which is directly bonded to the aryl via a covalent bond or linked through a spacer such as an alkyl chain; such as pyrazole or oxazole, optionally substituted by one or more alkyl groups and/or halogens.

6. The compound according to any one of claim 1 or 2 or 5, wherein the compound is of formula (IIIf): 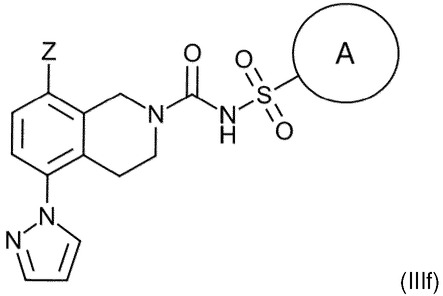
or a pharmaceutically acceptable salt thereof,
wherein Z represents a halogen; an alkyl group optionally substituted by 1 to 3 fluorine atoms; an alkoxy group; or a cycloalkyl group optionally substituted by one or more alkyl groups and/or halogens.

7. The compound according to any one of claim 1 to 3, wherein the compound is of formula (IV): 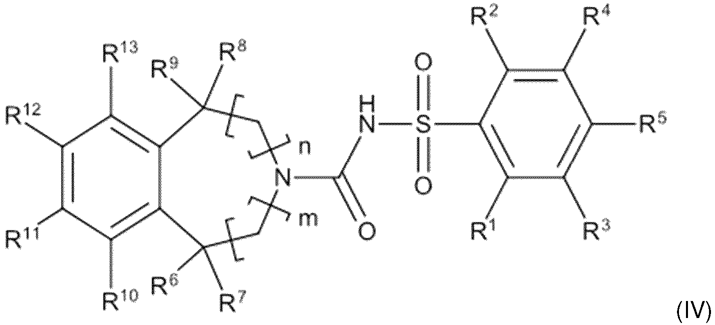 or a pharmaceutically acceptable salt thereof, wherein:
R¹, R², R³, R⁴, and R⁵ each independently represent H; an alkyl group, such as a C₁-C₄ alkyl group, optionally substituted by 1 to 3 fluorine atoms; a cycloalkyl group, optionally substituted by one or more alkyl groups and/or halogens; a heterocyclyl group, preferably a 4- to 6-member heterocyclyl group, optionally substituted by one or more alkyl groups, oxo groups and/or halogens; a halogen; an alkoxy group, such as a C₁-C₄ alkoxy group; a cycloakyloxy group, optionally substituted by one or more alkyl groups and/or halogens; a phenyl group, optionally substituted by one or more alkyl groups and/or halogens; or a substituted or unsubstituted amino group, such as dialkylamino groups, such as a dimethylamino group (-N(CH₃)₂);
alternatively, any one pair selected from R¹ and R³, R² and R⁴, R⁴ and R⁵, or R³ and R⁵, taken together, form a 5- or 6-member heterocycloalkyl group or an heteroaromatic ring or a 5- or 6-member cycloalkyl group, optionally substituted by one or more alkyl groups and/or halogens, while the remaining R¹, R², R³, R⁴,
and R⁵, each independently, represent H; an alkyl group, optionally substituted by 1 to 3 fluorine atoms; a cycloalkyl group, optionally substituted by one or more alkyl groups and/or halogens; a heterocyclyl group, preferably a 4- to 6-member heterocyclyl group, optionally substituted by one or more alkyl groups, oxo groups and/or halogens; a halogen; an alkoxy group such as a C₁-C₄ alkoxy group; a cycloakyloxy group, optionally substituted by one or more alkyl groups and/or halogens; a phenyl group, optionally substituted by one or more alkyl groups and/or halogens; or a substituted or unsubstituted amino group, such as dialkylamino groups, such as a dimethylamino group (-N(CH₃)₂);
R⁶, R⁷, R⁸, and R⁹ each independently represent H; or an alkyl group;
n and m each independently represent 0 or 1;
R¹⁰ and R¹³ each independently represent H; a halogen; an alkoxy group; a 4- to 6-member heterocycloalkyl group or a 5- or 6-member heteroaryl group, optionally substituted by one or more alkyl groups and/or halogens; a cycloalkyl group, optionally substituted by one or more alkyl groups and/or halogens; an alkyl group, optionally substituted by 1 to 3 fluorine atoms; and
R¹¹ and R¹² each independently represent H; a halogen; a dialkylamino group, such as a dimethylamino group (-N(CH₃)₂); a cycloalkyl group, optionally substituted by one or more alkyl groups and/or halogens; a 4- to 6-member heterocycloalkyl group, optionally substituted by one or more alkyl groups and/or halogens, such as azetidine, optionally substituted by one or more alkyl groups and/or halogens; or a 4- to 6-member heteroaryl group, optionally substituted by one or more alkyl groups and/or halogens, which is directly bonded to the aryl via a covalent bond or linked through a spacer such as an alkyl chain; such as pyrazole or oxazole, optionally substituted by one or more alkyl groups and/or halogens.

8. A compound of general formula (I) or a pharmaceutically acceptable salt thereof according to any of claims 1 to 4, wherein B is a tetrahydro-isoquinoline substituted on its aryl part of the bicyclic ring system by a 5- or 6-member heteroaryl group, which is directly bonded to the aryl part via a covalent bond or linked through a spacer such as an alkyl chain; wherein B is optionally further substituted by one or more members selected from the group consisting of:
- halogens;
- dialkylamino groups, such as a dimethylamino group (-N(CH₃)₂);
- cycloalkyl groups optionally substituted by one or more alkyl groups and/or halogens, such as a C₃-C₅ cycloalkyl group optionally substituted by 1 to 2 fluorine atoms;
- alkoxy groups, such as a C₁-C₄ alkoxy group;
- 3- to 6-member cycloalkyloxy groups, such as cyclopropyloxy, optionally substituted by one or more alkyl groups and/or halogens; and
- alkyl groups, such as a C₁-C₄ alkyl group, optionally substituted by 1 to 3 fluorine atoms.

9. A compound of general formula (I) or a pharmaceutically acceptable salt thereof according to any of claims 1 to 8, which is selected from: 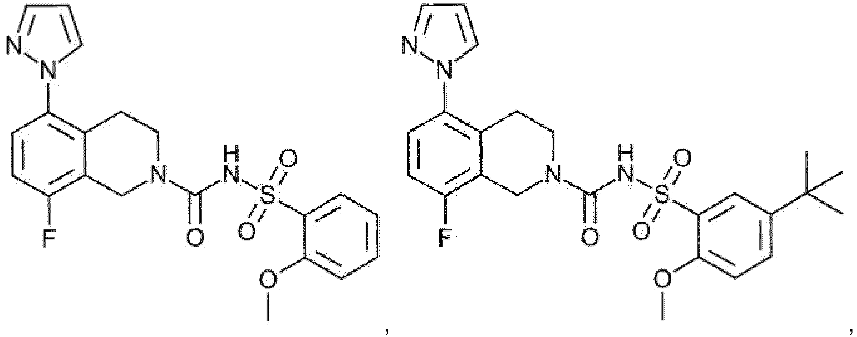 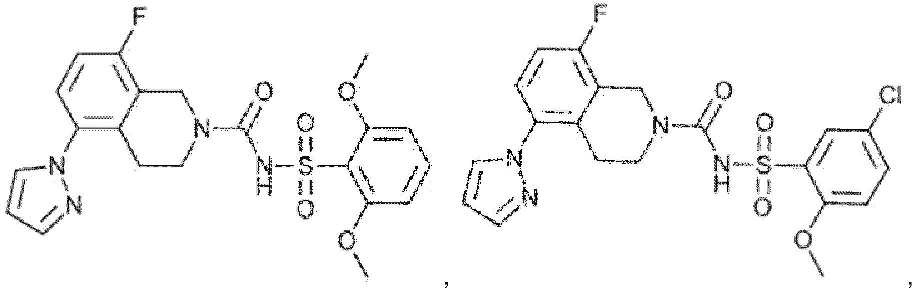 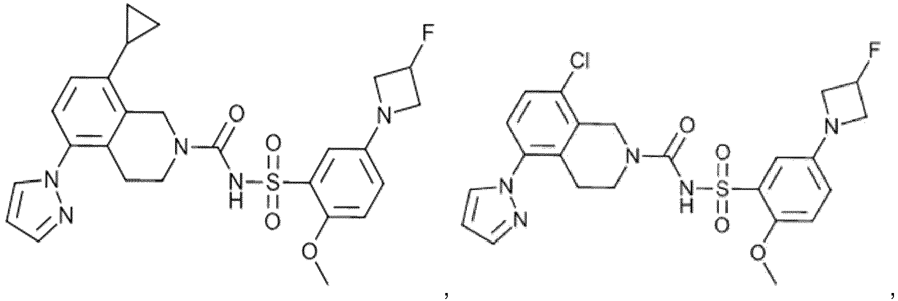 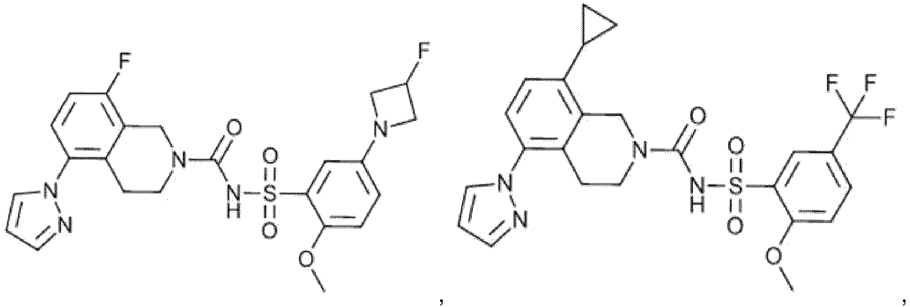 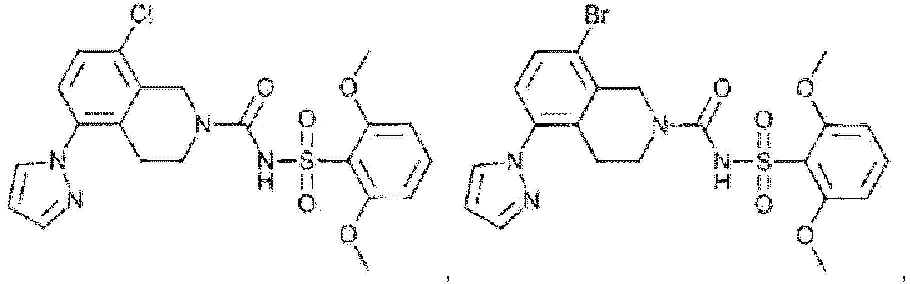 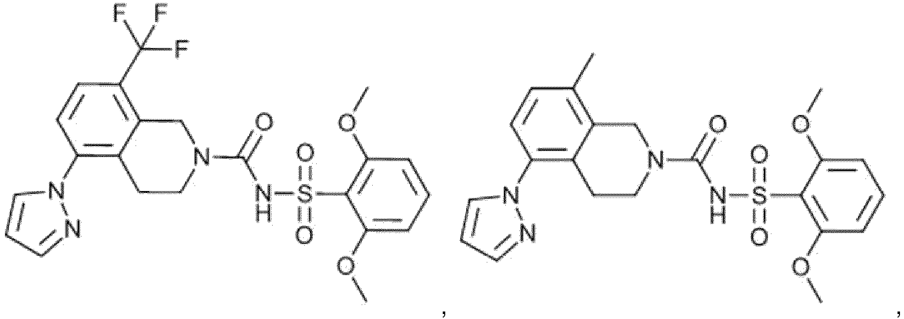 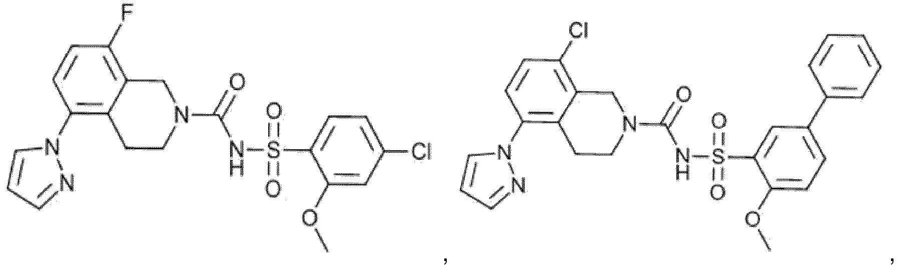 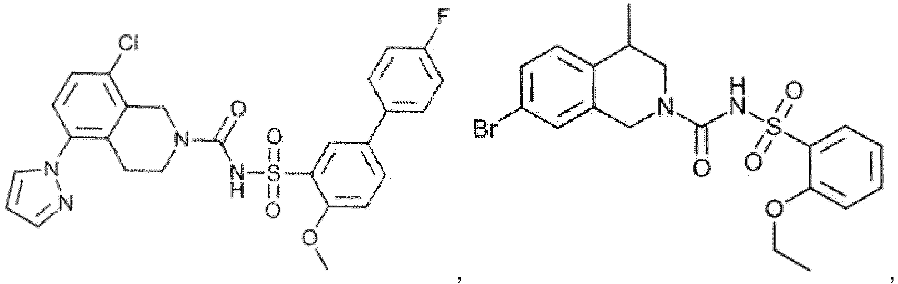 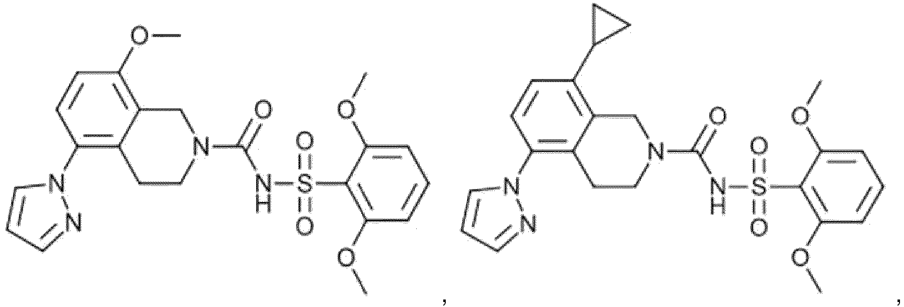 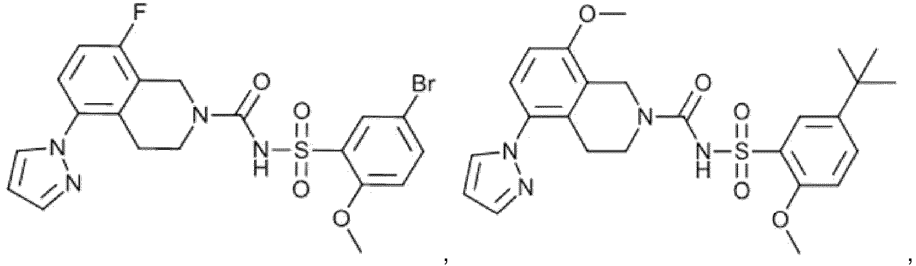 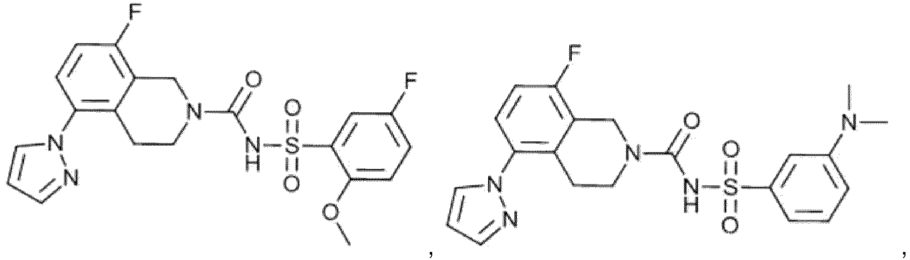 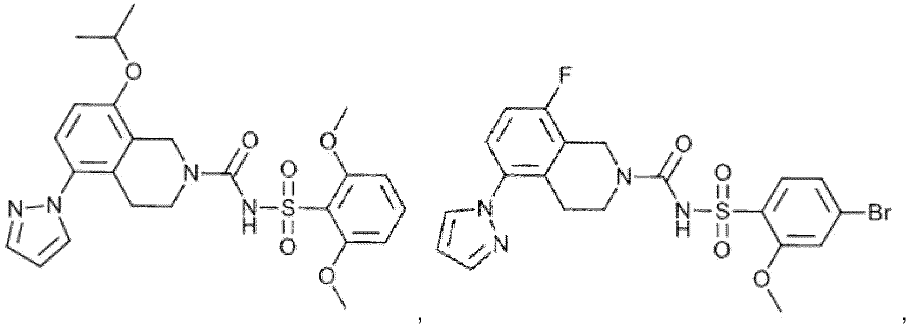 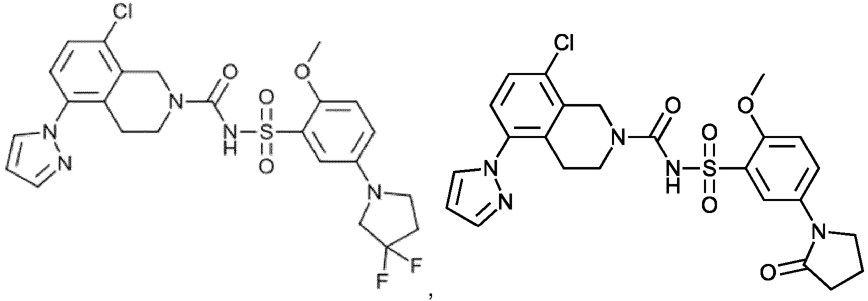 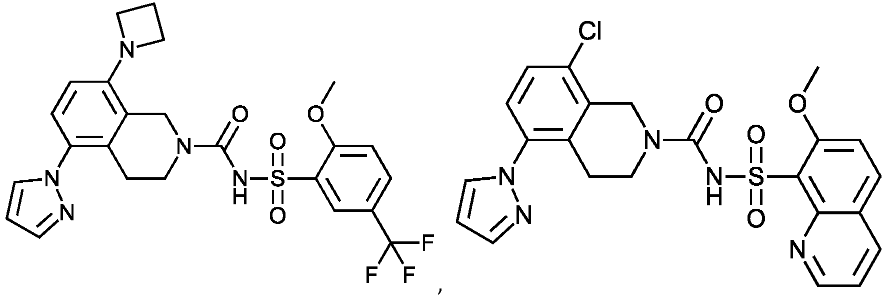 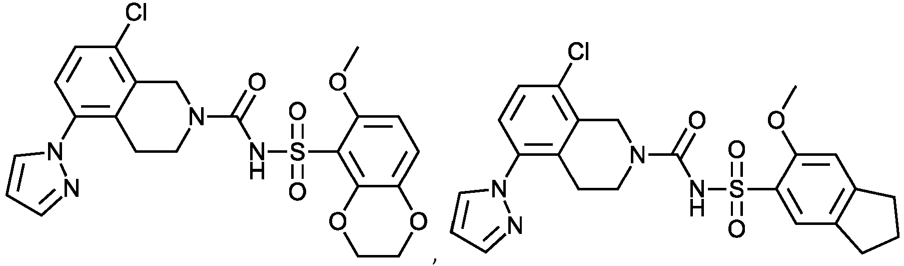 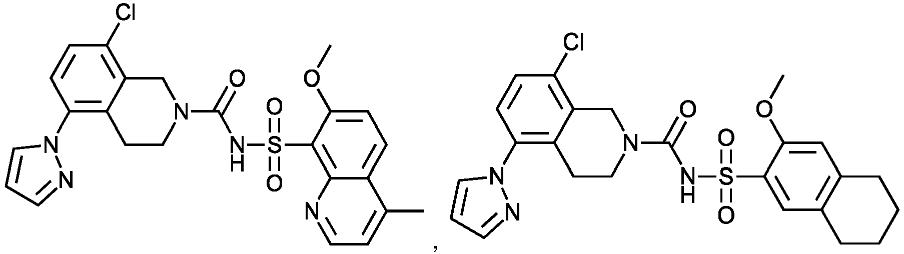 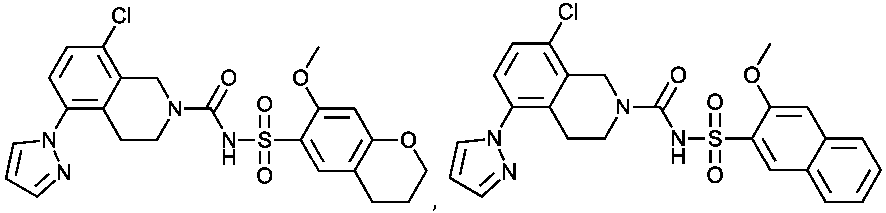 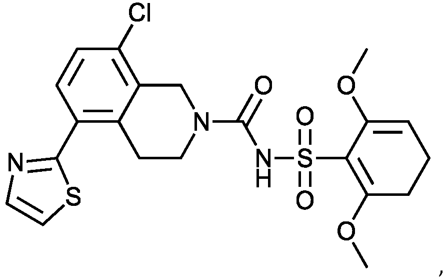 or

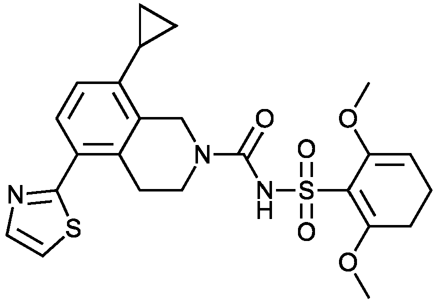

10. A compound of general formula (I) or a pharmaceutically acceptable salt thereof according to any of claims 1 to 8, which is selected from: 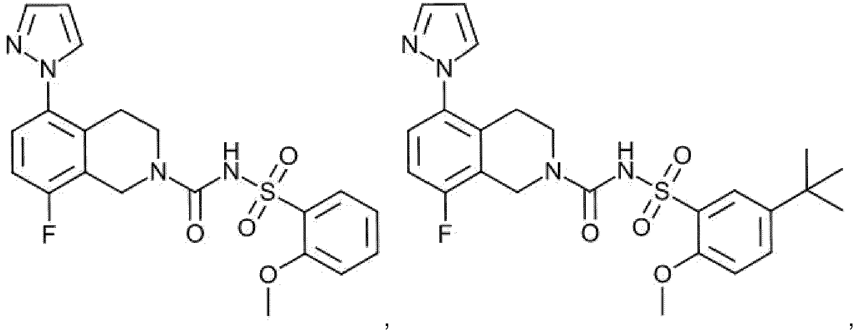 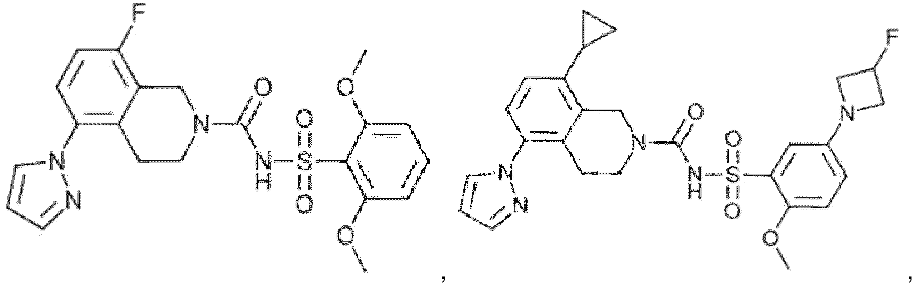 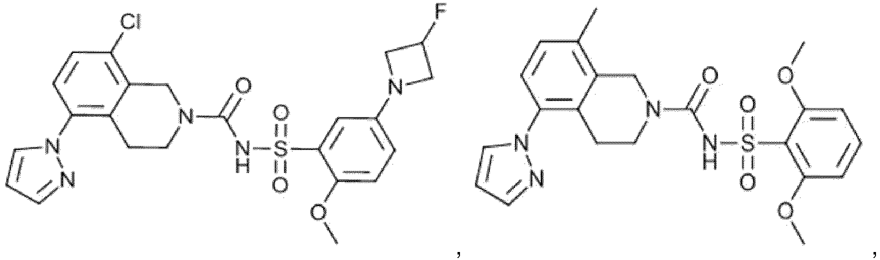 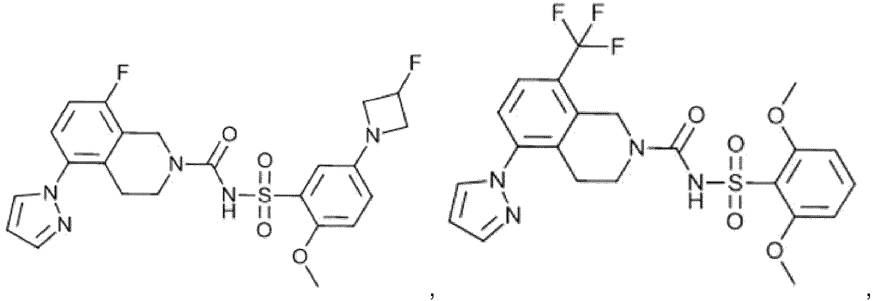 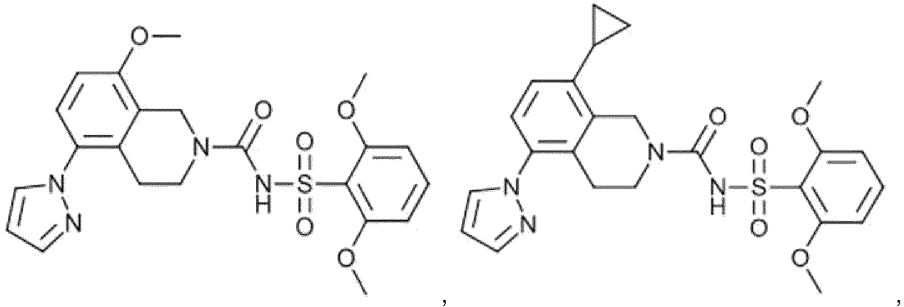 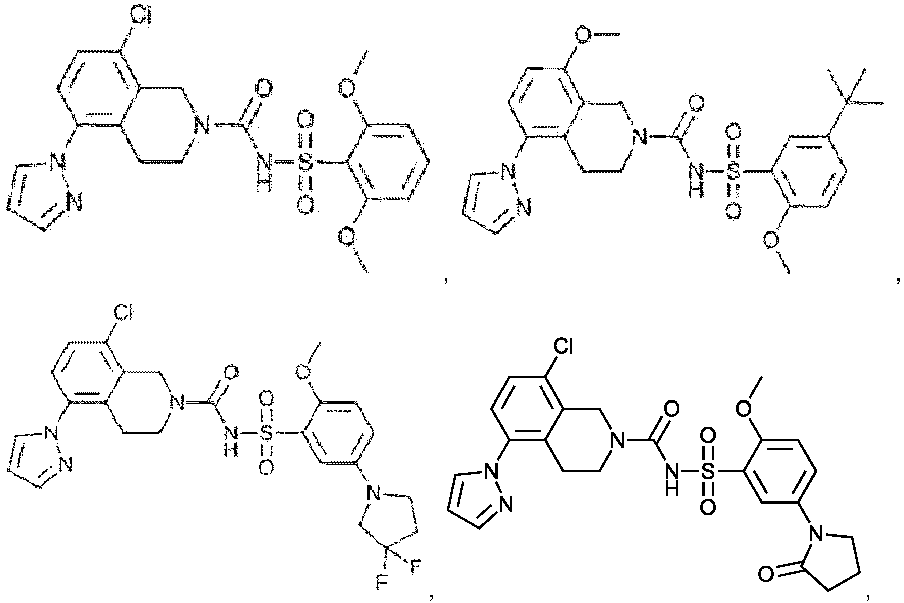 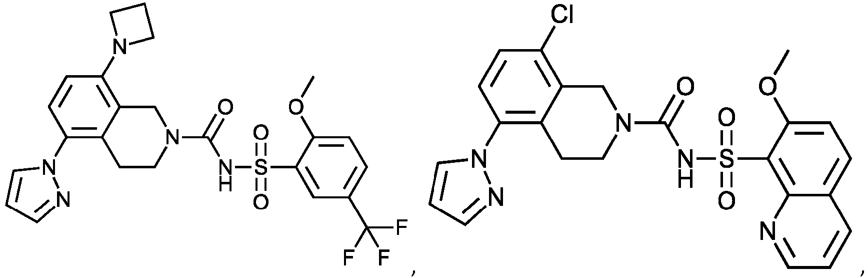 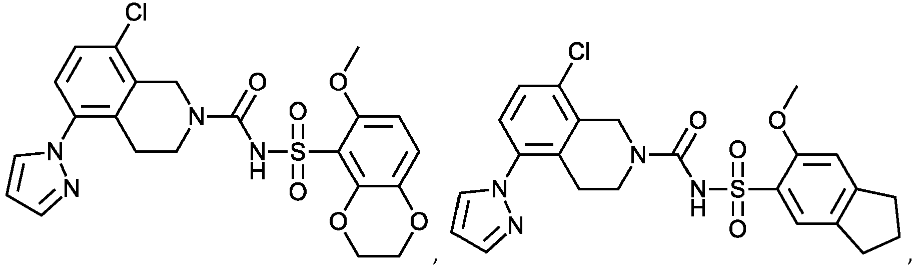 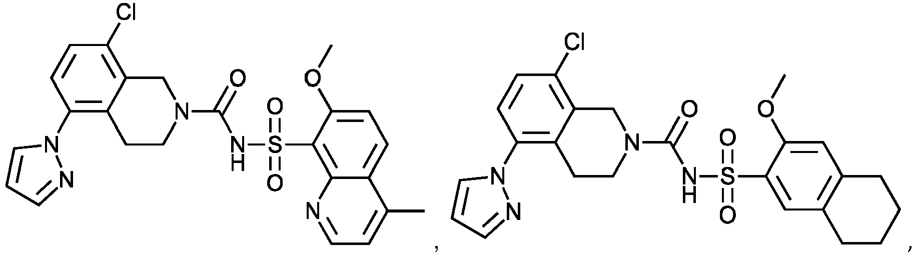 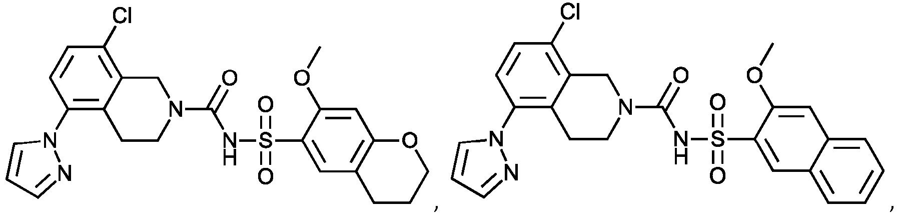 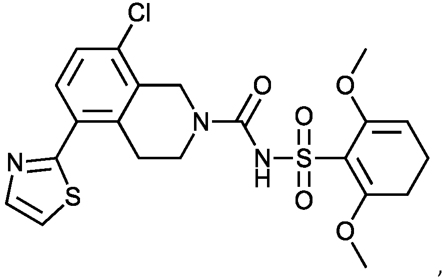 or

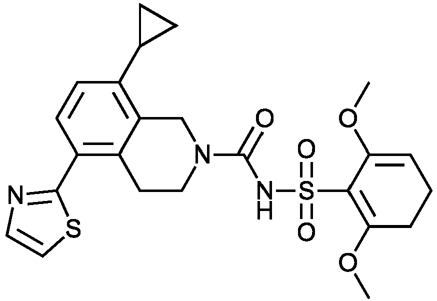

11. A pharmaceutical composition comprising a compound according to any one of claims 1 to 10, or a pharmaceutically acceptable salt thereof, and one or more pharmaceutically acceptable excipients.

12. The compound or a pharmaceutically acceptable salt thereof according to any one of claims 1 to 10, or the pharmaceutical composition according to claim 11, for use in the treatment or prophylaxis of a disease, preferably wherein the disease is a hyperproliferative disorder.

13. The compound, the pharmaceutically acceptable salt or the pharmaceutical composition for use according to claim 12, wherein it is for use in combination with a selective estrogen receptor degrader (SERD) inhibitor; an immune checkpoint inhibitor targeting PD1, PDL1, or CTLA4; and/or a cyclin-dependent kinase (CDK) inhibitor.

14. A method for treating or prophylaxis of a disease, preferably wherein the disease is a hyperproliferative disorder, comprising administering to a patient in need thereof a compound of general formula (I), or a pharmaceutically acceptable salt thereof, according to any one of claims 1 to 10, or a pharmaceutically acceptable composition according to claim 11.

15. A method for treating or prophylaxis of a disease according to claim 14, wherein it further comprises administering to the patient in need thereof a Selective Estrogen Receptor Degrader (SERD) inhibitor; an immune checkpoint inhibitor targeting PD1, PDL1, or CTLA4; and/or a Cyclin-Dependent Kinase (CDK) inhibitor.
